# EUROPEAN PATENT SPECIFICATION

(11) **EP 4 065 566 B1**
(45) Date of publication and mention of the grant of the patent: **01.01.2025**
(21) Application number: 20816144.8
(22) Date of filing: 26.11.2020
(51) Int. Cl.: C07D 261/20, A61K 31/423, A61P 13/10, A61Q 11/00, A61Q 19/00

(54) **TRPM8 MODULATORS**
TRPM8-MODULATOREN
MODULATEURS DE TRPM8

(30) Priority: 29.11.2019 WO PCT/CN2019/121927
(43) Date of publication of application: 05.10.2022
(73) Proprietor: Givaudan SA, 1214 Vernier (CH)
(72) Inventor: WANG, Chao, Shanghai, 201600 (CN); COCITO ARMANINO, Nicolas, 5400 Baden (CH); CHARPENTIER, Julie, 4057 Basel (CH); CHEN, Chun, Shanghai, 200062 (CN); EMTER, Roger, 8053 Zurich (CH); GOEKE, Andreas, 8600 Duebendorf (CH); HUANG, Feng, Shanghai, 201203 (CN); NATSCH, Andreas, 8707 Uetikon (CH); ZHOU, Lijun, Shanghai, 201203 (CN); ZOU, Yue, Shanghai, 200438 (CN)
(74) Representative: Global Patents
(86) International application number: PCT/EP2020/083453
(87) International publication number: WO 2021/105261

(56) References cited:
- US-B2- 8 344 025
- US-B2- 9 718 839
- M. JESÚS PÉREZ DE VEGA ET AL: "Transient Receptor Potential Melastatin 8 Channel (TRPM8) Modulation: Cool Entryway for Treating Pain and Cancer", JOURNAL OF MEDICINAL CHEMISTRY, vol. 59, no. 22, 1 August 2016 (2016-08-01), pages 10006 - 10029, XP055683129, ISSN: 0022-2623, DOI: 10.1021/acs.jmedchem.6b00305
- LIU Y ET AL: "Amino acid derived intramolecular 1,3-dipolar cycloadditions that form bridged medium ring systems with diastereoselective control", TETRAHEDRON, ELSEVIER SIENCE PUBLISHERS, AMSTERDAM, NL, vol. 58, no. 16, 15 April 2002 (2002-04-15), pages 3159 - 3170, XP004347815, ISSN: 0040-4020, DOI: 10.1016/S0040-4020(02)00242-9
- BANUCCI LEBEL ET AL: "Intramolecular Nitrone-Olefin Cycloadditions. The Stereochemistry of Hexahydro-2,l-benzisoxazoline Formation1", J. ORG. CHEM, vol. 36, no. 17, 1 January 1971 (1971-01-01), pages 2440 - 2448, XP055764952
- NORMAN A LEBEL ET AL: "The Addition of Nitrones to Olefins. Fused Bicyclic Isoxazolidines", JOURNAL OF THE AMERICAN CHEMICAL SOCIETY, AMERICAN CHEMICAL SOCIETY, US, vol. 86, no. 18, 20 September 1964 (1964-09-20), pages 3759 - 3767, XP002726005, ISSN: 0002-7863, DOI: 10.1021/JA01072A031
- BRION F ET AL: "STEREOSELECTIVE SYNTHESIS OF A TRANS-OCTAHYDROINDOLE DERIVATIVE, PRECURSOR OF TRANDOLAPRIL (RU 44 570), AN INHIBITOR OF ANGIOTENSIN3CONVERTING ENZYME", TETRAHEDRON LETTERS, ELSEVIER, AMSTERDAM , NL, vol. 33, no. 34, 1 January 1992 (1992-01-01), pages 4889 - 4892, XP000885877, ISSN: 0040-4039, DOI: 10.1016/S0040-4039(00)61225-X
- KALEVI PIHLAJA, JORMA MATTINEN, FERENC FIILOPTJ: "Conformational Analysis 30-A 'H and 13C NMR Stereochemical Study on N-Methyl-Substituted cis-and trans-Fused Octahydro-2H-1,3-and -3,l-benzoxazines", MAGNETIC RESONANCE IN CHEMISTRY, vol. 34, 1996, pages 998 - 1002, XP055764653
- P SOHIR ET AL: "Stereochemical Studies-XIV: Cyclic Amino Alcohols and Related Compounds-VII", ORGANIC MAGNETIC RESONSNCE, vol. 5, no. 3, 1 January 1973 (1973-01-01), pages 159 - 160, XP055764928
- FISYUK A S ET AL: "INTERACTION OF METHYL N-(3-OXOALKYL)CARBAMATES, S-METHYL -CARBAMATES, AND -DITHIOCARBAMATES WITH SODIUM BOROHYDRIDE. SYNTHESIS OF TETRAHYDRO- 1,3-OXAZIN-2-ONES AND -THIONES", CHEMISTRY OF HETEROCYCLIC COMPOUNDS, SPRINGER US, NEW YORK, vol. 37, 1 January 2001 (2001-01-01), pages 597 - 609, XP002526363, ISSN: 0009-3122, DOI: 10.1023/A:1011660706513

## Description

### TECHNICAL FIELD

The present invention relates to a particular class of compounds capable to activate TRPM8 ion channels. It further relates to the use of said compounds for inducing a sensation of coldness, and to consumer products comprising these compounds.

### BACKGROUND

TRPM8 (transient receptor potential melastatin member 8, also known as Trp-p8 or MCR1) is activated by innocuous cool and thus plays an important role as sensor for temperature. The channels are widely distributed in different tissues (such as human skin and mucosa (such as oral mucosa, throat mucosa, nasal mucosa), male urogenital tract, lung epithelium cells and artery myoctes). They are Ca²⁺-permeable, nonselective cation channels that exhibit polymodal gating mechanisms, being activated by innocuous cool to cold temperature, membrane depolarization, and molecules which are known as cooling agents including natural and synthetic compounds. The receptor was described for the first time in 2002 as cold receptor in a number of publications.

The present invention is based on the finding that a particular class of compounds can be used to drive a cooling response when brought into contact with TRPM8 receptor in-vitro and in-vivo.

Compounds providing a cooling sensation have for a long time played an important role in the flavor and fragrance industry in order to produce an association with freshness and cleanliness. Cooling compounds are widely used in a variety of products such as foodstuffs, tobacco products, beverages, dentifrices, mouthwashes, toothpastes, and toiletries. The cooling sensation provided contributed to the appeal and acceptability of consumer products. In particular, oral care products, such as dentifrices and mouthwashes are formulated with coolants because they provide breath freshening effects and a clean, cool, fresh feeling in the mouth.

A large number of compounds providing cooling sensations have been described. The most well-known natural occurring compound is menthol, in particular L-menthol. Among the synthetic compounds providing cooling sensations, many are derivatives of or are structurally related to menthol, i. e. containing the cyclohexane moiety, and derivatized with functional groups including carboxamide, ketal, ester, ether and alcohols.

Applicant surprisingly found a new class of chemical compounds which differ significantly in structural terms from the TRPM8 modulators known hitherto. It was surprisingly found that this class of chemical compounds as herein further described can provide long lasting cooling on the human skin and/or mucosa at very low concentrations.

### SUMMARY

There is provided in a first aspect a compound of formula (Ia), a salt or solvate thereof (in particular for use in providing cooling sensation) 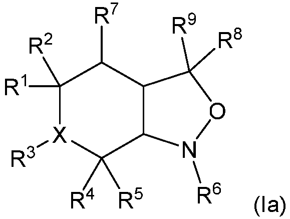 wherein
X isC,
   R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
**R¹** is selected from
C₁-C₆ alkyl,
C₁-C₇ alkyl substituted with one or two OH groups,
C₃-C₇ alkenyl,
phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl),
naphhtyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl), and
C₅-C₁₀ mono- or bicyclic aryl wherein 1 or 2 C-atoms are replaced by a hetero atom independently selected from sulfur, nitrogen, and oxygen, optionally substituted with up to three (e.g. 1 or 2) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl);
**R²** is selected from hydrogen and methyl;
**R⁴**, **R⁵**, **and R⁷** are independently selected from hydrogen and C₁-C₆ alkyl;
**R⁶ is** selected from hydrogen, C₁-C₆ alkyl (e.g. ethyl, isopropy), and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7-membered cycloalkyl ring.

In accordance with a second aspect there is provided a non-medical method of modulating (in-vitro and in-vivo modulation) of transient receptor potential melastatin member 8 (TRPM8) comprising bringing the receptor into contact with a compound of formula (Ia), or a salt or solvate thereof.

There is provided in a third aspect a non-medical method of inducing a cooling sensation in a human or animal comprising contacting the human or animal with a compound of formula (Ia), or a salt or solvate thereof.

There is provided in a fourth aspect consumer products, in particular consumer products which get into contact with the human skin and/or mucosa comprising a compound as defined by formula (Ia), or a salt or solvate thereof.

There is provided in a fifth aspect a composition comprising a cool sensation wherein the composition comprises at least one compound of formula (Ia), a salt or solvate thereof, and a further cooling compound.

There is provided in a sixth aspect pharmaceutical composition comprising one or more compounds as defined by formula (Ia), or a salt or solvate thereof.

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### DETAILED DESCRIPTION

The present invention is based, at least in part, on the surprising finding of a new class of chemical compounds which differ significantly in structural terms from the TRPM8 modulators known hitherto, that are capable to activate the TRPM8 ion channel, which brings about a Ca²⁺ influx into the cold-sensitive neurons. The electrical signal produced as a result is ultimately perceived as sensation of coldness. Applicant surprising fount that this class of chemical compounds as herein further described can provide long lasting cooling on the human skin and/or mucosa at very low concentrations.

Thus, there is provided in a first aspect a compound of formula (Ia), a salt or solvate thereof (in particular for use in providing cooling sensations) 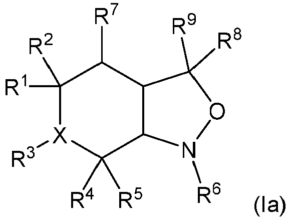 wherein
XisC,
R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
**R¹** is selected from
C₁-C₆ alkyl,
C₁-C₇ alkyl substituted with one or two OH groups,
C₃-C₇ alkenyl,
phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl),
naphthyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl), and
C₅-C₁₀ mono- or bicyclic aryl wherein 1 or 2 C-atoms are replaced by a hetero atom independently selected from sulfur, nitrogen, and oxygen, optionally substituted with up to three (e.g. 1 or 2) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl);
**R²** is selected from hydrogen and methyl;
**R⁴, R⁵**, **and R⁷** are independently selected from hydrogen and C₁-C₆ alkyl;
**R⁶ is** selected from hydrogen, C₁-C₆ alkyl (e.g. ethyl, isopropy), and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7-membered cycloalkyl ring.

Non-limiting examples are compounds of formula (Ia), a salt or solvate thereof, wherein R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl.

Further non-limiting examples are compound of formula (Ia), a salt or solvate thereof, wherein R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl.

Further non-limiting examples are compound of formula (Ia), a salt or solvate thereof, wherein R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl; and R¹ is phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl).

Further, non-limiting examples are compounds of formula (Ia), a salt or solvate thereof, 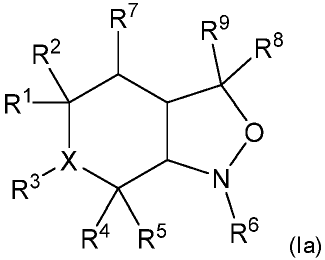 wherein
X is selected C;
R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
**R¹** is selected from
C₁-C₆ alkyl,
C₁-C₇ alkyl substituted with one or two OH groups,
C₃-C₇ alkenyl,
phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl),
naphthyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl), and
C₅-C₁₀ mono- or bicyclic aryl wherein 1 or 2 C-atoms are replaced by a hetero atom independently selected from sulfur, nitrogen, and oxygen, optionally substituted with up to three (e.g. 1 or 2) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl);
**R²** is selected from hydrogen and methyl;
**R⁴, R⁵**, **and R⁷** are independently selected from hydrogen and C₁-C₆ alkyl;
**R⁶ is** selected from hydrogen, C₁-C₆ alkyl (e.g. ethyl, isopropy), and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7-membered cycloalkyl ring.

Further non-limiting examples are compounds of formula (Ia), a salt or solvate thereof, wherein X is C; and R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl.

Further non-limiting examples are compound of formula (Ia), a salt or solvate thereof, wherein X is C;
**R¹** is selected from hydrogen,
C₁-C₆ alkyl,
C₁-C₇ alkyl substituted with one or two OH groups,
C₃-C₇ alkenyl,
phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl),
naphthyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl), and
C₅-C₁₀ mono- or bicyclic aryl wherein 1 or 2 C-atoms are replaced by a hetero atom independently selected from sulfur, nitrogen, and oxygen, optionally substituted with up to three (e.g. 1 or 2) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl);
**R²** is selected from hydrogen and methyl;
**R⁴, and R⁵** are independently selected from hydrogen and C₁-C₆ alkyl;
R³ is hydrogen;
**R⁶ is** selected from hydrogen, C₁-C₆ alkyl (e.g. ethyl, isopropy), and C₃-C₇ cycloalkyl;
**R⁷** is hydrogen;
**R⁸ and R⁹** are independently selected from hydrogen, and C₁-C₆ alkly, or
R⁸ and R⁹ form together with the C-atom to which they are attached a 3, 4, 5, 6, or 7-membered cycloalkyl ring.

Further non-limiting examples are compound of formula (Ia), a salt or solvate thereof, wherein X is C, and R³ is hydrogen; and R¹ is selected from phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, - C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl).

Further non-limiting examples are compound of formula (Ia), a salt or solvate thereof, wherein X is C; R¹ is selected from phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ is hydrogen; and R⁷ is hydrogen.

Further non-limiting examples are compound of formula (Ia), a salt or solvate thereof, wherein X is C; R¹ is selected from phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ is hydrogen; R⁴ is hydrogen, R⁵ is C₁-C₃ alkyl; and R⁷ is hydrogen.

In one particular embodiment the compounds of formula (Ia) wherein X is C, are compound of formula (Ib) have the relative stereochemistry as depicted 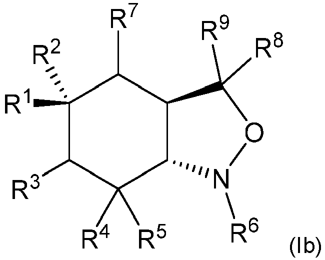 wherein
**R¹** is selected from
C₁-C₆ alkyl,
C₁-C₇ alkyl substituted with one or two OH groups,
C₃-C₇ alkenyl,
phenyl optionally substituted with up to five (e.g. one, two or three) substituents selected from the group consisting of halogen (including Br, Cl, and F), C≡N, NO₂, C₁-C₆ alkly, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl),
naphtyl optionally substituted with up to five (e.g. one, two or three) substituents selected from the group consisting of halogen (including Br, Cl, and F), C≡N, NO₂, C₁-C₆ alkly, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and - SR¹¹ wherein R¹¹ is selected from hydrogen and C₁-C₃ alkyl (including ethyl and isopropyl), and
C₅-C₁₀ mono- or bicyclic aryl wherein 1 or 2 C-atoms are replaced by a hetero atom independently selected from sulfur, nitrogen, and oxygen, optionally substituted with up to three (e.g. 1 or 2) substituents selected from the group consisting of halogen (including Br, Cl, and F), C≡N, NO₂, C₁-C₆ alkly, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl);
**R³** is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
**R²** is selected from hydrogen and methyl;
**R⁴, R⁵**, **and R⁷** are independently selected from hydrogen and C₁-C₆ alkyl;
**R⁶ is** selected from hydrogen, C₁-C₆ alkyl (e.g. ethyl, isopropy), and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7-membered cycloalkyl ring.

Further non-limiting examples are compound of formula (Ib), a salt or solvate thereof, wherein X is C; R¹ is selected from phenyl and phenyl substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); and R³ is hydrogen.

Further non-limiting examples are compound of formula (Ib), a salt or solvate thereof, wherein X is C; R¹ is selected from phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ is hydrogen; and R⁷ is hydrogen.

Further non-limiting examples are compound of formula (Ib), a salt or solvate thereof, wherein X is C; R¹ is selected from phenyl optionally substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ is hydrogen; R⁴ is hydrogen, R⁵ is C₁-C₃ alkyl; and R⁷ is hydrogen.

Further non-limiting examples are compound of formula (Ib), a salt or solvate thereof, wherein R³ and R⁷ are both hydrogen, and R⁸ and R⁹ are both methyl.

Further non-limiting examples are compound of formula (Ib), a salt or solvate thereof, wherein X is C, R¹ is selected from phenyl and phenyl substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ and R⁷ are both hydrogen; and R⁸ and R⁹ are both methyl.

Further non-limiting examples are compound of formula (Ib), a salt or solvate thereof, wherein X is C, R¹ is selected from phenyl and phenyl substituted with up to five (e.g. one, two or three) substituents selected from the group consisting of halogen (including Br, Cl, and F), C≡N, NO₂, C₁-C₆ alkyl, C₁-C₆ alkyl comprising up to 5 halogen atoms (including Br, Cl, and F), C₁-C₆ alkyloxy, C₁-C₆ alkyloxy comprising up to 5 halogen atoms (including Br, Cl, and F), C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl (including ethyl and isopropyl); R³ and R⁷ are both hydrogen; R⁶ is methyl or isopropy; and R⁸ and R⁹ are both methyl.

Further non-limiting examples are compound of formula (Ia) and (Ib) wherein either R¹ or R² is not hydrogen.

Further non-limiting examples are compound of formula (Ia) and (Ib) wherein either R¹ is not hydrogen.

Further non-limiting examples are compound of formula (Ia) and (Ib) wherein R¹ is selected from C₁-C₆ alkyl, C₁-C₇ alkyl substituted with one or two OH groups, and C₃-C₇ alkenyl.

Further non-limiting examples are compound of formula (Ia) and (Ib) wherein R¹ is selected from C₁-C₆ alkyl, C₁-C₇ alkyl substituted with one or two OH groups, and C₃-C₇ alkenyl; R² is hydrogen or methyl; and R⁸ and R⁹ are methyl.

Further non-limiting examples are compound of formula (Ia) and (Ib) wherein R¹ is selected from C₁-C₆ alkyl, C₁-C₇ alkyl substituted with one or two OH groups, and C₃-C₇ alkenyl; R² is hydrogen or methyl; R⁴ is hydrogen, R⁵ is C₁-C₃ alkyl; and R⁸ and R⁹ are methyl.

Further non-limiting examples are compound of formula (Ia) and (Ib) wherein R¹ is selected from C₁-C₆ alkyl, C₁-C₇ alkyl substituted with one or two OH groups, and C₃-C₇ alkenyl; R² is hydrogen or methyl; R⁴ is hydrogen, R⁵ is C₁-C₃ alkyl; R⁶ is methyl or isopropyl; and R⁸ and R⁹ are methyl.

Further non-limiting exmples are compounds of formula (Ia) or (Ib) selected from the group consisting of 1,3,3,5,7-pentamethyl-5-(3-methylbut-2-en-1-yl)octahydrobenzo[c]isoxazole; 1,3,3,5,7-pentamethyl-5-(4-methylpyridin-3-yl)octahydrobenzo[c]isoxazole; 1,3,3,5,7-pentamethyl-5-(pyridin-3-yl)octahydrobenzo[c]isoxazole; 1,3,3,5,7-pentamethyloctahydrobenzo[c]isoxazole; 1,3,3,5,7-pentamethyloctahydrobenzo[c]isoxazole hydrochloride; 1,3,3,6-tetramethyloctahydrobenzo[c]isoxazole; 1,3,3-trimethyl-6-((R)-4-methylcyclohex-3-en-1-yl)octahydrobenzo[c]isoxazole; 1,5,7-trimethylhexahydro-1H-spiro[benzo[c]isoxazole-3,1'-cyclopentane]; 1-ethyl-3,3,5,7-tetramethyl-5-(3-methylbut-2-en-1-yl)octahydrobenzo[c]isoxazole; 1-isopropyl-3,3,5,7-tetramethyl-5-(3-methylbut-2-en-1-yl)octahydrobenzo[c]isoxazole; 1-isopropyl-3,3,5,7-tetramethyloctahydrobenzo[c]isoxazol-1-ium chloride; 1-isopropyl-3,3,5,7-tetramethyloctahydrobenzo[c]isoxazole; 1-isopropyl-5,7-dimethylhexahydro-1H-spiro[benzo[c]isoxazole-3,1'-cyclopentane]; 2-(1,3,3,5,7-pentamethyloctahydrobenzo[c]isoxazol-5-yl)benzonitrile; 2-methyl-3-(1,3,3,5,7-pentamethyloctahydrobenzo[c]isoxazol-5-yl)benzonitrile; 5-(2,4-dimethoxyphenyl)-1,3,3,5,7-pentamethyloctahydrobenzo[c]isoxazole; 5-(2,5-dimethoxyphenyl)-1,3,3,5,7-pentamethyloctahydrobenzo[c]isoxazole; 5-(6-methoxypyridin-2-yl)-1,3,3,5,7-pentamethyloctahydrobenzo[c]isoxazole; 5,7-diethyl-1,3,3-trimethyloctahydrobenzo[c]isoxazole; 5-ethyl-1,3,3,5,7-pentamethyloctahydrobenzo[c]isoxazole; 6-isopentyl-1,4,4,8-tetramethyloctahydro-2H-benzo[d][1,3]oxazine; rac-(3aR,5R,7aR)-1,3,3-trimethyl-5-pentyloctahydrobenzo[c]isoxazole; rac-(3aR,5R,7aR)-1,3,3-trimethyl-5-propyloctahydrobenzo[c]isoxazole; rac-(3aR,5R,7aR)-1-ethyl-3,3-dimethyl-5-propyloctahydrobenzo[c]isoxazole; rac-(3aR,5R,7aR)-1-isopropyl-3,3-dimethyl-5-propyloctahydrobenzo[c]isoxazole; rac-(3aR,5R,7aR)-5-butyl-1,3,3-trimethyloctahydrobenzo[c]isoxazole; rac-(3aR,5R,7aR)-5-butyl-1-ethyl-3,3-dimethyloctahydrobenzo[c]isoxazole; rac-(3aR,5R,7aR)-5-butyl-1-isopropyl-3,3-dimethyloctahydrobenzo[c]isoxazole; rac-(3aR,5R,7aR)-5-ethyl-1-isopropyl-3,3-dimethyloctahydrobenzo[c]isoxazole; rac-(3aR,5R,7R,7aR)-1,3,3,5,7-pentamethyl-5-(2-(methylthio)phenyl)-octahydrobenzo[c]isoxazole; rac-(3aR,5R,7R,7aR)-1-ethyl-3,3,5,7-tetramethyl-5-(3-methylbut-2-en-1-yl)octahydrobenzo[c]isoxazole; rac-(3aR,5R,7R,7aR)-1-ethyl-3,3,5,7-tetramethyl-5-(o-tolyl)octahydrobenzo[c]isoxazole; rac-(3aR,5R,7R,7aR)-5-(3-chloro-2-methylphenyl)-1,3,3,5,7-pentamethyloctahydrobenzo[c]isoxazole; rac-(3aR,5R,7R,7aR)-5-(4-fluoro-2-methylphenyl)-1,3,3,5,7-pentamethyloctahydrobenzo[c]isoxazole; rac-(3aR,5R,7R,7aR)-5-(4-methoxyphenyl)-1,3,3,5,7-pentamethyloctahydrobenzo[c]isoxazole; rac-(3aR,5R,7S,7aR)-1,3,3,5,7-pentamethyl-5-(2-(methylthio)phenyl)octahydrobenzo[c]isoxazole; rac-(3aR,5R,7S,7aR)-1,3,3,5,7-pentamethyl-5-(2,4,5-trimethylphenyl)octahydrobenzo[c]isoxazole; rac-(3aR,5R,7S,7aR)-1,3,3,5,7-pentamethyl-5-(2-methyl-5-(trifluoromethyl)phenyl)octahydrobenzo[c]isoxazole; rac-(3aR,5R,7S,7aR)-1,3,3,5,7-pentamethyl-5-(4-(methylthio)phenyl)octahydrobenzo[c]isoxazole; rac-(3aR,5R,7S,7aR)-1,3,3,5,7-pentamethyl-5-(5-methylthiophen-2-yl)octahydrobenzo[c]isoxazole; rac-(3aR,5R,7S,7aR)-1,3,3,5,7-pentamethyl-5-(5-methylthiophen-3-yl)octahydrobenzo[c]isoxazole; rac-(3aR,5R,7S,7aR)-1,3,3,5,7-pentamethyl-5-(m-tolyl)octahydrobenzo[c]isoxazole; rac-(3aR,5R,7S,7aR)-1,3,3,5,7-pentamethyl-5-(naphthalen-1-yl)octahydrobenzo[c]isoxazole; rac-(3aR,5R,7S,7aR)-1,3,3,5,7-pentamethyl-5-(naphthalen-2-yl)octahydrobenzo[c]isoxazole; rac-(3aR,5R,7S,7aR)-1,3,3,5,7-pentamethyl-5-(o-tolyl)octahydrobenzo[c]isoxazole; rac-(3aR,5R,7S,7aR)-1,3,3,5,7-pentamethyl-5-(p-tolyl)octahydrobenzo[c]isoxazole; rac-(3aR,5R,7S,7aR)-1,3,3,5,7-pentamethyl-5-phenyloctahydrobenzo[c]isoxazole; rac-(3aR,5R,7S,7aR)-1,3,3,5,7-pentamethyl-5-propyloctahydrobenzo[c]isoxazole; rac-(3aR, 5R, 7S, 7a R)-1, 3, 3, 7-tetramethyl-5-(3-methyl but-2-en-1-yl)octahydrobenzo[c]isoxazole; rac-(3aR,5R,7S,7aR)-1,3,3,7-tetramethyl-5-(o-tolyl)octahydrobenzo[c]isoxazole; rac-(3aR,5R,7S,7aR)-1,3,3,7-tetramethyl-5-phenyloctahydrobenzo[c]isoxazole; rac-(3aR,5R,7S,7aR)-1-ethyl-3,3,5,7-tetramethyl-5-(3-methylbut-2-en-1-yl)octahydrobenzo[c]isoxazole; rac-(3aR,5R,7S,7aR)-1-ethyl-3,3,5,7-tetramethyl-5-(o-tolyl)octahydrobenzo[c]isoxazole; rac-(3aR,5R,7S,7aR)-1-ethyl-3,3,5,7-tetramethyl-5-phenyloctahydrobenzo[c]isoxazole; rac-(3aR,5R,7S,7aR)-1-ethyl-3,3,5,7-tetramethyloctahydrobenzo[c]isoxazole; rac-(3aR,5R,7S,7aR)-1-ethyl-5-(2-methoxyphenyl)-3,3,7-trimethyloctahydrobenzo[c]isoxazole; rac-(3aR,5R,7S,7aR)-1-isopropyl-3,3,5,7-tetramethyl-5-(3-methylbut-2-en-1-yl)octahydrobenzo[c]isoxazole; rac-(3aR,5R,7S,7aR)-1-isopropyl-3,3,5,7-tetramethyl-5-(o-tolyl)octahydrobenzo[c]isoxazole; rac-(3aR,5R,7S,7aR)-1-isopropyl-3,3,5,7-tetramethyl-5-phenyloctahydrobenzo[c]isoxazole; rac-(3aR,5R,7S,7aR)-1-isopropyl-3,3,5,7-tetramethyl-5-propyloctahydrobenzo[c]isoxazole; rac-(3aR,5R,7S,7aR)-1-isopropyl-3,3,7-trimethyl-5-(3-methylbut-2-en-1-yl)octahydrobenzo[c]isoxazole; rac-(3aR,5R,7S,7aR)-1-isopropyl-3,3,7-trimethyl-5-(o-tolyl)octahydrobenzo[c]isoxazole; rac-(3aR,5R,7S,7aR)-1-isopropyl-3,3,7-trimethyl-5-phenyloctahydrobenzo[c]isoxazole; rac-(3aR,5R,7S,7aR)-1-isopropyl-5-(2-methoxyphenyl)-3,3,5,7-tetramethyloctahydrobenzo[c]isoxazole; rac-(3aR,5R,7S,7aR)-1-isopropyl-5-(2-methoxyphenyl)-3,3,7-trimethyloctahydrobenzo[c]isoxazole; rac-(3aR,5R,7S,7aR)-1-isopropyl-5-(4-methoxyphenyl)-3,3,5,7-tetramethyloctahydrobenzo[c]isoxazole; rac-(3aR,5R,7S,7aR)-1-isopropyl-5-(6-methoxypyridin-2-yl)-3,3,5,7-tetramethyloctahydrobenzo[c]isoxazole; rac-(3aR,5R,7S,7aR)-5-(2,3-dimethylphenyl)-1,3,3,5,7-pentamethyloctahydrobenzo[c]isoxazole; rac-(3aR,5R,7S,7aR)-5-(2,4-difluorophenyl)-1,3,3,5,7-pentamethyloctahydrobenzo[c]isoxazole; rac-(3aR,5R,7S,7aR)-5-(2,4-dimethylphenyl)-1,3,3,5,7-pentamethyloctahydrobenzo[c]isoxazole; rac-(3aR,5R,7S,7aR)-5-(2,4-dimethylphenyl)-1,3,3,7-tetramethyloctahydrobenzo[c]isoxazole; rac-(3aR,5R,7S,7aR)-5-(2,5-difluorophenyl)-1,3,3,5,7-pentamethyloctahydrobenzo[c]isoxazole; rac-(3aR,5R,7S,7aR)-5-(2,5-dimethylphenyl)-1,3,3,5,7-pentamethyloctahydrobenzo[c]isoxazole; rac-(3aR,5R,7S,7aR)-5-(2,5-dimethylphenyl)-1,3,3,7-tetramethyloctahydrobenzo[c]isoxazole; rac-(3aR,5R,7S,7aR)-5-(2,6-dimethylphenyl)-1,3,3,5,7-pentamethyloctahydrobenzo[c]isoxazole; rac-(3aR,5R,7S,7aR)-5-(2-ethoxyphenyl)-1,3,3,5,7-pentamethyloctahydrobenzo[c]isoxazole; rac-(3aR,5R,7S,7aR)-5-(2-ethylphenyl)-1,3,3,5,7-pentamethyloctahydrobenzo[c]isoxazole; rac-(3aR,5R,7S,7aR)-5-(2-ethylphenyl)-1,3,3,7-tetramethyloctahydrobenzo[c]isoxazole; rac-(3aR,5R,7S,7aR)-5-(2-ethylphenyl)-1-isopropyl-3,3,7-trimethyloctahydrobenzo[c]isoxazole; rac-(3aR,5R,7S,7aR)-5-(2-fluorophenyl)-1,3,3,5,7-pentamethyloctahydrobenzo[c]isoxazole; rac-(3aR,5R,7S,7aR)-5-(2-methoxyphenyl)-1,3,3,5,7-pentamethyloctahydrobenzo[c]isoxazole; rac-(3aR,5R,7S,7aR)-5-(2-methoxyphenyl)-1,3,3,7-tetramethyloctahydrobenzo[c]isoxazole; rac-(3aR,5R,7S,7aR)-5-(3,4-difluorophenyl)-1,3,3,5,7-pentamethyloctahydrobenzo[c]isoxazole; rac-(3aR,5R,7S,7aR)-5-(3,5-difluorophenyl)-1,3,3,5,7-pentamethyloctahydrobenzo[c]isoxazole; rac-(3aR,5R,7S,7aR)-5-(3,5-dimethoxyphenyl)-1,3,3,5,7-pentamethyloctahydrobenzo[c]isoxazole; rac-(3aR,5R,7S,7aR)-5-(3,5-dimethylphenyl)-1,3,3,5,7-pentamethyloctahydrobenzo[c]isoxazole; rac-(3aR,5R,7S,7aR)-5-(3,5-dimethylphenyl)-1-isopropyl-3,3,5,7-tetramethyloctahydrobenzo[c]isoxazole; rac-(3aR,5R,7S,7aR)-5-(3-chloro-2-methylphenyl)-1,3,3,5,7-pentamethyloctahydrobenzo[c]isoxazole; rac-(3aR,5R,7S,7aR)-5-(3-chloro-2-methylphenyl)-1-isopropyl-3,3,7-trimethyloctahydrobenzo[c]isoxazole; rac-(3aR,5R,7S,7aR)-5-(3-fluoro-5-(trifluoromethyl)phenyl)-1,3,3,5,7-pentamethyloctahydrobenzo[c]isoxazole; rac-(3aR,5R,7S,7aR)-5-(3-fluorophenyl)-1,3,3,5,7-pentamethyloctahydrobenzo[c]isoxazole; rac-(3aR,5R,7S,7aR)-5-(3-isopropylphenyl)-1,3,3,5,7-pentamethyloctahydrobenzo[c]isoxazole; rac-(3aR,5R,7S,7aR)-5-(3-methoxyphenyl)-1,3,3,5,7-pentamethyloctahydrobenzo[c]isoxazole; rac-(3aR,5R,7S,7aR)-5-(4-(tert-butyl)phenyl)-1,3,3,5,7-pentamethyloctahydrobenzo[c]isoxazole; rac-(3aR,5R,7S,7aR)-5-(4-(tert-butyl)phenyl)-1,3,3,7-tetramethyloctahydrobenzo[c]isoxazole; rac-(3aR,5R,7S,7aR)-5-(4-(tert-butyl)phenyl)-1-isopropyl-3,3,7-trimethyloctahydrobenzo[c]isoxazole; rac-(3aR,5R,7S,7aR)-5-(4-chloro-2-methylphenyl)-1-isopropyl-3,3,7-trimethyloctahydrobenzo[c]isoxazole; rac-(3aR,5R,7S,7aR)-5-(4-fluoro-2-methoxyphenyl)-1,3,3,5,7-pentamethyloctahydrobenzo[c]isoxazole; rac-(3aR,5R,7S,7aR)-5-(4-fluoro-2-methylphenyl)-1,3,3,5,7-pentamethyloctahydrobenzo[c]isoxazole; rac-(3aR,5R,7S,7aR)-5-(4-fluoro-2-methylphenyl)-1,3,3,7-tetramethyloctahydrobenzo[c]isoxazole; rac-(3aR,5R,7S,7aR)-5-(4-fluorophenyl)-1,3,3,5,7-pentamethyloctahydrobenzo[c]isoxazole; rac-(3aR,5R,7S,7aR)-5-(4-fluorophenyl)-1-isopropyl-3,3,5,7-tetramethyloctahydrobenzo[c]isoxazole; rac-(3aR,5R,7S,7aR)-5-(4-methoxyphenyl)-1,3,3,5,7-pentamethyloctahydrobenzo[c]isoxazole; rac-(3aR,5R,7S,7aR)-5-(5-chloro-2-methylphenyl)-1,3,3,5,7-pentamethyloctahydrobenzo[c]isoxazole; rac-(3aR,5R,7S,7aR)-5-(5-chloro-2-methylphenyl)-1-isopropyl-3,3,7-trimethyloctahydrobenzo[c]isoxazole; rac-(3aR,5R,7S,7aR)-5-(5-fluoro-2-methylphenyl)-1,3,3,5,7-pentamethyloctahydrobenzo[c]isoxazole; rac-(3aR,5R,7S,7aR)-5-(5-fluoro-2-methylphenyl)-1,3,3,7-tetramethyloctahydrobenzo[c]isoxazole; rac-(3aR,5R,7S,7aR)-5-(benzo[d][1,3]dioxol-5-yl)-1,3,3,5,7-pentamethyloctahydrobenzo[c]isoxazole; rac-(3aR,5R,7S,7aR)-5-(furan-3-yl)-1,3,3,5,7-pentamethyloctahydrobenzo[c]isoxazole; rac-(3aR,5R,7S,7aR)-5,7-diethyl-1-isopropyl-3,3-dimethyloctahydrobenzo[c]isoxazole; rac-(3aR,5R,7S,7aR)-5-isopentyl-1-isopropyl-3,3,5,7-tetramethyloctahydrobenzo[c]isoxazole; rac-(3aR,5S,7aR)-1,3,3,5-tetramethyl-5-(3-methylbut-2-en-1-yl)octahydrobenzo[c]isoxazole; rac-(3aR,7aR)-1,3,3,4,5,7-hexamethyloctahydrobenzo[c]isoxazole; rac-(3aR,7aR)-1,3,3-trimethyl-6-phenyloctahydrobenzo[c]isoxazole; rac-(3aR,7aR)-1,5,7-triethyl-3,3-dimethyloctahydrobenzo[c]isoxazole; rac-(3aR,7aS)-1,3,3,4,5,7-hexamethyloctahydrobenzo[c]isoxazolerac-(3aS,5R,7S,7aS)-5-(4-fluorophenyl)-1,3,3,5,7-pentamethyloctahydrobenzo[c]isoxazole; rac-(3aS,5S,7S,7aS)-1-ethyl-3,3,7-trimethyl-5-(3-methylbut-2-en-1-yl)octahydrobenzo[c]isoxazole; rac-2-chloro-5-((3aR,5R,7S,7aR)-1,3,3,5,7-pentamethyloctahydrobenzo[c]isoxazol-5-yl)benzonitrile; rac-2-chloro-5-((3aS,5R,7R,7aS)-1,3,3,5,7-pentamethyloctahydrobenzo[c]isoxazol-5-yl)benzonitrile; rac-2-methyl-5-((3aR,5R,7S,7aR)-1,3,3,5,7-pentamethyloctahydrobenzo[c]isoxazol-5-yl)benzonitrile; rac-3-((3aR,5R,7S,7aR)-1,3,3,5,7-pentamethyloctahydrobenzo[c]isoxazol-5-yl)benzonitrile; rac-3-((3aR,5R,7S,7aR)-1-ethyl-3,3,5,7-tetramethyloctahydrobenzo[c]isoxazol-5-yl)-4-methylbenzonitrile; rac-3-((3aR,5R,7S,7aR)-1-isopropyl-3,3,5,7-tetramethyloctahydrobenzo[c]isoxazol-5-yl)-4-methylbenzonitrile; rac-3-((3aR,5R,7S,7aR)-1-isopropyl-3,3,5,7-tetramethyloctahydrobenzo[c]isoxazol-5-yl)benzonitrile; rac-3-((3aR,5R,7S,7aR)-1-isopropyl-3,3,7-trimethyloctahydrobenzo[c]isoxazol-5-yl)-4-methoxybenzonitrile; rac-3-chloro-5-((3aR,5R,7S,7aR)-1,3,3,5,7-pentamethyloctahydrobenzo[c]isoxazol-5-yl)benzonitrile; rac-3-methyl-4-((3aR,5R,7S,7aR)-1,3,3,5,7-pentamethyloctahydrobenzo[c]isoxazol-5-yl)benzonitrile; rac-4-((3aR,5R,7S,7aR)-1,3,3,5,7-pentamethyloctahydrobenzo[c]isoxazol-5-yl)benzonitrile; rac-4-methoxy-3-((3aR,5R,7R,7aR)-1,3,3,5,7-pentamethyloctahydrobenzo[c]isoxazol-5-yl)benzonitrile; rac-4-methoxy-3-((3aR,5R,7S,7aR)-1,3,3,5,7-pentamethyloctahydrobenzo[c]isoxazol-5-yl)benzonitrile; rac-4-methyl-2-((3aR,5R,7S,7aR)-1,3,3,5,7-pentamethyloctahydrobenzo[c]isoxazol-5-yl)benzonitrile; rac-4-methyl-3-((3aR,5R,7S,7aR)-1,3,3,5,7-pentamethyloctahydrobenzo[c]isoxazol-5-yl)benzonitrile; rac-4-methyl-3-((3aR,5R,7S,7aR)-1,3,3,7-tetramethyloctahydrobenzo[c]isoxazol-5-yl)benzonitrile; rac-methyl 4-methyl-3-((3aR,5R,7S,7aR)-1,3,3,5,7-pentamethyloctahydrobenzo[c]isoxazol-5-yl)benzoate; rel-4-methyl-3-((3aR,5R,7S,7aR)-1,3,3,5,7-pentamethyloctahydrobenzo[c]isoxazol-5-yl)benzonitrile and rel-4-methyl-3-((3aS,5S,7R,7aS)-1,3,3,5,7-pentamethyloctahydrobenzo[c]isoxazol-5-yl)benzonitrile.

As used hereinabove the prefix "rac" refers to the relative stereochemistry of the respective descriptors (R, S) disclosing the racemic mixture of both enantiomers; the prefix "rel" refers to the relative stereochemistry of the respective descriptors (R, S) referring to a single, unasigned enantiomer. Chemical names with no stereochemical descriptor (i.e. neither R nor S) refer to mixtures of diastereomers and enantiomers.

As used in relation to compounds of formula (Ia) (which encompass the compounds of formula (Ib)) unless otherwise indicated "alkyl" refers to linear or branched alkyl, "alkenyl" refers to linear or branched alkyl comprising at least one carbon-to-carbon double bond, e.g. 2 or 3 double bonds; and "cycloalkenyl" refers to cycloalkyl comrprising at least one carbon-to-carbon double bond with the proviso that the ring is not saturated.

The compounds as defined by formula (Ia) (which encompass the compounds of formula (Ib)) comprise several chiral centers and as such may exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or stereoselective synthesis. Accordingly, the chemical structures depicted herein encompass all possible stereoisomers forms of the illustrated compounds.

It is also noted that the compounds as defined by formula (Ia) (which encompass the compounds of formula (Ib)) may exist in unsolvated forms as well as solvated forms, including hydrated forms and as N-oxides. In general, compounds may be hydrated, solvated or N-oxides. Certain compounds may exist in multiple crystalline or amorphous forms. In general, all physical forms are equivalent for the uses contemplated herein and are intended to be within the scope of the present invention.

"Solvate" means a compound formed by solvation (the combination of solvent molecules with molecules or ions of the solute), or an aggregate that consists of a solute ion or molecule, i.e., a compound as defined by formula (Ia) (which encompass the compounds of formula (Ib)), with one or more solvent molecules. When water is the solvent, the corresponding solvate is "hydrate". Further suitable solvents can be but are not limited to: acetone, acetonitrile, benzene, cyclohexane, dihydrolevoglucosenone, methyl-tetrahydrofuran, pentylene glycol, ethylene glycol, petroleum ether, ethyl lactate, methyl lactate, propyl lactate, diethylether, tert-butyl methyl ether, dimethylsulfoxide, N,N-dimethylformamide, N,N-dimethylacetamide, dioxane, ethanol, ethyl acetate, ethylene glycol, diethylene glycol, propylene glycol, heptane, hexane, methanol, toluene and xylene.

"Salt" refers to a salt of a compound as defined by formula (Ia) (which encompass the compounds of formula (Ib)), which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as amino acids, acetic acid, trifluoroacetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-( 4-hydroxybenzoyl) benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced by a metal ion, e.g., an alkali metal ion, an alkaline earth ion, or an aluminum ion; or coordinates with an organic base such as ethanolamine, diethanolamine, triethanolamine, N-methylglucamine and the like.

The compounds as defined by formula (I) (which encompass the compounds of formula (Ia) and (Ib)) are "TRPM8 agonist", which means that they have an agonistic effect on the cellular Ca²⁺ ion permeability of the TRPM8 channels. Accordingly, by "TRPM8 agonist" is meant any compound, which when brought into contact with the TRPM8 receptor, produces an increase in fluorescence over background, using the FLIPR method as described, e.g., by Klein et al., (Chem. Senses 36: 649-658, 2011), which is also described in more details in the experimental part.

Accordingly there is provided in a second aspect a non-medical method of modulating (in-vitro and in-vivo modulation) of transient receptor potential melastatin member 8 (TRPM8) comprising bringing the receptor into contact with a compound of formula (Ia), or a salt or solvate thereof.

In certain embodiments of the second aspect of the invention the modulating method is an in-vitro method.

There is provided in a third aspect a non-medical method of inducing a cooling sensation in a human or animal comprising contacting the human or animal with a compound of formula (Ia) (which encompass the compounds of formula (Ib)), or a salt or solvate thereof. In this context it should be noted with regard to compounds of formula (Ib) that one stereoisomer may be more potent than the other stereoisomer.

In certain embodiments, the method is a method of achieving a cooling effect on the skin or mucosa comprising contacting the skin or mucosa with a product comprising one or more compounds of formula (Ia) (which encompass the compounds of formula (Ib)), or a salt or solvate thereof.

The compounds of formula (Ia) (which encompass the compounds of formula (Ib)), may be applied directly or as a solution or suspension, comprising an effective amount of a compound of formula (Ia). An amount to be effective depends, inter alia, upon the target TRPM8 area of the body but also on the cooling potency of compound or mixture of compounds.

There is provided in a fourth aspect consumer products, in particular consumer products which get into contact with the human skin and/or mucosa comprising a compound as defined by formula (Ia), which encompass the compounds of formula (Ib)).

Consumer products which get in contact with the mucosa include, but are not limited to food products, beverages, chewing gum, tobacco and tobacco replacement products, dental care products, personal care products, including lip care products, sexual health and intimate care products.

In certain embodiments dental care products are oral care products, tooth care products, cleaners for dental prostheses, adhesives for dental prostheses, and the like.

In certain embodiments food products are iced consumable products such as ice cream, sorbet; confectioneries such as candies and chocolates; food products containing mint or mint flavour, sauces, dairy products such as milk-based drinks and yoghurts; and snacks.

In certain embodiments tobacco replacement products are liquids or solids which are suitable to be consumed by electrical means, e.g., liquids to vape.

In certain embodiments personal care products getting in contact with the mucosa are lip balms, nose sprays and eye drops.

Consumer products which get in contact with the human skin include, but are not limited to cosmetic products. In certain embodiments cosmetic products are skincare products, especially bath products, skin washing and cleansing products, skincare products, eye makeup, nail care products, foot care products, and the like. In certain embodiments cosmetic products are products with specific effects, especially sunscreens, insect repellent products, tanning products, de-pigmenting products, deodorants, antiperspirants, hair removers, and shaving products. In a certain embodiments cosmetic products are hair care products, especially hair shampoos, hair care products, hair setting products, hair-shaping products, and hair coloring products as well as scalp-care products such as scalp-cooling shampoos and creams.

In certain embodiments, the consumer product is selected from air care products, such as an air freshener or a "ready to use" powdered air freshener which can be used in the home space (rooms, refrigerators, cupboards, shoes or car) and/or in a public space (halls, hotels, malls, etc .. ).

The consumer products can be in any physical form, such as a solid, semi-solid, plaster, solution, suspension, lotion, cream, foam, gel, paste, or a combination thereof. The physical form of the consumer product suitable manly depends on the specific actions, such as cleaning, softening, caring, cooling, and the like, such a consumer product should fulfill.

In a certain embodiment consumer products getting in contact with the human skin are fabric care products (such as fabric detergents, fabric conditioner (including tumble dryer sheets), and scent boosters (liquid or solid)) which in a first step are applied to a fabric, e.g., when washing the fabric, said treaded fabrics then getting in contact with the human skin.

The level of use for compounds of the present invention (compounds as defined by formula (Ia), which encompass compounds of formula (Ib)) depend, inter alia, upon the target TRPM8 area of the body but also on the cooling potency of compound or mixture of compounds. For examples in an oral application of a compound of the present invention, such as dentifrice, floss, chewing gum, or white strip, the levels of use may be from about 0.00001 % (0.01 ppm) to about 0.1 % (1000 ppm); from about 0.00005% (0.5 ppm) to about 0.1 % (1000 ppm); from about 0.0001 % (1 ppm) to about 0.05% (500 ppm); or from about 0.001 % (10 ppm) to about 0.01 % (100 ppm) by weight of the composition. When a compound of the present invention is used in a mouthwash, the level of use may be from about 0.000001 % (10 ppb) to about 0.01 % (100 ppm) or from about 0.0001 % (1 ppm) to about 0.001 % (10 ppm) by weight of the composition. When a compound of the present invention is delivered topically, for example in shampoos and lotions the levels may be from about 0.001 % (10 ppm) to about 0.5% (5000 ppm) by weight of the composition or from about 0.01 % (100 ppm) to about 0.4% (4000 ppm) by weight of the composition.

The cooling potency (strength) of a compound is defined by its EC₅₀ value. EC₅₀ (half maximal effective concentration) refers to the concentration of a compound which induces a response halfway between the baseline and maximum after a specified exposure time. It is commonly used as a measure of potency. EC₅₀ is a measure of concentration, expressed in µM (µmolar) unites, where 1 µM is equivalent to 1 µmol/L.

Compounds with an EC₅₀ of 10 µM or less are perceived by the human as cooling. The lower the EC₅₀ value the higher the cooling potency. For example, compounds having an EC₅₀ value of about 0.1 µM are perceived as strong cooling compounds.

Cooling properties of a compound however are not only defined by its strength (potency; EC50) but also its longevity, which refers to the period of time (in minutes) over which a cooling effect is perceived. The longevity can range from a few seconds after rinsing to several hours or even days. Another important property of cooling compounds is the onset speed, which refers to how fast the cooling effect is perceived after a compound gets in contact with with the mucosa or skin. Onset speeds can range from no perceivable delay, delivering the cooling sensation immediately upon contact, to a few seconds or even minutes after rinsing. The compounds of formula (Ia) (which encompass compounds of formula (Ib)) is a class of compounds which delivers the cooling sensation almost immediately upon contact.

The above described "onset speed" is typically sought-after in particular for oral care products, since a cooling sensation is already noticed during the use of the respective product (e.g. during brushing with toothpaste or rinsing with mouthwash). This so called "up-front" coooling sensation enhances the consumer experience during the use of oral care products. It reinforces that the oral care product is actively delivering on its claimed benefits and is therefore associated with cleanliness and oral health. Moreover, compounds delivering this "up-front" cooling sensation combine well with compounds that deliver long-lasting cooling sensation, creating a sustained effect that starts during product use and lasts for several minutes or even hours after use. Depending on the desired effects to be achieved, a flavorist skilled in the art will know how to combine compounds with different cooling properties to create the desired temporal profile of the cooling effect.

The compounds of formula (Ia) (which encompass the compounds of formula (Ib)) are very potent at relative low concentrations. Thus it is preferred to prepare a stock solution which is further diluted, before admixing it to a consumer product. Beside water, particular suitable solvents are triacetin and propylene glycol. One may also mention acetone, benzyl alcohol dihydrolevoglucosenone, methyl-tetrahydrofuran, pentylene glycol, ethylene glycol, ethyl lactate, methyl lactate, propyl lactate, dimethylsulfoxide, ethanol, ethyl acetate, ethylene glycol, diethylene glycol, propylene glycol, and triacetin which are suitable solvents for the compounds of formula (Ia) (which encompass the compounds of formula (lb)).But other solvent systems comprising surfactants may also be used.

To modify the cooling effect of a compound as defined herein by formula (Ia) (which encompass the compounds of formula (Ib)), the compound, a salt or solvate thereof may be combined with a compound selected from calcium ions and salts, magnesium ions and salts, arginine, or any chelating agent which is able to bind calcium or magnesium.

These compounds are known to be able to modulate the concentration of such ions in the extracellular space and therefore influence the response of the TRPM8 ion-channel, leading to a change in the perceived cooling effect.

According to Kizilbash et al. (WO2019/121193 A1) both, the cooling intensity and the flavour intensity may be enhanced when combined with agents which possess the property to potentiating said effects. Thus the compounds as defined herein by formula (Ia) may be combined in one particular embodiment with potentiating agents disclosed in WO2019/121193 which is incorporated by reference, in particular with regard to the potentiating agents. As a further enhancement agent one may cite N-lactoyl ethanolamine (2-hydroxy-N-(2-hydroxyethyl)propanamide; CAS 5422-34-4) which is known as an enhancer for cooling agents, for example, from PCT International publication WO 2008/107137 which is incorporated by reference, in particular with regard to the cooling enhancing substances as defined by formula (Ia).

For the reasons given above a combination of cooling compounds possessing different cooling profiles might be desired, depending on the effects to be desired by the consumer.

Thus there is provided in a fifth aspect a composition comprising a cool sensation wherein the composition comprises at least one compound of formula (Ia), a salt or solvate thereof, and a further cooling compound.

In one particular embodiment the compounds of formula (Ia) (which encompass the compounds of formula (Ib)) may be combined with menthol (e.g., in form of peppermint oil, and/or spearmint oil), menthone, p-menthanecarboxamides, N-2,3-trimethyl-2-isopropyl-butanamide (WS-23), menthyl lactate (Frescolat^{®} ML), menthone glycerol acetal (Frescolat^{®} MGA), 3-(1-menthoxy)-propane-1,2-diol (TK-10), p-menthane-3,8-diol (known as Coolact 38D), isopulegol (known as Coolact P), monomenthyl succinate (Physcool ^{®}), monomenthyl glutarate, o-menthylglycerol, menthyl N,N-dimethylsuccinamate, 2-(sec-butyl)cyclohexan-1-one (Freskomenthe), N-(pyrazol-3-yl)-N-(thiophen-2-ylmethyl)-2-(p-tolyloxy)acetamide, 2-(4-ethylphenoxy)-N-(pyrazol-3-yl)-N-(thiophen-2-ylmethyl)acetamide, 3-(benzo[d][1,3]dioxol-5-yl)-N,N-diphenylacrylamide, 4-(2-(4-allyl-2,6-dimethoxyphenoxy)-1-ethoxypropyl)-2-methoxyphenol, 4-(2-(4-allyl-2,6-dimethoxyphenoxy)-1-((2-isopropyl-5-methylcyclohexyl)oxy)propyl)-2-methoxyphenol (including 4-(2-(4-allyl-2,6-dimethoxyphenoxy)-1-(((1S,2R,5S)-2-isopropyl-5-methylcyclohexyl)oxy)propyl)-2-methoxyphenol) and 4-(2-(4-allyl-2,6-dimethoxyphenoxy)-1-(((1R,2S,5R)-2-isopropyl-5-methylcyclohexyl)oxy)propyl)-2-methoxyphenol), N-(2-Hydroxy-2-phenylethyl)-2-isopropyl-5,5-dimethylcyclohexane-1-carboxamide, N-(4-(Cyanomethyl)phenyl)-2-isopropyl-5,5-dimethylcyclohexanecarboxamide and N-(3-Hydroxy-4-methoxyphenyl )-2-isopropyl-5,5-dimethylcyclohexanecarboxamide. Further cooling compounds with which the compounds of formula (I) may be combined are those descrbed in the international patent application PCT/EP2020/079009 of the applicant.

Examples of p-methanecarboxamides include compounds such as N-ethyl-p-menthan-3-carboxamide (known commercially as WS-3), N-ethoxycarbonylmethyl-p-menthan-3-carboxamide (WS-5), N-( 4-methoxyphenyl)-p-menthan-3-carboxamide (WS-12) and N-tert-butyl-p-menthan-3-carboxamide (WS-14), N-(4-(cyanomethyl)phenyl)-2-isopropyl-5-methylcyclohexane-1-carboxamide (known commercially as Evercool 180), 2-isopropyl-5-methyl-N-(2-(pyridin-2-yl)ethyl)cyclohexane-1-carboxamide (known commercially as Evercool 190), and (1R,2S,5R)-N-((S)-2-((R)-2-aminopropanamido)-2-phenylethyl)-2-isopropyl-5-methylcyclohexane-1-carboxamide.

In order to achieve more than just a cooling effect, the compounds of formula (Ia) (which encompass the compounds of formula (Ib)), a salt or solvate thereof, may be combined with other actives, such as, flavours, fragrances, and sweetening agents.

Examples of flavour ingredients include natural flavors, artificial flavors, spices, seasonings, and the like. Exemplary flavor ingredients include synthetic flavor oils and flavoring aromatics and/or oils, oleoresins, essences, and distillates, and a combination comprising at least one of the foregoing.

Flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil; useful flavoring agents include artificial, natural and synthetic fruit flavors such as vanilla, and citrus oils including lemon, orange, lime, grapefruit, yuzu, sudachi, and fruit essences including apple, pear, peach, grape, raspberry, blackberry, gooseberry, blueberry, strawberry, cherry, plum, prune, raisin, cola, guarana, neroli, pineapple, apricot, banana, melon, apricot, cherry, tropical fruit, mango, mangosteen, pomegranate, papaya, and so forth.

Additional exemplary flavors imparted by a flavoring composition include a milk flavor, a butter flavor, a cheese flavor, a cream flavor, and a yogurt flavor; a vanilla flavor; tea or coffee flavors, such as a green tea flavor, an oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a chamomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; a nut flavor such as an almond flavor, a hazelnut flavor, a macadamia nut flavor, a peanut flavor, a pecan flavor, a pistachio flavor, and a walnut flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor.

Generally any flavoring or food additive (including food colors) such as those described in "Essential guide to food additives", Third edition2008, page 101 - 321 (ISBN: 978-1-905224-50-0) by Leatherhead Food International Ltd., can be used. The publication is incorporated herein by reference.

In one particular embodiment the compounds of formula (Ia) (which encompass the compounds of formula (Ib)) may be combined with anethole, menthol laevo, carvone laevo, ethyl maltol, vanillin, eucalyptol, eugenol, menthol racemic, cis-3-hexenol, linalol, mint oil (e.g. peppermint arvensis oil, peppermint piperita oil, spearmint native oil, spearmint scotch oil), corylone, ethyl butyrate, cis-3-hexenyl acetate, citral, eucalyptus oil, ethyl-vanillin, methyl salicylate, 2'-hydroxypropiophenone, ethyl acetate, methyl dihydro jasmonate, geraniol, lemon oil, iso amyl acetate, thymol, ionone beta, linalyl acetate, decanal, cis jasmone, ethyl hexanoate, melonal (2,6-dimethylhept-5-enal), citronellol, ethyl aceto acetate, nutmeg oil and clove oil, or mixtures thereof.

Examples of sweetening agents include, but are not limited to, sucrose, fructose, glucose, high fructose corn syrup, corn syrup, xylose, arabinose, rhamnose, erythritol, xylitol, mannitol, sorbitol, inositol, acesulfame potassium, aspartame, neotame, sucralose, and saccharine, and mixtures thereof; trilobatin, hesperetin dihydrochalcone glucoside, naringin dihydrochalcone, mogroside V, Luo Han Guo extract, rubusoside, rubus extract, glycyphyllin, isomogroside V, mogroside IV, siamenoside I, neomogroside, mukurozioside IIb, (+)-hernandulcin, 4 β -hydroxyhernandulcin, baiyunoside, phlomisoside I, bryodulcoside, bryoside bryonoside, abrusosides A-E, cyclocarioside A, cyclocaryoside I, albiziasaponins A-E, glycyrrhizin, araboglycyrrhizin, periandrins I-V, pterocaryosides A and B, osladin, polypodosides A and B, telosmoside A8-18, phyllodulcin, huangqioside E neoastilbin, monatin, 3-acetoxy-5,7-dihydroxy-4'-methoxyflavanone, 2R,3R-(+)-3-Acetoxy-5,7,4'-trihydroxyflavanone, (2R,3R)-dihydroquercetin 3-O-acetate, dihydroquercetin 3-O-acetate 4'-methyl ether, brazzein, curculin, mabinlin, monellin, neoculin, pentadin, thaumatin, and combinations thereof. Some of the compounds listed above are known sweetness enhancers as well as sweeteners. When used as sweetness enhancers they are normally used below their sweetness detection thresholds.

In certain embodiments, the compounds of formula (Ia) (which encompass the compounds of formula (Ib)) may be combined with additional ingredients collectively refereed to orally acceptable carrier materials.

In some aspects, the orally acceptable carrier may comprise one or more compatible solid or liquid excipients or diluents which are suitable for topical oral administration. By "compatible," as used herein, is meant that the components of the composition are capable of being commingled without interaction in a manner which would substantially reduce stability and/or efficacy. The carriers can include the usual and conventional components of dentifrices, non-abrasive gels, subgingival gels, mouthwashes or rinses, mouth sprays, chewing gums, lozenges and breath mints. The choice of a carrier to be used is basically determined by the way the composition is to be introduced into the oral cavity. Carrier materials for toothpaste, tooth gel or the like include abrasive materials, sudsing agents, binders, humectants, flavoring and sweetening agents, etc. as disclosed in e.g., U.S. Pat. No. 3,988,433, to Benedict. Carrier materials for biphasic dentifrice formulations are disclosed in U.S. Pat. Nos. 5,213,790; 5,145,666 and 5,281,410 all to Lukacovic et al., and in U. S. Patents 4,849,213 and 4,528,180 to Schaeffer. Mouthwash, rinse or mouth spray carrier materials typically include water, flavoring and sweetening agents, etc., as disclosed in, e.g., U.S. Pat. No. 3,988,433 to Benedict. Lozenge carrier materials typically include a candy base; chewing gum carrier materials include a gum base, flavoring and sweetening agents, as in, e.g., U.S. Pat. No. 4,083,955, to Grabenstetter et al.. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910 both to Damani. Carriers suitable for the preparation of compositions of the present disclosure are well known in the art. Their selection will depend on secondary considerations like taste, cost, and shelf stability, and the like.

Further suitable types of orally acceptable carrier materials or excipients are listed in WO2010/059289, in particular on page 17 - 31, which is incorporated by reference.

Scientific literature points out that the activation of TRPM8 channels may be useful for the treatment of most TRPM8-mediated pathologies (J. Med. Chem. 2016, 59 (22), 10006-10029). Thus one may assume that the compounds of formula (I) might also be suitable for treating prostate carcinomas, bladder weakness, inflammation, or pain comprising contacting a patient with one or more compounds of formula (Ia) as defined herein. One may also assume that the compounds of formula (Ia) as defined herein are suitable for alleviating the symptoms of coughs and colds, irritations, sore throat or hoarseness, as well as the treatment of laryngopharyngeal dysphagia (*Int. J. Mol. Sci. 2018, 19, 4113).*

Thus there is provided in a sixth aspect pharmaceutical composition comprising one or more compounds as defined by formula (Ia) (which encompass compounds of formula (Ib)), or a salt or solvate thereof.

Depending upon the particular treatment regimen contemplated, pharmaceutical compositions comprising one or more compounds of formula (Ia) may be administered parenterally, topically, orally, or locally. The pharmaceutical compositions may be a liquid, suspensions or a solid formulation.

In certain embodiments, the pharmaceutical composition is nasal spray, topical cream, skin sprays, throat spray, or eye drops.

The compounds of formula (Ia) are either compounds known per se or may be prepared by a person skilled in the art using known synthesis methods. For compounds of formula (Ia) may be prepared from aldehydes 2 by reacting them with a respective hydroxylamine R⁶NHOH or its salt form (e.g. hydrochloride), optionally in the presence of a base such as an inorganic base (e.g. potassium carbonate or sodium hydroxide) or an organic base. The reaction is usually performed under inert atmosphere such as argon or dinitrogen, in a suitable solvent such as toluene or xylene, typically at elevated temperatures such as 120°C or 140°C and often under removal of water by a Dean-Stark apparatus. The thus obtained compounds of formula (I) wherein n is 0, can be further converted into compounds of formula (I) wherein n is 1, e.g., by reducing the nitrogen-oxygen bond with typical reducing agents such as hydrogen under action of a palladium catalyst (e.g. palladium on charcoal) or zinc powder using an appropriate solvent such as ethyl acetate or tetrahydrofuran or toluene at suitable temperatures such as room temperature or elevated temperature (e.g. 50°C), and then condensing the resulting product with an appropriate reaction partner such as an aldehyde R'CHO or carbonyl diimidazole to give the desired substitution in formula (I). 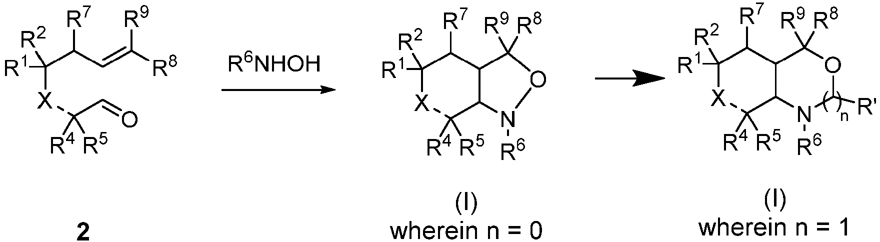 X is CR³, and R¹, R², R³, R⁴ - R⁹ and R' having the same meaning as provided for formula (Ia).

In general aldehydes 2 may be prepared by standard synthetic methods known to those skilled in the art. For example, aldehydes 2 wherein X is CHR³ and the dotted line represents a single bond, may be prepared by controlled reduction of the α,β-unsaturated aldehydes 3 by reacting them with hydrogen gas in the presence of a catalyst such as Lindlar catalyst (palladium on calcium carbonate, poisoned by lead) in an appropriate solvent such as ethyl acetate at an appropriate temperature such as room temperature. Other methods for controlled reduction are known to those skilled in the art and conditions may vary depending on the substrate.

In turn, aldehydes 3 may be prepared by condensation of aldehyde 4 and allylic alcohol 5 by reacting them together (as depicted below), optionally in the presence of an acid catalyst such as a protic acid (e.g. triethylamine-hydrochloride or p-toluenesulfonic acid) or a Lewis acid. This is done in a suitable solvent such as xylenes or toluene or no solvent and at elevated temperatures (e.g. 80°C, 120°C or 140°C), typically under removal of water by Dean-Stark apparatus. 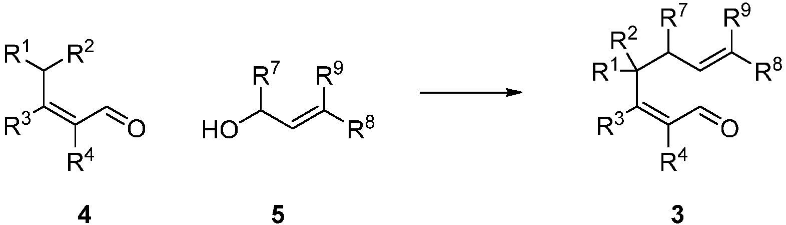 R¹ - R⁴, and R⁷ - R⁹ having the same meaning as provided for formula (I).

For compounds of formula (Ia) wherein R¹ is selected from phenyl (optionally substituted as defined above), naphthyl (optionally substituted as defined above) and C₅-C₁₀ mono- or bicyclic aryl wherein 1 or 2 C-atoms are replaced by a hetero atom independently selected from sulfur, nitrogen, and oxygen, aldehyde 3 may be prepared from aldehyde 6 (which in turn can be prepared as described above). The conversion of aldehyde 6 to aldehyde 3 can be done by reacting aldehyde 6 with the respective aryl bromide R¹Br (wherein R¹ has the same meaning as provided for formula (Ia) above) in the presence of a palladium catalyst such as palladium acetate and a phosphine ligand such as tricyclohexylphosphine or tri-*tert*-butylphosphine. The reaction is typically carried out under inert atmosphere such as dinitrogen or argon, in the presence of a base such as an inorganic base (e.g. cesium carbonate or potassium carbonate or sodium phosphate), in a suitable solvent such as N,N-dimethylformamide, dimethylsulfoxide or N,N-dimethylacetamide and at elevated temperatures (e.g. 100°C or 120°C). 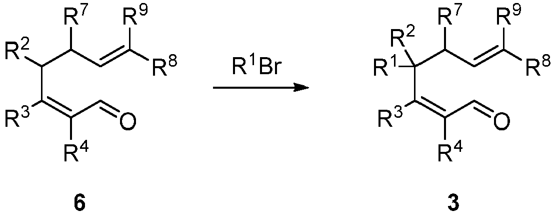 R² - R⁴, and R⁷ - R⁹ have the same meaning as provided for formula (Ia).

The invention is now further described with reference to the following non-limiting examples. These examples are for the purpose of illustration only and it is understood that variations and modifications can be made by one skilled in the art.

### EXAMPLES

### Example 1: (E)-2,4,7-trimethylocta-2,6-dienal (General Procedure)

A mixture of (*E*)-2-methylpent-2-enal(12.27 g, 125 mmol), 3-methylbut-2-en-1-ol(21.53 g, 250 mmol) and 1.5g Et₃N-HCl in xylene was refluxed for 20 hours. During this period, water was collected and removed by using a Dean-Stark apparatus, and the reaction was monitored by GC. After cooling to room temperature, the reaction mixture was diluted with MTBE (methyl ether tert-butyl ether), and washed with water and brine, dried with MgSO₄, filtered, then evaporated under reduced pressure to give crude product, which was purified by distillation to afford (*E*)-2,4,7-trimethylocta-2,6-dienal (14.20 g, yield: 68%).

### Example 2: (E)-4-methyl-3-(2,4,7-trimethyl-1-oxoocta-2,6-dien-4-yl)benzonitrile

### (General Procedure)

A mixture of 3-bromo-4-methylbenzonitrile (25.80 g, 132.00 mmol), (*E*)-2,4,7-trimethylocta-2,6-dienal (23.51 g, 132.00 mmol), diacetoxypalladium (1.48 g, 6.58 mmol), tri-*tert*-butylphosphane (2.66 g, 13.15 mmol), Cs₂CO₃ (42.80 g, 132.00 mmol) in DMF (N,N-dimethylformamide; 120 mL) was heated to 110-120 °C underAr atmosphere overnight, and the reaction was monitored by TLC, GC and GC-MS. After cooling to room temperature, the reaction mixture was diluted with MTBE, and filtered through a small pad of silica gel and the silica gel was washed with MTBE. The combined filtrates were concentrated in vacuo to give crude product, which was purified by distillation or flash chromatography (hexane/ MTBE = 50 : 1- 10 : 1) to afford aldehyde (E)-4-methyl-3-(2,4,7-trimethyl-1-oxoocta-2,6-dien-4-yl)benzonitrile (15.27 g, 52.60 mmol, 40 % yield) as light yellow oil.

GC/MS (El): *m*/*z(%):* 281 (1) [*M⁺*], 213 (100), 198 (44), 184 (24),170 (17), 154 (13), 140 (11), 127 (8), 115 (8), 69 (83). ¹H NMR (300 MHz, CDCI₃) δ 9.43 (s, 1H), 7.64 (s, 1H), 7.48 - 7.36 (m, 1H), 7.22 (d, *J* = 7.9 Hz, 1H), 6.80 (s, 1H), 4.89 (t, *J* = 7.4 Hz, 1H), 2.60 (d, *J* = 10.8 Hz, 2H), 2.30 (s, 3H), 1.67 (s, 3H), 1.54 (d, *J* = 7.2 Hz, 6H), 1.16 (s, 3H). ¹³C NMR (75 MHz, CDCI₃) δ 195.6 (t), 160.8 (t), 145.5 (q), 142.0 (q), 138.5 (q), 136.1 (q), 132.9 (t), 130.4 (t), 130.2 (t), 119.4 (q), 118.5 (t), 110.0 (q), 44.8 (q), 39.8 (d), 26.1 (s), 24.8 (s), 23.3 (s), 18.1 (s), 9.8 (s).

### Example 3: (E)-2,4,7-trimethyl-4-(o-tolyl)octa-2,6-dienal

Following the general procedure described in Example 2: 1-bromo-2-methylbenzene (37.60 g, 220.00 mmol), (*E*)-2,4,7-trimethylocta-2,6-dienal (18.26 g, 110.00 mmol), diacetoxypalladium (1.23 g, 5.49 mmol), tricyclohexylphosphane (3.08 g, 10.98 mmol), Cs₂CO₃ (42.90 g, 132.00 mmol) in DMF (150 mL) were reacted to give the title product (22.56 g, 80 % yield) as a light yellow oil.

GC/MS (El): *m*/*z* (%): 256 (1) [*M⁺*], 188 (93), 173 (100), 159(68), 128 (34), 115 (24), 105 (14), 91 (17), 77 (8), 69 (33). ¹H NMR (300 MHz, CDCI₃) δ 9.45 (s, 1H), 7.38 (d, *J* = 9.6, 8.0 Hz, 1H), 7.26 - 7.05 (m, 3H), 6.88 (s, 1H), 5.01 (t, 1H), 2.67 - 2.50 (m, 2H), 2.23 (s, 3H), 1.83 - 1.71 (m, 1H), 1.70 - 1.51 (m, 9H), 1.20 (s, 3H). ¹³C NMR (75 MHz, CDCI₃) δ 196.1 (t), 162.8 (t), 143.9 (q), 138.1 (q), 135.8 (q), 134.9 (q), 132.0 (t), 126.6 (t), 126.3 (t), 126.0 (t), 119.5 (t), 44.8 (q), 40.0 (d), 26.1 (s), 24.9 (s), 22.9 (s), 18.0 (s), 9.5 (s).

### Example 4: (E)-4-(4-fluoro-2-methylphenyl)-2,4,7-trimethylocta-2,6-dienal

Following the general procedure described in Example 2: 1-bromo-4-fluoro-2-methylbenzene (6.82 g, 36.10 mmol), (*E*)-2,4,7-trimethylocta-2,6-dienal (3.00g, 18.04 mmol), diacetoxypalladium (0.20 g, 0.90 mmol), tricyclohexylphosphane(0.51 g, 1.80 mmol), Cs₂CO₃ (7.05 g, 21.65 mmol) in DMF (20 mL) were reacted to give the title product (0.90 g, 18 % yield) as a yellow oil.

GC/MS (El): *m*/*z* (%): 274 (1) [*M⁺*], 206 (100), 191 (61), 177(72),146 (31), 133 (19), 123 (20), 109 (14), 77 (4),69 (53). ¹H NMR (300 MHz, CDCl₃) δ 9.41 (s, 1H), 7.34 - 7.27 (m, 1H), 6.86 - 6.80 (m, 2H), 4.95 (t, *J* = 7.4 Hz, 1H), 2.64 - 2.48 (m, 2H), 2.20 (s, 3H), 1.96 (d, 1H), 1.67 (s, 3H), 1.57 (s, 3H), 1.50 (s, 3H), 1.20 (s, 3H). ¹³C NMR (75 MHz, CDCI₃) δ 196.0 (t), 163.0 (q), 162.5 (t), 159.7 (q), 139.7 (q), 139.7 (q), 138.3 (q), 138.3 (q), 135.3 (q), 128.0 (t), 127.9 (t), 119.3 (t), 118.6 (t), 118.3 (t), 112.6 (t), 112.3 (t), 44.4 (q), 40.2 (d), 26.1 (s), 25.2 (s), 22.9 (s), 18.1 (s), 9.6 (s).

### Example 5: (E)-4-(5-chloro-2-methylphenyl)-2,4,7-trimethylocta-2,6-dienal

Following the general procedure described in Example 2: 2-bromo-4-chloro-1-methylbenzene (5.30 g, 25.80 mmol), (*E*)-2,4,7-trimethylocta-2,6-dienal (2.15 g, 12.90 mmol), diacetoxypalladium (0.15 g, 0.65 mmol), tri-*tert*-butylphosphane (0.26 g, 1.29 mmol), Cs₂CO₃ (5.04 g, 15.48 mmol) in DMF (20 mL) were reacted to give the title product (1.39 g, 37 % yield) as a light yellow oil.

GC/MS (El): *m*/*z* (%): 290 (1) [*M⁺*], 222 (100), 207 (54), 193 (24), 186 (18), 172 (12), 157 (14), 128 (23), 115 (22), 69 (92). ¹H NMR (300 MHz, CDCl₃) δ 9.42 - 9.37 (m, 1H), 7.32 (d, *J* = 2.0 Hz, 1H), 7.14 - 7.09 (m, 1H), 7.05 - 7.00 (m, 1H), 6.79 (s, 1H), 4.97 (t, *J=* 7.4 Hz, 1H), 2.69 - 2.44 (m, 2H), 2.17 (s, 3H), 1.67 (s, 3H), 1.57 (s, 3H), 1.49 (s, 3H), 1.20 (s, 3H). ¹³C NMR (75 MHz, CDCI₃) δ 195.8 (t), 161.5 (t), 145.9 (q), 138.3 (q), 134.3 (q), 133.2 (q), 131.7 (q), 131.4 (q), 126.6 (t), 126.5 (t), 121.5 (t), 119.0 (t), 44.8 (q), 39.8 (d), 26.1 (s), 24.8 (s), 22.3 (s), 18.1 (s), 9.7 (s).

### Example 6: (E)-3-(2,4,7-trimethyl-1-oxoocta-2,6-dien-4-yl)benzonitrile

Following the general procedure described in Example 2: 3-bromobenzonitrile (6.57 g, 36.10 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.80 mmol), Cs₂CO₃ (5.88 g, 18.04 mmol) in DMF (20 mL) were reacted to give the title product (1.11 g, 23 % yield) as a light yellow oil.

GC/MS (El): *m*/*z* (%): 267 (1) [*M⁺*], 199(87), 184 (39), 170 (13), 154 (14), 140 (9), 127 (11), 116 (10), 103 (4), 69 (100). ¹H NMR (300 MHz, CDCI₃) δ 9.42 (s, 1H), 7.56 - 7.36 (m, 4H), 6.75 (s, 1H), 5.04 - 4.82 (m, 1H), 2.49 (d, *J* = 7.4 Hz, 2H), 1.65 (s, 3H), 1.53 (s, 3H), 1.46 (s, 3H), 1.25 (s, 3H). ¹³C NMR (75 MHz, CDCI₃) δ 195.7 (t), 160.7 (t), 148.0 (q), 140.3 (q), 136.2 (q), 131.5 (t), 130.3 (t), 130.0 (t), 129.2 (t), 119.0 (q), 118.3 (t), 112.4 (q), 44.9 (q), 42.8 (d), 26.0 (s), 24.5 (s), 17.9 (s), 10.7 (s).

### Example 7: (E)-4-(2,5-dimethylphenyl)-2,4,7-trimethylocta-2,6-dienal

Following the general procedure described in Example 2: 2-bromo-1,4-dimethylbenzene (6.68 g, 36.10 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.80 mmol), Cs₂CO₃ (7.05 g, 21.65 mmol) in DMF (20 mL) were reacted to give the title product (2.44 g, 50 % yield) as a light yellow oil.

GC/MS (El): *m*/*z* (%): 270 (1) [*M⁺*], 202 (93), 187 (73), 173 (100), 159 (28), 143 (29), 128 (24), 115 (17), 91 (13), 69 (26). ¹H NMR (300 MHz, CDCl₃) δ 9.41 (s, 1H), 7.17 (s, 1H), 7.06 - 6.93 (m, 2H), 6.85 (s, 1H), 5.04 (t, *J* = 7.4 Hz, 1H), 2.69 - 2.45 (m, 2H), 2.33 (s, 3H), 2.15 (s, 3H), 1.72 (s, 3H), 1.60 (s, 3H), 1.52 (s, 3H), 1.21 (s, 3H). ¹³C NMR (75 MHz, CDCI₃) δ 196.2 (t), 163.0 (t), 143.9 (q), 138.1 (q), 135.2 (q), 134.9 (q), 132.6 (q), 132.0 (t), 127.3 (t), 127.0 (t), 119.7 (t), 44.7 (q), 40.1 (d), 26.1 (s), 25.0 (s), 22.5 (s), 21.4 (s), 18.1 (s), 9.6 (s).

### Example 8: (E)-4-(5-fluoro-2-methylphenyl)-2,4,7-trimethylocta-2,6-dienal

Following the general procedure described in Example 2: 2-bromo-4-fluoro-1-methylbenzene (6.34 g, 33.60 mmol), (*E*)-2,4,7-trimethylocta-2,6-dienal (3.00 g, 16.78 mmol), diacetoxypalladium (0.19 g, 0.84 mmol), tri-*tert*-butylphosphane (0.34 g, 1.68 mmol), Cs₂CO₃ (5.47 g, 16.78 mmol) in DMF (20 mL) were reacted to give the title product (1.00 g, 22 % yield) as a light yellow oil.

GC/MS (El): *m*/*z* (%): 274 (1) [*M⁺*], 206 (100), 191 (69), 177(29), 159(12), 146 (25), 133 (15), 123 (12), 109 (10), 69 (69). ¹H NMR (300 MHz, CDCI₃) δ 9.43 (s, 1H), 7.16 - 6.98 (m, 2H), 6.92 - 6.74 (m, 2H), 5.13 - 4.86 (m, 1H), 2.70 - 2.48 (m, 2H), 2.18 (s, 3H), 1.67 (s, 3H), 1.58 (s, 3H), 1.50 (s, 3H), 1.21 (s, 3H). ¹³C NMR (75 MHz, CDCI₃) δ 195.9 (t), 163.0 (t), 161.8 (t), 159.8 (t), 146.2 (q), 146.1 (q), 138.4 (q), 135.5 (q), 133.3 (t), 133.2 (t), 131.4 (q), 131.4 (q), 119.1 (t), 113.8 (t), 113.5 (t), 113.3 (t), 113.0 (t), 44.8 (q), 39.8 (d), 26.1 (s), 24.9 (s), 22.1 (s), 18.1 (s), 9.6 (s).

### Example 9: (E)-4-(2-methoxyphenyl)-2,4,7-trimethylocta-2,6-dienal

Following the general procedure described in Example 2: 1-bromo-2-methoxybenzene (6.75 g, 36.1 mmol), (*E*)-2,4,7-trimethylocta-2,6-dienal (3.00 g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMF (20 mL) were reacted to give the title product (2.50 g, 51 % yield) as a light yellow oil.

GC/MS (El): *m*/*z* (%): 272 (2) [*M⁺*], 203 (100), 188 (50), 175 (40), 161(17), 145 (12), 128 (14), 115 (15), 91 (16), 69 (9). ¹H NMR (300 MHz, CDCI₃) δ 9.27 (s, 1H), 7.20-7.15 (m, 1H), 7.13 - 7.05 (m, 1H), 6.83 (t, *J=* 7.5 Hz, 1H), 6.76 (d, *J=* 0.7 Hz, 1H), 6.70 (d, *J* = 8.1 Hz, 1H), 4.89 (t, *J=* 7.4 Hz, 1H), 3.55 (s, 3H), 2.55 (d, *J=* 7.6 Hz, 2H), 1.55 (s, 3H), 1.46 (s, 3H), 1.38 (s, 3H), 1.13 (s, 3H). ¹³C NMR (75 MHz, CDCI₃) δ 196.3 (t), 165.1 (t), 157.1 (q), 136.2 (q), 134.4 (q), 127.9 (t), 126.8 (t), 120.6 (t), 120.1 (t), 111.5 (t), 55.1 (s), 43.0 (q), 39.1 (d), 26.0 (s), 23.4 (s), 17.9 (s), 9.3 (s).

### Example 10: (E)-2,4,7-trimethyl-4-(p-tolyl)octa-2,6-dienal

Following the general procedure described in Example 2: 1-bromo-4-methylbenzene (6.09 g, 35.6 mmol), (*E*)-2,4,7-trimethylocta-2,6-dienal (2.96 g, 17.82 mmol), diacetoxypalladium (0.20 g, 0.89 mmol), triphenylphosphine (0.47 g, 1.78 mmol), Cs₂CO₃ (5.81 g, 17.82 mmol) in DMF (20 mL) were reacted to give the title product (1.75 g, 38 % yield) as a colorless oil.

GC/MS (El): *m*/*z* (%): 256 (1) [*M⁺*], 188 (70), 187 (70), 172 (80), 159 (100), 128 (30), 115 (18), 105 (14), 91 (13), 69 (10). ¹H NMR (300 MHz, CDCI₃) δ 9.40 (s, 1H), 7.17 - 7.07 (m, 4H), 6.79 (d, *J=* 5.7 Hz, 1H), 5.02 (t, *J=* 7.3 Hz, 1H), 2.64 - 2.43 (m, 2H), 2.31 (s, 3H), 1.68 (s, 3H), 1.56 (s, 3H), 1.51 (s, 3H), 1.33 (s, 3H). ¹³C NMR (75 MHz, CDCI₃) δ 196.4 (t), 163.3 (t), 143.6 (q), 139.9 (q), 135.7 (q), 135.0 (q), 129.1 (t), 126.5 (t), 119.6 (t), 44.6 (q), 42.7 (d), 26.1 (s), 25.0 (s), 21.0 (s), 18.1 (s), 10.5 (s).

### Example 11: (E)-4-(2,4-dimethylphenyl)-2,4,7-trimethylocta-2,6-dienal

Following the general procedure described in Example 2: 1-bromo-2,4-dimethylbenzene (4.78 g, 25.80 mmol), (*E*)-2,4,7-trimethylocta-2,6-dienal (2.15 g, 12.90 mmol), diacetoxypalladium (0.15 g, 0.65 mmol), tricyclohexylphosphane (0.36 g, 1.29 mmol), Cs₂CO₃ (4.20 g, 12.90 mmol) in DMF (35 mL) were reacted to give the title product (2.40 g, 69 % yield) as a light yellow oil.

GC/MS (El): *m*/*z* (%): 270 (2) [*M⁺*], 202 (53), 186 (71), 173 (29), 159 (21), 143 (23), 128 (19), 115 (14), 91 (10), 69 (12). ¹H NMR (300 MHz, CDCI₃) δ 9.42 (s, 1H), 7.23 (d, *J=* 8.0 Hz, 1H), 6.97 (d, *J=* 8.0 Hz, 1H), 6.91 (s, 1H), 6.83 (s, 1H), 5.01 (t, *J* = 7.4 Hz, 1H), 2.57 (qd, *J=* 14.5, 7.7 Hz, 2H), 2.26 (s, 3H), 2.18 (s, 3H), 1.67 (s, 3H), 1.58 (s, 3H), 1.49 (s, 3H), 1.18 (s, 3H). ¹³C NMR (75 MHz, CDCI₃) δ 195.9 (t), 162.9 (t), 140.9 (q), 138.1 (q), 135.9 (q), 135.5 (q), 134.7 (q), 132.8 (t), 126.6 (t), 126.2 (t), 119.7 (t), 44.4 (q), 40.2 (d), 26.0 (s), 25.0 (s), 22.7 (s), 20.7 (s), 18.0 (s), 9.5 (s).

### Example 12: (E)-4-(3-chloro-2-methylphenyl)-2,4,7-trimethylocta-2,6-dienal

Following the general procedure described in Example 2: 1-bromo-3-chloro-2-methylbenzene (5.30 g, 25.8 mmol), (E)-2,4,7-trimethylocta-2,6-dienal (2.15 g, 12.90 mmol), diacetoxypalladium (0.145 g, 0.645 mmol), tri-tert-butylphosphane (0.26 g, 1.29 mmol), Cs₂CO₃ (5.04 g, 15.48 mmol) in DMF (20 mL) were reacted to give the title product (1.51 g, 40 % yield) as a light yellow oil.

GC/MS (El): *m*/*z* (%): 290 (1) [*M⁺*], 222 (100), 207 (61), 195 (34), 186 (36), 157 (14), 141 (27), 128 (27), 115 (24), 69 (94). ¹H NMR (300 MHz, CDCI₃) δ 9.42 (s, 1H), 7.29 (d, *J* = 7.9 Hz, 2H), 7.18 - 7.10 (m, 1H), 6.86 (s, 1H), 4.97 (t, *J* = 8.0, 6.8 Hz, 1H), 2.65 - 2.50 (m, 2H), 2.23 (s, 3H), 1.67 (s, 3H), 1.57 (s, 3H), 1.51 (s, 3H), 1.17 (s, 3H). ¹³C NMR (75 MHz, CDCI₃) δ 195.8 (t), 162.1 (t), 146.3 (q), 137.8 (q), 136.4 (q), 135.3 (q), 133.9 (q), 127.8 (t), 126.6 (t), 124.9 (t), 119.0 (t), 45.2 (q), 40.2 (d), 26.0 (s), 25.2 (s), 20.3 (s), 18.0 (s), 9.5 (s).

### Example 13: (E)-4-(2-fluorophenyl)-2,4,7-trimethylocta-2,6-dienal

Following the general procedure described in Example 2: 1-bromo-2-fluorobenzene (6.32 g, 36.10 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.80 mmol), Cs₂CO₃ (7.05 g, 21.65 mmol) in DMF (20 mL) were reacted to give the title product (1.4 g, 30 % yield) as a light yellow oil.

GC/MS (El): *m*/*z* (%): 260 (1) [*M⁺*], 192 (100), 177 (99), 163 (31), 146 (21), 133 (14), 123 (14), 109 (30), 77(5), 69 (59). ¹H NMR (300 MHz, CDCI₃) δ 9.39 (s, 1H), 7.37 - 7.30 (m, 1H), 7.28 - 7.19 (m, 1H), 7.17 - 7.09 (m, 1H), 7.08 - 6.92 (m, 2H), 6.83 (s, 1H), 5.03 - 4.94 (m, 1H), 2.63 (d, *J=* 7.4 Hz, 2H), 1.67 (s, 3H), 1.57 (s, 3H), 1.53 (s, 3H), 1.28 (s, 3H). ¹³C NMR (75 MHz, CDCI₃) δ 196.2 (t), 162.2 (t), 138.1 (q), 138.1 (q), 135.3 (q), 133.2 (q), 133.1 (q), 128.5 (t), 128.4 (t), 127.6 (t), 127.6 (t), 123.9 (t), 123.9 (t), 119.0 (t), 116.1 (t), 115.8 (t), 42.5 (d), 39.8 (d), 39.8 (d), 26.0 (s), 23.4 (s), 17.9 (s), 9.5 (s).

### Example 14: (E)-4-(2,4,7-trimethyl-1-oxoocta-2,6-dien-4-yl)benzonitrile

Following the general procedure described in Example 2: 4-bromobenzonitrile (6.57 g, 36.10 mmol), (E*)*-2,4,7-trimethylocta-2,6-dienal (3.00 g, 18.04 mmol), diacetoxypalladium (0.20 g, 0.90 mmol), tri-*tert*-butylphosphane (0.37 g, 1.804 mmol), Cs₂CO₃ (5.88 g, 18.04 mmol) in DMF (20 mL) were reacted to give the title product (1.00 g, 21 % yield) as a light yellow oil.

GC/MS (El): *m*/*z* (%): 267 (1) [*M⁺*], 199 (86), 184 (38), 170 (11), 154 (13), 140 (9), 127 (11), 116 (10), 103 (4),69 (100). ¹H NMR (300 MHz, CDCI₃) δ 9.39 (s, 1H), 7.58 (d, *J* = 12.8 Hz, 2H), 7.32 (d, *J* = 8.2 Hz, 2H), 6.72 (s, 1H), 4.90 (t, *J* = 7.2 Hz, 1H), 2.46 (d, *J* = 7.4 Hz, 2H), 1.61 (s, 3H), 1.50 (s, 3H), 1.44 (s, 3H), 1.22 (s, 3H). ¹³C NMR (75 MHz, CDCI₃) δ 195.6 (t), 160.7 (t), 152.0 (q), 140.3 (q), 136.1 (q), 132.1 (t), 127.5 (t), 118.7 (q), 118.3 (t), 110.1 (q), 45.2 (q), 42.7 (d), 25.9 (s), 24.4 (s), 17.9 (s), 10.7 (s).

### Example 15: (E)-4-(2,5-dimethoxyphenyl)-2,4,7-trimethylocta-2,6-dienal

Following the general procedure described in Example 2: 2-bromo-1,4-dimethoxybenzene (7.83 g, 36.10 mmol), (*E*)-2,4,7-trimethylocta-2,6-dienal (3.00g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMF (20 mL) were reacted to give the title product (3.25 g, 60 % yield) as a yellow oil.

GC/MS (El): *m*/*z* (%): 274 (1) [*M⁺*], 206 (100), 191 (69), 177 (29), 159 (12), 146 (25), 133 (15), 123 (12), 109 (10), 69 (69). ¹H NMR (300 MHz, CDCI₃) δ 9.37 (s, 1H), 6.89 (s, 1H), 6.83 (s, 1H), 6.77 - 6.69 (m, 2H), 5.01 (t, *J* = 15.8, 8.4 Hz, 1H), 3.77 (s, 3H), 3.63 (s, 3H), 2.62 (d, *J* = 7.5 Hz, 2H), 1.68 (s, 3H), 1.58 (s, 3H), 1.45 (s, 3H), 1.26 (s, 3H). ¹³C NMR (75 MHz, CDCI₃) δ 196.6 (t), 165.1 (t), 153.6 (q), 151.6 (q), 136.4 (q), 136.2 (q), 134.7 (q), 120.0 (t), 114.6 (t), 112.4 (t), 110.8 (t), 55.8 (s), 55.7 (s), 43.1 (q), 39.2 (d), 26.1 (s), 23.4 (s), 18.0 (s), 9.5 (s).

### Example 16: (E)-2-(2,4,7-trimethyl-1-oxoocta-2,6-dien-4-yl)benzonitrile

Following the general procedure described in Example 2: 2-bromobenzonitrile (4.70 g, 25.80 mmol), (*E*)-2,4,7-trimethylocta-2,6-dienal (2.15 g, 12.90 mmol), diacetoxypalladium (0.15 g, 0.65 mmol), tri-*tert*-butylphosphane (0.26 g, 1.29 mmol), Cs₂CO₃ (5.04 g, 15.48 mmol) in DMF (20 mL) were reacted to give the title product (1.26 g, 37 % yield) as a light yellow oil.

GC/MS (El): *m*/*z* (%): 267 (1) [*M⁺*], 199 (43), 170 (100), 156 (23), 143 (7), 128 (14), 116 (9), 103 (4), 89 (3), 69 (58). ¹H NMR (300 MHz, CDCI₃) δ 9.48 (s, 1H), 7.67 - 7.48 (m, 3H), 7.38 - 7.29 (m, 1H), 6.95 (d, *J* = 1.2 Hz, 1H), 4.94 (t, *J* = 7.5 Hz, 1H), 2.73 (d, *J* = 11.2 Hz, 2H), 1.66 (s, 3H), 1.58 (d, *J* = 3.9 Hz, 6H), 1.17 (d, *J* = 1.1 Hz, 3H). ¹³C NMR (75 MHz, CDCI₃) δ 196.0 (t), 160.7 (t), 150.7 (q), 139.0 (q), 136.3 (q), 135.2 (t), 132.7 (t), 127.1 (t), 127.0 (t), 119.4 (q), 118.3 (t), 111.7 (q), 44.7 (q), 40.8 (d), 26.1 (s), 23.8 (s), 18.1 (s), 9.9 (s).

### Example 17: (E)-4-methyl-2-(2,4,7-trimethyl-1-oxoocta-2,6-dien-4-yl)benzonitrile

Following the general procedure described in Example 2: 2-bromo-4-methylbenzonitrile (3.28 g, 16.74 mmol), (*E*)-2,4,7-trimethylocta-2,6-dienal (1.56 g, 8.37 mmol), diacetoxypalladium (0.09 g, 0.42 mmol), tricyclohexylphosphane (0.24 g, 0.84 mmol), Cs₂CO₃ (3.27 g, 10.05 mmol) in DMF (20 mL) were reacted to give the title product (0.97 g, 41 % yield) as a light yellow oil.

GC/MS (El): *m*/*z(%):* 281 (1) [*M⁺*], 253 (2), 213 (30), 184 (100), 170 (30), 153 (7), 142 (9), 128 (6), 115 (8), 69 (46). ¹H NMR (300 MHz, CDCI₃) δ 9.46 (s, 1H), 7.50 (d, *J* = 7.8 Hz, 1H), 7.29 (s, 1H), 7.12 (d, *J* = 7.8 Hz, 1H), 6.92 (d, *J* = 1.1 Hz, 1H), 4.95 (t, *J* = 7.5 Hz, 1H), 2.71 (d, *J* = 7.5 Hz, 2H), 2.42 (s, 3H), 1.66 (s, 3H), 1.56 (d, *J* = 5.9 Hz, 6H), 1.18 (d, *J* = 1.0 Hz, 3H). ¹³C NMR (75 MHz, CDCI₃) δ 196.0 (t), 160.8 (t), 150.6 (q), 143.5 (q), 138.8 (q), 136.1 (q), 135.0 (t), 127.8 (t), 127.7 (t), 119.7 (q), 118.4 (t), 108.6 (q), 44.5 (q), 40.8 (d), 26.1 (s), 23.8 (s), 22.1 (s), 18.1 (s), 9.9 (s).

### Example 18: (E)-4-(3-methoxyphenyl)-2,4,7-trimethylocta-2,6-dienal

Following the general procedure described in Example 2: 1-bromo-3-methoxybenzene (6.75 g, 36.10 mmol), (*E*)-2,4,7-trimethylocta-2,6-dienal (3.00 g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMF (20 mL) were reacted to give the title product (1.20 g, 24 % yield) as a light yellow oil.

GC/MS (El): *m*/*z(%):* 272 (2) [*M⁺*], 204 (82), 188 (100), 175 (72), 161 (18), 145 (21), 128 (20), 115 (21), 91 (20), 69 (22). ¹H NMR (300 MHz, CDCI₃) δ 9.42 (s, 1H), 7.25 - 7.18 (m, 1H), 6.87 - 6.79 (m, 2H), 6.78 - 6.71 (m, 2H), 5.00 (t, 1H), 3.79 (s, 3H), 2.61 - 2.44 (m, 2H), 1.66 (s, 3H), 1.55 (s, 3H), 1.50 (s, 3H), 1.32 (s, 3H). ¹³C NMR (75 MHz, CDCI₃) δ 196.3 (t), 162.8 (t), 159.6 (q), 148.3 (q), 140.0 (q), 135.1 (q), 129.3 (t), 119.5 (t), 119.3 (t), 113.3 (t), 110.7 (t), 55.2 (s), 44.9 (q), 42.6 (d), 26.1 (s), 24.9 (s), 18.1 (s), 10.4 (s).

### Example 19: (E)-2,4,7-trimethyl-4-(naphthalen-2-yl)octa-2,6-dienal

Following the general procedure described in Example 2: 2-bromonaphthalene (6.23 g, 30.10 mmol), (*E*)-2,4,7-trimethylocta-2,6-dienal (2.50 g, 15.04 mmol), diacetoxypalladium (0.17 g, 0.752 mmol), tri-*tert*-butylphosphine (0.30 g, 1.50 mmol), Cs₂CO₃ (4.90 g, 15.04 mmol) in DMF (20 mL) were reacted to give the title product (1.00 g, 23 % yield) as yellow oil.

GC/MS (El): *m*/*z* (%): 292 (1) [*M⁺*], 224 (100), 208 (69), 195 (29), 179 (12), 165 (25), 153 (15), 141 (12), 128 (10), 69 (69). ¹H NMR (300 MHz, CDCI₃) δ 9.38 (s, 1H), 7.76-7.69 (m, 3H), 7.63 (s, 1H), 7.41 - 7.35 (m, 2H), 7.31 - 7.24 (m, 1H), 6.77 (d, *J* = 1.1 Hz, 1H), 4.94 (t, *J* = 7.4 Hz, 1H), 2.63 - 2.45 (m, 2H), 1.56 (d, *J* = 9.8 Hz, 6H), 1.47 (s, 3H), 1.20 (s, 3H). ¹³C NMR (75 MHz, CDCI₃) δ 196.4 (t), 162.7 (t), 144.0 (q), 140.2 (q), 135.2 (q), 133.3 (q), 132.0 (q), 128.1 (t), 128.0 (t), 127.5 (t), 126.1 (t), 125.9 (t), 125.8 (t), 124.6 (t), 119.4 (t), 45.1 (q), 42.6 (d), 26.1 (s), 24.9 (s), 18.1 (s), 10.5 (s).

### Example 20: (E)-4-(2-ethylphenyl)-2,4,7-trimethylocta-2,6-dienal

Following the general procedure described in Example 2: 1-bromo-2-ethylbenzene (5.00 g, 27.00 mmol), (*E*)-2,4,7-trimethylocta-2,6-dienal (2.25 g, 13.51 mmol), diacetoxypalladium (0.15 g, 0.68 mmol), tri-*tert*-butylphosphane (0.27 g, 1.35 mmol), Cs₂CO₃ (5.28 g, 16.21 mmol) in DMF (20 mL) were reacted to give the title product (1.52 g, 42 % yield) as a light yellow oil. GC/MS (El): *m*/*z* (%): 270(1) [*M⁺*], 202 (66), 187 (16), 173 (100), 145 (27), 128 (30), 115 (19), 91 (14), 69 (29). ¹H NMR (300 MHz, CDCI₃) δ 9.41 (s, 1H), 7.36 (d, *J=* 7.3 Hz, 1H), 7.24 - 7.14 (m, 3H), 6.95 (d, *J=* 1.2 Hz, 1H), 5.02 (t, *J* = 6.7, 1.3 Hz, 1H), 2.71 -2.43 (m, 4H), 1.70 (s, 3H), 1.59 (s, 3H), 1.52 (s, 3H), 1.21 (s, 3H), 1.09 (t, *J=* 7.6 Hz, 3H). ¹³C NMR (75 MHz, CDCI₃) δ 196.1 (t), 163.4 (t), 143.8 (q), 142.0 (q), 137.8 (q), 135.0 (q), 130.1 (t), 126.8 (t), 126.0 (t), 125.8 (t), 119.6 (t), 44.5 (q), 41.1 (d), 27.6 (d), 26.1 (s), 25.1 (s), 18.1 (s), 15.5 (s), 9.7 (s).

### Example 21: (E)-2,4,7-trimethyl-4-(m-tolyl)octa-2,6-dienal

Following the general procedure described in Example 2: 1-bromo-3-methylbenzene (4.41 g, 25.80 mmol), (*E*)-2,4,7-trimethylocta-2,6-dienal (2.15 g, 12.90 mmol), diacetoxypalladium (0.15 g, 0.65 mmol), tricyclohexylphosphine (0.36 g, 1.29 mmol), Cs₂CO₃ (4.20 g, 12.90 mmol) in DMF (15 mL) were reacted to give the title product (2.04 g, 62 % yield) as a light yellow oil.

GC/MS (El): *m*/*z* (%): 256(1) [*M⁺*], 188(100), 173 (84), 159 (89), 143 (20), 128 (34), 115 (21), 91 (17), 69 (22). ¹H NMR (300 MHz, CDCI₃) δ 9.43 (s, 1H), 7.23 - 7.13 (m, 1H), 7.10 - 6.98 (m, 3H), 6.79 (d, *J* = 1.1 Hz, 1H), 5.05 (t, 1H), 2.61 - 2.46 (m, 2H), 2.34 (s, 3H), 1.69 (s, 3H), 1.54 (d, *J* = 12.8 Hz, 6H), 1.34 (s, 3H). ¹³C NMR (75 MHz, CDCI₃) δ 196.3 (t), 163.2 (t), 146.5 (q), 139.8 (q), 137.7 (q), 134.9 (q), 128.1 (t), 127.3 (t), 126.9 (t), 123.6 (t), 119.6 (t), 44.8 (q), 42.6 (d), 26.0 (s), 24.9 (s), 21.7 (s), 18.0 (s), 10.4 (s).

### Example 22: (E)-4-(benzo[d][1,31dioxol-5-yl)-2,4,7-trimethylocta-2,6-dienal

Following the general procedure described in Example 2: 5-bromobenzo[*d*][1,3]dioxole (3.89 g, 19.35 mmol), (E)-2,4,7-trimethylocta-2,6-dienal (2.15 g, 12.90 mmol), diacetoxypalladium (0.15 g, 0.65 mmol), tricyclohexylphosphane (0.36 g, 1.29 mmol), Cs₂CO₃ (5.04 g, 15.48 mmol) in DMF (20 mL) were reacted to give the title product (1.26 g, 34 % yield) as a light yellow oil.

GC/MS (El): *m*/*z* (%): 286 (3) [*M⁺*], 217 (29), 202 (33), 189 (100), 159 (45), 115 (29), 91 (19), 69 (11). ¹H NMR (300 MHz, CDCI₃) δ 9.41 (s, 1H), 6.78 - 6.70 (m, 4H), 5.94 (s, 2H), 5.00 (t, *J* = 7.3 Hz, 1H), 2.55 - 2.41 (m, 2H), 1.68 (s, 3H), 1.56 (s, 3H), 1.47 (s, 3H), 1.36 (s, 3H). ¹³C NMR (75 MHz, CDCI₃) δ 196.3 (t), 163.0 (t), 147.8 (q), 145.8 (q), 140.6 (q), 139.9 (q), 135.1 (q), 119.6 (t), 119.4 (t), 107.9 (t), 107.5 (t), 101.0 (d), 44.7 (q), 42.9 (d), 26.1 (s), 25.0 (s), 18.1 (s), 10.4 (s).

### Example 23: (E)-4-(6-methoxypyridin-2-yl)-2,4,7-trimethylocta-2,6-dienal

Following the general procedure described in Example 2: 2-bromo-6-methoxypyridine (5.11 g, 27.20 mmol), (*E*)-2,4,7-trimethylocta-2,6-dienal (2.26 g, 13.59 mmol), diacetoxypalladium (0.15 g, 0.68 mmol), tri-*tert*-butylphosphane (0.28 g, 1.36 mmol), Cs₂CO₃ (5.32 g, 16.31 mmol) in DMF (20 mL) were reacted to give the title product (2.12 g, 57 % yield) as a light yellow oil.

GC/MS (El): *m*/*z* (%): 273 (2) [*M⁺*], 244 (16), 188 (100), 176 (49), 162 (53), 146 (27), 137 (28), 69 (39). ¹H NMR (300 MHz, CDCI₃) δ 9.39 (s, 1H), 7.45 (q, *J=* 7.7 Hz, 1H), 6.83 - 6.67 (m, 2H), 6.53 (d, *J=* 9.3 Hz, 1H), 5.01 (t, *J=* 7.4 Hz, 1H), 3.88 (s, 3H), 2.67 - 2.54 (m, 2H), 1.65 (s, 3H), 1.53 (s, 6H), 1.34 (s, 3H). ¹³C NMR (75 MHz, CDCI₃) δ 196.4 (t), 163.1 (q), 162.8 (q), 162.5 (t), 139.4 (q), 138.6 (t), 134.8 (q), 119.6 (t), 113.8 (t), 107.9 (t), 53.1 (s), 47.5 (q), 41.0 (d), 26.0 (s), 24.1 (s), 18.0 (s), 10.2 (s).

### Example 24: (E)-4-(2,4-dimethoxyphenyl)-2,4,7-trimethylocta-2,6-dienal

Following the general procedure described in Example 2: 1-bromo-2,4-dimethoxybenzene (7.83 g, 36.10 mmol), (*E*)-2,4,7-trimethylocta-2,6-dienal (3.00g, 18.04 mmol), diacetoxypalladium (0.20 g, 0.90 mmol), tri-*tert*-butylphosphine (0.37 g, 1.80 mmol), Cs₂CO₃ (7.05 g, 21.65 mmol) in DMF (20 mL) were reacted to give the title product (3.40 g, 62 % yield) as a light yellow oil.

GC/MS (El): *m*/*z* (%): 302 (1) [*M⁺*], 233 (100), 218 (34), 205 (97), 191 (26), 175 (18), 115 (9), 91 (8). ¹H NMR (300 MHz, CDCI₃) δ 9.43 (d, *J* = 52.8 Hz, 1H), 7.15 (d, *J* = 8.5 Hz, 1H), 6.81 (s, 1H), 6.45 (dd, *J* = 8.5, 2.4 Hz, 1H), 6.41 - 6.37 (m, 1H), 4.97 (t, *J* = 6.1 Hz, 1H), 3.75 (d, *J* = 5.2 Hz, 3H), 3.65 (d, *J* = 7.6 Hz, 3H), 2.65 - 2.55 (m, 2H), 1.64 (s, 3H), 1.54 (s, 3H), 1.43 (s, 3H), 1.24 (s, 3H). ¹³C NMR (75 MHz, CDCI₃) δ 196.5 (t), 165.5 (t), 159.6 (q), 158.0 (q), 136.2 (q), 134.2 (q), 127.2 (t), 126.8 (q), 120.1 (t), 103.8 (t), 99.3 (t), 55.1 (s), 55.1 (s), 42.5 (q), 39.2 (d), 25.9 (s), 23.5 (s), 17.9 (s), 9.2 (s).

### Example 25: (E)-4-(3,5-dimethylphenyl)-2,4,7-trimethylocta-2,6-dienal

Following the general procedure described in Example 2: 1-bromo-3,5-dimethylbenzene (4.78 g, 25.8 mmol), (*E*)-2,4,7-trimethylocta-2,6-dienal (2.15 g, 12.90 mmol), diacetoxypalladium (0.15 g, 0.65 mmol), tricyclohexylphosphane (0.36 g, 1.29 mmol), Cs₂CO₃ (4.20 g, 12.90 mmol) in DMF (15 mL) were reacted to give the title product (2.15 g, 62 % yield) as colorless oil.

GC/MS (El): *m*/*z* (%): 270 (1) [*M⁺*], 202 (57), 186 (100), 173 (89), 159 (22), 143 (27), 128 (17), 115 (12), 91 (9), 69 (10). ¹H NMR (300 MHz, CDCI₃) δ 9.45 (s, 1H), 6.88 (s, 3H), 6.78 (d, *J* = 1.0 Hz, 1H), 5.06 (t, *J* = 7.3 Hz, 1H), 2.63 - 2.45 (m, 2H), 2.32 (s, 6H), 1.71 (s, 3H), 1.60 (s, 3H), 1.51 (s, 3H), 1.38 (s, 3H). ¹³C NMR (75 MHz, CDCI₃) δ 196.6 (t), 163.4 (t), 146.6 (q), 139.8 (q), 137.6 (q), 134.9 (q), 127.8 (t), 124.4 (t), 119.7 (t), 44.8 (q), 42.6 (d), 26.1 (s), 25.1 (s), 21.6 (s), 21.4 (s), 18.1 (s), 10.5 (s).

### Example 26: (E)-4-(4-methoxyphenyl)-2,4,7-trimethylocta-2,6-dienal

Following the general procedure described in Example 2: 1-bromo-4-methoxybenzene (4.50 g, 24.06 mmol), (*E*)-2,4,7-trimethylocta-2,6-dienal (2.00 g, 12.03 mmol), bis(tri-*tert*-butylphosphine)palladium(0) (0.31 g, 0.60 mmol), Cs₂CO₃ (4.70 g, 14.44 mmol) in DMF (20 mL) were reacted to give the title product (2.81 g, 86 % yield) as a light yellow oil.

GC/MS (El): *m*/*z(%):* 272 (2) [*M⁺*], 203 (100), 188 (62), 175 (83), 160 (11), 145 (11), 115 (12), 91 (9). ¹H NMR (300 MHz, CDCI₃) δ 9.40 (s, 1H), 7.19 - 7.12 (m, 2H), 6.83 (d, 2H), 6.77 (s, 1H), 5.00 (t, 1H), 3.79 (s, 3H), 2.58 - 2.39 (m, 2H), 1.67 (s, 3H), 1.52 (d, *J* = 14.9 Hz, 6H), 1.31 (s, 3H). ¹³C NMR (75 MHz, CDCI₃) δ 196.4 (t), 163.5 (t), 157.8 (q), 139.8 (q), 138.6 (q), 135.0 (q), 127.8 (t), 127.7 (t), 119.6 (t), 114.2 (t), 113.6 (t), 55.4 (t), 55.2 (t), 44.3 (q), 42.8 (d), 26.1 (s), 25.0 (s), 18.1 (s), 10.4 (s).

### Example 27: (E)-2,4,7-trimethyl-4-(naphthalen-1-yl)octa-2,6-dienal

Following the general procedure described in Example 2: 1-bromonaphthalene (7.47 g, 36.10 mmol), (*E*)-2,4,7-trimethylocta-2,6-dienal (3.00 g, 18.04 mmol), diacetoxypalladium (0.20 g, 0.902 mmol), tricyclohexylphosphane (0.51 g, 1.80 mmol), Cs₂CO₃ (7.05 g, 21.65 mmol) in DMF (20 mL) were reacted to give the title product (2.1 g, 40 % yield) as a light yellow oil.

GC/MS (El): *m*/*z* (%): 292 (4) [*M⁺*], 224 (59), 209 (27), 195 (100), 179 (46), 165 (58), 153 (24), 69 (13). ¹H NMR (300 MHz, CDCI₃) δ 9.44 (s, 1H), 7.96 - 7.71 (m, 3H), 7.57 - 7.35 (m, 4H), 7.14 (s, 1H), 4.97 (s, 1H), 2.79 (s, 2H), 1.64 (d, *J* = 4.4 Hz, 3H), 1.50 (s, 3H), 1.19 (s, 3H), 1.00 (s, 3H). ¹³C NMR (75 MHz, CDCI₃) δ 195.8 (t), 162.9 (t), 142.3 (q), 138.5 (q), 135.0 (q), 134.6 (q), 130.5 (q), 129.4 (t), 128.0 (t), 126.0 (t), 125.2 (t), 124.9 (t), 124.1 (t), 119.4 (t), 45.0 (q), 40.9 (d), 26.0 (s), 25.5 (s), 18.0 (s), 9.3 (s).

### Example 28: (E)-4-(3,5-dimethoxyphenyl)-2,4,7-trimethylocta-2,6-dienal

Following the general procedure described in Example 2: 1-bromo-3,5-dimethoxybenzene (7.83 g, 36.1 mmol), (*E*)-2,4,7-trimethylocta-2,6-dienal (3.00g, 18.04 mmol), diacetoxypalladium (0.20 g, 0.90 mmol), tricyclohexylphosphane (0.51 g, 1.804 mmol), Cs₂CO₃ (7.05 g, 21.65 mmol) in DMF (20 mL) were reacted to give the title product (2.00 g, 29 % yield) as a yellow oil.

GC/MS (El): *m*/*z* (%): 302 (6) [*M⁺*], 233 (100), 218 (86), 205 (49), 191 (34), 175 (27), 115 (12), 69 (19). ¹H NMR (300 MHz, CDCI₃) δ 9.41 (s, 1H), 6.47 (t, *J* = 2.2 Hz, 1H), 6.42 (d, *J* = 2.1 Hz, 2H), 6.33 (t, *J* = 2.1 Hz, 1H), 5.03 (t, *J* = 7.3 Hz, 1H), 3.77 (s, 6H), 2.50 (t, 2H), 1.69 (s, 3H), 1.57 (s, 3H), 1.49 (s, 3H), 1.39 (s, 3H). ¹³C NMR (75 MHz, CDCI₃) δ 196.2 (t), 162.5 (t), 161.0 (q), 160.7 (q), 149.1 (q), 143.5 (q), 135.0 (q), 119.5 (t), 105.5 (t), 105.5 (t), 99.5 (t), 55.4 (s), 55.3 (q), 45.0 (d), 42.5 (s), 26.0 (s), 24.8 (s), 18.0 (s), 10.4 (s).

### Example 29: (E)-4-(3-isopropylphenyl)-2,4,7-trimethylocta-2,6-dienal

Following the general procedure described in Example 2: 1-bromo-3-isopropylbenzene (7.18 g, 36.1 mmol), (*E*)-2,4,7-trimethylocta-2,6-dienal (3.00g, 18.04 mmol), diacetoxypalladium (0.20 g, 0.90 mmol), tri-tert-butylphosphine (0.37 g, 1.80 mmol), Cs₂CO₃ (7.05 g, 21.65 mmol) in DMF (20 mL) were reacted to give the title product (5.00 g, 97 % yield) as a yellow oil.

GC/MS (El): *m*/*z* (%): 284(1) [*M⁺*], 216 (44), 187 (26), 173 (100), 158(21), 145 (46), 128 (20), 115 (12), 91 (11), 69 (14). ¹H NMR (300 MHz, CDCI₃) δ 9.41 (s, 1H), 7.26 - 7.18 (m, 1H), 7.08 (d, 3H), 6.79 (s, 1H), 5.04 (t, *J* = 7.4 Hz, 1H), 2.97-2.79 (m, 1H), 2.52 (d, 2H), 1.68 (s, 3H), 1.53 (s, 6H), 1.31 (s, 3H), 1.22 (d, *J* = 6.9 Hz, 6H). ¹³C NMR (75 MHz, CDCI₃) δ 196.4 (t), 163.3 (t), 148.8 (q), 146.3 (q), 139.8 (q), 135.0 (q), 128.2 (t), 125.2 (t), 124.1 (t), 124.0 (t), 119.6 (t), 45.1 (q), 42.7 (d), 34.3 (t), 26.1 (s), 25.1 (s), 24.2 (s), 24.1 (s), 18.0 (s), 10.5 (s).

### Example 30: (E)-2,4,7-trimethyl-4-(pyridin-3-yl)octa-2,6-dienal

Following the general procedure described in Example 2: 3-bromopyridine (5.70 g, 36.1 mmol), (*E*)-2,4,7-trimethylocta-2,6-dienal (3.00 g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMF (20 mL) were reacted to give the title product (0.6 g, 14 % yield) as a light yellow oil.

GC/MS (El): *m*/*z* (%): 243(2) [*M⁺*], 214 (10), 200 (11), 175 (55), 158 (39), 146 (100), 132 (61), 69 (47). ¹H NMR (300 MHz, CDCI₃) δ 9.42 (s, 1H), 8.52 (d, *J=* 2.3 Hz, 1H), 8.46 (dd, *J* = 4.7, 1.3 Hz, 1H), 7.58 - 7.49 (m, 1H), 7.25 - 7.20 (m, 1H), 6.77 (s, 1H), 4.99 (t, *J* = 7.4 Hz, 1H), 2.52 (d, *J=* 7.4 Hz, 2H), 1.66 (s, 3H), 1.56 (s, 3H), 1.47 (s, 3H), 1.30 (s, 3H). ¹³C NMR (75 MHz, CDCI₃) δ 195.9 (t), 161.0 (t), 148.5 (t), 147.7 (t), 141.8 (q), 140.3 (q), 136.1 (q), 134.3 (t), 123.2 (t), 118.5 (t), 43.7 (q), 42.8 (d), 26.1 (s), 24.5 (s), 18.0 (s), 10.8 (s).

### Example 31: (E)-4-methoxy-3-(2,4,7-trimethyl-1-oxoocta-2,6-dien-4-yl)benzonitrile

Following the general procedure described in Example 2: 3-bromo-4-methoxybenzonitrile (5.04 g, 23.77 mmol), (E)-2,4,7-trimethylocta-2,6-dienal (2.16 g, 11.89 mmol), diacetoxypalladium (0.13 g, 0.594 mmol), tri-*tert*-butylphosphane (0.24 g, 1.19 mmol), Cs₂CO₃ (7.75 g, 23.77 mmol) in DMF (50 mL) were reacted to give the title product (1.91 g, 54 % yield) as a light yellow oil.

GC/MS (El): *m*/*z* (%): 297 (1) [*M⁺*], 229 (100), 214 (15), 200 (33),186 (28), 170 (11), 160 (9), 146 (10), 116 (11),69 (48). ¹H NMR (300 MHz, CDCI₃) δ 9.33 (s, 1H), 7.64 - 7.45 (m, 2H), 6.87 (d, *J* = 7.7 Hz, 1H), 6.75 (s, 1H), 4.86 (t, *J* = 7.4 Hz, 1H), 3.74 (s, 3H), 2.78 - 2.46 (m, 2H), 1.61 (s, 3H), 1.48 (d, *J* = 11.8 Hz, 6H), 1.17 (s, 3H). ¹³C NMR (75 MHz, CDCI₃) δ 196.0 (t), 162.8 (t), 160.3 (q), 136.6 (q), 135.6 (q), 135.4 (q), 132.8 (t), 131.0 (t), 119.4 (q), 119.0 (t), 111.6 (t), 103.9 (q), 55.5 (s), 43.0 (q), 38.6 (d), 25.9 (s), 23.2 (s), 17.9 (s), 9.5 (s).

### Example 32: (E)-4-(2,5-difluorophenyl)-2,4,7-trimethylocta-2,6-dienal

Following the general procedure described in Example 2: 2-bromo-1,4-difluorobenzene (5.00 g, 25.90 mmol), (*E*)-2,4,7-trimethylocta-2,6-dienal (2.32 g, 12.95 mmol), diacetoxypalladium (0.09 g, 0.39 mmol), tri-*tert*-butylphosphane (0.16 g, 0.78 mmol), Cs₂CO₃ (8.44 g, 25.90 mmol) in DMF (30 mL) were reacted to give the title product (1.19 g, 33 % yield) as a light yellow oil.

GC/MS (El): *m*/*z* (%): 278 (1) [*M⁺*], 210 (100), 195 (15), 181 (11), 164 (15), 147 (10), 127 (20), 119 (4), 101 (5), 69 (71). ¹H NMR (300 MHz, CDCI₃) δ 9.37 (s, 1H), 7.08 - 6.84 (m, 3H), 6.76 (d, *J* = 1.9 Hz, 1H), 4.95 (t, *J* = 7.3 Hz, 1H), 2.57 (d, *J* = 11.2 Hz, 2H), 1.65 (s, 3H), 1.54 (s, 3H), 1.48 (s, 3H), 1.28 (s, 3H). ¹³C NMR (75 MHz, CDCI₃) δ 195.9 (t), 161.0 (t), 138.3 (q), 138.3 (q), 135.8 (q), 118.6 (t), 117.2 (t), 117.0 (t), 116.8 (t), 116.7 (t), 114.8 (t), 114.8 (t), 114.6 (t), 114.5 (t), 114.4 (t), 114.3 (t), 42.6 (q), 39.7 (d), 39.7 (d), 26.0 (s), 23.4 (s), 17.9 (s), 9.6 (s).

### Example 33: (E)-4-(3,5-difluorophenyl)-2,4,7-trimethylocta-2,6-dienal

Following the general procedure described in Example 2: 1-bromo-3,5-difluorobenzene (5.00g, 25.9 mmol), (*E*)-2,4,7-trimethylocta-2,6-dienal (2.32 g, 12.95 mmol), diacetoxypalladium (0.09 g, 0.39 mmol), tri-*tert*-butylphosphane (0.18 g, 0.78 mmol), Cs₂CO₃ (8.44 g, 25.90 mmol) in DMF (30 mL) were reacted to give the title product (1.48 g, 41 % yield) as a light yellow oil.

GC/MS (El): *m*/*z* (%): 278 (1) [*M⁺*], 210 (80), 192 (46), 181 (13), 164 (19), 151 (13), 127 (13), 119 (4), 101 (4), 69 (100). ¹H NMR (300 MHz, CDCI₃) δ 9.42 (s, 1H), 6.92 - 6.54 (m, 4H), 4.96 (t, *J* = 10.6, 4.3 Hz, 1H), 2.48 (d, *J* = 7.5 Hz, 2H), 1.67 (s, 3H), 1.53 (s, 3H), 1.49 (s, 3H), 1.33 (d, *J* = 1.1 Hz, 3H). ¹³C NMR (75 MHz, CDCI₃) δ 195.8 (t), 164.8 (q), 164.6 (q), 161.5 (q), 161.3 (q), 160.8 (t), 150.9 (q), 140.5 (q), 136.0 (q), 118.5 (t), 110.0 (t), 109.9 (t), 109.8 (t), 109.7 (t), 102.2 (t), 101.8 (t), 101.5 (t), 45.0 (q), 42.7 (d), 26.1 (s), 24.6 (s), 18.0 (s), 10.6 (s).

### Example 34: (E)-2-chloro-5-(2,4,7-trimethyl-1-oxoocta-2,6-dien-4-yl)benzonitrile

Following the general procedure described in Example 2: 5-bromo-2-chlorobenzonitrile (5.00 g, 23.10 mmol), (*E*)-2,4,7-trimethylocta-2,6-dienal (3.00 g, 18.04 mmol), diacetoxypalladium (0.20 g, 0.90 mmol), tri-tert-butylphosphane (0.37 g, 1.80 mmol), Cs₂CO₃ (7.05 g, 21.65 mmol) in DMF (20 mL) were reacted to give the title product (1.00 g, 18 % yield) as a yellow oil.

GC/MS (El): *m*/*z* (%): 301 (1) [*M*⁺], 233 (45), 218 (13), 198 (6), 190 (5), 166 (5), 153 (9), 140 (6), 127 (5), 69 (100). ¹H NMR (300 MHz, CDCl₃) δ 9.43 (s, 1H), 7.55 (d, *J =* 1.7 Hz, 1H), 7.47 - 7.37 (m, 2H), 6.72 (d, *J =* 1.0 Hz, 1H), 4.93 (t, *J =* 7.4 Hz, 1H), 2.48 (d, *J* = 7.4 Hz, 2H), 1.67 (s, 3H), 1.53 (s, 3H), 1.48 (s, 3H), 1.30 (s, 3H). ¹³C NMR (75 MHz, CDCl₃) δ 195.5 (t), 160.0 (t), 146.4 (q), 140.5 (q), 136.7 (q), 134.7 (q), 132.7 (t), 132.1 (t), 129.9 (t), 118.0 (t), 116.2 (q), 113.3 (q), 44.7 (q), 42.8 (d), 26.1 (s), 24.6 (s), 18.1 (s), 10.9 (s).

### Example 35: (E)-3-chloro-5-(2,4,7-trimethyl-1-oxoocta-2,6-dien-4-yl)benzonitrile

Following the general procedure described in Example 2: 3-bromo-5-chlorobenzonitrile (5.00 g, 23.10 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.80 mmol), Cs₂CO₃ (7.05 g, 21.65 mmol) in DMF (20 mL) were reacted to give the title product (0.23 g, 4 % yield) as a yellow oil.

GC/MS (El): *m*/*z* (%): 301 (1) [*M*⁺], 233 (35), 215 (6), 204 (5), 190 (3), 166 (4), 153 (8), 140 (5), 127 (4), 69 (100). ¹H NMR (300 MHz, CDCl₃) δ 9.41 (s, 1H), 7.52 - 7.37 (m, 3H), 6.70 (s, 1H), 4.93 (t, *J =* 7.4 Hz, 1H), 2.46 (d, *J =* 7.4 Hz, 2H), 1.65 (s, 3H), 1.51 (s, 3H), 1.45 (s, 3H), 1.28 (s, 3H). ¹³C NMR (75 MHz, CDCl₃) δ 195.4 (t), 159.4 (t), 150.2 (q), 140.5 (q), 136.7 (q), 135.2 (q), 131.7 (t), 129.6 (t), 128.6 (t), 117.9 (t), 117.5 (q), 113.7 (q), 44.9 (q), 42.6 (d), 26.0 (s), 24.4 (s), 17.9 (s), 10.8 (s).

### Example 36: (E)-4-(3,4-difluorophenyl)-2,4,7-trimethylocta-2,6-dienal

Following the general procedure described in Example 2: 4-bromo-1,2-difluorobenzene (5.00g, 25.9 mmol), (*E*)-2,4,7-trimethylocta-2,6-dienal (2.32 g, 12.95 mmol), diacetoxypalladium (0.09 g, 0.39 mmol), tri-*tert*-butylphosphane (0.16 g, 0.78 mmol), Cs₂CO₃ (8.44 g, 25.90 mmol) in DMF (30 mL) were reacted to give the title product (1.27 g, 35 % yield) as a light yellow oil.

GC/MS (El): *m*/*z* (%): 278 (1) [*M*⁺], 210 (88), 195 (36), 181 (28), 164 (21), 151 (14), 141 (11), 127 (24), 101 (5), 69 (100). ¹H NMR (300 MHz, CDCl₃) δ 9.41 (s, 1H), 7.15 - 6.89 (m, 3H), 6.72 (d, *J =* 1.0 Hz, 1H), 4.96 (t, *J =* 7.4 Hz, 1H), 2.47 (d, *J =* 7.4 Hz, 2H), 1.67 (s, 3H), 1.50 (d, *J* = 6.0 Hz, 6H), 1.31 (d, *J =* 0.8 Hz, 3H). ¹³C NMR (75 MHz, CDCl₃) δ 195.9 (t), 161.5 (t), 151.9 (q), 151.7 (q), 150.5 (q), 150.3 (q), 148.6 (q), 148.4 (q), 147.2 (q), 147.0 (q), 143.8 (q), 143.7 (q), 143.7 (q), 140.3 (q), 135.8 (q), 122.9 (t), 122.8 (t), 122.7 (t), 122.6 (t), 118.7 (t), 117.1 (t), 116.9 (t), 116.0 (t), 115.7 (t), 44.5 (q), 42.9 (d), 26.1 (s), 24.8 (s), 18.0 (s), 10.6 (s).

### Example 37: (E)-2-methyl-5-(2,4,7-trimethyl-1-oxoocta-2,6-dien-4-yl)benzonitrile

Following the general procedure described in Example 2: 5-bromo-2-methylbenzonitrile (5.00 g, 25.5 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.804 mmol), Cs₂CO₃ (7.05 g, 21.65 mmol) in DMF (20 mL) were reacted to give the title product (2.10 g, 41 % yield) as a yellow oil.

GC/MS (El): *m*/*z* (%): 281 (1) [*M*⁺], 213 (100), 198 (74), 184 (26), 170 (24), 154 (14), 140 (9), 130 (10), 115 (10), 69 (100). ¹H NMR (300 MHz, CDCl₃) δ 9.41 (s, 1H), 7.47 (d, *J =* 1.6 Hz, 1H), 7.34 (dd, *J =* 8.2, 1.8 Hz, 1H), 7.23 (d, *J =* 8.2 Hz, 1H), 6.73 (s, 1H), 4.93 (t, *J =* 7.3 Hz, 1H), 2.55 - 2.41 (m, 5H), 1.66 (s, 3H), 1.50 (d, *J =* 5.4 Hz, 6H), 1.27 (s, 3H). ¹³C NMR (75 MHz, CDCl₃) δ 195.8 (t), 161.1 (t), 145.0 (q), 140.2 (q), 139.9 (q), 136.0 (q), 131.4 (t), 130.5 (t), 130.3 (t), 118.5 (t), 118.3 (q), 112.7 (q), 44.5 (q), 42.7 (d), 26.0 (s), 24.6 (s), 20.0 (s), 18.0 (s), 10.7 (s).

### Example 38: (E)-2,4,7-trimethyl-4-(2-(methylthio)phenyl)octa-2,6-dienal

Following the general procedure described in Example 2: (2-bromophenyl)(methyl)-sulfane (5.71 g, 28.1 mmol), (*E*)-2,4,7-trimethylocta-2,6-dienal (2.51 g, 14.05 mmol), diacetoxypalladium (0.16 g, 0.70 mmol), tri-*tert*-butylphosphane (0.28 g, 1.41 mmol), Cs₂CO₃ (4.58 g, 14.05 mmol) in DMF (80 mL) were reacted to give the title product (2.31 g, 57 % yield) as a light yellow oil.

GC/MS (El): *m*/*z* (%): 288 (1) [*M*⁺], 273 (29), 245 (28), 217 (80), 205 (35), 172 (89), 163 (80), 144 (100), 129 (73), 69 (61). ¹H NMR (300 MHz, CDCl₃) δ 9.46 (s, 1H), 7.42 - 7.34 (m, 2H), 7.26 - 7.20 (m, 2H), 6.99 (d, *J =* 0.9 Hz, 1H), 5.02 (t, *J =* 7.5 Hz, 1H), 2.87 - 2.68 (m, 2H), 2.33 (s, 3H), 1.69 (s, 3H), 1.60 (s, 3H), 1.55 (s, 3H), 1.20 (d, *J =* 0.7 Hz, 3H). ¹³C NMR (75 MHz, CDCl₃) δ 196.3 (t), 162.6 (t), 146.4 (q), 136.7 (q), 136.3 (q), 134.9 (q), 131.0 (t), 127.2 (t), 126.4 (t), 126.1 (t), 119.6 (t), 45.1 (q), 39.6 (d), 26.0 (s), 24.0 (s), 19.2 (s), 18.0 (s), 9.5 (s).

### Example 39: (E)-4-(3-fluoro-5-(trifluoromethyl)phenyl)-2,4,7-trimethylocta-2,6-dienal

Following the general procedure described in Example 2: 1-bromo-3-fluoro-5-(trifluoromethyl)benzene (8.77 g, 36.10 mmol), (*E*)-2,4,7-trimethylocta-2,6-dienal (3.00 g, 18.04 mmol), diacetoxypalladium (0.20 g, 0.90 mmol), tri-*tert*-butylphosphane (0.37 g, 1.81 mmol), Cs₂CO₃ (7.05 g, 21.65 mmol) in DMF (20 mL) were reacted to give the title product (1.75 g, 30 % yield) as a light yellow oil.

GC/MS (El): *m*/*z* (%): 328 (1) [*M*⁺], 309 (3), 260 (100), 242 (15), 231 (9), 195 (6), 177 (6), 146 (6), 133 (3), 69 (41). ¹H NMR (300 MHz, CDCl₃) δ 9.44 (s, 1H), 7.28 (s, 1H), 7.17 (t, *J =* 10.4 Hz, 2H), 6.74 (s, 1H), 4.98 (t, *J =* 7.5 Hz, 1H), 2.50 (d, *J =* 7.5 Hz, 2H), 1.67 (s, 3H), 1.54 (s, 3H), 1.47 (s, 3H), 1.31 (s, 3H). ¹³C NMR (75 MHz, CDCl₃) δ 195.7 (t), 164.2 (q), 160.9 (q), 160.3 (t), 150.7 (q), 150.6 (q), 140.6 (q), 136.5 (q), 119.5 (t), 119.4 (t), 118.3 (t), 117.6 (t), 117.3 (t), 111.0 (t), 110.9 (t), 110.6 (t), 110.6 (t), 45.2 (q), 42.8 (d), 26.0 (s), 24.8 (s), 18.0 (s), 10.8 (s).

### Example 40: (E)-4-(2,3-dimethylphenyl)-2,4,7-trimethylocta-2,6-dienal

Following the general procedure described in Example 2: 1-bromo-2,3-dimethylbenzene (6.68 g, 36.10 mmol), (*E*)-2,4,7-trimethylocta-2,6-dienal (3.00g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMF (20 mL) were reacted to give the title product (1.65 g, 34 % yield) as a light yellow oil.

GC/MS (El): *m*/*z* (%): 270 (1) [*M*⁺], 202 (88), 201 (26), 187 (100), 186 (82), 173 (89), 171 (21), 159 (31), 157 (28), 143 (35), 141 (26), 69 (29). ¹H NMR (300 MHz, CDCl₃) δ 9.55 (s, 1H), 7.37 (d, *J =* 6.7 Hz, 1H), 7.25 - 7.17 (m, 2H), 7.04 (s, 1H), 5.16 (t, *J =* 7.4 Hz, 1H), 2.86 - 2.60 (m, 2H), 2.35 (s, 3H), 2.21 (s, 3H), 1.83 (s, 3H), 1.70 (s, 3H), 1.64 (s, 3H), 1.28 (s, 3H). ¹³C NMR (75 MHz, CDCl₃) δ 196.2 (t), 163.5 (t), 144.3 (q), 138.0 (q), 137.6 (q), 134.9 (q), 134.4 (q), 128.5 (t), 125.6 (t), 124.1 (t), 119.7 (t), 44.9 (q), 40.5 (d), 26.1 (s), 25.4 (s), 21.2 (s), 19.6 (s), 18.1 (s), 9.5 (s).

### Example 41: (E)-2,4,7-trimethyl-4-(2-methyl-5-(trifluoromethyl)phenyl)octa-2,6-dienal

Following the general procedure described in Example 2: 2-bromo-1-methyl-4-(trifluoromethyl)benzene (5.00 g, 20.92 mmol), (*E*)-2,4,7-trimethylocta-2,6-dienal (1.74 g, 10.46 mmol), diacetoxypalladium (0.12 g, 0.52 mmol), tri-*tert*-butylphosphane (0.21 g, 1.05 mmol), Cs₂CO₃ (4.09 g, 12.55 mmol) in DMF (20 mL) were reacted to give the title product (1.50 g, 44 % yield) as a yellow oil.

GC/MS (El): *m*/*z* (%): 324 (1) [*M*⁺], 305 (88), 256 (100), 241 (55), 236 (6), 22 (19), 225 (21), 159 (31), 221 (28), 191 (3), 177 (4), 69 (57). ¹H NMR (300 MHz, CDCl₃) δ 9.44 (s, 1H), 7.59 (s, 1H), 7.41 (d, *J =* 7.9 Hz, 1H), 7.22 (d, *J =* 7.9 Hz, 1H), 6.83 (s, 1H), 4.96 (t, *J =* 7.3 Hz, 1H), 2.70 - 2.49 (m, 2H), 2.29 (s, 3H), 1.67 (s, 3H), 1.56 (s, 6H), 1.18 (s, 3H). ¹³C NMR (75 MHz, CDCl₃) δ 195.8 (t), 161.4 (t), 144.9 (q), 140.2 (q), 138.4 (q), 135.8 (q), 132.4 (t), 128.6 (q), 128.2 (q), 123.4 (t), 123.4 (t), 123.2 (t), 123.2 (t), 118.8 (t), 44.9 (q), 39.9 (d), 26.0 (s), 24.9 (s), 22.9 (s), 18.0 (s), 9.7 (s).

### Example 42: (E)-4-(2-ethoxyphenyl)-2,4,7-trimethylocta-2,6-dienal

Following the general procedure described in Example 2: 1-bromo-2-ethoxybenzene (7.26 g, 36.10 mmol), (*E*)-2,4,7-trimethylocta-2,6-dienal (3.00 g, 18.04 mmol), diacetoxypalladium (0.20 g, 0.90 mmol), tricyclohexylphosphane (0.51 g, 1.80 mmol), Cs₂CO₃ (7.05 g, 21.65 mmol) in DMA (20 mL) were reacted to give the title product (2.16 g, 42 % yield) as a light yellow oil.

GC/MS (El): *m*/*z* (%): 286 (5) [*M*⁺], 218 (37), 217 (100), 189 (60), 188 (25), 161 (42), 147 (30), 133 (21), 91 (17), 69 (13). ¹H NMR (300 MHz, CDCl₃) δ 9.38 (s, 1H), 7.32 - 7.27 (m, 1H), 7.24 - 7.16 (m, 1H), 6.97 - 6.87 (m, 2H), 6.80 (d, *J =* 8.1 Hz, 1H), 5.01 (t, *J =* 7.5 Hz, 1H), 4.07 - 3.72 (m, 2H), 2.76 - 2.56 (m, 2H), 1.67 (s, 3H), 1.59 (s, 3H), 1.49 (s, 3H), 1.31 (t, *J =* 7.0 Hz, 3H), 1.23 (s, 3H). ¹³C NMR (75 MHz, CDCl₃) δ 196.7 (t), 165.8 (t), 156.4 (q), 136.1 (q), 134.6 (q), 134.4 (q), 127.8 (t), 126.9 (t), 120.4 (t), 120.2 (t), 111.8 (t), 63.4 (d), 43.1 (q), 39.2 (d), 26.1 (s), 23.4 (s), 18.0 (s), 15.0 (s), 9.5 (s).

### Example 43: (E)-2,4,7-trimethyl-4-(4-methylpyridin-3-yl)octa-2,6-dienal

Following the general procedure described in Example 2: 3-bromo-4-methylpyridine (5.00 g, 29.1 mmol), (*E*)-2,4,7-trimethylocta-2,6-dienal (2.42 g, 14.53 mmol), diacetoxypalladium (0.16 g, 0.73 mmol), tri-*tert*-butylphosphane (0.29 g, 1.45 mmol), Cs₂CO₃ (5.68 g, 17.44 mmol) in DMF (20 mL) were reacted to give the title product (1.18 g, 30 % yield) as a light yellow oil.

GC/MS (El): *m*/*z(%):* 257 (5) [*M*⁺], 228 (5), 214 (8), 189 (65),174 (28), 160 (100), 146 (71), 130 (14), 121 (10), 69 (45). ¹H NMR (300 MHz, CDCl₃) δ 9.36 (s, 1H), 8.51 (s, 1H), 8.30 (d, *J =* 4.9 Hz, 1H), 6.96 (d, *J* = 4.8 Hz, 1H), 6.74 (s, 1H), 4.88 (t, *J* = 6.9 Hz, 1H), 2.54 (d, *J =* 7.4 Hz, 2H), 2.17 (s, 3H), 1.59 (s, 3H), 1.50 (d, *J =* 10.2 Hz, 6H), 1.14 (s, 3H). ¹³C NMR (75 MHz, CDCl₃) δ 195.5 (t), 160.9 (t), 147.9 (t), 147.7 (q), 145.0 (q), 139.1 (q), 138.5 (q), 135.6 (q), 126.4 (t), 118.6 (t), 43.5 (q), 39.8 (d), 25.9 (s), 24.3 (s), 22.1 (s), 18.0 (s), 9.7 (s).

### Example 44: (E)-2,4,7-trimethyl-4-(5-methylthiophen-3-yl)octa-2,6-dienal

Following the general procedure described in Example 2: 4-bromo-2-methylthiophene (5.00 g, 28.20 mmol), (*E*)-2,4,7-trimethylocta-2,6-dienal (2.52 g, 14.12 mmol), diacetoxypalladium (0.10 g, 0.42 mmol), tri-*tert*-butylphosphane (0.17 g, 0.85 mmol), Cs₂CO₃ (9.20 g, 28.20 mmol) in DMF (30 mL) were reacted to give the title product (1.80 g, 49 % yield) as a light yellow oil.

GC/MS (El): *m*/*z* (%): 262 (1) [*M*⁺], 193 (43), 178 (65), 165 (100), 149 (11), 135 (7), 111 (7), 69 (9). ¹H NMR (300 MHz, CDCl₃) δ 9.36 (s, 1H), 6.71 (d, *J =* 1.4 Hz, 1H), 6.64 (d, *J* = 1.2 Hz, 1H), 6.54 (s, 1H), 5.04 (t, *J =* 7.3 Hz, 1H), 2.56 - 2.44 (m, 2H), 2.41 (d, *J =* 0.7 Hz, 3H), 1.68 (s, 3H), 1.56 (s, 3H), 1.46 (s, 3H), 1.42 (d, *J =* 1.1 Hz, 3H). ¹³C NMR (75 MHz, CDCl₃) δ 196.2 (t), 161.9 (t), 147.4 (q), 139.8 (q), 139.7 (q), 134.8 (q), 125.3 (t), 119.5 (t), 117.3 (t), 42.9 (q), 41.7 (d), 26.0 (s), 25.3 (s), 18.0 (s), 15.4 (s), 9.7 (s).

### Example 45: (E)-4-(furan-3-yl)-2,4,7-trimethylocta-2,6-dienal

Following the general procedure described in Example 2: 3-bromofuran (3.31 g, 22.51 mmol), (*E*)-2,4,7-trimethylocta-2,6-dienal (2.01 g, 11.25 mmol), diacetoxypalladium (0.08 g, 0.34 mmol), tri-*tert*-butylphosphane (0.14 g, 0.68 mmol), Cs₂CO₃ (7.33 g, 22.51 mmol) in DMF (30 mL) were reacted to give the title product (1.12 g, 43 % yield) as a light yellow oil.

GC/MS (El): *m*/*z* (%): 232 (2) [*M*⁺], 164 (46), 163 (30), 148 (7), 135 (100), 121 (7), 107 (12), 91 (40), 69 (24). ¹H NMR (300 MHz, CDCl₃) δ 9.36 (s, 1H), 7.35 (t, *J =* 1.4 Hz, 1H), 7.20 (s, 1H), 6.58 (d, *J =* 1.1 Hz, 1H), 6.21 (d, *J =* 0.7 Hz, 1H), 5.06 (t, *J =* 7.3 Hz, 1H), 2.55 - 2.33 (m, 2H), 1.68 (s, 3H), 1.61 - 1.49 (m, 6H), 1.44 (s, 3H). ¹³C NMR (75 MHz, CDCl₃) δ 196.2 (t), 161.0 (t), 143.1 (t), 139.7 (q), 138.4 (t), 134.9 (q), 131.3 (q), 119.3 (t), 109.7 (t), 41.4 (d), 39.4 (q), 25.9 (s), 25.3 (s), 18.0 (s), 9.9 (s).

### Example 46: (E)-4-(2,4-difluorophenyl)-2,4,7-trimethylocta-2,6-dienal

Following the general procedure described in Example 2: 1-bromo-2,4-difluorobenzene (5.44 g, 28.20 mmol), (*E*)-2,4,7-trimethylocta-2,6-dienal (2.52 g, 14.10 mmol), diacetoxypalladium (0.10 g, 0.42 mmol), tri-*tert*-butylphosphane (0.17 g, 0.85 mmol), Cs₂CO₃ (9.19 g, 28.20 mmol) in DMF (30 mL) were reacted to give the title product (1.35 g, 35 % yield) as a light yellow oil.

GC/MS (El): *m*/*z* (%):278(1) [*M*⁺], 276 (3), 210 (98), 195 (41), 181 (27), 164 (22), 141 (21), 127 (45), 69 (100). ¹H NMR (300 MHz, CDCl₃) δ 9.41 (s, 1H), 7.36 - 7.24 (m, 1H), 6.92 - 6.83 (m, 1H), 6.82 - 6.78 (m, 1H), 6.77 - 6.69 (m, 1H), 4.97 (t, *J* = 7.3 Hz, 1H), 2.61 (d, *J =* 7.6 Hz, 2H), 1.67 (s, 3H), 1.56 (s, 3H), 1.53 (s, 3H), 1.31 (s, 3H). ¹³C NMR (75 MHz, CDCl₃) δ 196.0 (t), 163.6 (q), 163.4 (q), 162.5 (q), 162.3 (q), 161.6 (t), 160.3 (q), 160.2 (q), 159.2 (q), 159.0 (q), 138.3 (q), 138.2 (q), 135.6 (q), 129.3 (q), 129.3 (q), 129.2 (q), 129.1 (q), 128.5 (t), 128.5 (t), 128.4 (t), 128.3 (t), 118.8 (t), 110.9 (t), 110.9 (t), 110.7 (t), 110.6 (t), 104.8 (t), 104.4 (t), 104.4 (t), 104.1 (t), 42.3 (q), 42.3 (q), 39.9 (d), 26.0 (s), 23.6 (s), 17.9 (s), 9.6 (s).

### Example 47: (E)-4-(4-chloro-2-methylphenyl)-2,4,7-trimethylocta-2,6-dienal

Following the general procedure described in Example 2: 1-bromo-4-chloro-2-methylbenzene (4.94 g, 24.06 mmol), (*E*)-2,4,7-trimethylocta-2,6-dienal (2.00g, 12.03 mmol), diacetoxypalladium (0.14 g, 0.60 mmol), tricyclohexylphosphane (0.34 g, 1.20 mmol), Cs₂CO₃ (4.70 g, 14.43 mmol) in DMF (20 mL) were reacted to give the title product (0.86 g, 25 % yield) as a yellow oil.

GC/MS (El): *m*/*z* (%):290 (1) [*M*⁺], 222 (100), 207 (69), 193 (50), 186 (63), 158 (26), 141 (34), 128 (32), 115 (32), 69 (98). ¹H NMR (300 MHz, CDCl₃) δ 9.42 (s, 1H), 7.34 - 7.23 (m, 1H), 7.20 - 7.05 (m, 2H), 6.80 (s, 1H), 4.93 (s, 1H), 2.55 (s, 2H), 2.20 (s, 3H), 1.66 (s, 3H), 1.56 (s, 3H), 1.49 (s, 3H), 1.20 (s, 3H). ¹³C NMR (75 MHz, CDCl₃) δ 196.0 (t), 162.2 (t), 142.6 (q), 138.4 (q), 138.0 (q), 135.5 (q), 132.3 (q), 131.8 (t), 128.0 (t), 126.1 (t), 119.2 (t), 44.6 (q), 40.1 (d), 26.2 (s), 25.1 (s), 22.8 (s), 18.2 (s), 9.8 (s).

### Example 48: (E)-4-(4-fluorophenyl)-2,4,7-trimethylocta-2,6-dienal

Following the general procedure described in Example 2: 1-bromo-4-fluorobenzene (2.93 g, 16.74 mmol), (*E*)-2,4,7-trimethylocta-2,6-dienal (1.56 g, 8.37 mmol), diacetoxypalladium (0.09 g, 0.42 mmol), tricyclohexylphosphane (0.24 g, 0.84 mmol), Cs₂CO₃ (3.27 g, 10.05 mmol) in DMF (20 mL) were reacted to give the title product (1.20 g, 55 % yield) as a yellow oil.

GC/MS (El): m/z(%):260 (1)[*M*⁺], 192 (100), 177 (57), 163 (25), 146 (22), 133 (14), 109 (16), 95 (2), 83 (2), 69 (65). ¹H NMR (300 MHz, CDCl₃) δ 9.40 (s, 1H), 7.23 - 7.14 (m, 2H), 6.95 (t, *J =* 8.7 Hz, 2H), 6.75 (d, *J =* 1.0 Hz, 1H), 4.97 (t, *J =* 7.4 Hz, 1H), 2.48 (d, *J* = 7.4 Hz, 2H), 1.64 (s, 3H), 1.49 (s, 6H), 1.28 (d, *J =* 0.9 Hz, 3H). ¹³C NMR (75 MHz, CDCl₃) δ 196.0 (t), 162.8 (q), 162.5 (t), 159.6 (q), 142.2 (q), 142.1 (q), 139.9 (q), 135.3 (q), 128.2 (t), 128.1 (t), 119.1 (t), 115.1 (t), 114.8 (t), 44.4 (q), 42.9 (d), 26.0 (s), 24.8 (s), 17.9 (s), 10.4 (s).

### Example 49: (E)-4-(3-fluorophenyl)-2,4,7-trimethylocta-2,6-dienal

Following the general procedure described in Example 2: 1-bromo-3-fluorobenzene (2.93 g, 16.74 mmol), (*E*)-2,4,7-trimethylocta-2,6-dienal (1.56 g, 8.37 mmol), diacetoxypalladium (0.094 g, 0.419 mmol), tricyclohexylphosphane (0.24 g, 0.84 mmol), Cs₂CO₃ (3.27 g, 10.05 mmol) in DMF (20 mL) were reacted to give the title product (1.18 g, 54 % yield) as a yellow oil.

GC/MS (El): *m*/*z* (%):260 (1) [*M*⁺], 192 (100), 177 (59), 163 (65), 146 (27), 133 (17), 109 (23), 96 (3), 83 (3), 69 (45). ¹H NMR (300 MHz, CDCl₃) δ 9.43 (s, 1H), 7.31 - 7.21 (m, 1H), 7.07 - 6.86 (m, 3H), 6.76 (d, *J =* 1.1 Hz, 1H), 5.00 (t, *J =* 7.4 Hz, 1H), 2.60 - 2.40 (m, 2H), 1.68 (s, 3H), 1.52 (d, *J =* 5.0 Hz, 6H), 1.32 (d, *J =* 1.0 Hz, 3H). ¹³C NMR (75 MHz, CDCl₃) δ 196.0 (t), 164.5 (q), 161.9 (t), 161.3 (q), 149.3 (q), 149.3 (q), 140.1 (q), 135.5 (q), 129.8 (t), 129.7 (t), 122.5 (t), 122.4 (t), 119.0 (t), 113.9 (t), 113.6 (t), 113.2 (t), 112.9 (t), 44.9 (q), 44.8 (q), 42.7 (d), 26.0 (s), 24.7 (s), 18.0 (s), 10.5 (s).

### Example 50: (E)-2,4,7-trimethyl-4-phenylocta-2,6-dienal

To a three-necked round-bottomeed flask equipped with a magnetic stirrer, Dean-Stark and Water condenser, was added 3-methylbut-2-en-1-ol (9.69 g, 110.00 mmol), (*E*)-2-methyl-4-phenylpent-2-enal (11.71 g, 55.10 mmol), and triethylamine hydrochloride (0.38 g, 2.76 mmol) in xylene (30 ml) to give a light colorless solution. The reaction mixture was stirred and heated to a refluxing temperature at 150°C. Water was collected from Dean-Stark. The reaction was heated at refluxing for 16 hours. Then cooled to room temperature and filtered through a short silica gel pad. Washed with MTBE( 150 mL). The crude product was concentrated in rotary evaporator remove excessive xylene and alcohol. Then purified by distillation ,kugelrohr (0.03mbar/155°C) to obtain the title product (5.691 g, 22.07 mmol, 40.0 % yield) as a light yellow oil.

GC/MS (El): *m*/*z* (%): 242 (1) [*M*⁺], 174 (100), 159 (57), 145(49), 128 (27), 115 (20), 105 (11), 91 (19), 77 (10), 69 (22). ¹H NMR (300 MHz, CDCl₃) δ 9.42 (s, 1H), 7.30 - 7.23 (m, 5H), 6.79 (d, *J =* 1.1 Hz, 1H), 5.09 - 4.93 (m, 1H), 2.60 - 2.44 (m, 2H), 1.67 (s, 3H), 1.53 (d, *J =* 3.5 Hz, 6H), 1.31 (d, *J =* 1.1 Hz, 3H). ¹³C NMR (75 MHz, CDCl₃) δ 196.3 (t), 163.0 (t), 146.5 (q), 139.9 (q), 135.1 (q), 128.3 (t), 126.6 (t), 126.2 (t), 119.4 (t), 44.9 (q), 42.7 (d), 26.0 (s), 24.8 (s), 18.0 (s), 10.4 (s).

### Example 51: (E)-2-methyl-3-(2,4,7-trimethyl-1-oxoocta-2,6-dien-4-yl)benzonitrile

Following the general procedure described in Example 2: 3-iodo-2-methylbenzonitrile (2.00 g, 8.23 mmol), (*E*)-2,4,7-trimethylocta-2,6-dienal (2.74 g, 16.46 mmol), diacetoxypalladium (0.09 g, 0.41 mmol), tri-*tert*-butylphosphane (0.17 g, 0.82 mmol), Cs₂CO₃ (3.22 g, 9.87 mmol) in DMF (60 mL) were reacted to give the title product (0.42 g, 18 % yield) as a light yellow oil.

GC/MS (El): *m*/*z* (%): 281 (1) [*M*⁺], 213 (62), 198 (38), 184 (13), 170 (10), 154 (11), 140 (8), 69 (100)

### Example 52: (E)-2,4,7-trimethyl-4-(5-methylthiophen-2-yl)octa-2,6-dienal

Following the general procedure described in Example 2: 2-bromo-5-methylthiophene (1.75 g, 9.88 mmol), (*E*)-2,4,7-trimethylocta-2,6-dienal (4.93 g, 29.7 mmol), diacetoxypalladium (0.11 g, 0.49 mmol), tricyclohexylphosphane (0.28 g, 0.99 mmol), Cs₂CO₃ (6.44 g, 19.77 mmol) in DMF (35 mL) were reacted to give the title product (0.54 g, 21 % yield) as a light yellow oil.

GC/MS (El): *m*/*z(%):* 262 (1) [*M*⁺], 193 (73), 178 (54), 165 (100), 149 (10), 135 (8), 125 (8), 111 (10), 91 (9), 59 (13).

### Example 53: (E)-2,4,7-trimethyl-4-(2,4,5-trimethylphenyl)octa-2,6-dienal

Following the general procedure described in Example 2: 1-bromo-2,4,5-trimethylbenzene (5.00 g, 25.10 mmol), (*E*)-2,4,7-trimethylocta-2,6-dienal (2.25 g, 12.56 mmol), diacetoxypalladium (0.09 g, 0.38 mmol), tri-*tert*-butylphosphane (0.15 g, 0.75 mmol), Cs₂CO₃ (8.18 g, 25.10 mmol) in DMF (30 mL) were reacted to give the title product (1.51 g, 42 % yield) as a light yellow oil.

GC/MS (El): *m*/*z* (%): 284 (1) [*M*⁺], 215 (54), 200 (68), 187 (100), 173 (31), 157 (26), 141 (15), 128 (10), 115 (9), 69 (11).

### Example 54: (E)-4-(2,6-dimethylphenyl)-2,4,7-trimethylocta-2,6-dienal

Following the general procedure described in Example 2: 2-bromo-1,3-dimethylbenzene (6.68 g, 36.1 mmol), (*E*)-2,4,7-trimethylocta-2,6-dienal (3.00 g, 18.0 mmol), diacetoxypalladium (0.20 g, 0.90 mmol), tri-tert-butylphosphane (0.37 g, 1.80 mmol), Cs₂CO₃ (7.05 g, 21.7 mmol) in DMF (20 mL) were reacted to give the title product (1.02 g, 21 % yield) as a yellow oil.

GC/MS (EI): *m*/*z* (%): 270 (1) [M⁺], 202 (48), 186 (90), 173 (100), 159 (51), 143 (49), 129 (56), 109 (51).

### Example 55: (E)-2,4,7-trimethylocta-2,6-dienal

A mixture of (*E*)-2-methylbut-2-enal (100 g, 1161 mmol), 3-methylbut-2-en-1-ol(195 g, 2322 mmol) and 7.99 g Et₃N-HCl in xylene was refluxed for 36 hours. During this period, water was collected and removed from Dean-Stark apparatus, and the reaction was monitored by GC. After cooled to room temperature, the reaction mixture was diluted with MTBE, and washed with water and brine, dried with MgSO₄, filtered, then evaporated under reduced pressure to give crude product, which was purified by distillation to afford (*E*)-2,7-dimethylocta-2,6-dienal (77.5 g, 44% yield) as a colorless oil.

### Example 56: (E)-4-methyl-3-(2,4,7-trimethyl-1-oxoocta-2,6-dien-4-yl)benzonitrile

### (General Procedure)

A mixture of 3-bromo-4-methylbenzonitrile (4.62 g, 23.55 mmol), (*E*)-2,7-dimethylocta-2,6-dienal (1.82 g, 11.77 mmol), diacetoxypalladium (0.13 g, 0.59 mmol), tri*-tert-*butylphosphane (0.24 g, 1.18 mmol), Cs₂CO₃ (3.84 g, 11.77 mmol) in DMF (20 mL) was heated to 110-120 °C under Ar atmosphere overnight, and the reaction was monitored by TLC, GC and GC-MS. After cooling to room temperature, the reaction mixture was diluted with MTBE, and filtered through a small pad of silica gel and the silica gel was washed with MTBE. Then the combined filtrates were concentrated in vacuo to give crude product, which was purified by distillation or flash chromatography (hexane/ MTBE = 50 : 1- 10 : 1) to afford (*E*)-3-(2,7-dimethyl-1-oxoocta-2,6-dien-4-yl)-4-methylbenzonitrile (1.03g yield: 31%) as light yellow oil.

GC/MS (El): *m*/*z* (%): 267 (1) [*M*⁺], 199 (100), 184 (21), 170 (11), 154 (11), 140 (13), 127 (9), 115 (8), 103 (5), 69 (83). ¹H NMR (300 MHz, CDCl₃) δ 9.44 (s, 1H), 7.55 (s, 1H), 7.47 -7.40 (m, 1H), 7.27 (d, *J =* 8.0 Hz, 1H), 6.50 (d, *J =* 9.6 Hz, 1H), 5.02 (t, *J* = 7.1 Hz, 1H), 4.04 (q, *J =* 16.7, 7.4 Hz, 1H), 2.49 (t, *J =* 7.2 Hz, 2H), 2.40 (s, 3H), 1.76 (s, 3H), 1.67 (s, 3H), 1.58 (s, 3H). ¹³C NMR (75 MHz, CDCl₃) δ 194.9 (t), 154.8 (t), 142.5 (q), 141.7 (q), 139.5 (q), 134.9 (q), 131.4 (t), 130.3 (t), 130.1 (t), 120.0 (t), 119.0 (q), 110.4 (q), 40.8 (t), 33.9 (d), 25.7 (s), 20.1 (s), 17.9 (s), 9.8 (s).

### Example 57: (E)-2,7-dimethyl-4-phenylocta-2,6-dienal

Following the general procedure described in Example 56: bromobenzene (4.07 g, 25.90 mmol), (*E*)-2,7-dimethylocta-2,6-dienal (2.15 g, 12.96 mmol), diacetoxypalladium (0.15 g, 0.65 mmol), tricyclohexylphosphine (0.36 g, 1.30 mmol), Cs₂CO₃ (5.07 g, 15.56 mmol) in DMF (20 mL) were reacted to give the title product (2.04 g, 69 % yield) as a light yellow oil.

GC/MS (El): *m*/*z* (%): 228 (1) [*M*⁺], 160 (100), 145 (41), 131(30), 115(28), 91 (40), 77 (12), 69 (71), 53 (12). ¹H NMR (300 MHz, CDCl₃) δ 9.42 (s, 1H), 7.36 - 7.28 (m, 2H), 7.26 - 7.21 (m, 3H), 6.59 (d, *J =* 9.9 Hz, 1H), 5.03 (t, *J =* 7.1 Hz, 1H), 3.82 (dd, *J =* 16.9, 7.7 Hz, 1H), 2.59 - 2.42 (m, 2H), 1.78 (s, 3H), 1.65 (s, 3H), 1.58 (s, 3H). ¹³C NMR (75 MHz, CDCl₃) δ 195.4 (t), 156.9 (t), 142.6 (q), 138.8 (q), 134.1 (q), 128.9 (t), 127.6 (t), 127.0 (t), 121.0 (t), 45.6 (t), 34.8 (d), 25.9 (s), 18.1 (s), 9.7 (s).

### Example 58: (E)-4-(2,4-dimethylphenyl)-2,7-dimethylocta-2,6-dienal

Following the general procedure described in Example 56: 1-bromo-2,4-dimethylbenzene (7.29 g, 39.4 mmol), (*E*)-2,7-dimethylocta-2,6-dienal (3.00g, 19.71 mmol), diacetoxypalladium (0.22 g, 0.99 mmol), tricyclohexylphosphane (0.55 g, 1.971 mmol), Cs₂CO₃ (7.70 g, 23.65 mmol) in DMF (20 mL) were reacted to give the title product (1.60 g, 32 % yield) as a yellow oil.

GC/MS (El): *m*/*z* (%): 256 (2) [*M*⁺], 188 (72), 173 (43), 172 (50), 159 (100), 144 (22), 129 (14), 69 (15). ¹H NMR (300 MHz, CDCl₃) δ 9.41 (s, 1H), 7.17 (d, *J =* 7.8 Hz, 1H), 7.06 - 6.98 (m, 2H), 6.53 (d, *J =* 13.2, 6.6 Hz, 1H), 5.07 (t, *J* = 7.1 Hz, 1H), 4.00 (q, *J* = 17.0, 7.5 Hz, 1H), 2.46 (t, *J =* 18.9, 11.5 Hz, 2H), 2.30 (d, *J =* 1.9 Hz, 6H), 1.78 (d, *J =* 0.9 Hz, 3H), 1.67 (s, 3H), 1.61 (s, 3H). ¹³C NMR (75 MHz, CDCl₃) δ 195.5 (t), 157.5 (t), 138.5 (q), 137.9 (q), 136.2 (q), 135.7 (q), 133.9 (q), 131.5 (t), 127.3 (t), 126.3 (t), 121.2 (t), 40.9 (t), 34.3 (d), 25.8 (s), 21.0 (s), 19.7 (s), 18.0 (s), 9.7 (s).

### Example 59: (E)-4-(4-chloro-2-methylphenyl)-2,7-dimethylocta-2,6-dienal

Following the general procedure described in Example 56: 1-bromo-4-chloro-2-methylbenzene (8.10 g, 39.40 mmol), (*E*)-2,7-dimethylocta-2,6-dienal (3.00g, 19.71 mmol), diacetoxypalladium (0.22 g, 0.99 mmol), tricyclohexylphosphane (0.55 g, 1.97 mmol), Cs₂CO₃ (7.70 g, 23.65 mmol) in DMF (20 mL) were reacted to give the title product (1.00 g, 18 % yield) as a yellow oil.

GC/MS (El): *m*/*z*(%):276 (1)[*M*⁺], 208 (88), 193 (37), 179 (30), 172 (40), 144 (27), 129 (18), 69 (100). ¹H NMR (300 MHz, CDCl₃) δ 9.41 (s, 1H), 7.22 - 7.10 (m, 3H), 6.51 (d, *J* = 9.5 Hz, 1H), 5.03 (t, *J =* 7.1 Hz, 1H), 3.97 (q, *J =* 16.7, 7.5 Hz, 1H), 2.45 (t, *J =* 7.2 Hz, 2H), 2.30 (s, 3H), 1.75 (s, 3H), 1.66 (s, 3H), 1.58 (s, 3H). ¹³C NMR (75 MHz, CDCl₃) δ 195.2 (t), 156.3 (t), 139.5 (q), 139.0 (q), 137.8 (q), 134.4 (q), 132.1 (q), 130.5 (t), 127.9 (t), 126.6 (t), 120.6 (t), 40.7 (t), 34.2 (d), 25.8 (s), 19.6 (s), 18.0 (s), 9.8 (s).

### Example 60: (E)-4-(2,5-dimethylphenyl)-2,7-dimethylocta-2,6-dienal

Following the general procedure described in Example 56: 2-bromo-1,4-dimethylbenzene (7.29 g, 39.4 mmol), (*E*)-2,7-dimethylocta-2,6-dienal (3.00 g, 19.71 mmol), diacetoxypalladium (0.22 g, 0.99 mmol), tricyclohexylphosphane (0.55 g, 1.97 mmol), Cs₂CO₃ (7.70 g, 23.65 mmol) in DMF (20 mL) were reacted to give the title product (1.44 g, 28 % yield) as a yellow oil.

GC/MS (El): *m*/*z* (%): 256 (2) [*M*⁺], 188 (100), 173 (86), 172 (52), 159 (76), 144 (23), 115 (10), 69 (36). ¹H NMR (300 MHz, CDCl₃) δ 9.41 (s, 1H), 7.06 (d, *J =* 8.5 Hz, 2H), 6.96 (d, *J =* 7.5 Hz, 1H), 6.55 (d, *J =* 11.8 Hz, 1H), 5.07 (t, *J =* 7.1 Hz, 1H), 4.08 - 3.94 (m, 1H), 2.47 (t, *J =* 17.3, 10.1 Hz, 2H), 2.34 (s, 3H), 2.29 (s, 3H), 1.78 (s, 3H), 1.67 (s, 3H), 1.61 (s, 3H). ¹³C NMR (75 MHz, CDCl₃) δ 195.4 (t), 157.3 (t), 140.7 (q), 138.5 (q), 135.9 (q), 133.9 (q), 132.5 (q), 130.5 (t), 127.2 (t), 127.0 (t), 121.1 (t), 41.1 (t), 34.3 (d), 25.7 (s), 21.1 (s), 19.2 (s), 17.9 (s), 9.7 (s).

### Example 61: (E)-4-(5-chloro-2-methylphenyl)-2,7-dimethylocta-2,6-dienal

Following the general procedure described in Example 56: 2-bromo-4-chloro-1-methylbenzene (8.10 g, 39.4 mmol), (*E*)-2,7-dimethylocta-2,6-dienal (3.00 g, 19.71 mmol), diacetoxypalladium (0.22 g, 0.99 mmol), tricyclohexylphosphane (0.55 g, 1.97 mmol), Cs₂CO₃ (7.70 g, 23.65 mmol) in DMF (20 mL) were reacted to give the title product (0.62 g, 11% yield) as a yellow oil.

GC/MS (El): *m*/*z* (%): 276 (1) [*M*⁺], 210 (25), 208 (78), 173 (28), 141 (12), 128 (13), 115 (8), 69 (100). ¹H NMR (300 MHz, CDCl₃) δ 9.42 (s, 1H), 7.21 (s, 1H), 7.14 - 7.04 (m, 2H), 6.48 (d, *J =* 11.3 Hz, 1H), 5.03 (t, *J =* 7.2 Hz, 1H), 3.97 (q, *J =* 16.9, 7.4 Hz, 1H), 2.46 (t, *J =* 7.3 Hz, 2H), 2.28 (s, 3H), 1.76 (s, 3H), 1.66 (s, 3H), 1.59 (s, 3H). ¹³C NMR (75 MHz, CDCl₃) δ 195.2 (t), 155.9 (t), 142.8 (q), 139.2 (q), 134.5 (q), 134.2 (q), 132.2 (q), 131.9 (t), 126.6 (t), 120.5 (t), 41.2 (t), 34.2 (d), 25.8 (s), 19.2 (s), 18.0 (s), 9.8 (s).

### Example 62: (E)-4-(4-fluoro-2-methylphenyl)-2,7-dimethylocta-2,6-dienal

Following the general procedure described in Example 56: 1-bromo-4-fluoro-2-methylbenzene (7.45 g, 39.4 mmol), (*E*)-2,7-dimethylocta-2,6-dienal (3.00 g, 19.71 mmol), diacetoxypalladium (0.22 g, 0.99 mmol), tricyclohexylphosphane (0.553 g, 1.971 mmol), Cs₂CO₃ (7.70 g, 23.65 mmol) in DMF (20 mL) were reacted to give the title product (1.03 g, 20 % yield) as a yellow oil.

GC/MS (El): *m*/*z* (%): 260 (1) [*M*⁺], 192 (100), 177 (46), 163 (59), 146 (26), 133 (19), 123 (26), 109 (10), 77 (5), 69 (50). ¹H NMR (300 MHz, CDCl₃) δ 9.41 (s, 1H), 7.24 - 7.16 (m, 1H), 6.94 - 6.82 (m, 2H), 6.51 (dd, *J* = 9.6, 1.0 Hz, 1H), 5.03 (t, *J =* 7.2 Hz, 1H), 3.97 (q, *J =* 16.9, 7.4 Hz, 1H), 2.45 (t, *J =* 7.2 Hz, 2H), 2.31 (s, 3H), 1.75 (s, 3H), 1.65 (s, 3H), 1.57 (s, 3H). ¹³C NMR (75 MHz, CDCl₃) δ 195.3 (t), 162.8 (q), 159.6 (q), 156.7 (t), 138.8 (q), 138.2 (q), 138.1 (q), 136.6 (q), 134.3 (q), 128.0 (t), 127.9 (t), 120.7 (t), 117.3 (t), 117.1 (t), 113.4 (t), 113.1 (t), 40.6 (t), 34.3 (d), 25.8 (s), 19.8 (s), 17.9 (s), 9.7 (s).

### Example 63: (E)-4-(3-chloro-2-methylphenyl)-2,7-dimethylocta-2,6-dienal

Following the general procedure described in Example 56: 1-bromo-3-chloro-2-methylbenzene (5.00 g, 24.33 mmol), (*E*)-2,7-dimethylocta-2,6-dienal (1.85 g, 12.17 mmol), diacetoxypalladium (0.14 g, 0.61 mmol), tricyclohexylphosphane (0.34 g, 1.22 mmol), Cs₂CO₃ (4.76 g, 14.60 mmol) in DMF (20 mL) were reacted to give the title product (0.40 g, 12 % yield) as a yellow oil.

GC/MS (El): *m*/*z* (%): 276 (1) [*M*⁺], 210 (22), 208 (66), 173 (55), 141 (12), 128 (13), 115 (9), 69 (100). ¹H NMR (300 MHz, CDCl₃) δ 9.43 (s, 1H), 7.31 - 7.26 (m, 1H), 7.21 - 7.11 (m, 2H), 6.54 (d, *J* = 9.3 Hz, 1H), 5.05 (t, *J* = 6.5 Hz, 1H), 4.22 - 3.96 (m, 1H), 2.48 (t, *J =* 7.1 Hz, 2H), 2.39 (s, 3H), 1.76 (s, 3H), 1.68 (s, 3H), 1.60 (s, 3H). ¹³C NMR (75 MHz, CDCl₃) δ 195.2 (t), 156.3 (t), 143.1 (q), 139.2 (q), 135.3 (q), 134.5 (q), 133.9 (q), 127.7 (t), 127.1 (t), 125.1 (t), 120.7 (t), 41.9 (t), 34.4 (d), 25.8 (s), 18.0 (s), 16.0 (s), 9.8 (s).

### Example 64: (E)-4-(2-methoxyphenyl)-2,7-dimethylocta-2,6-dienal

Following the general procedure described in Example 56: 1-bromo-2-methoxybenzene (7.37 g, 39.4 mmol), (*E*)-2,7-dimethylocta-2,6-dienal (3.00 g, 19.71 mmol), diacetoxypalladium (0.22 g, 0.99 mmol), tricyclohexylphosphane (0.55 g, 1.97 mmol), Cs₂CO₃ (7.70 g, 23.65 mmol) in DMF (20 mL) were reacted to give the title product (1.90 g, 37 % yield) as a yellow oil.

GC/MS (El): *m*/*z* (%): 258 (3) [*M*⁺], 189 (100), 174 (64), 161 (51), 145 (13), 128 (19), 115 (25), 107 (8), 91 (40), 69 (19). ¹H NMR (300 MHz, CDCl₃) δ 9.41 (s, 1H), 7.26 - 7.16 (m, 2H), 6.99 - 6.83 (m, 2H), 6.61 (dd, *J* = 9.8, 1.1 Hz, 1H), 5.06 (t, *J =* 7.1 Hz, 1H), 4.30-4.13 (m, 1H), 3.83 (s, 3H), 2.60-2.41 (m, 2H), 1.77 (d, *J =* 1.1 Hz, 3H), 1.66 (s, 3H), 1.60 (s, 3H). ¹³C NMR (75 MHz, CDCl₃) δ 195.8 (t), 157.4 (t), 157.1 (q), 138.9 (q), 133.6 (q), 130.7 (q), 128.0 (t), 127.8 (t), 121.5 (t), 120.8 (t), 110.8 (t), 55.4 (s), 39.4 (t), 33.1 (d), 25.8 (s), 18.0 (s), 9.5 (s).

### Example 65: (E)-4-(5-fluoro-2-methylphenyl)-2,7-dimethylocta-2,6-dienal

Following the general procedure described in Example 56: 2-bromo-4-fluoro-1-methylbenzene (7.45 g, 39.40 mmol), (*E*)-2,7-dimethylocta-2,6-dienal (3.00 g, 19.71 mmol), diacetoxypalladium (0.22 g, 0.99 mmol), tricyclohexylphosphane (0.55 g, 1.97 mmol), Cs₂CO₃ (7.70 g, 23.65 mmol) in DMF (20 mL) were reacted to give the title product (1.45 g, 28 % yield) as a yellow oil.

GC/MS (El): *m*/*z* (%): 260 (1) [*M*⁺], 192 (100), 177 (23), 163 (18), 146 (23), 133 (17), 123 (15), 109 (8), 69 (82). ¹H NMR (300 MHz, CDCl₃) δ 9.42 (s, 1H), 7.10 (dd, *J =* 8.1, 6.2 Hz, 1H), 6.96 (dd, *J =* 10.2, 2.6 Hz, 1H), 6.83 (td, *J =* 8.3, 2.7 Hz, 1H), 6.49 (dd, *J =* 9.6, 1.0 Hz, 1H), 5.04 (t, *J =* 7.2 Hz, 1H), 3.99 (dd, *J =* 15.9, 7.7 Hz, 1H), 2.46 (t, *J =* 7.3 Hz, 2H), 2.28 (s, 3H), 1.76 (d, *J =* 1.0 Hz, 3H), 1.66 (s, 3H), 1.59 (s, 3H). ¹³C NMR (75 MHz, CDCl₃) δ 195.2 (t), 163.3 (q), 160.1 (q), 156.0 (t), 142.9 (q), 142.9 (q), 139.1 (q), 134.4 (q), 131.8 (t), 131.7 (t), 131.3 (q), 131.3 (q), 120.6 (t), 113.5 (t), 113.4 (t), 113.2 (t), 113.1 (t), 41.34 (t), 41.3 (t), 34.1 (d), 25.8 (s), 19.0 (s), 17.9 (s), 9.8 (s).

### Example 66: (E)-2,7-dimethyl-4-(o-tolyl)octa-2,6-dienal

Following the general procedure described in Example 56: 1-bromo-2-methylbenzene (4.43 g, 25.9 mmol), (*E*)-2,7-dimethylocta-2,6-dienal (2.15 g, 12.96 mmol), diacetoxypalladium (0.15 g, 0.65 mmol), tricyclohexylphosphine (0.36 g, 1.30 mmol), Cs₂CO₃ (5.07 g, 15.56 mmol) in DMF (20 mL) were reacted to give the title product (1.89 g, 60 % yield) as a light yellow oil.

GC/MS (El): *m*/*z* (%): 242 (1) [*M*⁺], 174 (100), 159 (81), 145 (70), 128 (58), 115 (45), 105 (26), 91 (20), 69 (52). ¹H NMR (300 MHz, CDCl₃) δ 9.40 (s, 1H), 7.25 - 7.09 (m, 4H), 6.54 (d, *J =* 9.7, 1.1 Hz, 1H), 5.04 (t, *J =* 7.2 Hz, 1H), 4.01 (q, *J =* 9.5, 7.4 Hz, 1H), 2.53 - 2.42 (m, 2H), 2.31 (s, 3H), 1.75 (d, *J =* 1.1 Hz, 3H), 1.64 (s, 3H), 1.57 (s, 3H). ¹³C NMR (75 MHz, CDCl₃) δ 195.4 (t), 157.2 (t), 140.9 (q), 138.6 (q), 135.7 (q), 134.0 (q), 130.6 (t), 126.6 (t), 126.4 (t), 121.0 (t), 41.1 (t), 34.2 (d), 25.8 (s), 19.7 (s), 17.9 (s), 9.7 (s).

### Example 67: (E)-4-(2-ethylphenyl)-2,7-dimethylocta-2,6-dienal

Following the general procedure described in Example 56: 1-bromo-2-ethylbenzene (5.00 g, 27.0 mmol), (*E*)-2,7-dimethylocta-2,6-dienal (2.36 g, 13.51 mmol), diacetoxypalladium (0.15 g, 0.68 mmol), tricyclohexylphosphane (0.38 g, 1.35 mmol), Cs₂CO₃ (5.28 g, 16.21 mmol) in DMF (20 mL) were reacted to give the title product (2.01 g, 58 % yield) as a yellow oil. GC/MS (El): *m*/*z* (%): 256 (1)[*M*⁺], 188 (73), 173 (10), 159 (100), 141 (17), 131 (30), 115 (25), 103 (4), 91 (20), 69 (24).

### Example 68: (E)-4-(4-fluoro-2-methoxyphenyl)-2,4,7-trimethylocta-2,6-dienal

Following the general procedure described in Example 56: 1-bromo-4-fluoro-2-methoxybenzene (3.43 g, 16.7 mmol), (*E*)-2,4,7-trimethylocta-2,6-dienal (1.56 g, 8.37 mmol), diacetoxypalladium (0.094 g, 0.42 mmol), tricyclohexylphosphane (0.235 g, 0.84 mmol), Cs₂CO₃ (3.27 g, 10.1 mmol) in DMF (20 mL) were reacted to give the title product (1.86 g, 73 % yield) as a light yellow oil.

GC/MS (EI): *m*/*z* (%): 290 (1) [M⁺], 221 (100), 206 (35), 193 (46), 179 (18), 139 (19), 109 (18), 69 (10).

### Example 69: (E)-2,4,7-trimethyl-4-(3-methylbut-2-en-1-yl)octa-2,6-dienal

Following the general procedure described in Example 1: 2,4,7-trimethylocta-2,6-dienal, 3-methylbut-2-en-1-ol and Et₃N-HCl were reacted in xylene to give the title product as a colorless oil.

GC/MS (El): *m*/*z* (%): 234 (1) [*M*⁺], 166 (82), 151 (9), 137 (22), 123 (56), 108 (61), 95 (58), 81 (21), 69 (100), 55 (17). ¹H NMR (300 MHz, CDCl₃) δ 9.31 (s, 1H), 6.33 (s, 1H), 5.07 (t, *J =* 7.1 Hz, 2H), 2.33 - 2.06 (m, 4H), 1.84 (s, 3H), 1.69 (s, 6H), 1.59 (s, 6H), 1.18 (s, 3H). ¹³C NMR (75 MHz, CDCl₃) δ 196.8 (t), 163.6 (t), 138.6 (q), 134.2 (q), 120.1 (t), 42.5 (q), 39.4 (d), 26.1 (s), 24.4 (s), 18.1 (s), 10.1 (s).

### Example 70: (E)-2,7-dimethyl-4-(3-methylbut-2-en-1-yl)octa-2,6-dienal

Following the general procedure described in Example 1: 2,7-dimethylocta-2,6-dienal, 3-methylbut-2-en-1-ol and Et₃N-HCl were reacted in xylene to give the title product as a colorless oil.

GC/MS (El): *m*/*z* (%): 220 (4) [*M*⁺], 205(3), 177 (3), 152 (55), 137 (7), 109 (38), 95 (20), 82 (44), 77 (10), 69 (100). ¹H NMR (300 MHz, CDCl₃) δ 9.39 (s, 1H), 6.26 (d, *J =* 10.2 Hz, 1H), 5.04 (t, *J =* 9.0, 4.3 Hz, 2H), 2.74 - 2.53 (m, 1H), 2.30 - 2.12 (m, 2H), 2.09 - 1.94 (m, 2H), 1.69 (d, *J =* 14.5 Hz, 9H), 1.58 (s, 6H). ¹³C NMR (75 MHz, CDCl₃) δ 195.7 (t), 159.2 (t), 139.0 (q), 133.5 (q), 121.4 (t), 40.4 (t), 32.8 (d), 25.8 (s), 17.9 (s), 9.7 (s).

### Example 71: 4-ethyl-2-methylhex-2-enal

A solution of 2-ethylbutanal (50 g, 0.5 mol) in Methanol (100 mL) was treated at room temperature with a solution of sodium hydroxide (3.99 g, 100 mmol) in water (5 mL). The resulting solution was cooled to 0°C then treated dropwise over 3 h under vigorous stirring with propionaldehyde (72.5 g, 1.25 mol) and then allowed to warm to r.t. overnight. The resulting mixture was neutralized by addition of acetic acid and concentrated under reduced pressure. The residue was extracted with MTBE (3x 150 mL), the combined extracts washed with brine, dried over MgSO₄ and concentrated under reduced pressure to give an oil which was subjected to Kugelrohr distillation (0.9 mbar, 80°C) to give 4-ethyl-2-methylhex-2-enal (25 g, 36% yield) as a colorless oil.

GC/MS (EI): *m*/*z* (%): 140 (13) [M⁺], 125 (12), 111 (100), 97 (19), 83 (22), 69 (28), 55 (71).

### Example 72: (E)-4,4-diethyl-2,7-dimethylocta-2,6-dienal

Following the general procedure described in Example 1: 4-ethyl-2-methylhex-2-enal, 3-methylbut-2-en-1-ol and Et₃N-HCl were reacted in xylene to give the title product as a colorless oil.

GC/MS (EI): *m*/*z* (%): 208 (1) [M⁺], 140 (100), 125 (20), 111 (79), 95 (22), 81 (19), 69 (66), 55 (31).

### Example 73: (E)-4-ethyl-2,4,7-trimethylocta-2,6-dienal

Following the general procedure described in Example 1: 2,4-dimethylhept-2-enal, 3-methylbut-2-en-1-ol and Et₃N-HCl were reacted in xylene to give the title product as a colorless oil.

GC/MS (EI): *m*/*z* (%): 208 (1) [M⁺], 140 (100), 125 (9), 111 (40), 97 (42), 83 (10), 69 (66), 55 (17).

### Example 74: (E)-4-(3-methylbut-2-en-1-yl)non-2-enal

Following the general procedure described in Example 1: non-2-enal, 3-methylbut-2-en-1-o! and Et₃N-HCl were reacted in xylene to give the title product as a colorless oil.

GC/MS (El): *m*/*z* (%):208 (1) [M⁺], 140 (48), 111 (2), 96 (25), 83 (26), 69 (100), 55 (10).

### Example 75: (E)-4-butyl-7-methylocta-2,6-dienal

Following the general procedure described in Example 1: oct-2-enal, 3-methylbut-2-en-1-o! and Et₃N-HCl were reacted in xylene to give the title product as a colorless oil.

GC/MS (EI): *m*/*z* (%): 194 (2) [M⁺], 126 (67), 95 (6), 83 (34), 69 (17), 55 (10).

### Example 76: (E)-4-ethyl-2,4,7-trimethylocta-2,6-dienal

Following the general procedure described in Example 1: 2,4-dimethylhex-2-enal, 3-methylbut-2-en-1-ol and Et₃N-HCl were reacted in xylene to give the title product as a light yellow oil.

GC/MS (EI): *m*/*z* (%): 194 (1) [M⁺], 165 (2), 126 (100), 111 (22), 109 (22), 97 (53), 69 (54), 55 (25).

### Example 77: (E)-4,7-dimethyl-4-(3-methylbut-2-en-1-yl)octa-2,6-dienal

Following the general procedure described in Example 1: 4,7-dimethylocta-2,6-dienal, 3-methylbut-2-en-1-ol and Et₃N-HCl were reacted in xylene to give the title product as a colorless oil.

GC/MS (EI): *m*/*z* (%): 220 (1) [M⁺], 152 (67), 137 (14), 123 (25),109 (54), 94 (47), 81 (42), 69 (100), 59 (18), 53 (16).

### Example 78: 7-methyl-3-phenyloct-6-enal

A mixture of phenylboronic acid (1.76 g, 14.47 mmol), (E)-7-methylocta-2,6-dienal (1.00 g), and diacetoxypalladium (0.041 g, 0.18 mmol) was stirred in 50 mL toluene to give a red solution. Then tri-phenylphosphane (0.19 g, 0.72 mmol), K₂CO₃ (2.00 g, 14.47 mmol) and Cs₂CO₃ (0.24 g, 0.72 mmol) were added and the mixture stirred at 80°C under Ar atmosphere overnight, while monitoring the reaction by GC and GC-MS. The reaction was then quenched with water, and extracted with MTBE. The combined organic layers were washed with brine, dried over MgSO4, filtered and concentrated to give the crude product as a brown oil, which was purified by distillation (180°C, 0.12 mbar) to give 7-methyl-3-phenyloct-6-enal (1.20 g, 4.27 mmol, 59.0 % yield) as a colorless oil.

GC/MS (EI): *m*/*z* (%): 216 (18) [M⁺], 198 (29), 183 (24),157 (38), 142 (34), 129 (78), 118 (74), 105 (100), 91 (71), 77 (44), 69 (51), 55 (52).

### Example 79: 7-methyl-3-((R)-4-methylcyclohex-3-en-1-yl)oct-6-enal

A mixture of [Carbonyl(hydrido)tris(triphenylphosphane)rhodium(I)] ([RhH(CO)(PPh₃)₃]) (1.40 g, 1.52 mmol), triphenylphosphane (1.28 g, 4.89 mmol) and (R)-1-methyl-4-(6-methylhepta-1,5-dien-2-yl)cyclohex-1-ene (50.00 g, 245.00 mmol) were stirred in a 100mL autoclave and heated to 80°C under 4Mpa pressure of syngas atmosphere for about 36 h until the inside pressure did not decrease any more. The mixture was purified by distillation (0.068 mbar, 175°C) to give (S)-7-methyl-3-((R)-4-methylcyclohex-3-en-1-yl)oct-6-enal (35.00 g, 149.33 mmol) as a colorless oil.

GC/MS (El): *m*/*z(%):* 216 (38) [M⁺], 201 (16), 159 (23), 137 (38),121 (40), 105 (60), 95 (100), 79 (96), 67 (90), 55 (64).

### Example 80: 4-isopentyl-2,4,7-trimethyloct-6-enal

A mixture of Lindlar catalyst (Pd/CaCO3, lead poisoned, 5 wt%, 0.1g), (E)-2,4,7-trimethyl-4-(3-methylbut-2-en-1-yl)octa-2,6-dienal (4.12 g, 17.01 mmol), and and Ethyl acetate (40 mL) was stirred under hydrogen atmosphere at r.t. overnight, and the reaction was monitored by GC and GC-MS. The mixture was filtered through a small pad of MgSO4 and the filtrate was washed with MTBE. The combined filtrates were concentrated in vacuo to give crude product 4-isopentyl-2,4,7-trimethyloct-6-enal as a colorless oil (3.08 g, 12.9 mmol, 76% yield), which was used for next step without further purification.

GC/MS (EI): *m*/*z* (%): 238 (1) [M⁺], 220 (2), 169 (15), 151 (20),109 (30), 95 (100), 81 (28), 69 (98), 55 (49).

### Example 81: 4-methyl-3-(1,3,3,5,7-pentamethyloctahydrobenzofclisoxazol-5-vl)benzonitrile (General Procedure)

### Example 81a: 4-methyl-3-(2,4,7-trimethyl-1-oxooct-6-en-4-yl)benzonitrile

A mixture of lindlar catalyst (Pd/CaCO₃, lead poisoned, 5 wt%,0.2g), (*E*)-4-methyl-3-(2,4,7-trimethyl-1-oxoocta-2,6-dien-4-yl)benzonitrile (10.3 g, 35.4 mmol), and and Ethyl acetate (40 mL) was stirred under hydrogen atmosphere at r.t. and the reaction was monitored by GC and GC-MS until a conversion >85% was observed. The mixture was then filtered through a small pad of MgSO₄ and the filtrate was washed with MTBE. The combined filtrates were concentrated in vacuo to give crude product 4-methyl-3-(2,4,7-trimethyl-1-oxooct-6-en-4-yl)benzonitrile (7.1 g, 24. 9 mmol, 70 % yield), which was used for next step without further purification.

### Example 81b: 4-methyl-3-(1,3,3,5,7-pentamethyloctahydrobenzofclisoxazol-5-vl)benzonitrile

A mixture of *N*-methylhydroxylamine hydrochloride (5.06 g, 60.60 mmol) and K₂CO₃ (4.46 g, 32.30 mmol) was stirred in Toluene (50 mL) under Ar atmosphere at r.t. for 15 minutes, then 4-methyl-3-(2,4,7-trimethyl-1-oxooct-6-en-4-yl)benzonitrile (13.95 g, 40.4 mmol) was added and stirred at r.t. for 5 minutes. The mixture was heated to reflux under Ar atmosphere overnight and the reaction was monitored by GC and GC-MS. During this period, water was collected and removed by using Dean-Stark apparatus. After cooling to room temperature, excess K₂CO₃ was added, and the reaction mixture was filtered and the filter was washed with MTBE. The combined filtrates were concentrated in vacuo to give crude product, which was purified by flash chromatography (Hexane/MTBE = 4:1-1:3) to afford rac-4-methyl-3-((3a*R*,5*R*,7*S*,7a*R*)-1,3,3,5,7-pentamethyloctahydrobenzo[c]isoxazol-5-yl)benzonitrile (2.72 g, 8.52 mmol, 21% yield) as a yellow wax, rac-4-methyl-3-((3aS,5R,7S,7aS)-1,3,3,5,7-pentamethyloctahydrobenzo[c]isoxazol-5-yl)benzonitrile (0.41 g, 1.33 mmol, 21% yield) as a yellow oil and rac-4-methyl-3-((3aS,5R,7R,7aS)-1,3,3,5,7-pentamethyloctahydrobenzo[c]isoxazol-5-yl)benzonitrile (2.52 g, 8.08 mmol, 20% yield) as a yellow oil. The obtained rac-4-methyl-3-((3a*R*,5*R*,7*S*,7a*R*)-1,3,3,5,7-pentamethyloctahydrobenzo[c]isoxazol-5-yl)benzonitrile was further recrystalized from a mixture of ether and CH₂Cl₂ to give rac-4-methyl-3-((3a*R*,5*R*,7*S*,7a*R*)-1,3,3,5,7-pentamethyloctahydrobenzo[c]isoxazol-5-yl)benzonitrile as a white solid.

rac-4-methyl-3-((3a*R*,5*R*,7*S*,7a*R*)-1,3,3,5,7-pentamethyloctahydrobenzo[*c*]isoxazol-5-yl)benzonitrile: GC/MS (EI): *m*/*z* (%): 312 (37) [*M*⁺], 297 (4), 266 (20), 196 (6), 182 (6), 144 (14), 126 (100), 100 (47), 87 (55), 70 (17). ¹H NMR (300 MHz, CDCl₃) δ 7.67 (s, 1H), 7.50 - 7.35 (m, 1H), 7.23 (s, 1H), 2.99 - 2.53 (m, 6H), 2.32 (t, *J =* 10.7 Hz, 1H), 2.19 - 1.85 (m, 4H), 1.69-1.55 (m, 1H), 1.53 - 1.39 (m, 4H), 1.28 (s, 3H), 1.22 - 1.00 (m, 6H). ¹³C NMR (75 MHz, CDCl₃) δ 149.8 (q), 142.3 (q), 134.2 (t), 129.7 (t), 129.6 (t), 119.7 (q), 109.9 (q), 79.5 (q), 77.4 (t), 53.8 (t), 49.0 (s), 45.2 (d), 40.0 (q), 35.9 (d), 33.3 (t), 27.0 (s), 25.1 (s), 24.5 (s), 24.2 (s), 20.4 (s).

rac-4-methyl-3-((3aS,5R,7S,7aS)-1,3,3,5,7-pentamethyloctahydrobenzo[c]isoxazol-5-yl)benzonitrile: GC/MS (EI): *m*/*z* (%): 312 (37) [*M*⁺], 297 (6), 266 (4), 196 (3), 182 (4), 144 (9), 126 (100), 113 (44), 98 (95), 87 (21).

rac-4-methyl-3-((3aS,5R,7R,7aS)-1,3,3,5,7-pentamethyloctahydrobenzo[c]isoxazol-5-yl)benzonitrile: GC/MS (EI): *m*/*z* (%): 312 (58) [*M*⁺], 297 (6), 266 (22), 196 (7), 182 (7), 144 (17), 126 (89), 113 (25), 100 (38), 98 (45), 87 (100), 70 (20).

A sample of rac-4-methyl-3-((3a*R*,5*R*,7*S*,7a*R*)-1,3,3,5,7-pentamethyloctahydrobenzo[c]isoxazol-5-yl)benzonitrile could be further separated by chiral HPLC (Phenomenex Lux Cellulose-1, 4.6x150 mm, 3 um Hexane/Isopropanol, 96:4% v/v, isocratic for 20min) to give the two enantiomerically pure fractions rel-4-methyl-3-((3aR,5R,7S,7aR)-1,3,3,5,7-pentamethyloctahydrobenzo[c]isoxazol-5-yl)benzonitrile (first peak to elute - retention time ca. 9 min) and rel-4-methyl-3-((3aS,5S,7R,7aS)-1,3,3,5,7-pentamethyloctahydrobenzo[c]isoxazol-5-yl)benzonitrile (second peak to elute - retention time ca. 12 min).

In the preparation of similar examples, where *N*-methylhydroxylamine hydrochloride was replaced by *N*-ethylhydroxylamine or *N-*isopropylhydroxylamine, the same procedure was used, whereby the base (K₂CO₃) was omitted and toluene was occasionally substituted for xylenes as reaction solvent where indicated.

### Example 82: rac-(3aR,5R,7S,7aR)-1,3,3,5,7-pentamethyl-5-(o-tolyl)octahydrobenzof clisoxazole

Following the general procedure described in Example 81b: 2,4,7-trimethyl-4-(o-tolyl)oct-6-enal (3.15 g, 10.59 mmol) (obtained from the corresponding α,β-unsaturated aldehyde using the general procedure described in Example 81a), *N-*methylhydroxylamine hydrochloride (1.33 g, 15.88 mmol), K₂CO₃ (1.17 g, 8.47 mmol) in toluene (50 mL) were reacted to give the title product (0.70 g, 23 % yield) as a light yellow oil.

GC/MS (El): *m*/*z* (%): 287 (64) [*M*⁺], 272 (5), 171 (12), 157 (14), 126 (100), 119 (23), 100 (47), 87 (56), 70 (12). ¹H NMR (300 MHz, CDCl₃) δ 7.46 - 7.35 (m, 1H), 7.22 - 7.09 (m, 3H), 2.84 (s, 3H), 2.58 (s, 3H), 2.41 - 2.25 (m, 1H), 2.19 - 2.06 (m, 1H), 2.05 - 1.90 (m, 3H), 1.67 (t, *J=* 12.9 Hz, 1H), 1.59 - 1.49 (m, 1H), 1.46 (s, 3H), 1.28 (s, 3H), 1.18 - 1.01 (m, 6H). ¹³C NMR (75 MHz, CDCl₃) δ 148.3 (q), 136.0 (q), 133.5 (t), 126.2 (t), 126.0 (t), 125.5 (t), 79.5 (q), 77.2 (t), 53.9 (t), 48.9 (s), 45.3 (d), 39.7 (q), 35.9 (d), 33.3 (t), 27.0 (s), 25.1 (s), 24.6 (s), 23.8 (s), 20.4 (s).

### Example 83: rac-(3aR,5R,7S,7aR)-1-isopropyl-3,3,5,7-tetramethyl-5-(o-tolyl)octahydrobenzof clisoxazole

Following the general procedure described in Example 81b: 2,4,7-trimethyl-4-(*o-*tolyl)oct-6-enal (520 mg, 1.51 mmol) (obtained from the corresponding α,β-unsaturated aldehyde using the general procedure described in Example 81a), *N-*isopropylhydroxylamine hydrochloride, K₂CO₃ (170 mg, 1.21 mmol) in toluene (50 mL) were reacted to give the title product (320 mg, 67 % yield) as a light yellow oil.

GC/MS (El): *m*/*z* (%): 315 (44) [*M*⁺], 300 (100), 241 (1), 185 (9), 171 (8), 154 (69), 128 (22), 126 (25), 115 (24), 98 (3). ¹H NMR (300 MHz, CDCl₃) δ 7.40 (d, *J =* 7.7 Hz, 1H), 7.21 - 7.06 (m, 3H), 3.31 - 3.09 (m, 1H), 2.57 (s, 3H), 2.48 - 2.26 (m, 2H), 1.98 (dd, *J =* 10.3, 3.2 Hz, 3H), 1.69 - 1.51 (m, 2H), 1.47 (s, 3H), 1.31 (s, 3H), 1.26 (d, *J* = 6.7 Hz, 3H), 1.11 - 1.01 (m, 8H), 0.99 - 0.92 (m, 1H). ¹³C NMR (75 MHz, CDCl₃) δ 148.4 (q), 136.0 (q), 133.4 (t), 126.1 (t), 125.9 (t), 125.4 (t), 78.0 (q), 68.5 (t), 55.0 (t), 53.0 (t), 45.7 (d), 39.5 (t), 35.9 (d), 33.6 (t), 27.1 (s), 24.6 (s), 24.3 (s), 23.8 (s), 22.6 (s), 20.5 (s),13.4 (s).

### Example 84: rac-(3aR,5R,7S,7aR)-1-ethyl-3,3,5,7-tetramethyl-5-(o-tolyl)octahydrobenzof clisoxazole

Following the general procedure described in Example 81b: 2,4,7-trimethyl-4-(o-tolyl)oct-6-enal (298 mg, 0.81 mmol) (obtained from the corresponding α,β-unsaturated aldehyde using the general procedure described in Example 81a), *N-*ethylhydroxylamine hydrochloride (118 mg, 1.21 mmol), K₂CO₃ (89 mg, 0.65 mmol) in toluene (50 mL) were reacted to give the title product (95 mg, 39 % yield) as a light yellow oil.

GC/MS (El): *m*/*z* (%): 301 (2) [*M*⁺], 300 (8), 286 (77), 185 (10), 171 (12), 132 (100), 117 (20), 105 (20), 91 (15), 68 (9). ¹H NMR (300 MHz, CDCl₃) δ 7.40 (d, *J =* 7.7 Hz, 1H), 7.21 - 7.06 (m, 3H), 3.09 - 2.92 (m, 1H), 2.88 - 2.73 (m, 1H), 2.57 (s, 3H), 2.36 - 2.22 (m, 2H), 2.07 - 1.90 (m, 3H), 1.73 - 1.51 (m, 2H), 1.46 (s, 3H), 1.31 - 1.21 (m, 6H), 1.11 (s, 3H), 1.02 (d, *J* = 6.2 Hz, 3H). ¹³C NMR (75 MHz, CDCl₃) δ 148.3 (q), 136.0 (q), 133.4 (t), 126.1 (t), 126.0 (t),125.5 (t), 79.5 (q), 74.8 (t), 56.5 (d), 53.2 (t), 45.4 (d), 39.7 (q), 36.0 (d), 33.9 (t), 31.0 (s), 26.6 (s), 24.8 (s), 23.8 (s), 20.5 (s), 13.9 (s).

### Example 85: rac-(3aR,5R,7S,7aR)-5-(4-fluoro-2-methylphenyl)-1,3,3,5,7-pentamethyloctahydrobenzofclisoxazole

Following the general procedure described in Example 81b: 4-(4-fluoro-2-methylphenyl)-2,4,7-trimethyloct-6-enal (400 mg, 1.45 mmol) (obtained from the corresponding α,β-unsaturated aldehyde using the general procedure described in Example 81a), *N*-methylhydroxylamine hydrochloride (181 mg, 2.17 mmol), K₂CO₃ (160 mg, 1.16 mmol) in toluene (50 mL) were reacted to give the title product (110 mg, 25 % yield) as a yellow oil.

GC/MS (El): *m*/*z* (%): 305 (100) [*M*⁺], 150 (42), 137 (38), 126 (85), 123 (22), 100 (42), 98 (28), 87 (48). ¹H NMR (300 MHz, CDCl₃) δ 7.49 - 7.21 (m, 1H), 7.01 - 6.65 (m, 2H), 3.27 - 2.67 (m, 3H), 2.60 - 2.51 (m, 2H), 2.31 (s, 1H), 2.21 - 1.81 (m, 4H), 1.65 - 1.38 (m, 5H), 1.36-0.99 (m, 10H). ¹³C NMR (75 MHz, CDCl₃) δ 162.5 (q), 159.2 (q), 144.1 (q), 138.7 (q), 127.3 (t), 119.7 (t), 112.1 (t), 79.4 (q), 73.8 (t), 53.9 (t), 48.9 (s), 45.5 (d), 39.4 (q), 36.1 (d), 33.3 (t), 26.9 (s), 25.0 (s), 24.8 (s), 23.7 (s), 20.3 (s).

From the same reaction, an additional diastereomer was isolated: rac-(3aR,5R,7R,7aR)-5-(4-fluoro-2-methylphenyl)-1,3,3,5,7-pentamethyloctahydrobenzo[c]isoxazole (138 mg, 31% yield) as a yellow oil. GC/MS (El): *m*/*z(%):* 305 (71) [M⁺], 150 (30), 126 (100), 113 (29), 100 (52), 98 (49), 87 (96), 70 (25).

### Example 86: rac-(3aR,5R,7S,7aR)-5-(5-chloro-2-methylphenyl)-1,3,3,5,7-pentamethyloctahydrobenzo[c]isoxazole

Following the general procedure described in Example 81b: 4-(5-chloro-2-methylphenyl)-2,4,7-trimethyloct-6-enal (412 mg, 1.15 mmol) (obtained from the corresponding α,β-unsaturated aldehyde using the general procedure described in Example 81a), *N*-methylhydroxylamine hydrochloride (145 mg, 1.73 mmol), K₂CO₃ (128 mg, 0.92 mmol) in toluene (50 mL) were reacted to give the title product (120 mg, 32 % yield) as a light yellow oil.

GC/MS (El): *m*/*z* (%): 321 (46) [*M*⁺], 306 (3), 275 (2), 205 (4), 191 (5), 153 (26), 126 (100), 100 (44), 87 (56), 70 (12). ¹H NMR (300 MHz, CDCl₃) δ 7.33 (s, 1H), 7.15 - 7.00 (m, 2H), 2.83 (s, 3H), 2.52 (s, 3H), 2.38 - 2.21 (m, 1H), 2.14 - 2.05 (m, 1H), 1.95 (d, *J* = 12.8 Hz, 3H), 1.60 (t, *J =* 12.8 Hz, 1H), 1.48 - 1.39 (m, 4H), 1.29 (s, 3H), 1.17 (s, 3H), 1.09 - 1.01 (m, 3H). ¹³C NMR (75 MHz, CDCl₃) δ 150.2 (q), 134.6 (t), 134.5 (t), 131.7 (q), 126.0 (t), 125.9 (t), 79.5 (q), 77.1 (t), 53.8 (t), 49.1 (s), 45.1 (d), 39.8 (q), 35.7 (d), 33.3 (t), 27.0 (s), 25.0 (s), 24.4 (s), 23.2 (s), 20.4 (s).

### Example 87: rac-3-((3aR,5R,7S,7aR)-1,3,3,5,7-pentamethyloctahydrobenzofclisoxazol-5-yl)benzonitrile

Following the general procedure described in Example 81b: 3-(2,4,7-trimethyl-1-oxooct-6-en-4-yl)benzonitrile (6.00 g, 13.48 mmol) (obtained from the corresponding α,β-unsaturated aldehyde using the general procedure described in Example 81a), *N-*methylhydroxylamine hydrochloride (1.69 g, 20.23 mmol), K₂CO₃ (1.49 g, 10.79 mmol) in toluene (50 mL) were reacted to give the title product (1.20 g, 30 % yield) as a yellow oil.

GC/MS (El): *m*/*z(%):* 298 (36) [*M*⁺], 283 (6), 252 (11), 182 (10),168 (9), 126 (100), 100 (41), 87 (50), 70 (16). ¹H NMR (300 MHz, CDCl₃) δ 7.72 - 7.57 (m, 2H), 7.53 - 7.35 (m, 2H), 2.90 - 2.59 (m, 3H), 2.43 - 2.21 (m, 1H), 2.14 - 1.90 (m, 2H), 1.89 - 1.71 (m, 2H), 1.58 - 1.45 (m, 1H), 1.40 (d, *J =* 12.7 Hz, 1H), 1.34 (s, 3H), 1.25 (s, 3H), 1.19 - 0.96 (m, 6H). ¹³C NMR (75 MHz, CDCl₃) δ 152.5 (q), 129.9 (t), 129.7 (t), 129.2 (t), 129.1 (t), 119.2 (q), 112.4 (q), 79.3 (q), 77.4 (t), 53.7 (t), 48.9 (s), 45.8 (d), 38.8 (q), 36.8 (d), 33.1 (t), 26.9 (s), 26.4 (s), 24.9 (s), 20.1 (s).

### Example 88: rac-(3aR,5R,7S,7aR)-5-(2,5-dimethylphenyl)-1,3,3,5,7-pentamethyloctahydrobenzo[c]isoxazole

Following the general procedure described in Example 81b: 4-(2,5-dimethylphenyl)-2,4,7-trimethyloct-6-enal (500 mg, 1.84 mmol) (obtained from the corresponding α,β-unsaturated aldehyde using the general procedure described in Example 81a), *N-*methylhydroxylamine hydrochloride (230 mg, 2.75 mmol), K₂CO₃ (203 mg, 1.47 mmol) in toluene (50 mL) were reacted to give the title product (168 mg, 30 % yield) as a yellow oil.

GC/MS (El): *m*/*z* (%): 301 (68) [*M*⁺], 286 (3), 255 (2), 171 (20), 126 (100), 119 (19), 100 (38), 87 (47), 70 (11). ¹H NMR (300 MHz, CDCl₃) δ 7.20 (s, 1H), 7.06 (d, *J =* 7.6 Hz, 1H), 6.96 (d, *J =* 7.6 Hz, 1H), 2.91-2.67 (m, 3H), 2.53 (s, 3H), 2.39 - 2.25 (m, 4H), 2.18 - 2.06 (m, 1H), 1.97 (d, *J =* 12.8 Hz, 3H), 1.68 (d, *J =* 12.8 Hz, 1H), 1.59 - 1.48 (m, 1H), 1.45 (s, 3H), 1.34 - 1.22 (m, 4H), 1.18 - 1.13 (m, 2H), 1.06 (d, *J* = 6.0 Hz, 3H). ¹³C NMR (75 MHz, CDCl₃) δ 148.0 (q), 135.2 (q), 133.4 (t), 132.7 (q), 126.7 (t), 126.3 (t), 79.5 (q), 77.2 (t), 53.9 (t), 48.9 (s), 45.3 (d), 39.5 (s), 35.8 (d), 33.3 (t), 27.1 (s), 27.0 (s), 25.0 (s), 24.6 (s), 23.4 (s), 21.4 (s), 20.4 (s).

### Example 89: rac-3-((3aR,5R,7S,7aR)-1-ethyl-3,3,5,7-tetramethyloctahydrobenzo[c]isoxazol-5-yl)-4-methylbenzonitrile

Following the general procedure described in Example 81b: 4-methyl-3-(2,4,7-trimethyl-1-oxooct-6-en-4-yl)benzonitrile (300 mg, 0.79 mmol) (obtained from the corresponding α,β-unsaturated aldehyde using the general procedure described in Example 81a), *N-*ethylhydroxylamine (73 mg, 1.19 mmol) in toluene (50 mL) were reacted to give the title product (50 mg, 19 % yield) as a yellow oil.

GC/MS (El): *m*/*z* (%): 326 (24) [*M*⁺], 311 (23), 266 (14), 196 (5), 182 (5), 140 (100), 114 (37), 101 (38). ¹H NMR (300 MHz, CDCl₃) δ 7.64 (s, 1H), 7.39 (d, *J =* 7.9 Hz, 1H), 7.22 (d, *J* = 7.9 Hz, 1H), 3.07 - 2.91 (m, 1H), 2.87 - 2.72 (m, 1H), 2.59 (s, 3H), 2.33 - 2.22 (m, 2H), 2.04 - 1.87 (m, 3H), 1.62 - 1.51 (m, 1H), 1.45 - 1.37 (m, 4H), 1.30 - 1.21 (m, 6H), 1.18 - 1.07 (m, 3H), 1.02 (d, *J* = 6.1 Hz, 3H). ¹³C NMR (75 MHz, CDCl₃) δ 149.5 (q), 142.1 (q), 133.9 (t), 129.5 (t), 129.4 (t), 119.5 (q), 109.7 (q), 79.3 (q), 74.5 (t), 56.4 (d), 52.8 (t), 45.0 (d), 39.8 (q), 35.8 (d), 33.6 (t), 26.5 (s), 24.6 (s), 24.3 (s), 24.0 (s), 20.3 (s), 13.7 (s).

### Example 90: rac-(3aR,5R,7S,7aR)-5-(5-fluoro-2-methylphenyl)-1,3,3,5,7-pentamethyloctahydrobenzo[c]isoxazole

Following the general procedure described in Example 81b: 4-(5-fluoro-2-methylphenyl)-2,4,7-trimethyloct-6-enal (400 mg, 1.45 mmol) (obtained from the corresponding α,β-unsaturated aldehyde using the general procedure described in Example 81a), *N*-methylhydroxylamine hydrochloride (181 mg, 2.17 mmol), K₂CO₃ (160 mg, 1.16 mmol) in toluene (50 mL) were reacted to give the title product (60 mg, 14 % yield) as a yellow oil.

GC/MS (El): *m*/*z* (%): 305 (71) [*M*⁺], 150 (11), 137 (30), 126 (100), 123 (23), 100 (45), 98 (34), 87 (57). ¹H NMR (300 MHz, CDCl₃) δ 7.16 - 6.96 (m, 2H), 6.79 (td, *J =* 8.1, 2.6 Hz, 1H), 2.93 - 2.64 (m, 3H), 2.51 (s, 3H), 2.36 - 2.21 (m, 1H), 2.09 (t, *J =* 10.3 Hz, 1H), 2.03 - 1.84 (m, 3H), 1.60 (t, *J =* 12.9 Hz, 1H), 1.51 - 1.39 (m, 4H), 1.25 (s, 3H), 1.19 - 0.96 (m, 6H). ¹³C NMR (75 MHz, CDCl₃) δ 162.8 (q), 159.6 (q), 150.5 (q), 150.4 (q), 134.4 (t), 134.3 (t), 131.4 (q), 131.3 (q), 112.9 (t), 112.6 (t), 112.5 (t), 112.2 (t), 79.4 (q), 77.1 (t), 53.8 (t), 48.9 (s), 45.0 (d), 39.7 (q), 35.6 (d), 33.2 (t), 26.9 (s), 25.0 (s), 24.3 (s), 23.0 (s), 20.3 (s).

### Example 91: rac-3-((3aR,5R,7S,7aR)-1-isopropyl-3,3,5,7-tetramethyloctahydrobenzo[c]isoxazol-5-yl)benzonitrile

Following the general procedure described in Example 81b: 3-(2,4,7-trimethyl-1-oxooct-6-en-4-yl)benzonitrile (200 mg, 0.74 mmol) (obtained from the corresponding α,β-unsaturated aldehyde using the general procedure described in Example 81a), *N-*isopropylhydroxylamine (167 mg, 2.23 mmol) in xylene (70 mL) were reacted to give the title product (55 mg, 23 % yield) as a yellow oil.

GC/MS (El): *m*/*z* (%): 326 (24) [*M*⁺], 311 (100), 252 (4), 196 (10), 182 (5), 154 (51), 126 (20), 115 (9). ¹H NMR (300 MHz, CDCl₃) δ 7.69 - 7.58 (m, 2H), 7.52 - 7.36 (m, 2H), 3.27 - 3.10 (m, 1H), 2.46 - 2.25 (m, 2H), 2.06 - 1.91 (m, 1H), 1.86 - 1.72 (m, 2H), 1.54 - 1.39 (m, 2H), 1.34 (s, 3H), 1.30 (s, 3H), 1.23 (s, 3H), 1.10 - 0.98 (m, 9H). ¹³C NMR (75 MHz, CDCl₃) δ 152.7 (q), 130.0 (t), 129.6 (t), 129.2 (t), 129.1 (t), 119.3 (q), 112.4 (q), 77.9 (q), 68.8 (t), 55.0 (t), 52.9 (t), 46.1 (d), 38.7 (q), 36.7 (d), 33.5 (t), 27.2 (s), 26.5 (s), 24.3 (s), 22.6 (s), 20.3 (s), 13.7 (s).

### Example 92: rac-3-((3aR,5R,7S,7aR)-1-isopropyl-3,3,5,7-tetramethyloctahydrobenzo[c]isoxazol-5-yl)-4-methylbenzonitrile

Following the general procedure described in Example 81b: 4-methyl-3-(2,4,7-trimethyl-1-oxooct-6-en-4-yl)benzonitrile (3.534 g, 12.38 mmol) (obtained from the corresponding α,β-unsaturated aldehyde using the general procedure described in Example 81a), *N-*isopropylhydroxylamine (2.79 g, 37.10 mmol) in xylene (70 mL) were reacted to give the title product rac-3-((3a*R*,5*R*,7*S*,7a*R*)-1-isopropyl-3,3,5,7-tetramethyloctahydrobenzo[c]isoxazol-5-yl)-4-methylbenzonitrile (589 mg, 14 % yield) as a white solid.

GC/MS (EI): *m*/*z* (%): 340 (23) [*M*⁺], 325 (89), 266 (7), 196 (5), 182 (5), 154 (100), 126 (37), 115 (30). ¹H NMR (300 MHz, CDCl₃) δ 7.65 (s, 1H), 7.40 (d, *J =* 7.8 Hz, 1H), 7.23 (d, *J =* 7.8 Hz, 1H), 3.35 - 3.05 (m, 1H), 2.62 (s, 3H), 2.48 - 2.22 (m, 2H), 2.05 - 1.85 (m, 3H), 1.61 - 1.37 (m, 5H), 1.31 (s, 3H), 1.25 (d, *J* = 6.6 Hz, 3H), 1.14 - 0.94 (m, 9H). ¹³C NMR (75 MHz, CDCl₃) δ 149.8 (q), 142.2 (q), 134.0 (t), 129.5 (t), 129.4 (t), 119.5 (q), 109.7 (q), 77.9 (q), 68.3 (t), 55.0 (t), 52.8 (t), 45.4 (d), 39.7 (q), 35.7 (d), 33.5 (t), 27.1 (s), 24.2 (s), 24.1 (s), 22.6 (s), 20.4 (s),13.4 (s).

From the same reaction, two additioal isomers were isolated: rac-3-((3aS,5R,7S,7aS)-1-isopropyl-3,3,5,7-tetramethyloctahydrobenzo[c]isoxazol-5-yl)-4-methylbenzonitrile (234 mg, 6 % yield) and rac-3-((3aS,5R,7R,7aS)-1-isopropyl-3,3,5,7-tetramethyloctahydrobenzo[c]isoxazol-5-yl)-4-methylbenzonitrile (765 mg, 18 % yield) as a light yellow oils.

rac-3-((3aS,5R,7S,7aS)-1-isopropyl-3,3,5,7-tetramethyloctahydrobenzo[c]isoxazol-5-yl)-4-methylbenzonitrile: GC/MS (EI): *m*/*z(%):* 340 (11) [*M*⁺], 325 (24), 247 (27), 154 (63), 141 (43), 126 (100), 115 (24), 84(30).

rac-3-((3aS,5R,7R,7aS)-1-isopropyl-3,3,5,7-tetramethyloctahydrobenzo[c]isoxazol-5-yl)-4-methylbenzonitrile: GC/MS (El): *m*/*z* (%): 340 (20) [*M*⁺], 325 (100), 266 (6), 210(9), 154 (52), 141(23), 126 (45), 115 (43).

### Example 93: rac-(3aR,5R,7S,7aR)-5-(2-methoxyphenyl)-1,3,3,5,7-pentamethyloctahydrobenzo[c]isoxazole

Following the general procedure described in Example 81b: 4-(2-methoxyphenyl)-2,4,7-trimethyloct-6-enal (500 mg, 1.82 mmol) (obtained from the corresponding α,β-unsaturated aldehyde using the general procedure described in Example 81a), *N-*methylhydroxylamine hydrochloride (228 mg, 2.73 mmol), K₂CO₃ (201 mg, 1.46 mmol) in toluene (50 mL) were reacted to give the title product (123 mg, 22 % yield) as a yellow oil.

GC/MS (El): *m*/*z(%):* 303 (92) [*M*⁺], 288 (5), 257 (9), 201 (15), 180 (17), 135 (31), 126 (100), 100 (37), 87 (44). ¹H NMR (300 MHz, CDCl₃) δ 7.31 (d, *J* = 7.8 Hz, 1H), 7.25 - 7.16 (m, 1H), 6.99 - 6.83 (m, 2H), 3.84 (s, 3H), 2.93 - 2.61 (m, 3H), 2.40 - 2.26 (m, 1H), 2.17 - 1.84 (m, 4H), 1.73 (t, *J =* 12.7 Hz, 1H), 1.57 (t, *J =* 12.7 Hz, 1H), 1.43 (s, 3H), 1.30 - 1.25 (m, 3H), 1.19 - 1.11 (m, 3H), 1.07 - 0.98 (m, 3H). ¹³C NMR (75 MHz, CDCl₃) δ 158.3 (q), 138.3 (q), 127.3 (t), 126.0 (t), 120.5 (t), 111.9 (t), 79.5 (q), 77.5 (t), 55.0 (s), 53.7 (t), 48.9 (s), 44.1 (d), 38.7 (q), 34.6 (d), 33.0 (t), 26.9 (s), 25.0 (s), 23.6 (s), 20.3 (s).

### Example 94: rac-(3aR,5R,7S,7aR)-1,3,3,5,7-pentamethyl-5-phenyloctahydrobenzo[c]isoxazole

Following the general procedure described in Example 81b: 2,4,7-trimethyl-4-phenyloct-6-enal (2.11 g, 8.04 mmol) (obtained from the corresponding α,β-unsaturated aldehyde using the general procedure described in Example 81a), *N*-methylhydroxylamine hydrochloride (1.01 g, 12.06 mmol), K₂CO₃ (0.89 mg, 6.43 mmol) in toluene (50 mL) were reacted to give the title product (0.78 g, 35 % yield) as a light yellow oil.

GC/MS (El): *m*/*z(%):* 273 (52) [*M*⁺], 258 (5), 227 (3), 171 (11), 180 (9), 157 (15), 126 (100), 100 (40), 87 (44). ¹H NMR (300 MHz, CDCl₃) δ 7.45 - 7.38 (m, 2H), 7.37 - 7.29 (m, 2H), 7.24 - 7.16 (m, 1H), 2.90 - 2.66 (m, 3H), 2.43 - 2.25 (m, 1H), 2.13 - 2.02 (m, 1H), 2.01 - 1.90 (m, 1H), 1.87 - 1.75 (m, 2H), 1.65 - 1.52 (m, 1H), 1.50 - 1.42 (m, 1H), 1.36 (s, 3H), 1.27 (s, 3H), 1.19 - 0.99 (m, 6H). ¹³C NMR (75 MHz, CDCl₃) δ 151.2 (q), 128.3 (t), 125.9 (t), 125.1 (t), 79.4 (q), 77.7 (t), 53.9 (t), 48.9 (s), 46.1 (d), 38.6 (q), 37.0 (d), 33.2 (t), 27.0 (s), 26.6 (s), 25.0 (s), 20.2 (s).

### Example 95: rac-(3aR,5R,7S,7aR)-5-(2,4-dimethylphenyl)-1,3,3,5,7-pentamethyloctahydrobenzo[c]isoxazole

Following the general procedure described in Example 81b: 4-(2,4-dimethylphenyl)-2,4,7-trimethyloct-6-enal (1.02 g, 1.88 mmol) (obtained from the corresponding α,β-unsaturated aldehyde using the general procedure described in Example 81a), *N-*methylhydroxylamine hydrochloride (0.24 g, 2.82 mmol), K₂CO₃ (0.21 g, 1.50 mmol) in toluene (50 mL) were reacted to give the title product (0.15 g, 27 % yield) as a light yellow oil.

GC/MS (El): *m*/*z* (%): 301 (54) [*M*⁺], 286 (6), 255 (3), 185 (12), 171 (14), 126 (100), 119 (23), 100 (42), 87 (58), 70 (12). ¹H NMR (300 MHz, CDCl₃) δ 7.34 - 7.22 (m, 1H), 7.04 - 6.92 (m, 2H), 2.89 - 2.65 (m, 3H), 2.54 (s, 3H), 2.35 - 2.24 (m, 4H), 2.14 - 2.05 (m, 1H), 2.05 - 1.85 (m, 3H), 1.65 (t, *J =* 12.9 Hz, 1H), 1.56 - 1.48 (m, 1H), 1.44 (s, 3H), 1.27 (d, *J =* 10.0 Hz, 3H), 1.18 - 0.96 (m, 6H). ¹³C NMR (75 MHz, CDCl₃) δ 145.3 (q), 135.8 (q), 135.5 (q), 134.3 (t), 126.6 (t), 125.5 (t), 79.5 (q), 77.2 (t), 53.9 (t), 48.9 (s), 45.5 (d), 39.4 (q), 36.0 (d), 33.3 (t), 27.0 (s), 25.0 (s), 24.7 (s), 23.6 (s), 20.5 (s), 20.4 (s).

### Example 96: rac-(3aR,5R,7S,7aR)-1-ethyl-3,3,5,7-tetramethyl-5-phenyloctahydrobenzo[c]isoxazole

Following the general procedure described in Example 81b: 2,4,7-trimethyl-4-phenyloct-6-enal (1.03 g, 2.93 mmol) (obtained from the corresponding α,β-unsaturated aldehyde using the general procedure described in Example 81a), N-ethylhydroxylamine hydrochloride (0.43 g, 4.39 mmol), K₂CO₃ (0.32 g, 2.34 mmol) in toluene (50 mL) were reacted to give the title product (0.34 g, 40 % yield) as a light yellow oil.

GC/MS (El): *m*/*z* (%): 287 (28) [*M*⁺], 272 (94), 171 (19), 157 (24), 140 (87), 129 (20), 118 (100), 105 (37), 91 (51). ¹H NMR (300 MHz, CDCl₃) δ 7.44 - 7.38 (m, 2H), 7.37 - 7.29 (m, 2H), 7.24 - 7.16 (m, 1H), 3.10 - 2.71 (m, 2H), 2.38 - 2.16 (m, 2H), 2.05 - 1.92 (m, 1H), 1.90 - 1.77 (m, 2H), 1.56 (t, *J =* 12.4 Hz, 1H), 1.50 - 1.37 (m, 1H), 1.36 (s, 3H), 1.32 - 1.18 (m, 6H), 1.12 (s, 3H), 1.02 (d, *J* = 6.4 Hz, 3H). ¹³C NMR (75 MHz, CDCl₃) δ 151.3 (q), 128.3 (t), 125.9 (t), 125.1 (t), 79.4 (q), 75.3 (t), 56.5 (d), 53.2 (t), 46.2 (d), 38.6 (q), 37.1 (d), 33.7 (t), 26.7 (s), 26.6 (s), 24.7 (s), 20.4 (s), 13.9 (s).

### Example 97: rac-(3aR,5R,7S,7aR)-5-(3-chloro-2-methylphenyl)-1,3,3,5,7-pentamethyloctahydrobenzo[c]isoxazole

Following the general procedure described in Example 81b: 4-(3-chloro-2-methylphenyl)-2,4,7-trimethyloct-6-enal (289 mg, 0.83 mmol) (obtained from the corresponding α,β-unsaturated aldehyde using the general procedure described in Example 81a), *N*-methylhydroxylamine hydrochloride (104 mg, 1.24 mmol), K₂CO₃ (92 mg, 0.66 mmol) in toluene (50 mL) were reacted to give the title product (121 mg, 45 % yield) as a light yellow oil.

GC/MS (El): *m*/*z* (%): 321 (10) [*M*⁺], 320(21), 306 (7), 234 (31), 195 (22), 180 (21), 166 (100), 126 (53), 115 (13), 96 (15), 68 (16). ¹H NMR (300 MHz, CDCl₃) δ 7.38 - 7.22 (m, 2H), 7.15 - 7.05 (m, 1H), 2.90 - 2.67 (m, 3H), 2.57 (s, 3H), 2.40 - 2.26 (m, 1H), 2.21 - 2.08 (m, 1H), 2.05 - 1.84 (m, 3H), 1.68 (t, *J =* 12.9 Hz, 1H), 1.52 - 1.45 (m, 4H), 1.27 (s, 3H), 1.20 - 1.14 (m, 3H), 1.05 (d, *J* = 6.2 Hz, 3H). ¹³C NMR (75 MHz, CDCl₃) δ 150.2 (q), 137.4 (q), 134.2 (q), 127.5 (t), 126.4 (t), 123.9 (t), 79.4 (q), 76.9 (t), 53.8 (t), 48.9 (s), 45.6 (d), 40.0 (s), 36.3 (d), 33.3 (t), 27.0 (s), 26.9 (s), 25.1 (s), 25.0 (s), 20.3 (s), 20.2 (s).

### Example 98: rac-(3aR,5R,7S,7aR)-5-(2-fluorophenyl)-1,3,3,5,7-pentamethyloctahydrobenzo[c]isoxazole

Following the general procedure described in Example 81b: 4-(2-fluorophenyl)-2,4,7-trimethyloct-6-enal (500 mg, 1.91 mmol) (obtained from the corresponding α,β-unsaturated aldehyde using the general procedure described in Example 81a), *N-*methylhydroxylamine hydrochloride (240 mg, 2.86 mmol), K₂CO₃ (211 mg, 1.53 mmol) in toluene (50 mL) were reacted to give the title product (120 mg, 22 % yield) as a light yellow oil.

GC/MS (El): *m*/*z* (%): 291 (41) [*M*⁺], 276 (7), 136 (35), 126 (100), 123 (29), 109 (41), 98 (35), 87 (40). ¹H NMR (300 MHz, CDCl₃) δ 7.26 (td, *J =* 8.2, 1.5 Hz, 1H), 7.18 - 7.08 (m, 1H), 7.06 - 6.88 (m, 2H), 2.85 - 2.56 (m, 3H), 2.34 - 2.14 (m, 1H), 2.04 (t, *J =* 10.3 Hz, 1H), 1.85 (d, *J =* 12.6 Hz, 3H), 1.59 (t, *J =* 12.6 Hz, 1H), 1.52 - 1.41 (m, 1H), 1.36 (s, 3H), 1.26 - 1.16 (m, 3H), 1.14 - 1.03 (m, 3H), 0.96 (d, *J =* 6.1 Hz, 3H). ¹³C NMR (75 MHz, CDCl₃) δ 163.4 (q), 160.2 (q), 137.3 (q),137.1 (q), 127.9 (t), 127.8 (t), 126.6 (t), 126.5 (t), 124.1 (t), 124.0 (t), 116.8 (t), 116.5 (t), 79.4 (q), 77.4 (t), 53.5 (t), 48.9 (s), 44.5 (d), 44.4 (d), 38.3 (q), 38.2 (q), 35.2 (d), 35.1 (d), 33.0 (t), 26.9 (s), 24.9 (s), 24.2 (s), 24.1 (s), 20.2 (s).

### Example 99: rac-(3aR,5R,7S,7aR)-1,3,3,5,7-pentamethyl-5-(p-tolyl)octahydrobenzo[c]isoxazole

Following the general procedure described in Example 81b: 2,4,7-trimethyl-4-(*p-*tolyl)oct-6-enal (284 mg, 1.01 mmol) (obtained from the corresponding α,β-unsaturated aldehyde using the general procedure described in Example 81a), *N-*methylhydroxylamine hydrochloride (127 mg, 1.52 mmol), K₂CO₃ (112 mg, 0.81 mmol) in toluene (50 mL) were reacted to give the title product (112 mg, 40 % yield) as a light yellow oil.

GC/MS (El): *m*/*z* (%): 287 (1) [*M*⁺], 286(3), 195 (35), 180 (22),171 (4), 132 (100), 126 (28), 117 (14), 105 (13), 98 (12), 91 (10). ¹H NMR (300 MHz, CDCl₃) δ 7.30 (d, *J =* 8.2 Hz, 2H), 7.15 (d, *J =* 8.2 Hz, 2H), 2.89 - 2.65 (m, 3H), 2.39 - 2.26 (m, 4H), 2.08 (t, *J =* 10.3 Hz, 1H), 2.01 - 1.90 (m, 1H), 1.88 - 1.74 (m, 2H), 1.55 (t, *J =* 12.9 Hz, 1H), 1.48 - 1.40 (s, 1H), 1.37 (d, *J =* 12.9 Hz, 3H), 1.27 (s, 3H), 1.16 - 1.01 (m, 6H). ¹³C NMR (75 MHz, CDCl₃) δ 148.4 (q), 135.5 (q), 129.1 (t), 125.0 (t), 79.5 (q), 77.7 (t), 54.0 (t), 49.0 (s), 46.2 (d), 38.3 (q), 37.2 (d), 33.2 (t), 27.0 (s), 26.6 (s), 25.0 (s), 20.9 (s), 20.3 (s).

### Example 100: rac-(3aR,5R,7S,7aR)-1-isopropyl-3,3,5,7-tetramethyl-5-phenyloctahydrobenzo[c]isoxazole

Following the general procedure described in Example 81b: 2,4,7-trimethyl-4-phenyloct-6-enal (258 mg, 0.91 mmol) (obtained from the corresponding α,β-unsaturated aldehyde using the general procedure described in Example 81a), *N-*isopropylhydroxylamine (102 mg, 1.36 mmol) in xylene (50 mL) were reacted to give the title product rac-(3a*R*,5*R*,7*S*,7a*R*)-1-isopropyl-3,3,5,7-tetramethyl-5-phenyloctahydrobenzo[c]isoxazole (92 mg, 34 % yield) as a light yellow oil.

GC/MS (El): *m*/*z* (%): 301 (35) [*M*⁺], 286 (100), 171 (11), 154 (71), 126 (28), 115 (24), 105 (20), 91 (23). ¹H NMR (300 MHz, CDCl₃) δ 7.45 - 7.37 (m, 2H), 7.33 (t, *J =* 7.7 Hz, 2H), 7.24 - 7.16 (m, 1H), 3.32 - 3.08 (m, 1H), 2.48 - 2.26 (m, 2H), 2.05 - 1.90 (m, 1H), 1.87 - 1.72 (m, 2H), 1.62 - 1.41 (m, 2H), 1.36 (s, 3H), 1.31 (s, 3H), 1.26 (d, *J =* 6.7 Hz, 3H), 1.13 - 0.97 (m, 9H). ¹³C NMR (75 MHz, CDCl₃) δ 151.4 (q), 128.3 (t), 125.9 (t), 125.1 (t), 78.0 (q), 69.0 (t), 55.0 (t), 53.1 (t), 46.5 (d), 38.5 (q), 37.0 (d), 33.6 (t), 27.2 (s), 26.7 (s), 24.3 (s), 22.6 (s), 20.4 (s).

From the same reaction, additional isomers were also isolated: rac-(3aS,5R,7S,7aS)-1-isopropyl-3,3,5,7-tetramethyl-5-phenyloctahydrobenzo[c]isoxazole (19 mg, 6 % yield) and rac-(3aS,5R,7R,7aS)-1-isopropyl-3,3,5,7-tetramethyl-5-phenyloctahydrobenzo[c]isoxazole (68 mg, 24 % yield) as light yellow oils.

rac-(3aS,5R,7S,7aS)-1-isopropyl-3,3,5,7-tetramethyl-5-phenyloctahydrobenzo[c]isoxazole: GC/MS (El): *m*/*z* (%): 301 (24) [*M*⁺], 286 (38), 169 (14), 154 (80), 141 (37), 126 (100), 115 (25), 105 (38), 91 (44).

rac-(3aS,5R,7R,7aS)-1-isopropyl-3,3,5,7-tetramethyl-5-phenyloctahydrobenzo[c]isoxazole: GC/MS (El): *m*/*z* (%): 301 (35) [*M*⁺], 286 (100), 171 (11), 154 (71), 126 (28), 115 (24), 105 (20), 91 (23).

### Example 101: rac-(3aR,5R,7S,7aR)-1-isopropyl-5-(2-methoxyphenyl)-3,3,5,7-tetramethyloctahydrobenzo[c]isoxazole

Following the general procedure described in Example 81b: 4-(2-methoxyphenyl)-2,4,7-trimethyloct-6-enal (700 mg, 2.55 mmol) (obtained from the corresponding α,β-unsaturated aldehyde using the general procedure described in Example 81a), propan-2-ol (460 mg, 7.65 mmol) in toluene (50 mL) were reacted to give the title product (183 mg, 22 % yield) as a light yellow oil.

GC/MS (El): *m*/*z* (%): 331 (45) [*M*⁺], 316 (94), 257 (4), 201 (12), 154 (100), 135 (23), 126 (33), 115 (24), 91 (16). ¹H NMR (300 MHz, CDCl₃) δ 7.30 (d, *J =* 7.7 Hz, 1H), 7.25 - 7.16 (m, 1H), 6.97 - 6.87 (m, 2H), 3.84 (s, 3H), 2.49 - 2.26 (m, 2H), 1.98 (d, *J =* 12.1 Hz, 3H), 1.74 - 1.48 (m, 3H), 1.43 (s, 3H), 1.30 (s, 3H), 1.26 (d, *J* = 6.7 Hz, 3H), 1.08 (d, *J =* 7.2 Hz, 6H), 1.01 (d, *J* = 6.2 Hz, 3H). ¹³C NMR (75 MHz, CDCl₃) δ 158.4 (q), 138.6 (q), 127.3 (t), 126.0 (t), 120.6 (t), 112.0 (t), 78.2 (q), 68.9 (t), 55.1 (t), 54.9 (s), 52.8 (t), 44.6 (d), 38.7 (q), 34.7 (d), 27.2 (s), 27.0 (s), 24.3 (s), 23.6 (s), 22.6 (s), 20.5 (s),13.7 (s).

### Example 102: rac-4-((3aR,5R,7S,7aR)-1,3,3,5,7-pentamethyloctahydrobenzofclisoxazol-5-yl)benzonitrile

Following the general procedure described in Example 81b: 4-(2,4,7-trimethyl-1-oxooct-6-en-4-yl)benzonitrile (300 mg, 1.11 mmol) (obtained from the corresponding α,β-unsaturated aldehyde using the general procedure described in Example 81a), N-methylhydroxylamine hydrochloride (140 mg, 1.67 mmol), K₂CO₃ (123 mg, 0.89 mmol) in toluene (50 mL) were reacted to give the title product (75 mg, 23 % yield) as a light yellow oil.

GC/MS (El): *m*/*z* (%): 298 (5) [*M*⁺], 297(20), 283 (6), 240 (13), 195 (21), 180 (18), 143(35), 126 (70), 116 (31), 98 (37), 68 (100). ¹H NMR (300 MHz, CDCl₃) δ 7.60 (d, *J =* 8.4 Hz, 2H), 7.48 (d, *J =* 8.4 Hz, 2H), 2.88 - 2.61 (m, 3H), 2.39 - 2.21 (m, 1H), 2.13 - 2.01 (m, 1H), 1.91 - 1.73 (m, 2H), 1.51 (t, *J =* 12.9 Hz, 1H), 1.41 (d, *J =* 12.2 Hz, 1H), 1.34 (s, 3H), 1.29 - 1.17 (m, 4H), 1.14 (d, *J =* 9.7 Hz, 3H), 1.03 (d, *J =* 6.0 Hz, 3H). ¹³C NMR (75 MHz, CDCl₃) δ 156.5 (q), 132.2 (t), 126.1 (t), 119.0 (q), 109.8 (q), 79.3 (q), 77.3 (t), 53.6 (t), 48.9 (s), 45.6 (d), 39.2 (q), 36.6 (d), 33.1 (t), 26.9 (s), 26.3 (s), 24.9 (s), 20.2 (s).

### Example 103: rac-(3aR,5R,7S,7aR)-5-(3-methoxyphenyl)-1,3,3,5,7-pentamethyloctahydrobenzo[c]isoxazole

Following the general procedure described in Example 81b: 4-(3-methoxyphenyl)-2,4,7-trimethyloct-6-enal (700 mg, 2.55 mmol) (obtained from the corresponding α,β-unsaturated aldehyde using the general procedure described in Example 81a), *N-*methylhydroxylamine hydrochloride (320 mg, 3.83 mmol), K₂CO₃ (282 mg, 2.04 mmol) in toluene (50 mL) were reacted to give the title product (238 mg, 31 % yield) as a yellow oil.

GC/MS (El): *m*/*z* (%): 303 (5) [*M*⁺], 302 (8), 288 (3), 216(20), 195 (28), 180 (18), 148 (100), 126 (38), 98 (12), 96 (13). ¹H NMR (300 MHz, CDCl₃) δ 7.18 (t, *J =* 8.0 Hz, 1H), 6.89 (dd, *J =* 11.6, 5.0 Hz, 2H), 6.66 (dd, *J =* 8.0, 2.2 Hz, 1H), 3.72 (s, 3H), 2.82 - 2.55 (m, 3H), 2.33 - 2.11 (m, 1H), 1.99 (t, *J =* 10.2 Hz, 1H), 1.91 - 1.81 (m, 1H), 1.72 (dd, *J* = 15.2, 7.1 Hz, 2H), 1.48 (t, *J =* 12.9 Hz, 1H), 1.36 (dd, *J=* 20.6, 8.2 Hz, 1H), 1.26 (s, 3H), 1.19 (d, *J =* 8.7 Hz, 3H), 1.12 - 1.02 (m, 3H), 0.99 - 0.90 (m, 3H). ¹³C NMR (75 MHz, CDCl₃) δ 159.5 (q), 153.0 (q), 129.2 (t), 117.5 (t), 112.1 (t), 110.0 (t), 79.3 (q), 77.6 (t), 55.1 (q), 53.8 (t), 48.9 (s), 46.0 (d), 38.6 (q), 36.9 (d), 33.1 (t), 26.9 (s), 26.5 (s), 24.9 (s), 20.2 (s).

### Example 104: rac-(3aR,5R,7S,7aR)-1,3,3,5,7-pentamethyl-5-(naphthalen-2-yl)octahydrobenzo[c]isoxazole

Following the general procedure described in Example 81b: 2,4,7-trimethyl-4-(naphthalen-2-yl)oct-6-enal (500 mg, 1.70 mmol) (obtained from the corresponding α,β-unsaturated aldehyde using the general procedure described in Example 81a), *N-*methylhydroxylamine hydrochloride (213 mg, 2.55 mmol), K₂CO₃ (188 mg, 1.36 mmol) in toluene (50 mL) were reacted to give the title product (160 mg, 29 % yield) as a light yellow oil.

GC/MS (El): *m*/*z* (%): 323 (14) [*M*⁺], 308 (5), 236 (12), 195 (72), 180 (70), 168 (100), 126 (35), 98 (14), 96 (19). ¹H NMR (300 MHz, CDCl₃) δ 7.87 - 7.68 (m, 4H), 7.56 (dd, *J* = 8.7, 1.5 Hz, 1H), 7.49 - 7.37 (m, 2H), 2.93 - 2.63 (m, 3H), 2.37 (dd, *J =* 16.9, 6.5 Hz, 1H), 2.16 - 1.84 (m, 4H), 1.66 (s, 1H), 1.56 (d, *J =* 12.4 Hz, 1H), 1.44 (s, 3H), 1.32 (s, 3H), 1.20 - 1.14 (m, 3H), 1.07 (d, *J =* 5.8 Hz, 3H). ¹³C NMR (75 MHz, CDCl₃) δ 148.5 (q), 133.4 (q), 131.8 (q), 128.0 (t), 127.9 (t), 127.4 (t), 126.0 (t), 125.6 (t), 124.4 (t), 122.8 (t), 79.5 (q), 77.7 (t), 53.6 (t), 48.9 (s), 46.1 (d), 38.7 (q), 37.0 (d), 33.2 (t), 27.0 (s), 26.4 (s), 25.0 (s), 20.3 (s).

### Example 105: rac-(3aR,5R,7S,7aR)-5-(2-ethylphenyl)-1,3,3,5,7-pentamethyloctahydrobenzo[c]isoxazole

Following the general procedure described in Example 81b: 4-(2-ethylphenyl)-2,4,7-trimethyloct-6-enal (500 mg, 1.84 mmol) (obtained from the corresponding α,β-unsaturated aldehyde using the general procedure described in Example 81a), *N-*methylhydroxylamine hydrochloride (230 mg, 2.75 mmol), K₂CO₃ (203 mg, 1.47 mmol) in toluene (50 mL) were reacted to give the title product (146 mg, 26 % yield) as a light yellow oil.

GC/MS (El): *m*/*z* (%): 301 (82) [*M*⁺], 286 (5), 185 (10), 171 (13), 147 (20), 126 (100), 100 (47), 87 (52). ¹H NMR (300 MHz, CDCl₃) δ 7.45 - 7.33 (m, 1H), 7.28 - 7.09 (m, 3H), 3.00 - 2.67 (m, 5H), 2.35 (s, 1H), 2.14 (dd, *J=* 13.8, 6.8 Hz, 1H), 1.94 (ddd, *J =* 23.8, 8.1, 2.6 Hz, 3H), 1.72 (d, *J =* 12.9 Hz, 1H), 1.56 (d, *J =* 12.5 Hz, 1H), 1.47 (s, 3H), 1.29 (t, *J =* 7.4 Hz, 6H), 1.17 (d, *J =* 10.4 Hz, 3H), 1.05 (d, *J =* 6.2 Hz, 3H). ¹³C NMR (75 MHz, CDCl₃) δ 147.4 (q), 142.7 (q), 131.9 (t), 126.3 (t), 125.6 (t), 125.2 (t), 79.4 (q), 77.0 (t), 53.9 (t), 48.8 (s), 46.1 (d), 39.6 (q), 36.6 (d), 33.3 (t), 27.5 (d), 26.9 (s), 25.4 (s), 25.0 (s), 20.3 (s), 17.1 (s).

### Example 106: rac-(3aR,5R,7S,7aR)-5-(benzo[d][1,3]dioxol-5-yl)-1,3,3,5,7-pentamethyloctahydrobenzo[c]isoxazole

Following the general procedure described in Example 81b: 4-(benzo[*d*][1,3]dioxol-5-yl)-2,4,7-trimethyloct-6-enal (259 mg, 0.90 mmol) (obtained from the corresponding α,β-unsaturated aldehyde using the general procedure described in Example 81a), *N-*methylhydroxylamine hydrochloride (113 mg, 1.35 mmol), K₂CO₃ (99 mg, 0.72 mmol) in toluene (50 mL) were reacted to give the title product (87 mg, 31 % yield) as a light yellow oil.

GC/MS (El): *m*/*z* (%): 317 (5) [*M*⁺], 302 (4), 258 (6), 230 (10), 195 (73), 180 (79), 162 (100), 138 (10), 126 (26), 98 (10), 96 (14). ¹H NMR (300 MHz, CDCl₃) δ 6.91 (d, *J =* 1.4 Hz, 1H), 6.84 (dd, *J* = 8.2, 1.6 Hz, 1H), 6.76 (d, *J =* 8.2 Hz, 1H), 5.93 (s, 2H), 2.86 - 2.58 (m, 3H), 2.36 - 2.19 (m, 1H), 2.06 (t, *J =* 10.3 Hz, 1H), 1.99 - 1.85 (m, 1H), 1.76 (t, *J =* 11.3 Hz, 2H), 1.51 (t, *J =* 12.9 Hz, 1H), 1.40 (d, *J =* 12.7 Hz, 1H), 1.32 (d, *J =* 7.6 Hz, 3H), 1.26 (d, *J =* 8.6 Hz, 3H), 1.15 (s, 3H), 1.02 (d, *J =* 6.2 Hz, 3H). ¹³C NMR (75 MHz, CDCl₃) δ 147.7 (q), 145.6 (q), 145.5 (q), 117.8 (t), 107.9 (t), 106.2 (t), 101.0 (d), 79.4 (q), 77.7 (t), 54.0 (t), 49.0 (d), 46.5 (d), 38.6 (q), 37.4 (d), 33.2 (t), 27.0 (d), 26.9 (d), 25.0 (d), 20.3 (d).

### Example 107: rac-(3aR,5R,7S,7aR)-5-(3,5-dimethylphenyl)-1,3,3,5,7-pentamethyloctahydrobenzo[c]isoxazole

Following the general procedure described in Example 81b: 4-(3,5-dimethylphenyl)-2,4,7-trimethyloct-6-enal (311 mg, 1.14 mmol) (obtained from the corresponding α,β-unsaturated aldehyde using the general procedure described in Example 81a), N-methylhydroxylamine hydrochloride (143 mg, 1.71 mmol), K₂CO₃ (126 mg, 0.91 mmol) in toluene (50 mL) were reacted to give the title product (124 mg, 36 % yield) as a light yellow oil.

GC/MS (El): *m*/*z* (%): 301 (97) [*M*⁺], 286 (5), 255 (5), 185 (19), 171 (23), 126 (100), 119 (16), 100 (38), 87 (45), 70 (10). ¹H NMR (300 MHz, CDCl₃) δ 7.01 (s, 2H), 6.87 (s, 1H), 2.95 - 2.57 (m, 3H), 2.44 - 2.22 (m, 7H), 2.08 (t, *J =* 10.3 Hz, 1H), 2.01 - 1.89 (m, 1H), 1.81 (dd, *J =* 14.8, 7.1 Hz, 2H), 1.55 (t, *J =* 12.9 Hz, 1H), 1.47 (d, *J =* 12.1 Hz, 1H), 1.35 (s, 3H), 1.28 (d, *J =* 11.5 Hz, 3H), 1.18 - 1.02 (m, 6H). ¹³C NMR (75 MHz, CDCl₃) δ 151.3 (q), 137.7 (q), 127.6 (t), 122.9 (t), 79.5 (q), 77.7 (t), 54.0 (t), 49.0 (s), 46.1 (d), 38.4 (q), 37.1 (d), 33.2 (t), 27.0 (s), 26.6 (s), 25.0 (s), 21.7 (s), 20.3 (s).

### Example 108: rac-(3aR,5R,7R,7aR)-1-ethyl-3,3,5,7-tetramethyl-5-(o-tolyl)octahydrobenzo[c]isoxazole

Following the general procedure described in Example 81b: 2,4,7-trimethyl-4-(*o-*tolyl)oct-6-enal (300 mg, 0.81 mmol) (obtained from the corresponding α,β-unsaturated aldehyde using the general procedure described in Example 81a), *N-*ethylhydroxylamine hydrochloride (120 mg, 1.21 mmol), K₂CO₃ (90 mg, 0.65 mmol) in toluene (50 mL) were reacted to give the title product (80 mg, 33 % yield) as a light yellow oil.

GC/MS (El): *m*/*z* (%): 301 (27) [*M*⁺], 286 (100), 243 (10), 185 (10), 171 (10), 140 (25), 132 (38), 119 (14), 101 (18), 91 (10). ¹H NMR (300 MHz, CDCl₃) δ 7.17 (d, *J =* 8.7 Hz, 1H), 7.00 (t, *J =* 8.7 Hz, 3H), 2.99 - 2.62 (m, 2H), 2.61 - 2.48 (m, 1H), 2.40 (d, *J =* 12.3 Hz, 3H), 2.24 (dd, *J =* 14.2, 6.1 Hz, 1H), 2.13 - 1.93 (m, 1H), 1.90 - 1.59 (m, 2H), 1.41 - 0.69 (m, 17H). ¹³C NMR (75 MHz, CDCl₃) δ 143.6 (q), 135.6 (q), 133.6 (t), 127.9 (t), 126.2 (t), 126.0 (t), 78.8 (q), 75.8 (t), 56.2 (d), 53.4 (t), 47.7 (d), 41.8 (q), 37.0 (d), 33.8 (t), 30.4 (s), 26.5 (s), 24.5 (s), 24.0 (s), 20.2 (s), 13.7 (s).

### Example 109: rac-(3aR,5R,7S,7aR)-5-(3,5-dimethylphenyl)-1-isopropyl-3,3,5,7-tetramethyloctahydrobenzo[c]isoxazole

Following the general procedure described in Example 81b: 4-(3,5-dimethylphenyl)-2,4,7-trimethyloct-6-enal (260 mg, 0.95 mmol) (obtained from the corresponding α,β-unsaturated aldehyde using the general procedure described in Example 81a), *N-*isopropylhydroxylamine (210 mg, 2.84 mmol), K₂CO₃ (90 mg, 0.65 mmol) in xylene (80 mL) were reacted to give the title product (220 mg, 69 % yield) as a light yellow oil.

GC/MS (El): *m*/*z(%):* 329 (58) [*M*⁺], 314 (100), 199 (8), 185 (10), 171 (11), 154 (88), 126 (28), 115 (22), 112 (18), 91 (9). ¹H NMR (300 MHz, CDCl₃) δ 7.02 (s, 2H), 6.87 (s, 1H), 3.41 -2.90 (m, 1H), 2.48-2.24 (m, 8H), 2.08 - 1.90 (m, 1H), 1.81 (ddd, *J =* 12.2, 5.6, 2.7 Hz, 2H), 1.50 (dt, *J* = 17.9, 12.7 Hz, 2H), 1.39 - 1.22 (m, 9H), 1.08 (dd, *J =* 13.7, 5.8 Hz, 9H). ¹³C NMR (75 MHz, CDCl₃) δ 151.3 (q), 137.5 (q), 127.4 (t), 122.8 (t), 77.9 (q), 68.9 (t), 54.8 (t), 53.0 (t), 46.3 (d), 38.1, 36.9 (d), 33.4 (t), 27.1 (s), 26.6 (s), 24.2 (s), 22.5 (s), 21.5 (s), 20.4 (s), 13.6 (s).

### Example 110: rac-(3aR,5R,7S,7aR)-5-(4-methoxyphenyl)-1,3,3,5,7-pentamethyloctahydrobenzo[c]isoxazole

Following the general procedure described in Example 81b: 4-(4-methoxyphenyl)-2,4,7-trimethyloct-6-enal (500 mg, 1.82 mmol) (obtained from the corresponding α,β-unsaturated aldehyde using the general procedure described in Example 81a), *N-*methylhydroxylamine hydrochloride (230 mg, 2.73 mmol), K₂CO₃ (200 mg, 1.46 mmol) in toluene (50 mL) were reacted to give the title product (130 mg, 24 % yield) as a light yellow oil.

GC/MS (El): *m*/*z* (%): 303 (1) [*M*⁺], 302 (3), 288 (5), 244 (5), 216 (6), 195 (61), 180 (53), 148 (100), 126 (20), 96 (10). ¹H NMR (300 MHz, CDCl₃) δ 7.32 (d, *J =* 8.8 Hz, 2H), 6.87 (d, *J =* 8.8 Hz, 2H), 3.79 (s, 3H), 2.88 - 2.64 (m, 3H), 2.42 - 2.20 (m, 1H), 2.07 (t, *J =* 10.3 Hz, 1H), 2.00 - 1.89 (m, 1H), 1.79 (dd, *J =* 14.9, 6.9 Hz, 2H), 1.55 (t, *J =* 12.9 Hz, 1H), 1.48 - 1.39 (m, 1H), 1.38 - 1.23 (m, 6H), 1.20 - 1.11 (m, 3H), 1.04 (d, *J =* 6.2 Hz, 3H). ¹³C NMR (75 MHz, CDCl₃) δ 157.5 (q), 143.4 (q), 126.0 (t), 113.5 (t), 79.3 (q), 77.6 (t), 55.1 (s), 53.9 (t), 48.9 (s), 46.3 (d), 37.9 (q), 37.2 (d), 33.1 (t), 26.9 (s), 26.9 (s), 26.6 (s), 24.9 (s), 20.2 (s).

### Example 111: rac-(3aR,5R,7S,7aR)-1,3,3,5,7-pentamethyl-5-(naphthalen-1-yl)octahydrobenzo[c]isoxazole

Following the general procedure described in Example 81b: 2,4,7-trimethyl-4-(naphthalen-1-yl)oct-6-enal (500 mg, 1.70 mmol) (obtained from the corresponding α,β-unsaturated aldehyde using the general procedure described in Example 81a), *N-*methylhydroxylamine hydrochloride (210 mg, 2.55 mmol), K₂CO₃ (190 mg, 1.36 mmol) in toluene (50 mL) were reacted to give the title product (170 mg, 31 % yield) as a light yellow oil.

GC/MS (El): *m*/*z* (%): 323 (2) [*M*⁺], 322 (5), 308 (4), 236 (6), 195 (82), 180 (100), 168 (64), 153 (49), 126 (19), 98 (12), 96 (11). ¹H NMR (300 MHz, CDCl₃) δ 8.44 (d, *J =* 8.5 Hz, 1H), 7.90 (dd, *J* = 7.9, 1.3 Hz, 1H), 7.75 (d, *J* = 8.0 Hz, 1H), 7.62 - 7.36 (m, 4H), 3.01 - 2.68 (m, 3H), 2.49 (d, *J =* 10.5 Hz, 1H), 2.32 - 2.12 (m, 3H), 1.92 (t, *J =* 12.9 Hz, 1H), 1.76 - 1.69 (m, 3H), 1.39 - 1.26 (m, 5H), 1.20 (d, *J =* 8.5 Hz, 3H), 1.14 - 1.06 (m, 3H). ¹³C NMR (75 MHz, CDCl₃) δ 146.0 (q), 135.3 (q), 131.1 (q), 130.0 (t), 127.9 (t), 126.4 (t), 125.2 (t), 124.7 (t), 124.7 (t),122.8 (t), 79.4 (q), 77.3 (t), 53.9 (t), 48.8 (s), 46.5 (d), 39.9 (q), 37.1 (d), 33.3 (t), 26.9 (s), 25.7 (s), 24.9 (s), 20.2 (s).

### Example 112: rac-(3aR,5R,7R,7aR)-5-(3-chloro-2-methylphenyl)-1,3,3,5,7-pentamethyloctahydrobenzofclisoxazole

Following the general procedure described in Example 81b: 4-(3-chloro-2-methylphenyl)-2,4,7-trimethyloct-6-enal (290 mg, 0.83 mmol) (obtained from the corresponding α,β-unsaturated aldehyde using the general procedure described in Example 81a), *N*-methylhydroxylamine hydrochloride (100 mg, 1.24 mmol), K₂CO₃ (90 mg, 0.66 mmol) in toluene (50 mL) were reacted to give the title product (100 mg, 39 % yield) as a light yellow oil.

GC/MS (El): *m*/*z* (%): 321 (12) [*M*⁺], 320 (44), 306 (7), 263 (44), 234 (20), 166 (100), 153 (14), 126 (23), 108 (21), 98 (18), 68 (12). ¹H NMR (300 MHz, CDCl₃) δ 7.30 - 7.21 (m, 1H), 7.18 (d, *J=* 7.8 Hz, 1H), 7.05 (t, *J =* 8.0 Hz, 1H), 2.92 - 2.65 (m, 3H), 2.59 - 2.49 (m, 3H), 2.47 - 2.34 (m, 1H), 2.16 (dd, *J =* 12.1, 8.1 Hz, 1H), 1.81 (t, *J =* 11.0 Hz, 2H), 1.36 (d, *J =* 12.8 Hz, 3H), 1.30 (d, *J* = 6.3 Hz, 1H), 1.26 (s, 1H), 1.23 - 1.09 (m, 6H), 1.03 (t, *J =* 7.3 Hz, 4H). ¹³C NMR (75 MHz, CDCl₃) δ 145.7 (q), 137.4 (q), 133.9 (q), 127.5 (t), 126.6 (t), 126.4 (t), 79.0 (q), 78.1 (t), 54.2 (t), 48.7 (s), 48.2 (d), 42.0 (q), 37.2 (d), 33.4 (t), 30.6 (s), 26.8 (s), 24.7 (s), 20.1 (s), 19.9 (s).

### Example 113: rac-(3aR,5R,7S,7aR)-1-isopropyl-5-(4-methoxyphenyl)-3,3,5,7-tetramethyloctahydrobenzo[c]isoxazole

Following the general procedure described in Example 81b: 4-(4-methoxyphenyl)-2,4,7-trimethyloct-6-enal (700 mg, 2.55 mmol) (obtained from the corresponding α,β-unsaturated aldehyde using the general procedure described in Example 81a), *N-*isopropylhydroxylamine (290 mg, 3.83 mmol), K₂CO₃ (280 mg, 2.04 mmol) in toluene (50 mL) were reacted to give the title product (150 mg, 18 % yield) as a light yellow oil.

GC/MS (El): *m*/*z* (%): 331 (50) [*M*⁺], 316 (100), 208 (12), 187 (7), 154 (69), 126 (22), 115 (17). ¹H NMR (300 MHz, CDCl₃) δ 7.24 (d, *J =* 8.8 Hz, 2H), 6.79 (d, *J =* 8.8 Hz, 2H), 3.71 (s, 3H), 3.22 - 2.94 (m, 1H), 2.27 (dd, *J =* 17.7, 5.9 Hz, 2H), 1.95 - 1.77 (m, 1H), 1.75 - 1.62 (m, 2H), 1.39 (dt, *J* = 25.8, 12.7 Hz, 2H), 1.24 (d, *J* = 8.6 Hz, 6H), 1.18 (d, *J* = 6.7 Hz, 3H), 0.97 (dd, *J* = 16.1, 6.3 Hz, 9H). ¹³C NMR (75 MHz, CDCl₃) δ 157.5 (q), 143.6 (q), 126.0 (t), 113.5 (t), 77.9 (q), 68.9 (t), 55.2, 54.9 (t), 53.0 (t), 46.7 (d), 37.8 (q), 37.1 (d), 33.5 (t), 27.1 (s), 26.6 (s), 24.2 (s), 22.5 (s), 20.3 (s), 13.6 (s).

### Example 114: rac-(3aR,5R,7S,7aR)-5-(3-isopropylphenyl)-1,3,3,5,7-pentamethyloctahydrobenzo[c]isoxazole

Following the general procedure described in Example 81b: 4-(3-isopropylphenyl)-2,4,7-trimethyloct-6-enal (500 mg, 1.75 mmol) (obtained from the corresponding α,β-unsaturated aldehyde using the general procedure described in Example 81a), *N-*methylhydroxylamine hydrochloride (220 mg, 2.62 mmol), K₂CO₃ (190 mg, 1.40 mmol) in toluene (50 mL) were reacted to give the title product (130 mg, 24 % yield) as a light yellow oil.

GC/MS (El): *m*/*z(%):* 315 (62) [*M*⁺], 180 (13), 161 (14), 126 (100), 100 (38), 87 (40). ¹H NMR (300 MHz, CDCl₃) δ 7.23 - 7.08 (m, 3H), 7.00 (d, *J* = 6.9 Hz, 1H), 2.91 - 2.78 (m, 1H), 2.77 - 2.57 (m, 3H), 2.34 - 2.17 (m, 1H), 2.08 - 1.67 (m, 4H), 1.49 (t, *J =* 12.9 Hz, 1H), 1.37 (t, *J =* 12.6 Hz, 1H), 1.28 (s, 3H), 1.23 - 1.10 (m, 9H), 1.09 - 0.91 (m, 6H). ¹³C NMR (75 MHz, CDCl₃) δ 151.1 (q), 148.6 (q), 128.1 (t), 123.6 (t), 123.3 (t), 122.5 (t), 79.3 (q), 77.6 (t), 53.9 (t), 48.9 (s), 46.0 (d), 38.5 (q), 37.0 (d), 34.4 (t), 33.1 (t), 26.9 (s), 26.6 (s), 24.9 (s), 24.1 (s), 24.1 (s), 20.2 (s).

### Example 115: rac-(3aR,5R,7R,7aR)-5-(4-methoxyphenyl)-1,3,3,5,7-pentamethyloctahydrobenzo[c]isoxazole

Following the general procedure described in Example 81b: 4-(4-methoxyphenyl)-2,4,7-trimethyloct-6-enal (500 mg, 1.82 mmol) (obtained from the corresponding α,β-unsaturated aldehyde using the general procedure described in Example 81a), *N-*methylhydroxylamine hydrochloride (230 mg, 2.73 mmol), K₂CO₃ (200 mg, 1.46 mmol) in toluene (50 mL) were reacted to give the title product (120 mg, 21 % yield) as a light yellow oil.

GC/MS (El): *m*/*z* (%): 303 (20) [*M*⁺], 302 (39), 288 (5), 245 (13), 216 (42), 195 (10), 180 (19), 148 (100), 126 (20), 98 (15). ¹H NMR (300 MHz, CDCl₃) δ 7.18 (d, *J =* 8.7 Hz, 2H), 6.81 (d, *J =* 8.7 Hz, 2H), 3.74 (s, 3H), 2.74 (s, 3H), 2.33 (d, *J =* 12.7 Hz, 1H), 2.24 - 2.04 (m, 2H), 1.94 (dd, *J* = 17.2, 6.4 Hz, 1H), 1.39 - 1.20 (m, 3H), 1.15 (dd, *J* = 8.3, 4.0 Hz, 9H), 0.97 (d, *J =* 6.4 Hz, 3H). ¹³C NMR (75 MHz, CDCl₃) δ 157.3 (q), 138.8 (q), 126.7 (t), 113.9 (t), 78.9, 78.1 (t), 55.2 (t), 54.0 (s), 48.7 (s), 46.1 (d), 39.4, 36.9 (d), 34.6 (s), 33.1 (t), 26.6 (s), 24.8 (s), 20.1 (s).

### Example 116: rac-4-methoxy-3-((3aR,5R,7S,7aR)-1,3,3,5,7-pentamethyloctahydrobenzo[c]isoxazol-5-yl)benzonitrile

Following the general procedure described in Example 81b: 4-methoxy-3-(2,4,7-trimethyl-1-oxooct-6-en-4-yl)benzonitrile (350 mg, 1.07 mmol) (obtained from the corresponding α,β-unsaturated aldehyde using the general procedure described in Example 81a), *N*-methylhydroxylamine hydrochloride (130 mg, 1.60 mmol), K₂CO₃ (120 mg, 0.85 mmol) in toluene (50 mL) were reacted to give the title product (100 mg, 28 % yield) as a light yellow oil.

GC/MS (El): *m*/*z* (%): 328 (72) [*M*⁺], 282 (28), 241 (26), 226 (67), 160 (39), 126 (100), 100 (41), 87 (47). ¹H NMR (300 MHz, CDCl₃) δ 7.58 - 7.42 (m, 2H), 6.90 (d, *J =* 8.4 Hz, 1H), 3.86 (s, 3H), 2.84 - 2.58 (m, 3H), 2.35 - 2.16 (m, 1H), 2.05 - 1.82 (m, 4H), 1.57 (t, *J =* 12.7 Hz, 1H), 1.49 - 1.28 (m, 5H), 1.27 - 1.18 (m, 3H), 1.12 - 0.94 (m, 6H). ¹³C NMR (75 MHz, CDCl₃) δ 161.0 (q), 139.6 (q), 132.1 (t), 130.2 (t), 119.6 (q), 112.1 (t), 103.8 (q), 79.3 (q), 77.3 (t), 55.5 (s), 53.4 (t), 48.9 (s), 43.7 (d), 39.0 (q), 34.3 (d), 32.9 (t), 26.9 (s), 24.9 (s), 23.1 (s), 20.2 (s).

### Example 117: rac-(3aR,5R,7S,7aR)-5-(3,5-difluorophenyl)-1,3,3,5,7-pentamethyloctahydrobenzo[c]isoxazole

Following the general procedure described in Example 81b: 4-(3,5-difluorophenyl)-2,4,7-trimethyloct-6-enal (550 mg, 0.69 mmol) (obtained from the corresponding α,β-unsaturated aldehyde using the general procedure described in Example 81a), *N-*methylhydroxylamine hydrochloride (90 mg, 1.04 mmol), K₂CO₃ (80 mg, 0.55 mmol) in toluene (50 mL) were reacted to give the title product (90 mg, 40 % yield) as a light yellow oil.

GC/MS (El): *m*/*z* (%): 309 (56) [*M*⁺], 294 (8), 207 (18), 193 (18),179 (18), 141 (20), 126 (100), 113 (18), 100 (46), 87 (56). ¹H NMR (300 MHz, CDCl₃) δ 6.88 (d, *J =* 7.7 Hz, 2H), 6.63 (tt, *J =* 8.7, 2.2 Hz, 1H), 2.92 - 2.58 (m, 3H), 2.40 - 2.19 (m, 1H), 2.12 - 1.86 (m, 2H), 1.85 - 1.63 (m, 2H), 1.55 - 1.39 (m, 2H), 1.34 - 1.25 (m, 6H), 1.18 - 1.12 (m, 3H), 1.08 - 1.00 (m, 3H). ¹³C NMR (75 MHz, CDCl₃) δ 164.8 (q), 164.6 (q), 161.5 (q), 161.4 (q), 155.6 (q), 108.5 (t), 108.2 (t), 101.7 (t), 101.3 (t), 101.0 (t), 79.3 (q),77.5 (t), 53.8 (t), 49.0 (s), 45.9 (d), 39.1 (q), 36.9 (d), 33.2 (t), 27.1 (s), 27.0 (s), 26.5 (s), 25.0 (s), 20.2 (s).

### Example 118: rac-2-chloro-5-((3aR,5R,7S,7aR)-1,3,3,5,7-pentamethyloctahydrobenzo[c]isoxazol-5-yl)benzonitrile

Following the general procedure described in Example 81b: 2-chloro-5-(2,4,7-trimethyl-1-oxooct-6-en-4-yl)benzonitrile (400 mg, 1.32 mmol) (obtained from the corresponding α,β-unsaturated aldehyde using the general procedure described in Example 81a), *N-*methylhydroxylamine hydrochloride (170 mg, 1.98 mmol), K₂CO₃ (150 mg, 1.05 mmol) in toluene (50 mL) were reacted to give the title product (80 mg, 18 % yield) as a light yellow oil.

GC/MS (El): *m*/*z* (%): 332 (23) [*M*⁺], 286 (9), 244 (5), 230 (8),164 (13), 150 (11), 126 (100), 113 (16), 100 (46),87 (55). Or GC/MS (EI): *m*/*z* (%): 331 (16) [*M*⁺], 274 (19), 195 (20), 177 (34),150 (15), 126 (88), 98 (300), 68 (100). ¹H NMR (300 MHz, CDCl₃) δ 7.65 (s, 1H), 7.59 - 7.50 (m, 1H), 7.47 - 7.39 (m, 1H), 2.88 - 2.57 (m, 3H), 2.28 (t, *J =* 11.7 Hz, 1H), 2.10 - 1.69 (m, 4H), 1.54 - 1.38 (m, 2H), 1.32 (s, 3H), 1.26 (s, 3H), 1.12 (s, 3H), 1.03 (s, 3H). ¹³C NMR (75 MHz, CDCl₃) δ 150.9 (q), 134.2 (q), 131.2 (t), 130.9 (t), 129.8 (t), 116.3 (q), 113.1 (q), 79.2 (q), 77.3 (t), 53.6 (t), 48.9 (s), 45.7 (d), 38.7 (q), 36.8 (d), 33.1 (t), 26.9 (s), 26.4 (s), 24.9 (s), 20.1 (s).

### Example 119: rac-3-chloro-5-((3aR,5R,7S,7aR)-1,3,3,5,7-pentamethyloctahydrobenzo[c]isoxazol-5-yl)benzonitrile

Following the general procedure described in Example 81b: 3-chloro-5-(2,4,7-trimethyl-1-oxooct-6-en-4-yl)benzonitrile (200 mg, 0.66 mmol) (obtained from the corresponding α,β-unsaturated aldehyde using the general procedure described in Example 81a), *N-*methylhydroxylamine hydrochloride (82 mg, 0.99 mmol), K₂CO₃ (73 mg, 0.53 mmol) in toluene (50 mL) were reacted to give the title product (36 mg, 16 % yield) as a light yellow oil.

GC/MS (El): *m*/*z* (%): 334 (16) [*M*⁺], 332 (48) [*M*⁺], 286 (24), 244 (20), 230 (33), 126 (100), 100 (49), 98 (36), 87 (66), 70 (22). ¹H NMR (300 MHz, CDCl₃) δ 7.56 (d, *J =* 7.9 Hz, 2H), 7.48 (s, 1H), 2.88 - 2.63 (m, 3H), 2.41 - 2.23 (m, 1H), 2.13 - 1.92 (m, 2H), 1.86 - 1.72 (m, 2H), 1.55 - 1.41 (m, 2H), 1.34 (s, 3H), 1.28 (s, 3H), 1.15 (s, 3H), 1.05 (d, *J =* 6.1 Hz, 3H). ¹³C NMR (75 MHz, CDCl₃) δ 154.7 (q), 135.3 (q), 130.6 (t), 129.4 (t), 127.5 (t), 117.9 (q), 113.9 (q), 79.3 (q), 77.3 (t), 53.6 (t), 49.0 (s), 45.7 (d), 39.1 (q), 36.8 (d), 33.1 (s), 26.9 (s), 26.4 (s), 25.0 (s), 20.1 (s).

### Example 120: rac-(3aR,5R,7S,7aR)-5-(3,4-difluorophenyl)-1,3,3,5,7-pentamethyloctahydrobenzo[c]isoxazole

Following the general procedure described in Example 81b: 4-(3,4-difluorophenyl)-2,4,7-trimethyloct-6-enal (854 mg, 2.13 mmol) (obtained from the corresponding α,β-unsaturated aldehyde using the general procedure described in Example 81a), *N-*methylhydroxylamine hydrochloride (267 mg, 3.20 mmol), K₂CO₃ (236 mg, 1.71 mmol) in toluene (50 mL) were reacted to give the title product (189 mg, 29 % yield) as a yellow oil.

GC/MS (El): *m*/*z* (%): 309 (40) [*M*⁺], 294 (5), 207 (14), 193 (10),179 (8), 141 (28), 126 (100), 113 (15), 100 (45), 87 (54). ¹H NMR (300 MHz, CDCl₃) δ 7.22 - 7.13 (m, 1H), 7.12 - 7.05 (m, 2H), 2.88 - 2.63 (m, 3H), 2.37 - 2.22 (m, 1H), 2.12 - 1.86 (m, 2H), 1.84 - 1.69 (m, 2H), 1.57 - 1.39 (m, 2H), 1.34 - 1.25 (m, 6H), 1.19 - 1.11 (m, 3H), 1.08 - 1.01 (m, 3H). ¹³C NMR (75 MHz, CDCl₃) δ 151.8 (q), 150.0 (q), 148.5 (q), 121.1 (t), 121.0 (t), 121.0 (t), 120.9 (t), 116.9 (t), 116.7 (t), 114.6 (t), 114.4 (t), 79.3 (q), 77.5 (t), 53.8 (t), 49.0 (s), 46.2 (d), 38.5 (q), 37.1 (d), 33.2 (t), 27.0 (s), 26.7 (s), 25.0 (s), 20.2 (s).

### Example 121: rac-2-methyl-5-((3aR,5R,7S,7aR)-1,3,3,5,7-pentamethyloctahydrobenzofclisoxazol-5-yl)benzonitrile

Following the general procedure described in Example 81b: 2-methyl-5-(2,4,7-trimethyl-1-oxooct-6-en-4-yl)benzonitrile (300 mg, 1.06 mmol) (obtained from the corresponding α,β-unsaturated aldehyde using the general procedure described in Example 81a), N-methylhydroxylamine hydrochloride (133 mg, 1.59 mmol), K₂CO₃ (117 mg, 0.85 mmol) in toluene (50 mL) were reacted to give the title product (135 mg, 41 % yield) as a light yellow oil.

GC/MS (El): *m*/*z* (%): 312 (73) [*M*⁺], 266 (33), 207 (34), 157 (27), 144 (32), 126 (100), 100 (45), 98 (33), 87 (55), 70 (19). ¹H NMR (300 MHz, CDCl₃) δ 7.60 (s, 1H), 7.52 (d, *J* = 8.1 Hz, 1H), 7.27 (d, *J* = 8.3 Hz, 1H), 2.90 - 2.64 (m, 3H), 2.51 (s, 3H), 2.31 (t, *J* = 10.7 Hz, 1H), 2.14 - 1.89 (m, 2H), 1.88 - 1.70 (m, 2H), 1.58 - 1.40 (m, 2H), 1.31 (d, *J* = 15.1 Hz, 6H), 1.16 (d, *J =* 8.5 Hz, 3H), 1.05 (d, *J =* 5.9 Hz, 3H). ¹³C NMR (75 MHz, CDCl₃) δ 149.6 (q), 139.3 (q), 130.2 (t), 129.8 (t), 129.2 (t), 118.5 (q), 112.6 (q), 79.2 (q), 77.4 (t), 53.7 (t), 49.4 (q), 48.9 (s), 45.9 (d), 38.4 (q), 36.8 (d), 33.1 (t), 27.0 (s), 26.9 (s), 26.4 (s), 24.9 (s), 20.1 (s), 19.8 (s).

### Example 122: rac-methyl 4-methyl-3-((3aR,5R,7S,7aR)-1,3,3,5,7-pentamethyloctahydrobenzofclisoxazol-5-yl)benzoate

Following the general procedure described in Example 81b: methyl (*E*)-4-methyl-3-(2,4,7-trimethyl-1-oxoocta-2,6-dien-4-yl)benzoate (1.40 g, 2.40 mmol) (obtained from the corresponding α,β-unsaturated aldehyde using the general procedure described in Example 81a), *N*-methylhydroxylamine hydrochloride (0.30 g, 3.61 mmol), K₂CO₃ (0.27 g, 1.92 mmol) in toluene (50 mL) were reacted to give the title product (0.25 g, 30 % yield) as a light yellow oil.

GC/MS (El): *m*/*z* (%): 345 (68) [*M*⁺], 267 (47), 225 (29), 190 (30), 126 (100), 100 (47), 98 (32), 87 (68), 70 (17). ¹H NMR (300 MHz, CDCl₃) δ 8.02 (s, 1H), 7.70 (d, *J =* 7.8 Hz, 1H), 7.13 (d, *J =* 7.9 Hz, 1H), 3.82 (s, 3H), 2.81 -2.58 (m, 3H), 2.54 (s, 3H), 2.31 - 2.16 (m, 1H), 2.10 - 1.85 (m, 4H), 1.64 - 1.44 (m, 2H), 1.39 (s, 3H), 1.21 (s, 3H), 1.09 (d, *J* = 5.6 Hz, 3H), 0.99 (d, *J =* 5.8 Hz, 3H). ¹³C NMR (75 MHz, CDCl₃) δ 167.2 (q), 148.3 (q), 141.6 (q), 133.3 (t), 127.5 (t), 127.0 (t), 126.7 (t), 79.2 (q), 76.9 (t), 53.6 (t), 51.8 (s), 48.7 (s), 45.0 (d), 39.6 (q), 35.6 (d), 33.1 (t), 26.8 (s), 26.7 (s), 24.8 (s), 24.3 (s), 23.7 (s), 20.1 (s).

### Example 123: rac-(3aR,5R,7S,7aR)-1,3,3,5,7-pentamethyl-5-(2-(methylthio)phenyl)octahydrobenzo[c]isoxazole

Following the general procedure described in Example 81b: 2,4,7-trimethyl-4-(2-(methylthio)phenyl)oct-6-enal (792 mg, 0.76 mmol) (obtained from the corresponding α,β-unsaturated aldehyde using the general procedure described in Example 81a), *N-*methylhydroxylamine hydrochloride (96 mg, 1.15 mmol), K₂CO₃ (84 mg, 0.61 mmol) in toluene (50 mL) were reacted to give the title product (80 mg, 33 % yield) as a light yellow oil.

GC/MS (El): *m*/*z* (%): 319 (46) [*M*⁺], 304 (9), 273 (13), 217 (8), 180 (11), 149 (35), 126 (100), 113 (16), 100 (36), 87 (56). ¹H NMR (300 MHz, CDCl₃) δ 7.47 - 7.32 (m, 2H), 7.24 - 7.10 (m, 2H), 2.92 - 2.71 (m, 3H), 2.50 (s, 3H), 2.36 - 2.24 (m, 2H), 2.08 - 1.91 (m, 4H), 1.90 - 1.77 (m, 1H), 1.56 (s, 3H), 1.27 (s, 3H), 1.17 (d, *J* = 4.9 Hz, 3H), 1.03 (d, *J =* 5.9 Hz, 3H). ¹³C NMR (75 MHz, CDCl₃) δ 148.7 (q), 137.1 (q), 130.4 (t), 126.8 (t), 126.1 (t), 125.7 (t), 79.6 (q), 76.5 (t), 54.0 (t), 48.9 (s), 44.4 (d), 40.4 (q), 34.7 (d), 33.5 (t), 27.0 (s), 27.0 (s), 25.0 (s), 24.3 (s), 20.3 (s), 18.8 (s).

### Example 124: rac-(3aR,5R,7S,7aR)-5-(2,5-difluorophenyl)-1,3,3,5,7-pentamethyloctahydrobenzo[c]isoxazole

Following the general procedure described in Example 81b: 4-(2,5-difluorophenyl)-2,4,7-trimethyloct-6-enal (614 mg, 0.88 mmol) (obtained from the corresponding α,β-unsaturated aldehyde using the general procedure described in Example 81a), N-methylhydroxylamine hydrochloride (110 mg, 1.31 mmol), K₂CO₃ (97 mg, 0.70 mmol) in toluene (50 mL) were reacted to give the title product (108 mg, 38 % yield) as a light yellow oil.

GC/MS (El): *m*/*z(%):* 309 (25) [*M*⁺], 294 (4), 263 (8), 207 (11), 153 (10), 141 (23), 126 (100), 113 (16), 100 (46), 87 (64). ¹H NMR (300 MHz, CDCl₃) δ 7.14 - 6.78 (m, 3H), 2.88 - 2.61 (m, 3H), 2.41 - 2.22 (m, 1H), 2.15 - 1.79 (m, 4H), 1.69 - 1.46 (m, 2H), 1.41 (s, 3H), 1.30 - 1.23 (m, 3H), 1.19 - 1.10 (m, 3H), 1.08 - 0.99 (m, 3H). ¹³C NMR (75 MHz, CDCl₃) δ 160.3 (q), 159.3 (q), 157.1 (q), 117.8 (t), 117.6 (t), 117.4 (t), 117.3 (t), 113.9 (t), 113.8 (t), 113.6 (t), 113.6 (t), 113.5 (t), 79.4 (q), 77.4 (t), 53.5 (t), 49.0 (s), 44.3 (d), 44.2 (d), 38.6 (d), 38.5 (q), 35.1 (d), 35.0 (d), 33.1 (t), 27.0 (s), 25.0 (s), 24.0 (s), 23.9 (s), 20.2 (s).

### Example 125: rac-(3aR,5R,7S,7aR)-5-(2,3-dimethylphenyl)-1,3,3,5,7-pentamethyloctahydrobenzo[c]isoxazole

Following the general procedure described in Example 81b: 4-(2,3-dimethylphenyl)-2,4,7-trimethyloct-6-enal (420 mg, 1.54 mmol) (obtained from the corresponding α,β-unsaturated aldehyde using the general procedure described in Example 81a), N-methylhydroxylamine hydrochloride (193 mg, 2.31 mmol), K₂CO₃ (193 mg, 2.31 mmol) in toluene (50 mL) were reacted to give the title product (89 mg, 19 % yield) as a light yellow oil.

GC/MS (El): *m*/*z* (%): 301 (100) [*M*⁺], 180 (22), 171 (28), 146 (37), 126 (93), 100 (48), 98 (34), 87 (69), 70 (17). ¹H NMR (300 MHz, CDCl₃) δ 7.33 - 7.27 (m, 1H), 7.11 - 7.06 (m, 2H), 2.89 - 2.65 (m, 3H), 2.46 - 2.40 (m, 3H), 2.29 (s, 3H), 2.21 - 2.10 (m, 1H), 2.08 - 1.89 (m, 4H), 1.78 - 1.66 (m, 1H), 1.63 - 1.54 (m, 1H), 1.51 (s, 3H), 1.33 - 1.27 (m, 3H), 1.16 (s, 3H), 1.08 - 1.03 (m, 3H). ¹³C NMR (75 MHz, CDCl₃) δ 148.0 (q), 138.9 (q), 134.9 (q), 128.3 (t), 125.4 (t), 123.3 (t), 79.5 (q), 77.1 (t), 54.0 (t), 48.9 (s), 45.9 (d), 39.5 (q), 36.5 (d), 33.4 (t), 27.0 (s), 25.3 (s), 25.0 (s), 21.6 (s), 20.4 (s), 19.3 (s).

### Example 126: rac-(3aR,5R,7S,7aR)-1,3,3,5,7-pentamethyl-5-(2,4,5-trimethylphenyl)octahvdrobenzo[c]isoxazole

Following the general procedure described in Example 81b: 2,4,7-trimethyl-4-(2,4,5-trimethylphenyl)oct-6-enal (458 mg, 1.47 mmol) (obtained from the corresponding α,β-unsaturated aldehyde using the general procedure described in Example 81a), N-methylhydroxylamine hydrochloride (184 mg, 2.21 mmol), K₂CO₃ (163 mg, 1.18 mmol) in toluene (50 mL) were reacted to give the title product (180 mg, 39 % yield) as a light yellow oil.

GC/MS (El): *m*/*z* (%): 315 (53) [*M*⁺], 199 (9), 180 (24), 160 (15), 149 (36), 133 (23), 126 (100), 113 (16), 100 (41), 87 (59). ¹H NMR (300 MHz, CDCl₃) δ 7.15 (s, 1H), 6.95 (s, 1H), 2.91 - 2.65 (m, 3H), 2.51 (s, 3H), 2.38 - 2.29 (m, 1H), 2.23 (d, *J=* 13.2 Hz, 6H), 2.15 - 2.05 (m, 1H), 2.03 - 1.82 (m, 3H), 1.72 - 1.48 (m, 2H), 1.45 (s, 3H), 1.28 (s, 3H), 1.20 (s, 1H), 1.15 (s, 2H), 1.06 (d, *J* = 6.0 Hz, 3H). ¹³C NMR (75 MHz, CDCl₃) δ 145.7 (q), 135.0 (t), 134.2 (q), 133.7 (q), 133.1 (q), 126.9 (t), 79.5 (q), 77.3 (t), 54.0 (t), 49.0 (s), 45.6 (d), 39.3 (q), 36.0 (d), 33.3 (t), 27.0 (s), 25.1 (s), 24.8 (s), 23.2 (s), 20.4 (s), 19.6 (s), 18.9 (s).

### Example 127: rac-(3aR,5R,7S,7aR)-1,3,3,5,7-pentamethyl-5-(4-(methylthio)phenyl)octahvdrobenzo[c]isoxazole

Following the general procedure described in Example 81b: 2,4,7-trimethyl-4-(4-(methylthio)phenyl)oct-6-enal (350 mg, 1.21 mmol) (obtained from the corresponding α,β-unsaturated aldehyde using the general procedure described in Example 81a), N-methylhydroxylamine hydrochloride (151 mg, 1.81 mmol), K₂CO₃ (133 mg, 0.96 mmol) in toluene (50 mL) were reacted to give the title product (68 mg, 18 % yield) as a yellow oil.

GC/MS (El): *m*/*z* (%): 319 (99) [*M*⁺], 195 (32), 180 (76), 165 (34), 164 (42), 137 (29), 126 (100), 100 (38), 98 (33), 87 (54). ¹H NMR (300 MHz, CDCl₃) δ 7.32 (d, *J =* 8.5 Hz, 2H), 7.22 (d, *J =* 8.5 Hz, 2H), 2.88 - 2.63 (m, 3H), 2.46 (s, 3H), 2.39 - 2.23 (m, 1H), 2.12 - 1.87 (m, 2H), 1.86 - 1.72 (m, 2H), 1.60 - 1.46 (m, 1H), 1.42 (d, *J =* 12.3 Hz, 1H), 1.33 (s, 3H), 1.26 (s, 3H), 1.13 (s, 3H), 1.03 (d, *J* = 6.1 Hz, 3H). ¹³C NMR (75 MHz, CDCl₃) δ 148.4 (q), 135.6 (q), 126.9 (t), 125.7 (t), 79.4 (q), 77.6 (t), 53.9 (t), 48.9 (s), 46.1 (d), 38.3 (q), 37.0 (d), 33.1 (t), 27.0 (s), 26.5 (s), 25.0 (s), 20.2 (s), 16.1 (s).

### Example 128: rac-4-methoxy-3-((3aR,5R,7R,7aR)-1,3,3,5,7-pentamethyloctahydrobenzo[c]isoxazol-5-yl)benzonitrile

Following the general procedure described in Example 81b: 4-methoxy-3-(2,4,7-trimethyl-1-oxooct-6-en-4-yl)benzonitrile (351 mg, 1.07 mmol) (obtained from the corresponding α,β-unsaturated aldehyde using the general procedure described in Example 81a), *N*-methylhydroxylamine hydrochloride (134 mg, 1.60 mmol), K₂CO₃ (118 mg, 0.85 mmol) in toluene (50 mL) were reacted to give the title product (84 mg, 24 % yield) as a light yellow oil.

GC/MS (El): *m*/*z* (%): 328 (100) [*M*⁺], 282 (33), 241 (37), 226 (87), 160 (45), 126 (98), 100 (38), 87 (80). ¹H NMR (300 MHz, CDCl₃) δ 7.59 - 7.35 (m, 2H), 6.91 (d, *J=* 8.1 Hz, 1H), 3.87 (s, 3H), 2.70 (s, 4H), 2.47 - 2.29 (m, 1H), 2.20 - 2.05 (m, 1H), 1.85 - 1.62 (m, 2H), 1.34 - 0.95 (m, 14H). ¹³C NMR (75 MHz, CDCl₃) δ 161.7 (q), 135.4 (q), 132.9 (t), 132.1 (t), 119.5 (q), 112.4 (t), 104.1 (q), 78.9 (q), 78.1 (t), 55.4 (s), 54.7 (t), 48.7 (s), 46.5 (d), 40.9 (q), 35.6 (d), 33.4 (t), 29.5 (s), 26.6 (s), 24.7 (s), 20.2 (s).

### Example 129: rac-(3aR,5R,7S,7aR)-5-(3-fluoro-5-(trifluoromethyl)phenyl)-1,3,3,5,7-pentamethyloctahydrobenzo[c]isoxazole

Following the general procedure described in Example 81b: 4-(3-fluoro-5-(trifluoromethyl)phenyl)-2,4,7-trimethyloct-6-enal (400 mg, 1.21 mmol) (obtained from the corresponding α,β-unsaturated aldehyde using the general procedure described in Example 81a), *N*-methylhydroxylamine hydrochloride (152 mg, 1.82 mmol), K₂CO₃ (134 mg, 0.97 mmol) in toluene (50 mL) were reacted to give the title product (140 mg, 32 % yield) as a light yellow oil.

GC/MS (El): *m*/*z* (%): 359 (52) [*M*⁺], 313 (25), 271 (17), 257 (39), 243 (23), 177 (17), 126 (100), 100 (46), 98 (36), 87 (58). ¹H NMR (300 MHz, CDCl₃) δ 7.44 (s, 1H), 7.30 (d, *J =* 10.5 Hz, 1H), 7.18 (d, *J =* 8.0 Hz, 1H), 2.92 - 2.66 (m, 3H), 2.45 - 2.27 (m, 1H), 2.18 - 1.93 (m, 2H), 1.91 - 1.77 (m, 2H), 1.61 - 1.42 (m, 2H), 1.38 (s, 3H), 1.29 (s, 3H), 1.17 (s, 3H), 1.08 (d, *J =* 6.0 Hz, 3H). ¹³C NMR (75 MHz, CDCl₃) δ 164.2 (q), 160.9 (q), 155.4 (q), 155.3 (q), 132.5 (q), 132.4 (q), 132.1 (q), 132.0 (q), 125.3 (q), 125.3 (q), 121.7 (q), 121.7 (q), 117.8 (t), 117.7 (t), 117.7 (t), 116.2 (t), 115.9 (t), 110.6 (t), 110.5 (t), 110.2 (t), 110.1 (t), 79.3 (q), 77.4 (t), 53.7 (t), 48.9 (s), 45.7 (d), 39.1 (q), 36.9 (d), 33.1 (t), 26.9 (s), 26.5 (s), 24.9 (s), 20.1 (s).

### Example 130: rac-(3aR,5R,7S,7aR)-1,3,3,5,7-pentamethyl-5-(2-methyl-5-(trifluoromethyl)phenyl)octahydrobenzo[c] isoxazole

Following the general procedure described in Example 81b: 2,4,7-trimethyl-4-(2-methyl-5-(trifluoromethyl)phenyl)oct-6-enal (300 mg, 0.92 mmol) (obtained from the corresponding α,β-unsaturated aldehyde using the general procedure described in Example 81a), *N*-methylhydroxylamine hydrochloride (115 mg, 1.38 mmol), K₂CO₃ (102 mg, 0.74 mmol) in toluene (50 mL) were reacted to give the title product (114 mg, 35 % yield) as a yellow oil.

GC/MS (El): *m*/*z* (%): 355 (66) [*M*⁺], 309 (17), 253 (25), 187 (33), 173 (25), 126 (100), 100 (45), 98 (32), 87 (53) . ¹H NMR (300 MHz, CDCl₃) δ 7.63 (s, 1H), 7.39 (d, *J =* 7.8 Hz, 1H), 7.29 (s, 1H), 2.91 - 2.68 (m, 3H), 2.63 (s, 3H), 2.44 - 2.27 (m, 1H), 2.20 - 2.09 (m, 1H), 2.06 - 1.94 (m, 3H), 1.71 - 1.53 (m, 2H), 1.49 (s, 3H), 1.32 (s, 3H), 1.21 (s, 3H), 1.09 (d, *J =* 6.0 Hz, 3H). ¹³C NMR (75 MHz, CDCl₃) δ 149.0 (q), 140.3 (q), 133.7 (t), 128.3 (q), 127.9 (q), 126.4 (q), 122.9 (t), 122.8 (t), 122.4 (t), 122.3 (t), 118.2 (t), 79.4 (q), 77.0 (t), 53.8 (t), 48.9 (s), 45.1 (d), 39.9 (q), 35.7 (d), 33.3 (t), 27.0 (s), 26.9 (s), 25.0 (s), 24.5 (s), 23.7 (s), 20.3 (s).

### Example 131: rac-(3aR,5R,7S,7aR)-5-(2-ethoxyphenyl)-1,3,3,5,7-pentamethyloctahydrobenzo[c]isoxazole

Following the general procedure described in Example 81b: 4-(2-ethoxyphenyl)-2,4,7-trimethyloct-6-enal (600 mg, 1.66 mmol) (obtained from the corresponding α,β-unsaturated aldehyde using the general procedure described in Example 81a), N-methylhydroxylamine hydrochloride (208 mg, 2.50 mmol), K₂CO₃ (184 mg, 1.33 mmol) in toluene (50 mL) were reacted to give the title product (201 mg, 38 % yield) as a light yellow oil.

GC/MS (El): *m*/*z* (%): 317 (94) [*M*⁺], 271 (15), 195 (16), 180 (33), 149 (45), 135 (35), 126 (100), 100 (41), 98 (29), 87 (52). ¹H NMR (300 MHz, CDCl₃) δ 7.31 (d, *J =* 7.8 Hz, 1H), 7.23 - 7.13 (m, 1H), 6.95 - 6.83 (m, 2H), 4.15 - 3.97 (m, 2H), 2.89 - 2.63 (m, 3H), 2.44 - 2.24 (m, 1H), 2.15 (t, *J=* 10.3 Hz, 1H), 2.02 - 1.73 (m, 5H), 1.51 - 1.43 (m, 6H), 1.26 (s, 3H), 1.14 (s, 3H), 1.05 - 0.99 (m, 3H). ¹³C NMR (75 MHz, CDCl₃) δ 157.4 (q), 137.8 (q), 127.2 (t), 126.2 (t), 120.3 (t), 112.2 (t), 79.5 (q), 77.3 (t), 63.2 (d), 53.9 (t), 48.9 (s), 43.9 (d), 38.8 (q), 34.2 (d), 33.2 (t), 26.9 (s), 25.0 (s), 23.9 (s), 20.3 (s), 15.1 (s).

### Example 132: rac-(3aR,5R,7S,7aR)-1,3,3,5,7-pentamethyl-5-(5-methylthiophen-2-vl)octahvdrobenzo[c]isoxazole

Following the general procedure described in Example 81b: 2,4,7-trimethyl-4-(5-methylthiophen-2-yl)oct-6-enal (854 mg, 1.55 mmol) (obtained from the corresponding α,β-unsaturated aldehyde using the general procedure described in Example 81a), N-methylhydroxylamine hydrochloride (194 mg, 2.33 mmol), K₂CO₃ (171 mg, 1.24 mmol) in toluene (50 mL) were reacted to give the title product (142 mg, 31 % yield) as a light yellow oil.

GC/MS (El): *m*/*z* (%): 293 (23) [*M*⁺], 195 (35), 180 (100), 149 (13), 138 (14), 137 (15), 126 (15), 100 (8), 98 (13), 87 (18). ¹H NMR (300 MHz, CDCl₃) δ 6.64 (d, *J =* 3.4 Hz, 1H), 6.60 - 6.52 (m, 1H), 2.86 - 2.63 (m, 3H), 2.44 (s, 3H), 2.35 - 2.20 (m, 1H), 2.14 - 2.03 (m, 1H), 1.79 - 1.71 (m, 2H), 1.68 - 1.60 (m, 2H), 1.54 - 1.46 (m, 1H), 1.41 (s, 3H), 1.29 - 1.25 (m, 3H), 1.14 (s, 3H), 1.01 (d, *J* = 6.3 Hz, 3H). ¹³C NMR (75 MHz, CDCl₃) δ 155.3 (q), 136.9 (q), 124.4 (t), 120.8 (t), 79.4 (q), 77.7 (t), 54.0 (t), 49.0 (s), 48.1 (d), 39.0 (d), 38.5 (q), 33.3 (t), 27.3 (s), 27.0 (s), 25.0 (s), 20.1 (s), 15.3 (s).

### Example 133: rac-(3aR,5R,7S,7aR)-1,3,3,5,7-pentamethyl-5-(2-(methylthio)phenyl)octahydrobenzo[c]isoxazole

Following the general procedure described in Example 81b: 2,4,7-trimethyl-4-(2-(methylthio)phenyl)oct-6-enal (792 mg, 0.76 mmol) (obtained from the corresponding α,β-unsaturated aldehyde using the general procedure described in Example 81a), *N-*methylhydroxylamine hydrochloride (96 mg, 1.15 mmol), K₂CO₃ (84 mg, 0.61 mmol) in toluene (50 mL) were reacted to give the title product (90 mg, 37 % yield) as a light yellow oil.

GC/MS (EI): *m*/*z* (%): 319 (56) [*M*⁺], 304 (9), 273 (13), 217 (10), 180 (8), 161 (14), 149 (36), 126 (80), 113 (22), 87 (100). ¹H NMR (300 MHz, CDCl₃) δ 7.34 - 7.22 (m, 2H), 7.18 - 7.04 (m, 2H), 3.25 - 3.07 (m, 1H), 2.81 - 2.57 (m, 3H), 2.49 (s, 3H), 2.21 - 2.07 (m, 1H), 1.94 - 1.73 (m, 2H), 1.48 (s, 3H), 1.33 - 1.10 (m, 9H), 1.05 - 0.98 (m, 3H). ¹³C NMR (75 MHz, CDCl₃) δ 144.3 (q), 136.9 (q), 130.1 (t), 128.6 (t), 126.7 (t), 125.5 (t), 79.2 (q), 78.3 (t), 54.4 (t), 48.8 (s), 47.7 (d), 42.2 (q), 36.6 (d), 33.5 (t), 29.4 (s), 26.9 (s), 24.8 (s), 20.2 (s), 18.7 (s).

### Example 134: rac-2-chloro-5-((3aR,5R,7R,7aR)-1,3,3,5,7-pentamethyloctahydrobenzo[c]isoxazol-5-yl)benzonitrile

Following the general procedure described in Example 81b: 2-chloro-5-(2,4,7-trimethyl-1-oxooct-6-en-4-yl)benzonitrile (400 mg, 1.32 mmol) (obtained from the corresponding α,β-unsaturated aldehyde using the general procedure described in Example 81a), N-methylhydroxylamine hydrochloride (165 mg, 1.98 mmol), K₂CO₃ (146 mg, 1.05 mmol) in toluene (50 mL) were reacted to give the title product (94 mg, 21 % yield) as a light yellow oil.

GC/MS (El): *m*/*z* (%): 332 (25) [*M*⁺], 286 (7), 230 (8), 164 (14), 126 (96), 113 (32), 100 (41), 98 (51), 87 (100), 70 (20). ¹H NMR (300 MHz, CDCl₃) δ 7.58 (s, 1H), 7.52 - 7.40 (m, 2H), 2.78 (s, 3H), 2.16 (dd, *J =* 20.7, 11.4 Hz, 2H), 1.75 (t, *J =* 11.2 Hz, 1H), 1.66 - 1.56 (m, 1H), 1.49 - 1.36 (m, 1H), 1.31 - 1.12 (m, 11H), 1.02 (d, *J* = 6.4 Hz, 3H). ¹³C NMR (75 MHz, CDCl₃) δ 147.2 (q), 134.2 (q), 131.8 (t), 131.7 (t), 130.3 (t), 116.3 (q), 113.6 (q), 78.8 (q), 77.7 (t), 54.2 (t), 48.7 (s), 45.5 (d), 40.2 (q), 36.7 (d), 34.2 (s), 33.3 (t), 26.7 (s), 24.8 (s), 20.0 (s).

### Example 135: rac-3-methyl-4-((3aR,5R,7S,7aR)-1,3,3,5,7-pentamethyloctahydrobenzo[c]isoxazol-5-yl)benzonitrile

Following the general procedure described in Example 81b: 3-methyl-4-(2,4,7-trimethyl-1-oxooct-6-en-4-yl)benzonitrile (1.26 g, 0.67 mmol) (obtained from the corresponding α,β-unsaturated aldehyde using the general procedure described in Example 81a), N-methylhydroxylamine hydrochloride (0.08 g, 0.100 mmol), K₂CO₃ (0.07 g, 0.53 mmol) in toluene (50 mL) were reacted to give the title product (0.045 g, 21 % yield) as a colorless oil.

GC/MS (El): *m*/*z(%):* 312 (43) [*M*⁺], 297 (6), 266 (7), 210 (10), 182 (11), 144 (30), 126 (100), 100 (53), 98 (40), 87 (71), 70 (19). ¹H NMR (300 MHz, CDCl₃) δ 7.56 (d, *J=* 7.8 Hz, 1H), 7.28 (s, 1H), 7.09 (d, *J =* 7.8 Hz, 1H), 2.91 - 2.61 (m, 3H), 2.38 (s, 3H), 2.33 - 2.17 (m, 1H), 2.06 - 1.79 (m, 3H), 1.69 (t, *J =* 12.8 Hz, 1H), 1.60 - 1.40 (m, 3H), 1.28 - 1.21 (m, 3H), 1.16 (s, 3H), 1.04 (d, *J =* 6.2 Hz, 3H). ¹³C NMR (75 MHz, CDCl₃) δ 153.8 (q), 137.6 (q), 136.5 (t), 129.8 (t), 126.5 (t), 119.0 (q), 110.1 (q), 79.4 (q), 77.3 (t), 53.7 (t), 48.9 (s), 45.0 (d), 40.4 (q), 35.7 (d), 33.3 (t), 27.0 (s), 25.0 (s), 24.4 (s), 23.7 (s), 20.3 (s).

### Example 136: rac-4-methyl-2-((3aR,5R,7S,7aR)-1,3,3,5,7-pentamethyloctahydrobenzo[c]isoxazol-5-yl)benzonitrile

Following the general procedure described in Example 81b: 4-methyl-2-(2,4,7-trimethyl-1-oxooct-6-en-4-yl)benzonitrile (271 mg, 0.96 mmol) (obtained from the corresponding α,β-unsaturated aldehyde using the general procedure described in Example 81a), N-methylhydroxylamine hydrochloride (120 mg, 1.43 mmol), K₂CO₃ (106 mg, 0.77 mmol) in toluene (40 mL) were reacted to give the title product (145 mg, 49 % yield) as a light yellow oil.

GC/MS (El): *m*/*z* (%):312 (38) [*M*⁺], 297 (6), 281 (7), 266 (28), 249 (5), 207 (45), 126 (100), 100 (34), 87 (55), 70 (12). ¹H NMR (300 MHz, CDCl₃) δ 7.56 (d, *J =* 7.8 Hz, 1H), 7.28 (s, 1H), 7.09 (d, *J =* 7.8 Hz, 1H), 2.91 - 2.61 (m, 3H), 2.38 (s, 3H), 2.33 - 2.17 (m, 1H), 2.06 - 1.79 (m, 3H), 1.69 (t, *J=* 12.8 Hz, 1H), 1.60 - 1.40 (m, 3H), 1.28 - 1.21 (m, 3H), 1.16 (s, 3H), 1.04 (d, *J* = 6.2 Hz, 3H). ¹³C NMR (75 MHz, CDCl₃) δ 153.3 (q), 143.6 (q), 136.0 (t), 127.2 (t), 127.1 (t), 120.6 (q), 107.6 (q), 79.5 (q), 76.6 (t), 53.8 (t), 48.9 (s), 44.9 (d), 39.5 (q), 35.5 (d), 33.4 (t), 27.0 (s), 26.9 (s), 24.9 (s), 24.6 (s), 22.2 (s), 20.0 (s).

### Example 137: rac-(3aR,5R,7S,7aR)-1,3,3,5,7-pentamethyl-5-(5-methylthiophen-3-vl)octahvdrobenzo[c]isoxazole

Following the general procedure described in Example 81b: 2,4,7-trimethyl-4-(5-methylthiophen-3-yl)oct-6-enal (758 mg, 1.29 mmol) (obtained from the corresponding α,β-unsaturated aldehyde using the general procedure described in Example 81a), *N-*methylhydroxylamine hydrochloride (162 mg, 1.94 mmol), K₂CO₃ (143 mg, 1.03 mmol) in toluene (40 mL) were reacted to give the title product (83 mg, 22 % yield) as a colorless oil.

GC/MS (El): *m*/*z* (%): 293 (44) [*M*⁺], 180 (27), 149 (27), 139 (44), 126 (100), 100 (29), 98 (51), 87 (80). ¹H NMR (300 MHz, CDCl₃) δ 6.72 (t, *J =* 10.0 Hz, 2H), 2.88-2.61 (m, 3H), 2.46 (s, 3H), 2.35 - 2.19 (m, 1H), 2.07 (t, *J =* 10.3 Hz, 1H), 1.99 - 1.82 (m, 1H), 1.78 - 1.63 (m, 2H), 1.61 - 1.34 (m, 4H), 1.34 - 1.21 (m, 7H), 1.15 (s, 3H), 1.01 (*d, J =* 6.3 Hz, 3H). ¹³C NMR (75 MHz, CDCl₃) δ 153.1 (q), 139.8 (q), 124.3 (t), 115.4 (t), 79.4 (q), 77.8 (t), 53.7 (t), 49.0 (s), 46.5 (d), 37.6 (q), 37.3 (d), 33.1 (t), 27.0 (s), 26.0 (s), 25.0 (s), 20.2 (s), 15.5 (s).

### Example 138: rac-(3aR,5R,7S,7aR)-5-(furan-3-yl)-1,3,3,5,7-pentamethyloctahydrobenzo[c]isoxazole

Following the general procedure described in Example 81b: 4-(furan-3-yl)-2,4,7-trimethyloct-6-enal (558 mg, 1.19 mmol) (obtained from the corresponding α,β-unsaturated aldehyde using the general procedure described in Example 81a), N-methylhydroxylamine hydrochloride (149 mg, 1.79 mmol), K₂CO₃ (132 mg, 0.95 mmol) in toluene (40 mL) were reacted to give the title product (100 mg, 32 % yield) as a colorless oil.

GC/MS (El): *m*/*z(%):* 263 (46) [*M*⁺], 248 (8), 180 (18), 149 (10), 126 (100), 109 (21), 100 (41), 98 (35), 87 (60). ¹H NMR (300 MHz, CDCl₃) δ 7.37 (t, *J =* 1.6 Hz, 1H), 7.22 (s, 1H), 6.36 (s, 1H), 2.90 - 2.70 (m, 3H), 2.33 - 2.18 (m, 1H), 2.08 (t, *J=* 10.3 Hz, 1H), 1.91 (s, 1H), 1.72 - 1.60 (m, 2H), 1.54 - 1.36 (m, 2H), 1.28 (d, *J =* 10.8 Hz, 6H), 1.13 (s, 3H), 1.00 (d, *J =* 6.3 Hz, 3H). ¹³C NMR (75 MHz, CDCl₃) δ 143.0 (t), 136.9 (t), 136.7 (q), 108.6 (t), 79.4 (q), 77.9 (t), 53.6 (t), 49.0 (s), 46.5 (d), 37.4 (d), 34.1 (q), 33.0 (t), 27.0 (s), 25.6 (s), 25.0 (s), 20.1 (s).

### Example 139: rac-(3aR,5R,7S,7aR)-5-(4-(tert-butyl)phenyl)-1,3,3,5,7-pentamethyloctahydrobenzo[c]isoxazole

Following the general procedure described in Example 81b: 4-(4-(*tert*-butyl)phenyl)-2,4,7-trimethyloct-6-enal (658 mg, 1.97 mmol) (obtained from the corresponding α,β-unsaturated aldehyde using the general procedure described in Example 81a), *N-*methylhydroxylamine hydrochloride (247 mg, 2.96 mmol), K₂CO₃ (218 mg, 1.58 mmol) in toluene (40 mL) were reacted to give the title product (267 mg, 41 % yield) as a colorless oil.

GC/MS (El): *m*/*z* (%): 329 (37) [*M*⁺], 180 (27), 159 (19), 126 (100), 100 (36), 98 (24), 87 (39), 57 (43). ¹H NMR (300 MHz, CDCl₃) δ 7.40 - 7.29 (m, 4H), 2.87 - 2.65 (m, 3H), 2.40 - 2.24 (m, 1H), 2.13 - 1.91 (m, 2H), 1.82 (t, *J =* 11.2 Hz, 2H), 1.65 - 1.52 (m, 1H), 1.51 - 1.42 (m, 1H), 1.36 (s, 3H), 1.32 (s, 9H), 1.28 (s, 3H), 1.13 (s, 3H), 1.04 (d, *J =* 6.0 Hz, 3H). ¹³C NMR (75 MHz, CDCl₃) δ 148.6 (q), 148.2 (q), 125.1 (t), 124.7 (t), 79.4 (q), 77.7 (t), 53.9 (t), 49.0 (s), 46.2 (d), 38.2 (q), 37.1 (d), 34.3 (q), 33.2 (t), 31.4 (s), 27.1 (s), 27.0 (s), 26.6 (s), 25.0 (s), 20.3 (s).

### Example 140: rac-(3aS,5R,7S,7aS)-5-(4-fluorophenyl)-1,3,3,5,7-pentamethyloctahydrobenzo[c]isoxazole

Following the general procedure described in Example 81b: 4-(4-fluorophenyl)-2,4,7-trimethyloct-6-enal (323 mg, 1.23 mmol) (obtained from the corresponding α,β-unsaturated aldehyde using the general procedure described in Example 81a), N-methylhydroxylamine hydrochloride (154 mg, 1.85 mmol), K₂CO₃ (136 mg, 0.98 mmol) in toluene (50 mL) were reacted to give the title product (44 mg, 12 % yield) as a light yellow oil.

GC/MS (El): *m*/*z* (%): 291(34) [*M*⁺], 276 (4), 189 (4), 163 (4), 147 (5), 126 (100), 113 (29), 98 (66), 87 (13). ¹H NMR (300 MHz, CDCl₃) δ 7.32 - 7.22 (m, 2H), 6.99 (t, *J =* 8.7 Hz, 2H), 2.86 - 2.68 (m, 3H), 2.44 (t, *J* = 6.6 Hz, 1H), 2.25 - 2.12 (m, 1H), 2.05 - 1.94 (m, 1H), 1.85 - 1.59 (m, 4H), 1.30 - 1.23 (m, 9H), 0.85 (d, *J* = 6.7 Hz, 3H). ¹³C NMR (75 MHz, CDCl₃) δ 162.6 (q), 159.3 (q), 145.7 (q), 145.6 (q), 127.2 (t), 127.1 (t), 115.1 (t), 114.9 (t), 80.4 (q), 75.0 (t), 48.6 (t), 46.5 (s), 42.6 (d), 36.9 (q), 34.8 (s), 33.8 (d), 30.0 (s), 29.3 (t), 23.5 (s), 21.2 (s).

### Example 141: rac-(3aR,5R,7S,7aR)-5-(4-fluorophenyl)-1,3,3,5,7-pentamethyloctahydrobenzo[c]isoxazole

Following the general procedure described in Example 81b: 4-(4-fluorophenyl)-2,4,7-trimethyloct-6-enal (323 mg, 1.23 mmol) (obtained from the corresponding α,β-unsaturated aldehyde using the general procedure described in Example 81a), N-methylhydroxylamine hydrochloride (154 mg, 1.85 mmol), K₂CO₃ (136 mg, 0.98 mmol) in toluene (50 mL) were reacted to give the title product rac-(3aR,5R,7 S,7aR)-5-(4-fluorophenyl)-1,3,3,5,7-pentamethyloctahydrobenzo[c]isoxazole (164 mg, 46 % yield), as a light yellow oil.

GC/MS (EI): *m*/*z* (%): 291 (50) [*M*⁺], 276 (5), 189 (8), 161 (8), 149 (10), 126 (100), 109 (30), 100 (41), 87 (42). ¹H NMR (300 MHz, CDCl₃) δ 7.40-7.28 (m, 2H), 6.99 (t, *J =* 8.7 Hz, 2H), 2.89 - 2.60 (m, 3H), 2.40 - 2.21 (m, 1H), 2.14 - 1.87 (m, 2H), 1.85 - 1.69 (m, 2H), 1.52 (t, *J =* 12.9 Hz, 1H), 1.39 (d, *J =* 10.2 Hz, 1H), 1.35 - 1.30 (m, 3H), 1.29 - 1.22 (m, 3H), 1.13 (s, 3H), 1.02 (d, *J =* 6.1 Hz, 3H). ¹³C NMR (75 MHz, CDCl₃) δ 162.6 (q), 159.4 (q), 146.9 (q), 126.7 (t), 126.6 (t), 115.0 (t), 114.7 (t), 79.4 (q), 77.6 (t), 53.9 (t), 48.9 (s), 46.3 (d), 38.3 (q), 37.2 (d), 33.1 (t), 26.9 (s), 26.7 (s), 24.9 (s), 20.2 (s).

In the same reaction, additional isomers were also obtained: rac-(3aS,5R,7S,7aS)-5-(4-fluorophenyl)-1,3,3,5,7-pentamethyloctahydrobenzo[c]isoxazole (48 mg, 12 % yield) and rac-(3aS,5R,7R,7aS)-5-(4-fluorophenyl)-1,3,3,5,7-pentamethyloctahydrobenzo[c]isoxazole (79 mg, 20 % yield) as light yellow oils.

rac-(3aS,5R,7S,7aS)-5-(4-fluorophenyl)-1,3,3,5,7-pentamethyloctahydrobenzo[c]isoxazole: GC/MS (El): *m*/*z* (%): 291 (33) [*M*⁺], 276 (4), 126 (100), 113 (30), 98 (73), 87 (14).

rac-(3aS,5R,7R,7aS)-5-(4-fluorophenyl)-1,3,3,5,7-pentamethyloctahydrobenzo[c]isoxazole: GC/MS (El): *m*/*z* (%): 291 (67) [*M*⁺], 276 (5), 189 (10), 161 (9), 149 (11), 126 (100), 109 (35), 100 (39), 98(40), 87 (71).

### Example 142: rac-(3aR,5R,7S,7aR)-5-(4-fluorophenyl)-1-isopropyl-3,3,5,7-tetramethyloctahydrobenzo[c]isoxazole

Following the general procedure described in Example 81b: 4-(4-fluorophenyl)-2,4,7-trimethyloct-6-enal (619 mg, 1.27 mmol) (obtained from the corresponding α,β-unsaturated aldehyde using the general procedure described in Example 81a), N-isopropylhydroxylamine (144 mg, 1.91 mmol) in toluene (40 mL) were reacted to give the title product (114 mg, 28 % yield) as a light yellow oil.

GC/MS (El): *m*/*z(%):* 319 (30) [*M*⁺], 304 (100), 262 (2), 208 (3), 189 (11), 175 (8), 154 (78), 126 (30), 109 (33), 84 (9). ¹H NMR (300 MHz, CDCl₃) δ 7.32 (q, *J =* 8.9, 5.3 Hz, 2H), 6.96 (t, *J =* 8.7 Hz, 2H), 3.26 - 3.09 (m, 1H), 2.49 - 2.18 (m, 2H), 2.06 - 1.85 (m, 1H), 1.83 - 1.68 (m, 2H), 1.57 - 1.33 (m, 2H), 1.29 (d, *J =* 7.3 Hz, 6H), 1.22 (d, *J =* 6.7 Hz, 3H), 1.08 - 0.95 (m, 9H). ¹³C NMR (75 MHz, CDCl₃) δ 162.5 (q), 159.3 (q), 147.1 (q), 147.0 (q), 126.6 (t), 126.5 (t), 114.9 (t), 114.7 (t), 77.9 (q), 68.8 (t), 54.9 (t), 53.0 (t), 46.5 (d), 38.1 (q), 37.0 (d), 33.5 (t), 27.1 (s), 26.9 (s), 26.7 (s), 24.2 (s), 22.5 (s), 20.3 (s), 13.6 (s).

### Example 143: rac-(3aR,5R,7S,7aR)-5-(3-fluorophenyl)-1,3,3,5,7-pentamethyloctahydrobenzo[c]isoxazole

Following the general procedure described in Example 81b: 4-(3-fluorophenyl)-2,4,7-trimethyloct-6-enal (377 mg, 1.44 mmol) (obtained from the corresponding α,β-unsaturated aldehyde using the general procedure described in Example 81a), *N-*methylhydroxylamine hydrochloride (180 mg, 2.16 mmol), K₂CO₃ (159 mg, 1.15 mmol) in toluene (40 mL) were reacted to give the title product (184 mg, 44 % yield) as a light yellow oil.

GC/MS (EI): *m*/*z* (%): 291 (49) [*M*⁺], 276 (5), 189 (13), 161 (15), 147 (7), 126 (100), 109 (26), 100 (31), 87 (51). ¹H NMR (300 MHz, CDCl₃) δ 7.20 (q, *J =* 14.4, 7.4 Hz, 1H), 7.09 (d, *J =* 8.0 Hz, 1H), 7.00 (d, *J =* 11.3 Hz, 1H), 6.80 (td, *J =* 8.2, 1.6 Hz, 1H), 2.85 - 2.55 (m, 3H), 2.32 - 2.16 (m, 1H), 2.06 - 1.82 (m, 2H), 1.79 - 1.64 (m, 2H), 1.46 (t, *J =* 12.9 Hz, 1H), 1.35 (d, *J =* 12.3 Hz, 1H), 1.26 (s, 3H), 1.23 - 1.16 (m, 3H), 1.06 (s, 3H), 0.96 (d, *J* = 6.1 Hz, 3H). ¹³C NMR (75 MHz, CDCl₃) δ 164.5 (q), 161.2 (q), 154.0 (q), 154.0 (q), 129.6 (t), 129.5 (t), 120.7 (t), 120.6 (t), 112.7 (t), 112.5 (t), 112.4 (t), 112.1 (t), 79.2 (q), 77.5 (t), 53.7 (t), 48.8 (s), 45.9 (d), 38.6 (q), 36.8 (d), 33.0 (t), 26.9 (s), 26.5 (s), 24.9 (s), 20.1 (s).

### Example 144: rac-(3aR,5R,7S,7aR)-5-(2,4-difluorophenyl)-1,3,3,5,7-pentamethyloctahydrobenzo[c]isoxazole

Following the general procedure described in Example 81b: 4-(2,4-difluorophenyl)-2,4,7-trimethyloct-6-enal (401 mg, 0.14 mmol), *N*-methylhydroxylamine hydrochloride (18 mg, 0.22 mmol) (obtained from the corresponding α,β-unsaturated aldehyde using the general procedure described in Example 81a), K₂CO₃ (16 mg, 0.11 mmol) in toluene (50 mL) were reacted to give the title product (26 mg, 60 % yield) as a light yellow oil.

GC/MS (EI): *m*/*z* (%): 309 (38) [*M*⁺], 294 (5), 207 (12), 141 (33), 126 (100), 113 (14), 100 (43), 98 (36), 87 (51).

### Example 145: rac-1,3,3,5,7-pentamethyl-5-(4-methylpyridin-3-vl)octahydrobenzo[c]isoxazole

Following the general procedure described in Example 81b: 2,4,7-trimethyl-4-(4-methylpyridin-3-yl)oct-6-enal (654 mg, 0.71 mmol) (obtained from the corresponding α,β-unsaturated aldehyde using the general procedure described in Example 81a), *N-*methylhydroxylamine hydrochloride (88 mg, 1.06 mmol), K₂CO₃ (78 mg, 0.57 mmol) in toluene (50 mL) were reacted to give the title product (115 mg, 53 % yield) as a light yellow oil.

GC/MS (Isomer 1) (EI): *m*/*z* (%): 288 (52) [*M*⁺], 271 (32), 242 (13), 228 (24), 134 (100), 126 (21), 98 (22) . And GC/MS (Isomer 2) (EI): *m*/*z* (%): 288 (51) [*M*⁺], 271 (31), 242 (36), 228 (5), 134 (100), 126 (58), 98 (28). ¹H NMR (300 MHz, CDCl₃) δ 8.60 - 8.36 (m, 1H), 8.33 - 8.15 (m, 1H), 7.06 - 6.89 (m, 1H), 2.76 (d, *J =* 20.5 Hz, 2H), 2.56 - 2.41 (m, 3H), 2.40 - 2.20 (m, 1H), 2.20 - 1.89 (m, 2H), 1.86 - 1.58 (m, 2H), 1.57 - 1.35 (m, 3H), 1.33 - 1.06 (m, 9H), 1.04 - 0.93 (m, 3H). ¹³C NMR (75 MHz, CDCl₃) δ 149.2 (t), 147.2 (t), 147.1 (t), 146.8 (t), 145.4 (q), 145.1 (q), 143.3 (q), 139.0 (q), 127.9 (t), 127.7 (t), 79.4 (q), 78.9 (q), 78.1 (t), 77.0 (t), 54.2 (t), 53.5 (t), 48.8 (s), 48.6 (s), 46.9 (d), 44.9 (d), 40.8 (q), 38.9 (q), 36.7 (d), 35.7 (d), 33.2 (t), 33.0 (t), 30.4 (s), 26.9 (s), 24.9 (s), 24.7 (s), 24.4 (s), 23.3 (s), 23.2 (s), 20.2 (s), 19.9 (s).

### Example 146: rac-2-(1,3,3,5,7-pentamethyloctahydrobenzo[c]isoxazol-5-yl)benzonitrile

Following the general procedure described in Example 81b: 2-(2,4,7-trimethyl-1-oxooct-6-en-4-yl)benzonitrile (389 mg, 1.44 mmol) (obtained from the corresponding α,β-unsaturated aldehyde using the general procedure described in Example 81a), *N-*methylhydroxylamine hydrochloride (181 mg, 2.17 mmol), K₂CO₃ (160 mg, 1.16 mmol) in toluene (50 mL) were reacted to give the title product (229 mg, 53 % yield) as a light yellow oil.

GC/MS (Isomer 1) (El): *m*/*z* (%): 298 (29) [*M*⁺], 283 (3), 252 (21), 126 (50), 113 (19), 98 (36), 87 (100), 70 (18). And GC/MS (Isomer 2) (El): *m*/*z* (%): 298 (14) [*M*⁺], 297 (62), 252 (40), 213 (100),198 (71), 158 (50), 144 (44), 126 (64), 98 (49), 68 (52). And GC/MS (Isomer 3) (EI): *mlz* (%):298 (6) [*M*⁺], 297 (24), 252 (16), 240 (19), 196 (33), 144 (28), 126 (100), 98 (30), 68 (43). ¹H NMR (300 MHz, CDCl₃) δ 7.74 - 7.65 (m, 1H), 7.58 - 7.40 (m, 2H), 7.36 - 7.27 (m, 1H), 2.98 - 2.75 (m, 3H), 2.72 - 2.52 (m, 1H), 2.43 - 2.20 (m, 1H), 2.17 (d, *J =* 8.5 Hz, 2H), 2.02 - 1.92 (m, 1H), 1.78 (s, 1H), 1.57 (s, 1H), 1.49-1.39 (m, 2H), 1.36 - 1.24 (m, 3H), 1.20 - 1.14 (m, 4H), 1.06 (d, *J* = 6.2 Hz, 3H). ¹³C NMR (75 MHz, CDCl₃) δ 153.3 (q), 149.8 (q), 136.3 (t), 135.8 (t), 132.8 (t), 132.7 (t), 128.3 (t), 126.5 (t), 126.4 (t), 126.1 (t), 120.1 (q), 119.9 (q), 110.5 (q), 110.1 (q), 79.3 (q), 78.9 (q), 77.5 (t), 76.4 (t), 54.3 (t), 53.6 (t), 48.8 (q), 48.6 (q), 45.9, 44.7, 41.1 (q), 39.5 (q), 36.3 (d), 35.4 (d), 33.4 (s), 33.2 (s), 31.2 (s), 30.8 (s), 26.7 (s), 26.5 (s), 24.7 (s), 24.5 (s), 24.4 (s), 19.8 (s), 19.7 (s).

### Example 147: rac-5-(3-methoxypyridin-2-yl)-1,3,3,5,7-pentamethyloctahydrobenzo[c]isoxazole

Following the general procedure described in Example 81b: 4-(6-methoxypyridin-2-yl)-2,4,7-trimethyloct-6-enal (485 mg, 0.92 mmol) (obtained from the corresponding α,β-unsaturated aldehyde using the general procedure described in Example 81a), *N-*methylhydroxylamine hydrochloride (115 mg, 1.37 mmol), K₂CO₃ (101 mg, 0.73 mmol) in toluene (50 mL) were reacted to give the title product (234 mg, 84 % yield) as a light yellow oil.

GC/MS (Isomer 1) (EI): *m*/*z* (%): 304 (30) [*M*⁺], 258 (20), 244 (17), 150 (100), 123 (14), 98 (11). And GC/MS (Isomer 2) (El): *m*/*z* (%): 304 (4) [*M*⁺], 303 (8), 258 (100), 242 (52), 218 (60), 150 (78), 123 (28), 98 (21).And GC/MS (Isomer 3) (EI): *m*/*z* (%): 304 (4) [*M*⁺], 258 (10), 247 (16), 231 (18), 150 (52), 123 (13), 97 (100).And GC/MS (Isomer 4) (EI): *m*/*z* (%): 304 (20) [*M*⁺], 258 (21), 217 (42), 202 (62), 150 (100), 123 (20), 98 (21). ¹H NMR (300 MHz, CDCl₃) δ 7.46 (q, *J =* 8.0 Hz, 1H), 6.82 (t, *J =* 6.8 Hz, 1H), 6.49 (dd, *J* = 16.6, 8.2 Hz, 1H), 3.87 (d, *J =* 11.3 Hz, 3H), 2.86 - 2.71 (m, 3H), 2.68 - 2.52 (m, 1H), 2.32 (d, *J =* 19.4 Hz, 1H), 2.14 - 2.01 (m, 1H), 2.00 - 1.78 (m, 2H), 1.72 (d, *J =* 12.9 Hz, 1H), 1.61 (d, *J =* 19.4 Hz, 1H), 1.30 (d, *J =* 30.5 Hz, 4H), 1.19 (d, *J =* 6.8 Hz, 3H), 1.12 (t, *J =* 9.5 Hz, 5H). ¹³C NMR (75 MHz, CDCl₃) δ 166.9 (q), 164.0 (q), 163.2 (q), 163.0 (q), 138.9 (t), 138.8 (t), 112.6 (t), 111.3 (t), 107.5 (t), 107.4 (t), 79.4 (q), 79.1 (q), 77.7 (t), 77.3 (t), 54.6 (t), 53.9 (t), 53.1 (s), 52.9 (s), 48.9 (s), 48.8 (s), 45.4 (d), 45.1 (d), 42.6 (q), 41.4 (q), 35.8 (d), 35.7 (d), 33.5 (t), 33.1 (t), 32.6 (s), 32.5 (s), 27.0 (s), 26.7 (s), 25.0 (s), 24.9 (s), 21.9 (s), 20.2 (s), 20.1 (s).

### Example 148: rac-(3aR,7aR)-1,3,3-trimethyl-6-phenyloctahydrobenzo[c]isoxazole

Following the general procedure described in Example 81b: 7-methyl-3-phenyloct-6-enal (500 mg, 2.31 mmol), *N*-methylhydroxylamine hydrochloride (290 mg, 3.47 mmol), K₂CO₃ (256 mg, 1.85 mmol) in toluene (50 mL) were reacted to give the title product (423 mg, 75 % yield) as a yellow oil.

GC/MS (El): *m*/*z(%):* 245 (81) [*M*⁺], 230 (19), 199 (18), 162 (100), 143 (20), 104 (22), 91 (36). ¹H NMR (300 MHz, CDCl₃) δ 7.35 - 7.27 (m, 2H), 7.25 - 7.16 (m, 3H), 2.76 - 2.53 (m, 4H), 2.38 (td, *J =* 10.9, 3.4 Hz, 1H), 2.10 - 1.76 (m, 4H), 1.65 - 1.39 (m, 3H), 1.39 - 1.30 (m, 3H), 1.18 (d, *J =* 6.8 Hz, 3H). ¹³C NMR (75 MHz, CDCl₃) δ 145.7 (q), 128.5 (t), 126.9 (t), 126.4 (t), 79.6 (q), 71.8 (t), 57.3 (t), 44.6 (q), 42.5 (t), 36.1 (d), 33.9 (d), 28.0 (s), 27.0 (s), 25.2 (s), 24.8 (d).

### Example 149: rac-(3aR,5R,7S,7aR)-1-isopropyl-3,3,7-trimethyl-5-phenyloctahydrobenzo[c]isoxazole

Following the general procedure described in Example 81b: 2,7-dimethyl-4-phenyloct-6-enal (258 mg, 0.92 mmol) (obtained from the corresponding α,β-unsaturated aldehyde using the general procedure described in Example 81a), *N*-isopropylhydroxylamine (207 mg, 2.76 mmol) in xylene (80 mL) were reacted to give the title product (153 mg, 58 % yield) as a light yellow oil.

GC/MS (EI): *m*/*z* (%): 287 (31) [*M*⁺], 272 (100), 230 (5), 157 (12), 154 (43), 126 (21), 115 (18), 91 (27). ¹H NMR (300 MHz, CDCl₃) δ 7.48 - 6.99 (m, 5H), 3.35 - 3.08 (m, 1H), 2.69 (d, *J =* 12.0 Hz, 1H), 2.51 (t, *J =* 9.7 Hz, 1H), 2.13 (t, *J =* 10.7 Hz, 1H), 1.86 (d, *J =* 12.6 Hz, 3H), 1.43 (t, *J =* 12.3 Hz, 1H), 1.35 - 1.19 (m, 7H), 1.07 (dd, *J* = 24.9, 7.3 Hz, 9H). ¹³C NMR (75 MHz, CDCl₃) δ 146.0 (q), 128.5 (t), 126.9 (t), 126.3 (t), 78.0 (q), 68.4 (t), 57.7 (t), 54.9 (t), 44.0 (t), 42.5 (d), 37.4 (t), 32.9 (s), 27.3 (s), 24.4 (s), 22.7 (s), 20.2 (s), 13.7 (s).

### Example 150: rac-(3aR,5R,7S,7aR)-1,3,3,7-tetramethyl-5-phenyloctahydrobenzo[c]isoxazole

Following the general procedure described in Example 81b: 2,7-dimethyl-4-phenyloct-6-enal (301 mg, 1.07 mmol) (obtained from the corresponding α,β-unsaturated aldehyde using the general procedure described in Example 81a), *N*-methylhydroxylamine hydrochloride (134 mg, 1.61 mmol), K₂CO₃ (118 mg, 0.86 mmol) in toluene (50 mL) were reacted to give the title product (105 mg, 38 % yield) as a light yellow oil.

GC/MS (El): *m*/*z* (%): 259 (13) [*M*⁺], 258 (32), 201 (23), 172 (24),126 (100), 104 (84), 91 (31), 68 (13). ¹H NMR (300 MHz, CDCl₃) δ 7.35 - 7.27 (m, 2H), 7.22 (dd, *J* = 7.6, 3.6 Hz, 3H), 2.85 (s, 3H), 2.73 - 2.60 (m, 1H), 2.16 (dt, *J =* 12.2, 6.4 Hz, 2H), 2.00 - 1.71 (m, 3H), 1.52 - 1.33 (m, 2H), 1.25 (d, *J =* 12.2 Hz, 3H), 1.21 - 0.96 (m, 6H). ¹³C NMR (75 MHz, CDCl₃) δ 145.8 (q), 128.5 (t), 126.8 (t), 126.3 (t), 79.3 (q), 77.0 (t), 58.5 (t), 48.9 (s), 44.0 (t), 42.2 (d), 37.0 (t), 32.9 (d), 27.0 (s), 25.1 (s), 20.0 (s).

### Example 151: rac-(3aR,5R,7S,7aR)-1,3,3,7-tetramethyl-5-(o-tolly)octahydrobenzofclisoxazole

Following the general procedure described in Example 81b: 2,7-dimethyl-4-(o-tolyl)oct-6-enal (289 mg, 1.18 mmol) (obtained from the corresponding α,β-unsaturated aldehyde using the general procedure described in Example 81a), N-methylhydroxylamine hydrochloride (148 mg, 1.77 mmol), K₂CO₃ (131 mg, 0.95 mmol) in toluene (50 mL) were reacted to give the title product (114 mg, 35 % yield) as a light yellow oil.

GC/MS (El): *m*/*z* (%): 273 (7) [*M*⁺], 272 (16), 215 (11), 186 (29), 126 (78), 118 (100), 105 (22), 98 (24), 91 (16). ¹H NMR (300 MHz, CDCl₃) δ 7.33 - 6.97 (m, 4H), 2.95 - 2.79 (m, 3H), 2.36 (s, 3H), 2.16 (dd, *J* = 19.9, 5.6 Hz, 2H), 1.80 (d, *J =* 13.2 Hz, 3H), 1.44 (dd, *J =* 13.5, 10.3 Hz, 2H), 1.33 - 1.21 (m, 4H), 1.19 - 1.01 (m, 6H). ¹³C NMR (75 MHz, CDCl₃) δ 143.6 (q), 135.2 (q), 130.5 (t), 126.2 (t), 126.0 (t), 125.3 (t), 79.3 (q), 77.2 (t), 58.7 (t), 48.9 (s), 41.4 (d), 39.3 (t), 37.1 (t), 31.9 (d), 27.0 (s), 25.1 (s), 20.0 (s), 19.5 (s).

### Example 152: rac-(3aR,5R,7S,7aR)-5-(4-(tert-butyl)phenyl)-1,3,3,7-tetramethyloctahydrobenzo[c]isoxazole

Following the general procedure described in Example 81b: 4-(4-(*tert*-butyl)phenyl)-2,7-dimethyloct-6-enal (558 mg, 1.79 mmol) (obtained from the corresponding α,β-unsaturated aldehyde using the general procedure described in Example 81a), N-methylhydroxylamine hydrochloride (224 mg, 2.69 mmol), K₂CO₃ (198 mg, 1.43 mmol) in toluene (40 mL) were reacted to give the title product (224 mg, 40 % yield) as a light yellow oil.

GC/MS (El): *m*/*z(%):* 315 (38) [*M*⁺], 300 (11), 145 (18), 126 (100), 100 (57), 98 (27), 87 (45), 57 (43). ¹H NMR (300 MHz, CDCl₃) δ 7.33 (d, *J =* 8.1 Hz, 2H), 7.17 (d, *J =* 8.1 Hz, 2H), 2.91 - 2.73 (m, 3H), 2.68 (d, *J =* 13.3 Hz, 1H), 2.22 - 2.07 (m, 2H), 1.95 - 1.74 (m, 3H), 1.51 - 1.35 (m, 2H), 1.33 - 1.25 (m, 12H), 1.18 (d, *J =* 7.1 Hz, 3H), 1.04 (d, *J =* 6.3 Hz, 3H). ¹³C NMR (75 MHz, CDCl₃) δ 149.1 (q), 142.8 (q), 126.5 (t), 125.4 (t), 79.4 (q), 77.1 (t), 58.6 (t), 49.0 (s), 43.5 (t), 42.2 (d), 37.0 (t), 34.4 (q), 33.1 (d), 31.4 (s), 27.1 (s), 27.0 (s), 25.2 (s), 20.1 (s).

### Example 153: rac-(3aR,5R,7S,7aR)-5-(4-(tert-butyl)phenyl)-1-isopropyl-3,3,7-trimethyloctahydrobenzo[c]isoxazole

Following the general procedure described in Example 81b: 4-(4-(*tert*-butyl)phenyl)-2,7-dimethyloct-6-enal (558 mg, 1.79 mmol) (obtained from the corresponding α,β-unsaturated aldehyde using the general procedure described in Example 81a), N-isopropylhydroxylamine (202 mg, 2.69 mmol) in toluene (40 mL) were reacted to give the title product (203 mg, 33 % yield) as a light yellow oil.

GC/MS (El): *m*/*z(%):* 343 (30) [*M*⁺], 328 (100), 213 (5), 154 (65), 145 (15), 128 (21), 126 (20), 115 (16), 57 (26). ¹H NMR (300 MHz, CDCl₃) δ 7.33 (d, *J =* 8.2 Hz, 2H), 7.17 (d, *J* = 8.2 Hz, 2H), 3.28 - 3.20 (m, 1H), 2.77 - 2.60 (m, 1H), 2.51 (t, *J =* 9.9 Hz, 1H), 2.21 - 2.06 (m, 1H), 1.92 - 1.69 (m, 3H), 1.48 - 1.36 (m, 1H), 1.33 - 1.24 (m, 16H), 1.15 - 1.07 (m, 6H), 1.01 (d, *J =* 7.8 Hz, 3H). ¹³C NMR (75 MHz, CDCl₃) δ 149.1 (q), 142.9 (q), 126.5 (t), 125.3 (t), 77.9 (q), 68.4 (t), 57.8 (t), 54.9 (t), 43.5 (t), 42.5 (d), 37.4 (t), 34.4 (q), 33.0 (d), 31.4 (s), 27.3 (s), 27.0 (s), 24.4 (s), 22.7 (s), 20.2 (s), 13.7 (s).

### Example 154: rac-3-((3aR,5R,7S,7aR)-1-isopropyl-3,3,7-trimethyloctahydrobenzo[c]isoxazol-5-yl)-4-methoxybenzonitrile

Following the general procedure described in Example 81b: 3-(2,7-dimethyl-1-oxooct-6-en-4-yl)-4-methoxybenzonitrile (528 mg, 1.57 mmol) (obtained from the corresponding α,β-unsaturated aldehyde using the general procedure described in Example 81a), N-isopropylhydroxylamine (177 mg, 2.36 mmol) in toluene (40 mL) were reacted to give the title product (233 mg, 43 % yield) as a light yellow oil.

GC/MS (EI): m/z(%):342 (25) [*M*⁺], 327 (100), 212 (12), 154 (54), 146 (24), 128 (21), 126 (27), 116 (19), 84 (9). ¹H NMR (300 MHz, CDCl₃) δ 7.55 - 7.39 (m, 2H), 6.87 (d, *J =* 8.3 Hz, 1H), 3.85 (s, 3H), 3.24 - 3.06 (m, 2H), 2.47 (t, *J =* 9.9 Hz, 1H), 2.19 - 2.01 (m, 1H), 1.88 - 1.67 (m, 3H), 1.41 - 1.30 (m, 1H), 1.28 - 1.19 (m, 6H), 1.15 (t, *J =* 6.7 Hz, 1H), 1.10 - 1.04 (m, 6H), 0.99 (d, *J* = 6.3 Hz, 3H). ¹³C NMR (75 MHz, CDCl₃) δ 160.2 (q), 135.5 (q), 131.9 (t), 130.5 (t), 119.5 (q), 110.8 (t), 103.8 (q), 77.9 (q), 68.4 (t), 57.5 (t), 55.7 (s), 54.9 (t), 40.7 (d), 37.3 (t), 35.9 (t), 30.9 (d), 27.2 (s), 27.0 (s), 24.4 (s), 22.6 (s), 20.1 (s).

### Example 155: rac-(3aR,5R,7S,7aR)-1-ethyl-5-(2-methoxyphenyl)-3,3,7-trimethyloctahydrobenzo[c]isoxazole

ollowing the general procedure described in Example 81b: 4-(2-methoxyphenyl)-2,7-dimethyloct-6-enal (558 mg, 1.82 mmol) (obtained from the corresponding α,β-unsaturated aldehyde using the general procedure described in Example 81a), N-ethylhydroxylamine (167 mg, 2.73 mmol) in toluene (40 mL) were reacted to give the title product (189 mg, 34 % yield) as a light yellow oil.

GC/MS (EI): *m*/*z* (%): 303 (36) [*M*⁺], 288 (24), 187 (7), 140 (100), 121 (28), 114 (44), 112 (36), 101 (39), 91 (26). ¹H NMR (300 MHz, CDCl₃) δ 7.23 - 7.12 (m, 2H), 6.97 - 6.80 (m, 2H), 3.82 (s, 3H), 3.26 - 3.09 (m, 1H), 3.07 - 2.93 (m, 1H), 2.91 - 2.68 (m, 1H), 2.33 (t, *J=* 10.1 Hz, 1H), 2.18 - 2.04 (m, 1H), 1.92 - 1.74 (m, 3H), 1.51 - 1.34 (m, 2H), 1.30 - 1.19 (m, 6H), 1.18 - 0.95 (m, 6H). ¹³C NMR (75 MHz, CDCl₃) δ 156.7 (q), 133.9 (q), 127.0 (t), 126.5 (t), 120.5 (t), 110.4 (t), 79.3 (q), 74.8 (t), 57.9 (t), 56.5 (d), 55.3 (s), 40.8 (d), 37.6 (t), 36.2 (t), 31.3 (d), 26.6 (s), 24.9 (s), 20.0 (s), 13.8 (s).

### Example 156: rac-(3aR,5R,7S,7aR)-5-(2-ethylphenyl)-1,3,3,7-tetramethyloctahydrobenzo[c]isoxazole

Following the general procedure described in Example 81b: 4-(2-ethylphenyl)-2,7-dimethyloct-6-enal (658 mg, 1.91 mmol) (obtained from the corresponding α,β-unsaturated aldehyde using the general procedure described in Example 81a), *N-*methylhydroxylamine hydrochloride (239 mg, 2.86 mmol), K₂CO₃ (211 mg, 1.53 mmol) in toluene (40 mL) were reacted to give the title product (213 mg, 39 % yield) as a light yellow oil.

GC/MS (El): *m*/*z(%):* 287 (38) [*M*⁺], 272 (11), 185 (6), 171 (10), 143 (9), 126 (100), 117 (19), 100 (65), 87 (41). ¹H NMR (300 MHz, CDCl₃) δ 7.25 - 7.11 (m, 4H), 3.01 -2.83 (m, 3H), 2.70 (q, *J =* 7.4 Hz, 3H), 2.27 - 2.07 (m, 2H), 1.75 (M, 3H), 1.57 - 1.37 (m, 2H), 1.33 - 1.15 (m, 9H), 1.05 (M, 3H). ¹³C NMR (75 MHz, CDCl₃) δ 143.2 (q), 141.3 (q), 128.9 (t), 126.3 (t), 126.2 (t), 125.9 (t), 79.4 (q), 77.3 (t), 58.8 (t), 49.0 (s), 42.2 (d), 38.7 (t), 37.2 (t), 32.9 (d), 27.1 (s), 26.0 (d), 25.2 (s), 20.1 (s), 16.1 (s).

### Example 157: rac-(3aR,5R,7S,7aR)-5-(2-ethylphenyl)-1-isopropyl-3,3,7-trimethyloctahydrobenzo[c]isoxazole

Following the general procedure described in Example 81b: 4-(2-ethylphenyl)-2,7-dimethyloct-6-enal (528 mg, 1.53 mmol) (obtained from the corresponding α,β-unsaturated aldehyde using the general procedure described in Example 81a), N-isopropylhydroxylamine (173 mg, 2.30 mmol) in toluene (40 mL) were reacted to give the title product (165 mg, 34 % yield) as a light yellow oil.

GC/MS (El): *mlz* (%):315 (34) [*M*⁺], 300 (100), 258 (3), 185 (8), 171 (6), 154 (60), 128 (28), 115 (24), 91 (11). ¹H NMR (300 MHz, CDCl₃) δ 7.25 - 7.11 (m, 4H), 3.31 - 3.14 (m, 1H), 3.04 - 2.89 (m, 1H), 2.69 (q, *J =* 7.5 Hz, 2H), 2.54 (t, *J =* 9.9 Hz, 1H), 2.21 - 2.07 (m, 1H), 1.84 - 1.70 (m, 3H), 1.45 (q, *J =* 24.8, 12.6 Hz, 1H), 1.32 - 1.19 (m, 10H), 1.15 - 1.07 (m, 6H), 1.06 - 1.00 (m, 3H). ¹³C NMR (75 MHz, CDCl₃) δ 143.4 (q), 141.4 (q), 128.9 (t), 126.2 (t), 126.2 (t), 125.9 (t), 78.0 (q), 68.6 (t), 58.0 (t), 55.0 (t), 42.6 (d), 38.7 (t), 37.6 (t), 32.8 (d), 27.3 (s), 26.0 (d), 24.5 (s), 22.7 (s), 20.3 (s), 16.0 (s).

### Example 158: rac-(3aR,5R,7S,7aR)-5-(2,5-dimethylphenyl)-1,3,3,7-tetramethyloctahydrobenzo[c]isoxazole

Following the general procedure described in Example 81b: 4-(2,5-dimethylphenyl)-2,7-dimethyloct-6-enal (400 mg, 1.55 mmol) (obtained from the corresponding α,β-unsaturated aldehyde using the general procedure described in Example 81a), N-methylhydroxylamine hydrochloride (194 mg, 2.32 mmol), K₂CO₃ (0.171 mg, 1.238 mmol) in toluene (50 mL) were reacted to give the title product (125 mg, 28 % yield) as a yellow oil.

GC/MS (EI): *m*/*z* (%): 287 (100) [*M*⁺], 272 (20), 171 (17), 157 (15), 126 (75), 119 (20), 100 (50), 98 (27), 87 (52). ¹H NMR (300 MHz, CDCl₃) δ 7.09 - 7.02 (m, 2H), 6.97 - 6.90 (m, 1H), 3.01 - 2.62 (m, 4H), 2.33 (d, *J* = 2.3 Hz, 6H), 2.27 - 2.03 (m, 2H), 1.93 - 1.72 (m, 3H), 1.55 - 1.32 (m, 2H), 1.31 - 1.26 (m, 3H), 1.17 (s, 3H), 1.12 - 1.02 (m, 3H). ¹³C NMR (75 MHz, CDCl₃) δ 143.4 (q), 135.5 (q), 132.0 (q), 130.4 (t), 126.7 (t), 126.0 (t), 79.3 (q), 77.2 (t), 58.7 (t), 48.9 (s), 41.4 (d), 39.2 (t), 37.2 (t), 32.0 (d), 27.0 (s), 25.2 (s), 21.2 (s), 20.0 (s), 19.0 (s).

### Example 159: rac-(3aR,5R,7S,7aR)-5-(3-chloro-2-methylphenyl)-1-isopropyl-3,3,7-trimethyloctahydrobenzo[c]isoxazole

Following the general procedure described in Example 81b: 4-(3-chloro-2-methylphenyl)-2,7-dimethyloct-6-enal (300 mg, 1.08 mmol) (obtained from the corresponding α,β-unsaturated aldehyde using the general procedure described in Example 81a), *N-*isopropylhydroxylamine (121 mg, 1.61 mmol) in toluene (50 mL) were reacted to give the title product (90 mg, 25 % yield) as a light yellow oil.

GC/MS (EI): *m*/*z* (%): 337 (9) [*M*⁺], 335 (28) [*M*⁺], 322 (33), 320 (100), 154 (67), 141 (21), 139 (21), 126 (20), 115 (17). ¹H NMR (300 MHz, CDCl₃) δ 7.22 (d, *J =* 7.5 Hz, 1H), 7.17 - 7.05 (m, 2H), 3.29 - 3.14 (m, 1H), 3.06 - 2.89 (m, 1H), 2.52 (t, *J =* 9.9 Hz, 1H), 2.42 - 2.32 (m, 3H), 2.22 - 2.06 (m, 1H), 1.91 - 1.68 (m, 3H), 1.50 - 1.33 (m, 1H), 1.32 - 1.23 (m, 7H), 1.14 - 1.07 (m, 6H), 1.02 (d, *J =* 6.3 Hz, 3H). ¹³C NMR (75 MHz, CDCl₃) δ 145.8 (q), 135.2 (q), 133.5 (q), 127.1 (t), 126.7 (t), 123.9 (t), 77.9 (q), 68.5 (t), 57.8 (t), 54.9 (t), 41.8 (d), 40.2 (s), 37.5 (t), 31.9 (d), 27.3 (s), 24.5 (s), 22.7 (s), 20.2 (s), 15.7 (s), 13.7 (s).

### Example 160: rac-(3aR,5R,7S,7aR)-5-(2,4-dimethylphenyl)-1,3,3,7-tetramethyloctahydrobenzo[c]isoxazole

Following the general procedure described in Example 81b: 4-(2,4-dimethylphenyl)-2,7-dimethyloct-6-enal (400 mg, 1.55 mmol) (obtained from the corresponding α,β-unsaturated aldehyde using the general procedure described in Example 81a), N-methylhydroxylamine hydrochloride (194 mg, 2.32 mmol), K₂CO₃ (171 mg, 1.24 mmol) in toluene (50 mL) were reacted to give the title product (170 mg, 38 % yield) as a yellow oil.

GC/MS (EI): *m*/*z* (%): 287 (100) [*M*⁺], 288 (21), 171 (20), 126 (74), 119 (21), 100 (46), 98 (25), 87 (47). ¹H NMR (300 MHz, CDCl₃) δ 7.18 - 7.10 (m, 1H), 7.06 - 6.96 (m, 2H), 2.99 - 2.64 (m, 4H), 2.39 - 2.26 (m, 6H), 2.26 - 2.07 (m, 2H), 1.93 - 1.72 (m, 3H), 1.54 - 1.37 (m, 2H), 1.29 (s, 3H), 1.19 (s, 3H), 1.05 (d, *J =* 6.3 Hz, 3H). ¹³C NMR (75 MHz, CDCl₃) δ 140.6 (q), 135.3 (q), 135.0 (q), 131.2 (t), 126.8 (t), 125.2 (t), 79.3 (q), 77.2 (t), 58.7 (t), 48.9 (s), 41.5 (d), 38.9 (t), 37.1 (t), 32.0 (s), 27.0 (s), 25.1 (s), 20.8 (s), 19.9 (s), 19.3 (s).

### Example 161: rac-4-methyl-3-((3aR,5R,7S,7aR)-1,3,3,7-tetramethyloctahydrobenzo[c]isoxazol-5-yl)benzonitrile

Following the general procedure described in Example 81b: 3-(2,7-dimethyl-1-oxooct-6-en-4-yl)-4-methylbenzonitrile (492 mg, 1.08 mmol) (obtained from the corresponding α,β-unsaturated aldehyde using the general procedure described in Example 81a), *N-*methylhydroxylamine hydrochloride (135 mg, 1.62 mmol), K₂CO₃ (119 mg, 0.86 mmol) in toluene (50 mL) were reacted to give the title product (115 mg, 36 % yield) as a light yellow oil.

GC/MS (EI): *m*/*z* (%): 298 (50) [*M*⁺], 283 (24), 252 (10), 196 (10), 182 (10), 142 (10), 126 (100), 113 (18), 100 (79), 87 (63). ¹H NMR (300 MHz, CDCl₃) δ 7.54 (s, 1H), 7.47 - 7.39 (m, 1H), 7.29 (s, 1H), 3.05 - 2.64 (m, 4H), 2.45 (s, 3H), 2.30 - 2.03 (m, 2H), 1.92 - 1.76 (m, 3H), 1.57 - 1.38 (m, 2H), 1.34 - 1.28 (m, 3H), 1.23 (s, 3H), 1.08 (d, *J =* 6.2 Hz, 3H). ¹³C NMR (75 MHz, CDCl₃) δ 145.2 (q), 141.3 (q), 131.3 (t), 129.7 (t), 129.5 (t), 119.4 (q), 110.2 (q), 79.4 (q), 77.3 (t), 58.6 (t), 49.1 (s), 41.3 (d), 39.3 (t), 37.2 (t), 31.8 (d), 27.1 (s), 25.2 (s), 20.1 (s), 20.0 (s).

### Example 162: rac-(3aR,5R,7S,7aR)-5-(5-chloro-2-methylphenyl)-1-isopropyl-3,3,7-trimethyloctahydrobenzo[c]isoxazole

Following the general procedure described in Example 81b: 4-(5-chloro-2-methylphenyl)-2,7-dimethyloct-6-enal (600 mg, 2.15 mmol) (obtained from the corresponding α,β-unsaturated aldehyde using the general procedure described in Example 81a), *N-*isopropylhydroxylamine (242 mg, 3.23 mmol) in xylene (50 mL) were reacted to give the title product (211 mg, 29 % yield) as a light yellow oil.

GC/MS (El): *m*/*z* (%): 337 (9) [*M*⁺], 335 (27) [*M*⁺], 322 (33), 320 (100), 154 (68), 141 (19), 139 (21), 126 (18), 115 (15). ¹H NMR (300 MHz, CDCl₃) δ 7.16 (s, 1H), 7.02 (s, 2H), 3.26 - 3.11 (m, 1H), 2.85 (t, 1H), 2.56 - 2.42 (m, 1H), 2.26 (s, 3H), 2.18 - 1.95 (m, 2H), 1.85 - 1.62 (m, 3H), 1.44 - 1.28 (m, 1H), 1.26 - 1.19 (m, 6H), 1.12 - 1.04 (m, 6H), 0.99 (d, *J =* 6.3 Hz, 3H). ¹³C NMR (75 MHz, CDCl₃) δ 145.7 (q), 133.6 (q), 131.7 (q), 131.6 (t), 125.8 (t), 125.5 (t), 77.8 (q), 68.3 (t), 57.7 (t), 54.8 (t), 41.5 (d), 39.4 (t), 37.4 (t), 31.6 (d), 27.2 (s), 24.4 (s), 22.5 (s), 20.0 (s), 18.8 (s), 13.6 (s).

### Example 163: rac-(3aR,5R,7S,7aR)-5-(5-fluoro-2-methylphenyl)-1,3,3,7-tetramethyloctahydrobenzo[c]isoxazole

Following the general procedure described in Example 81b: 4-(5-fluoro-2-methylphenyl)-2,7-dimethyloct-6-enal (300 mg, 1.14 mmol) (obtained from the corresponding α,β-unsaturated aldehyde using the general procedure described in Example 81a), *N*-methylhydroxylamine hydrochloride (143 mg, 1.72 mmol), K₂CO₃ (126 mg, 0.92 mmol) in toluene (50 mL) were reacted to give the title product (102 mg, 31 % yield) as a yellow oil.

GC/MS (El): *m*/*z* (%): 291 (100) [*M*⁺], 276 (33), 175 (17), 136 (23), 126 (83), 100 (62), 98 (32), 87 (55). ¹H NMR (300 MHz, CDCl₃) δ 7.11 - 7.01 (m, 1H), 6.94 - 6.85 (m, 1H), 6.81 - 6.70 (m, 1H), 2.97 - 2.57 (m, 4H), 2.28 (s, 3H), 2.22 - 1.99 (m, 2H), 1.89 - 1.68 (m, 3H), 1.46 - 1.29 (m, 2H), 1.24 (s, 3H), 1.16 (s, 3H), 1.02 (d, *J* = 6.3 Hz, 3H). ¹³C NMR (75 MHz, CDCl₃) δ 163.2 (q), 160.0 (q), 145.8 (q), 145.7 (q), 131.6 (t), 131.5 (t), 130.6 (t), 112.7 (t), 112.4 (t), 112.4 (t), 112.1 (t), 79.3 (q), 77.1 (t), 58.6 (t), 48.9 (s), 41.2 (d), 39.5 (t), 37.1 (t), 31.8 (d), 27.1 (s), 25.1 (s), 19.9 (s), 18.7 (s).

### Example 164: rac-(3aR,5R,7S,7aR)-5-(4-fluoro-2-methylphenyl)-1,3,3,7-tetramethyloctahydrobenzo[c]isoxazole

Following the general procedure described in Example 81b: 4-(4-fluoro-2-methylphenyl)-2,7-dimethyloct-6-enal (300 mg, 1.14 mmol) (obtained from the corresponding α,β-unsaturated aldehyde using the general procedure described in Example 81a), *N*-methylhydroxylamine hydrochloride (143 mg, 1.72 mmol), K₂CO₃ (126 mg, 0.92 mmol) in toluene (50 mL) were reacted to give the title product (115 mg, 35 % yield) as a yellow oil.

GC/MS (EI): *m*/*z* (%): 291 (100) [*M*⁺], 276 (27), 136 (28), 126 (83), 123 (28), 100 (57), 98 (31), 87 (52), 70 (15). ¹H NMR (300 MHz, CDCl₃) δ 7.19 - 7.10 (m, 1H), 6.88 - 6.77 (m, 2H), 2.93 - 2.59 (m, 4H), 2.31 (s, 3H), 2.23 - 2.03 (m, 2H), 1.88 - 1.67 (m, 3H), 1.50 - 1.30 (m, 2H), 1.28 - 1.19 (m, 3H), 1.15 (s, 3H), 1.05 - 0.97 (m, 3H). ¹³C NMR (75 MHz, CDCl₃) δ 162.4 (q), 159.2 (q), 139.3 (q), 139.3 (q), 137.5 (q), 137.4 (q), 126.8 (t), 126.6 (t), 117.0 (t), 116.7 (t), 112.8 (t), 112.5 (t), 79.3 (q), 79.1 (t), 58.7 (t), 48.9 (s), 41.6 (t), 38.8 (t), 37.1 (t), 32.1 (d), 27.0 (s), 25.1 (s), 19.9 (s), 19.5 (s).

### Example 165: rac-(3aR,5R,7S,7aR)-5-(4-chloro-2-methylphenyl)-1-isopropyl-3,3,7-trimethyloctahydrobenzo[c]isoxazole

Following the general procedure described in Example 81b: 4-(4-chloro-2-methylphenyl)-2,7-dimethyloct-6-enal (700 mg, 2.51 mmol) (obtained from the corresponding α,β-unsaturated aldehyde using the general procedure described in Example 81a), *N-*isopropylhydroxylamine (283 mg, 3.77 mmol) in xylene (50 mL) were reacted to give the title product (288 mg, 34 % yield) as a light yellow oil.

GC/MS (EI): *m*/*z* (%): 337 (10) [*M*⁺], 335 (30) [*M*⁺], 322 (33), 320 (100), 154 (73), 141 (22), 139 (31), 126 (19), 115 (16). ¹H NMR (300 MHz, CDCl₃) δ 7.17-7.05 (m, 3H), 3.27 - 3.12 (m, 1H), 2.86 (t, *J =* 12.0, 7.8, 3.3 Hz, 1H), 2.50 (t, *J =* 9.9 Hz, 1H), 2.31 (s, 3H), 2.18 - 2.04 (m, 2H), 1.84 - 1.63 (m, 3H), 1.44 - 1.31 (m, 1H), 1.28 - 1.20 (m, 6H), 1.12 - 1.04 (m, 6H), 1.00 (d, *J =* 6.4 Hz, 3H). ¹³C NMR (75 MHz, CDCl₃) δ 142.3 (q), 137.2 (q), 131.2 (q), 130.1 (t), 126.7 (t), 126.1 (t), 77.9 (q), 68.4 (t), 57.8 (t), 54.9 (t), 41.7 (d), 38.9 (t), 37.4 (t), 31.8 (d), 27.2 (s), 24.4 (s), 22.6 (s), 20.1 (s), 19.3 (s), 13.6 (s).

### Example 166: rac-(3aR,5R,7S,7aR)-1-isopropyl-5-(2-methoxyphenyl)-3,3,7-trimethyloctahydrobenzo[c]isoxazole

Following the general procedure described in Example 81b: 4-(2-methoxyphenyl)-2,7-dimethyloct-6-enal (525 mg, 2.02 mmol) (obtained from the corresponding α,β-unsaturated aldehyde using the general procedure described in Example 81a), N-isopropylhydroxylamine (227 mg, 3.02 mmol) in toluene (40 mL) were reacted to give the title product (280 mg, 44 % yield) as a colorless oil.

GC/MS (EI): *m*/*z(%):* 317 (35) [*M*⁺], 302 (100), 187 (16), 154 (89),141 (14), 128 (33), 126 (46), 121 (49), 112 (18), 91 (41). ¹H NMR (300 MHz, CDCl₃) δ 7.24 - 7.12 (m, 2H), 6.92 (t, *J =* 7.4 Hz, 1H), 6.85 (d, *J =* 8.2 Hz, 1H), 3.87 - 3.74 (m, 3H), 3.30 - 3.11 (m, 2H), 2.51 (t, *J* = 9.8 Hz, 1H), 2.19 - 2.09 (m, 1H), 1.93 - 1.74 (m, 3H), 1.62 - 1.32 (m, 2H), 1.31 - 1.21 (m, 6H), 1.16 - 0.95 (m, 9H). ¹³C NMR (75 MHz, CDCl₃) δ 156.7 (q), 133.9 (q), 126.9 (t), 126.5 (t), 120.5 (t), 110.4 (t), 78.0 (q), 68.5 (t), 57.5 (t), 55.2 (s), 54.7 (t), 41.1 (d), 37.2 (t), 36.0 (t), 31.2 (d), 27.2 (s), 24.3 (s), 22.4 (s), 20.1 (s), 13.6 (s).

### Example 167: rac-(3aR,5R,7S,7aR)-5-(2-methoxyphenyl)-1,3,3,7-tetramethyloctahydrobenzo[c]isoxazole

Following the general procedure described in Example 81b: 4-(2-methoxyphenyl)-2,7-dimethyloct-6-enal (300 mg, 1.15 mmol) (obtained from the corresponding α,β-unsaturated aldehyde using the general procedure described in Example 81a), N-methylhydroxylamine hydrochloride (144 mg, 1.73 mmol), K₂CO₃ (127 mg, 0.92 mmol) in toluene (50 mL) were reacted to give the title product (155 mg, 47 % yield) as a light yellow oil.

GC/MS (El): *m*/*z* (%): 289 (65) [*M*⁺], 274 (16), 243 (6), 187 (11),126 (100), 121 (38), 100 (58), 91 (38), 87 (55),70 (13). ¹H NMR (300 MHz, CDCl₃) δ 7.18 (t, *J=* 8.0 Hz, 2H), 7.01 - 6.78 (m, 2H), 3.82 (s, 3H), 3.27 - 3.06 (m, 1H), 2.91 - 2.55 (m, 3H), 2.23 - 2.07 (m, 2H), 1.95 - 1.71 (m, 3H), 1.53 - 1.28 (m, 2H), 1.27 - 0.95 (m, 9H). ¹³C NMR (75 MHz, CDCl₃) δ 156.7 (q), 133.8 (q), 127.0 (t), 126.5 (t), 120.5 (t), 110.4 (t), 79.4 (q), 77.2 (t), 58.6 (t), 55.3 (s), 48.9 (s), 40.7 (d), 37.0 (t), 36.2 (t), 31.3 (d), 27.0 (s), 25.1 (s), 20.0 (s).

### Example 168: rac-(3aR,5R,7S,7aR)-1-isopropyl-3,3,7-trimethyl-5-(o-tolyl)octahydrobenzo[c]isoxazole

Following the general procedure described in Example 81b: 2,7-dimethyl-4-(o-tolyl)oct-6-enal (258 mg, 1.06 mmol) (obtained from the corresponding α,β-unsaturated aldehyde using the general procedure described in Example 81a), N-isopropylhydroxylamine (238 mg, 3.17 mmol) in xylene (80 mL) were reacted to give the title product (208 mg, 66 % yield) as a light yellow oil.

GC/MS (EI): *m*/*z* (%): 301(63) [*M*⁺], 286 (100), 244 (4), 171 (17), 154 (71), 126 (38), 115 (30), 105 (37), 91 (13). ¹H NMR (300 MHz, CDCl₃) δ 7.28 - 7.05 (m, 4H), 3.23 (dd, *J =* 13.0, 6.5 Hz, 1H), 3.03 - 2.83 (m, 1H), 2.54 (t, *J =* 9.9 Hz, 1H), 2.35 (s, 3H), 2.23 - 2.04 (m, 2H), 1.78 (d, *J =* 12.4 Hz, 3H), 1.43 (dd, *J =* 24.5, 12.3 Hz, 1H), 1.26 (dd, *J =* 13.5, 6.7 Hz, 7H), 1.10 (t, *J* = 7.5 Hz, 5H), 1.07-0.96 (m, 3H). ¹³C NMR (75 MHz, CDCl₃) δ 143.9 (q), 135.3 (q), 130.5 (t), 126.3 (t), 126.0 (t), 125.4 (t), 78.1 (q), 68.5 (t), 57.9 (t), 54.9 (t), 41.8 (d), 39.3 (t), 37.5 (t), 31.9 (d), 27.3 (s), 24.5 (s), 22.6 (s), 20.2 (s), 19.5 (s), 13.6 (s).

### Example 169 (not in accordance with the invention): rac-(3aR,7S,7aR)-5,5-diethyl-1,3,3,7-tetramethyloctahydrobenzor[c]lisoxazole

Following the general procedure described in Example 81b: 4,4-diethyl-2,7-dimethyloct-6-enal (500 mg, 2.38 mmol) (obtained from the corresponding α,β-unsaturated aldehyde using the general procedure described in Example 81a), *N-*methylhydroxylamine hydrochloride (298 mg, 3.57 mmol), K₂CO₃ (263 mg, 1.90 mmol) in toluene (50 mL) were reacted to give the title product (236 mg, 42 % yield) as a colorless oil.

GC/MS (El): *m*/*z* (%): 239 (44) [*M*⁺], 224 (36), 210 (20), 193 (14), 137 (17), 126 (100), 100 (50), 55 (16). ¹H NMR (300 MHz, CDCl₃) δ 2.77 - 2.52 (m, 3H), 1.97 (ddd, *J* = 31.6, 17.0, 6.8 Hz, 2H), 1.76 - 1.63 (m, 1H), 1.34 (ddd, *J* = 13.5, 10.2, 7.7 Hz, 4H), 1.14 (dd, *J* = 26.2, 20.4 Hz, 7H), 0.98 - 0.81 (m, 5H), 0.80 - 0.59 (m, 7H). ¹³C NMR (75 MHz, CDCl₃) δ 79.2 (q), 78.1 (t), 53.1 (t), 48.8 (s), 43.9 (d), 36.5 (q), 33.4 (d), 32.9 (d), 32.4 (t), 26.9 (s), 25.4 (d), 24.8 (s), 20.2 (s), 7.6 (s), 7.5 (s).

### Example 170: rac-(3aR,5R,7aR)-5-ethyl-1-isopropyl-3,3-dimethyloctahydrobenzo[c]isoxazole

Following the general procedure described in Example 81b: 4-ethyl-7-methyloct-6-enal (1.00 g, 4.22 mmol) (obtained from the corresponding α,β-unsaturated aldehyde using the general procedure described in Example 81a), *N*-isopropylhydroxylamine hydrochloride (0.71 g, 6.33 mmol), K₂CO₃ (0.47 g, 3.38 mmol) in toluene (50 mL) were reacted to give the title product (0.47 g, 50 % yield) as a light yellow oil.

GC/MS (El): *m*/*z* (%): 225 (18) [*M*⁺], 210 (100), 151 (7), 114 (21), 95 (29), 81 (11), 67 (8). ¹H NMR (300 MHz, CDCl₃) δ 3.06 - 2.80 (m, 1H), 2.48 (dd, *J* = 10.8, 3.5 Hz, 1H), 1.96 - 1.85 (m, 1H), 1.82 - 1.73 (m, 1H), 1.70 - 1.62 (m, 1H), 1.55 - 1.15 (m, 8H), 1.13 - 0.99 (m, 9H), 0.93 - 0.75 (m, 5H). ¹³C NMR (75 MHz, CDCl₃) δ 78.5 (q), 66.4 (t), 57.8 (t), 57.2 (t), 39.0 (t), 31.3 (d), 31.2 (d), 30.9 (d), 29.5 (d), 26.9 (s), 24.6 (s), 20.6 (s), 19.3 (s), 11.7 (s).

### Example 171: rac-(3aR,7aS)-1,3,3,4,5,7-hexamethyloctahydrobenzo[c]isoxazole

Following the general procedure described in Example 81b: 2,4,5,7-tetramethyloct-6-enal (235 mg, 1.08 mmol) (obtained from the corresponding α,β-unsaturated aldehyde using the general procedure described in Example 81a), *N*-methylhydroxylamine hydrochloride (136 mg, 1.62 mmol), K₂CO₃ (120 mg, 0.87 mmol) in toluene (50 mL) were reacted to give the title product (77 mg, 34 % yield) as a colorless oil.

GC/MS (EI): *m*/*z*(%): 211 (63) [*M*⁺], 196 (91), 165 (10), 140 (100), 123 (19), 109 (25), 100 (80), 84 (29), 69 (16), 55 (33). ¹H NMR (300 MHz, CDCl₃) δ 2.71 - 2.57 (m, 3H), 2.50 - 2.39 (m, 1H), 1.91 - 1.73 (m, 2H), 1.70 - 1.55 (m, 2H), 1.39 - 1.25 (m, 8H), 0.99 - 0.85 (m, 9H). ¹³C NMR (75 MHz, CDCl₃) δ 81.1 (q), 74.7 (t), 57.2 (t), 46.2 (s), 37.4 (d), 34.8 (t), 32.8 (t), 30.6 (s), 29.4 (t), 23.7 (s), 20.6 (s), 19.9 (s), 19.2 (s).

### Example 172: rac-(3aR,7aR)-1,3,3,4,5,7-hexamethyloctahydrobenzo[c]isoxazole

Following the general procedure described in Example 81b: 2,4,5,7-tetramethyloct-6-enal (235 mg, 1.08 mmol) (obtained from the corresponding α,β-unsaturated aldehyde using the general procedure described in Example 81a), *N*-methylhydroxylamine hydrochloride (136 mg, 1.62 mmol), K₂CO₃ (120 mg, 0.87 mmol) in toluene (50 mL) were reacted to give the title product (77 mg, 34 % yield) as a colorless oil.

GC/MS (EI): *m*/*z*(%): 211 (55) [*M*⁺], 196 (61), 165 (14), 140 (55), 123 (19), 109 (28), 100 (100), 87 (24), 69 (16), 55 (25). ¹H NMR (300 MHz, CDCl₃) δ 2.85 - 2.51 (m, 3H), 2.17 - 1.98 (m, 1H), 1.78 - 1.56 (m, 3H), 1.49 - 1.39 (m, 1H), 1.36 - 1.23 (m, 3H), 1.21 - 1.07 (m, 5H), 1.02 - 0.88 (m, 9H). ¹³C NMR (75 MHz, CDCl₃) δ 79.8 (q), 77.8 (t), 63.3 (t), 49.0 (s), 44.2 (d), 39.0 (t), 38.7 (t), 36.6 (t), 29.1 (s), 24.7 (s), 20.1 (s), 19.1 (s), 16.2 (s).

### Example 173: rac-(3aR,5R,7aR)-1-ethyl-3,3-dimethyl-5-propyloctahydrobenzo[c]isoxazole

Following the general procedure described in Example 81b: 7-methyl-4-propyloct-6-enal (700 mg, 3.84 mmol) (obtained from the corresponding α,β-unsaturated aldehyde using the general procedure described in Example 81a), *N*-ethylhydroxylamine hydrochloride (562 mg, 5.76 mmol), K₂CO₃ (425 mg, 3.07 mmol) in toluene (50 mL) were reacted to give the title product (310 mg, 36 % yield) as a colorless oil.

GC/MS (EI): *m*/*z* (%): 225 (50) [*M*⁺], 210 (100), 165 (25), 123 (12), 109 (36), 100 (87), 95 (24), 81 (17), 74 (17), 67 (15). ¹H NMR (300 MHz, CDCl₃) δ 2.91 (dd, *J =* 12.7, 7.1 Hz, 1H), 2.65 (dq, *J =* 13.7, 6.9 Hz, 1H), 2.37 - 2.19 (m, 1H), 1.83 (t, *J =* 10.2 Hz, 3H), 1.69 (d, *J =* 12.3 Hz, 1H), 1.41 - 1.12 (m, 12H), 1.06 (s, 3H), 0.98 - 0.70 (m, 5H). ¹³C NMR (75 MHz, CDCl₃) δ 79.2 (q), 69.9 (t), 57.2 (t), 53.1 (d), 39.2 (d), 37.2 (t), 31.6 (d), 31.1 (d), 29.3 (d), 27.6 (s), 25.2 (s), 20.3, 14.4 (s), 13.1 (s).

### Example 174: rac-(3aR,5R,7S,7aR)-1,3,3,5,7-pentamethyl-5-propyloctahydrobenzo[c]isoxazole

Following the general procedure described in Example 81b: 2,4,7-trimethyl-4-propyloct-6-enal (842 mg, 3.12 mmol) (obtained from the corresponding α,β-unsaturated aldehyde using the general procedure described in Example 81a), *N-*methylhydroxylamine hydrochloride (391 mg, 4.68 mmol), K₂CO₃ (345 mg, 2.50 mmol) in toluene (50 mL) were reacted to give the title product (246 mg, 33 % yield) as a colorless oil.

GC/MS (EI): *m*/*z* (%): 239 (42) [*M*⁺], 224 (50), 193 (15), 137 (18), 126 (100), 109 (14), 100 (64), 81 (13), 69 (18), 55 (23). ¹H NMR (300 MHz, CDCl₃) δ 2.81 - 2.54 (m, 3H), 2.20 - 1.85 (m, 2H), 1.82 - 1.63 (m, 1H), 1.42 - 1.13 (m, 9H), 1.05 (d, *J =* 13.8 Hz, 3H), 1.01 - 0.79 (m, 11H). ¹³C NMR (75 MHz, CDCl₃) δ 79.4 (q), 78.2 (t), 53.8 (t), 48.9 (t), 48.6 (d), 46.7 (d), 36.5 (d), 34.5 (q), 32.9 (t), 26.9 (s), 24.9 (s), 23.6 (s), 20.2 (s), 16.8 (d), 15.1 (s).

### Example 175: rac-(3aR,5R,7aR)-1,3,3-trimethyl-5-propyloctahydrobenzo[c]isoxazole

Following the general procedure described in Example 81b: 7-methyl-4-propyloct-6-enal (859 mg, 4.50 mmol) (obtained from the corresponding α,β-unsaturated aldehyde using the general procedure described in Example 81a), *N*-methylhydroxylamine hydrochloride (564 mg, 6.76 mmol), K₂CO₃ (498 mg, 3.60 mmol) in toluene (50 mL) were reacted to give the title product (200 mg, 21 % yield) as a colorless oil.

GC/MS (EI): *m*/*z(%):* 211 (23) [*M*⁺], 210 (100), 196 (33), 165 (48), 153 (85), 126 (61), 109 (33), 96 (41), 82 (66), 67 (34), 55 (26). ¹H NMR (300 MHz, CDCl₃) δ 2.62 (s, 3H), 2.11 (td, *J =* 10.9, 3.1 Hz, 1H), 1.78 (dd, *J =* 15.9, 7.2 Hz, 3H), 1.66 (d, *J =* 12.3 Hz, 1H), 1.38 - 1.13 (m, 9H), 1.05 (s, 3H), 0.95 - 0.73 (m, 5H). ¹³C NMR (75 MHz, CDCl₃) δ 79.4 (q), 72.0 (t), 57.6 (s), 44.8 (s), 39.1 (d), 37.1 (t), 31.5 (d), 30.9 (d), 28.5 (d), 27.9 (s), 25.2 (s), 20.3 (d), 14.4 (s).

### Example 176: rac-(3aR,5R,7S,7aR)-1-isopropyl-3,3,5,7-tetramethyl-5-propyloctahydrobenzo[c]isoxazole

Following the general procedure described in Example 81b: 2,4,7-trimethyl-4-propyloct-6-enal (748 mg, 2.77 mmol) (obtained from the corresponding α,β-unsaturated aldehyde using the general procedure described in Example 81a), N-isopropylhydroxylamine (312 mg, 4.16 mmol), K₂CO₃ (383 mg, 2.77 mmol) in toluene (50 mL) were reacted to give the title product (216 mg, 29 % yield) as a colorless oil.

GC/MS (El): *m*/*z* (%): 267 (15) [*M*⁺], 252 (100), 210 (5), 154 (48), 137 (10), 128 (18), 95 (13), 81 (10), 69 (14), 55 (20). ¹H NMR (300 MHz, CDCl₃) δ 3.26 - 3.05 (m, 1H), 2.35 - 2.22 (m, 1H), 2.20 - 2.07 (m, 1H), 1.83 - 1.54 (m, 2H), 1.35 - 1.25 (m, 4H), 1.24 (d, *J* = 7.2 Hz, 5H), 1.19 (dd, *J* = 11.9, 2.8 Hz, 3H), 1.04 (d, *J =* 6.2 Hz, 3H), 1.01 (s, 3H), 0.96 - 0.90 (m, 7H), 0.89 - 0.85 (m, 3H). ¹³C NMR (75 MHz, CDCl₃) δ 78.1 (q), 69.5 (t), 54.8 (t), 52.7 (t), 48.6 (d), 47.0 (d), 36.4 (d), 34.4 (q), 33.0 (t), 27.1 (s), 24.2 (s), 23.6 (s), 22.5 (s), 20.4 (s), 16.8 (d), 15.1 (s),13.6 (s).

### Example 177: rac-(3aR,5R,7S,7aR)-1,5,7-triethyl-3,3-dimethyloctahydrobenzo[c]isoxazole

Following the general procedure described in Example 81b: 2,4-diethyl-7-methyloct-6-enal (2.01 g, 2.04 mmol) (obtained from the corresponding α,β-unsaturated aldehyde using the general procedure described in Example 81a), *N*-ethylhydroxylamine (0.19 g, 3.06 mmol), K₂CO₃ (0.23 g, 1.63 mmol) in toluene (50 mL) were reacted to give the title product (0.39 g, 78 % yield) as a coloeless oil.

GC/MS (El): *m*/*z(%):* 239 (24) [*M*⁺], 224 (100), 179 (6), 140 (24), 128 (18), 112 (11), 74 (14). ¹H NMR (300 MHz, CDCl₃) δ 2.93 - 2.53 (m, 2H), 2.21 (t, *J =* 10.0 Hz, 1H), 2.02 - 1.74 (m, 2H), 1.74 - 1.35 (m, 4H), 1.34 - 1.19 (m, 4H), 1.15 (dd, *J =* 7.9, 5.6 Hz, 5H), 1.08 - 0.94 (m, 4H), 0.91 - 0.72 (m, 7H). ¹³C NMR (75 MHz, CDCl₃) δ 79.3 (q), 73.7 (t), 57.9 (t), 56.7 (d), 43.9 (t), 39.1 (t), 36.5 (d), 31.2 (d), 29.5 (d), 26.4 (s), 26.2 (d), 24.6 (s), 13.9 (s), 11.7 (s), 10.7 (s).

### Example 178: rac-(3aR,5R,7S,7aR)-1-ethyl-3,3,5,7-tetramethyloctahydrobenzo[c]isoxazole

Following the general procedure described in Example 81b: 2,4,7-trimethyloct-6-enal (400 mg, 2.38 mmol) (obtained from the corresponding α,β-unsaturated aldehyde using the general procedure described in Example 81a), *N*-ethylhydroxylamine hydrochloride (348 mg, 3.57 mmol), K₂CO₃ (263 mg, 1.90 mmol) in toluene (50 mL) were reacted to give the title product (123 mg, 24 % yield) as a light yellow oil.

GC/MS (EI): *m*/*z(%):* 211 (23) [*M*⁺], 196 (100), 151 (10), 140 (16), 114 (27), 109 (11), 95 (14). ¹H NMR (300 MHz, CDCl₃) δ 3.08 - 2.53 (m, 2H), 2.14 (t, *J =* 10.1 Hz, 1H), 1.90 (t, *J =* 10.8 Hz, 1H), 1.72 - 1.44 (m, 4H), 1.19 (dd, *J* = 9.1, 4.8 Hz, 6H), 1.05 (d, *J =* 16.4 Hz, 3H), 0.95 (t, *J =* 8.4 Hz, 6H), 0.88 - 0.63 (m, 2H). ¹³C NMR (75 MHz, CDCl₃) δ 79.2 (q), 74.9 (t), 57.8 (t), 56.5 (d), 43.4 (d), 37.2 (t), 33.8 (d), 32.6 (t), 26.7 (s), 24.9 (s), 21.9 (s), 20.2 (s), 13.8 (s).

### Example 179: rac-(3aR,5R,7aR)-5-butyl-1-isopropyl-3,3-dimethyloctahydrobenzo[c]isoxazole

Following the general procedure described in Example 81b: 4-butyl-7-methyloct-6-enal (700 mg, 3.57 mmol) (obtained from the corresponding α,β-unsaturated aldehyde using the general procedure described in Example 81a), *N-*isopropylhydroxylamine hydrochloride (597 mg, 5.35 mmol), K₂CO₃ (394 mg, 2.85 mmol) in toluene (50 mL) were reacted to give the title product (42 mg, 5 % yield) as a brown oil.

GC/MS (EI): *m*/*z* (%): 253(15) [*M*⁺], 238 (100), 196 (3), 123 (8), 114 (16), 95 (8), 81 (9), 67 (7), 55 (6). ¹H NMR (300 MHz, CDCl₃) δ 3.07 - 2.79 (m, 1H), 2.51 (td, *J =* 10.8, 3.2 Hz, 1H), 1.84 (ddd, *J* = 25.2, 15.4, 7.9 Hz, 3H), 1.67 (d, *J =* 14.1 Hz, 1H), 1.26 (dd, *J =* 25.5, 6.2 Hz, 11H), 1.13 - 0.98 (m, 9H), 0.93 - 0.75 (m, 5H). ¹³C NMR (75 MHz, CDCl₃) δ 78.6 (q), 66.4 (t), 57.7 (t), 57.1 (t), 37.3 (t), 36.6 (d), 31.7 (d), 31.3 (d), 31.1 (d), 29.4 (d), 26.9 (s), 24.6 (s), 23.0 (d), 20.5 (s), 19.1 (s), 14.1 (s).

### Example 180: rac-(3aR,5R,7aR)-5-butyl-1-ethyl-3,3-dimethyloctahydrobenzo[c]isoxazole

Following the general procedure described in Example 81b: 4-butyl-7-methyloct-6-enal (812 mg, 4.14 mmol) (obtained from the corresponding α,β-unsaturated aldehyde using the general procedure described in Example 81a), *N*-ethylhydroxylamine hydrochloride (605 mg, 6.20 mmol), K₂CO₃ (457 mg, 3.31 mmol) in toluene (50 mL) were reacted to give the title product (519 mg, 52 % yield) as a colorless oil.

GC/MS (El): *m*/*z(%):* 239 (44) [*M*⁺], 224 (100), 179 (17), 140 (8), 123 (20), 109 (21), 100 (82), 81 (20), 67 (16). ¹H NMR (300 MHz, CDCl₃) δ 2.90 (dd, *J* = 12.7, 7.1 Hz, 1H), 2.64 (dd, *J =* 12.7, 6.8 Hz, 1H), 2.35 -2.19 (m, 1H), 1.90 - 1.75 (m, 3H), 1.68 (d, *J =* 12.5 Hz, 1H), 1.39 -1.19 (m, 12H), 1.16 (t, *J* = 7.1 Hz, 3H), 1.05 (s, 3H), 0.91 - 0.81 (m, 4H). ¹³C NMR (75 MHz, CDCl₃) δ 79.1 (q), 69.9 (t), 57.2 (t), 53.1 (d), 37.5 (t), 36.6 (d), 31.6 (d), 31.1 (d), 29.5 (d), 29.3 (d), 27.5 (s), 25.2 (s), 23.0 (d), 14.1 (s), 13.1 (s).

### Example 181: 1,3,3-trimethyl-6-((R)-4-methylcyclohex-3-en-1-yl)octahydrobenzo[c]isoxazole

Following the general procedure described in Example 81b: 7-methyl-3-((*R*)-4-methylcyclohex-3-en-1-yl)oct-6-enal (700 mg, 2.99 mmol), *N*-methylhydroxylamine hydrochloride (374 mg, 4.48 mmol), K₂CO₃ (330 mg, 2.39 mmol) in toluene (50 mL) were reacted to give the title product (648 mg, 82 % yield) as a light yellow oil.

GC/MS (El): *m*/*z* (%): 263 (100) [*M*⁺], 248 (23), 217 (19), 166 (32), 121 (22), 112 (22), 95 (40), 81 (28), 67 (30), 55 (20). ¹H NMR (300 MHz, CDCl₃) δ 5.29 (s, 1H), 2.59 (s, 3H), 2.11 (d, *J =* 9.2 Hz, 2H), 1.99 - 1.61 (m, 8H), 1.56 (s, 3H), 1.41 - 1.11 (m, 7H), 1.09 - 0.86 (m, 5H). ¹³C NMR (75 MHz, CDCl₃) δ 133.9 (q), 120.6 (t), 79.3 (q), 71.9 (t), 57.8 (t), 44.5 (s), 40.4 (t), 38.6 (t), 38.5 (t), 32.2 (d), 32.0 (d), 30.8 (t), 30.7 (d), 29.3 (d), 29.2 (d), 29.1 (d), 27.9 (s), 26.9 (d), 26.6 (d), 25.1 (s), 24.4 (d), 23.4 (s).

### Example 182: rac-(3aR,5R,7aR)-1,3,3-trimethyl-5-pentyloctahvdrobenzo[c]isoxazole

Following the general procedure described in Example 81b: 4-(3-methylbut-2-en-1-yl)nonanal (412 mg, 1.74 mmol) (obtained from the corresponding α,β-unsaturated aldehyde using the general procedure described in Example 81a), N-methylhydroxylamine hydrochloride (218 mg, 2.61 mmol), K₂CO₃ (193 mg, 1.39 mmol) in toluene (50 mL) were reacted to give the title product (295 mg, 70 % yield) as a colorless oil.

GC/MS (El): *m*/*z(%):* 239 (31) [*M*⁺], 224 (27), 193 (13), 137 (9), 123 (15), 109 (28), 86 (100), 67 (22), 55 (28). ¹H NMR (300 MHz, CDCl₃) δ 2.57 (s, 3H), 2.07 (td, *J=* 11.0, 3.2 Hz, 1H), 1.74 (dd, *J=* 15.6, 7.6 Hz, 3H), 1.63 (d, *J=* 12.5 Hz, 1H), 1.31 - 1.09 (m, 13H), 1.01 (s, 3H), 0.91 - 0.67 (m, 5H). ¹³C NMR (75 MHz, CDCl₃) δ 79.4 (q), 71.9 (t), 57.5 (t), 44.6 (s), 37.3 (t), 36.7 (d), 32.1 (d), 31.4 (d), 30.8 (d), 28.4 (d), 27.8 (s), 26.9 (d), 25.1 (s), 22.6 (d), 14.1 (s).

### Example 183: rac-(3aR,5R,7S,7aR)-5,7-diethyl-1-isopropyl-3,3-dimethyloctahydrobenzo[c]isoxazole

Following the general procedure described in Example 81b: 2,4-diethyl-7-methyloct-6-enal (1000 mg, 5.09 mmol) (obtained from the corresponding α,β-unsaturated aldehyde using the general procedure described in Example 81a), *N*-isopropylhydroxylamine hydrochloride (852 mg, 7.64 mmol), K₂CO₃ (563 mg, 4.07 mmol) in toluene (50 mL) were reacted to give the title product (125 mg, 10 % yield) as a light yellow oil.

GC/MS (EI): *m*/*z* (%): 253 (20) [*M*⁺], 238 (100), 196 (28), 179 (5), 154 (12), 123 (13), 109 (8), 95 (10), 81 (8), 69 (9), 55 (11). ¹H NMR (300 MHz, CDCl₃) δ 3.09 (dt, *J =* 13.0, 6.5 Hz, 1H), 2.42 (t, *J =* 9.8 Hz, 1H), 1.99-1.85 (m, 1H), 1.78 (d, *J =* 13.2 Hz, 1H), 1.60 (dd, *J =* 37.5, 28.7 Hz, 3H), 1.34 - 1.13 (m, 10H), 1.04 (dd, *J =* 12.8, 6.4 Hz, 6H), 0.92 - 0.73 (m, 7H), 0.61 (dd, *J =* 24.6, 11.6 Hz, 1H). ¹³C NMR (75 MHz, CDCl₃) δ 77.9 (q), 66.7 (t), 57.9 (t), 55.0 (t), 43.6 (t), 39.1 (t), 36.6 (d), 31.3 (d), 29.6 (d), 27.1 (s), 25.9 (d), 24.3 (s), 22.7 (s), 13.9 (s), 11.8 (s), 10.4 (s).

### Example 184: rac-(3aR,5R,7S,7aR)-1-isopropyl-3,3,7-trimethyl-5-(3-methylbut-2-en-1-vDoctahydrobenzofclisoxazole

Following the general procedure described in Example 81b: 2,7-dimethyl-4-(3-methylbut-2-en-1-yl)oct-6-enal (853 mg, 3.64 mmol) (obtained from the corresponding α,β-unsaturated aldehyde using the general procedure described in Example 81a), N-isopropylhydroxylamine hydrochloride (610 mg, 5.47 mmol), K₂CO₃ (403 mg, 2.92 mmol) in toluene (50 mL) were reacted to give the title product (250 mg, 25 % yield) as a light yellow oil.

GC/MS (El): *m*/*z* (%): 279 (23) [*M*⁺], 264 (100), 222 (4), 154 (6), 128 (4), 107 (4), 95 (7), 69 (13), 55 (5). ¹H NMR (300 MHz, CDCl₃) δ 5.08 (t, *J =* 7.3 Hz, 1H), 3.19 - 2.99 (m, 1H), 2.28 (t, *J =* 9.9 Hz, 1H), 1.86 (dd, *J =* 17.2, 5.5 Hz, 3H), 1.69 - 1.46 (m, 9H), 1.38 (dd, *J =* 7.2, 3.6 Hz, 1H), 1.24 - 1.10 (m, 6H), 1.00 (dd, *J =* 10.3, 3.9 Hz, 6H), 0.90 (d, *J* = 6.4 Hz, 3H), 0.73 (dd, *J* = 25.1, 12.8 Hz, 2H). ¹³C NMR (75 MHz, CDCl₃) δ 132.2 (q), 122.8 (t), 77.8 (q), 68.7 (t), 57.4 (t), 54.7 (t), 41.3 (d), 38.3 (t), 36.9 (t), 35.1 (d), 31.4 (d), 27.2 (s), 25.8 (s), 24.3 (s), 22.6 (s), 20.2 (s), 17.8 (s), 13.6 (s).

### Example 185: rac-(3aR,5R,7S,7aR)-1-ethyl-3,3,7-trimethyl-5-(3-methylbut-2-en-1-yl)octahydrobenzo[c]isoxazole

Following the general procedure described in Example 81b: 2,7-dimethyl-4-(3-methylbut-2-en-1-yl)oct-6-enal (2.00 g, 8.99 mmol) (obtained from the corresponding α,β-unsaturated aldehyde using the general procedure described in Example 81a), N-ethylhydroxylamine (0.82 g, 13.49 mmol), K₂CO₃ (0.99 g, 7.20 mmol) in toluene (50 mL) were reacted to give the title product (0.91 g, 38 % yield) as a light yellow oil.

GC/MS (El): *m*/*z* (%): 265 (42) [*M*⁺], 250 (100), 222 (3), 194 (6), 140 (18), 114 (18), 95 (10), 81 (8), 69 (22). ¹H NMR (300 MHz, CDCl₃) δ 5.11 (t, *J =* 7.3 Hz, 1H), 3.03 - 2.55 (m, 2H), 2.14 (t, *J =* 10.1 Hz, 1H), 1.97 - 1.79 (m, 3H), 1.66 (d, *J =* 8.6 Hz, 5H), 1.56 (s, 3H), 1.50 - 1.25 (m, 2H), 1.24 - 1.09 (m, 6H), 1.08 - 0.87 (m, 6H), 0.85 - 0.59 (m, 2H). ¹³C NMR (75 MHz, CDCl₃) δ 132.3 (q), 122.8 (t), 79.2 (q), 75.1 (t), 57.6 (t), 56.4 (d), 41.1 (d), 38.4 (t), 37.2 (t), 35.1 (d), 31.5 (d), 26.6 (s), 25.8 (s), 24.8 (s), 20.2 (s), 17.8 (s), 13.8 (s).

### Example 186: rac-(3aR,5R,7S,7aR)-1-ethyl-3,3,5,7-tetramethyl-5-(3-methylbut-2-en-1-yl)octahydrobenzo[c]isoxazole

Following the general procedure described in Example 81b: 2,4,7-trimethyl-4-(3-methylbut-2-en-1-yl)oct-6-enal (2.00 g, 7.95 mmol) (obtained from the corresponding α,β-unsaturated aldehyde using the general procedure described in Example 81a), *N-*ethylhydroxylamine hydrochloride (1.16 g, 11.93 mmol), K₂CO₃ (0.88 g, 6.36 mmol) in toluene (50 mL) were reacted to give the title product (1.03 g, 46 % yield) as a light yellow oil.

GC/MS (EI): *m*/*z(%):* 279 (55) [*M*⁺], 264 (80), 210 (7), 149 (11),140 (100), 114 (42), 69 (35), 55 (20). ¹H NMR (300 MHz, CDCl₃) δ 5.19 (t, *J =* 7.6 Hz, 1H), 3.08 - 2.60 (m, 2H), 2.18 - 2.00 (m, 2H), 1.91 (d, *J =* 7.5 Hz, 2H), 1.72 (s, 4H), 1.58 (s, 3H), 1.48 - 1.26 (m, 3H), 1.26 - 1.14 (m, 6H), 1.12 - 0.99 (m, 4H), 0.91 (d, *J =* 4.3 Hz, 6H). ¹³C NMR (75 MHz, CDCl₃) δ 133.5 (q), 120.4 (t), 79.3 (q), 75.7 (t), 56.4 (d), 53.0 (t), 46.2 (d), 43.6 (d), 36.2 (d), 35.8 (q), 33.4 (t), 26.6 (s), 26.2 (s), 24.7 (s), 24.0 (s), 20.3 (s), 18.0 (s), 13.9 (s).

From the same reaction, an additional diastereomer was isolated: rac-(3aR,5R,7R,7aR)-1-ethyl-3,3,5,7-tetramethyl-5-(3-methylbut-2-en-1-yl)octahydrobenzo[c]isoxazole (320 mg, 14% yield) as a yellow oil. GC/MS (El): *m*/*z* (%): 279 (63) [M⁺], 264 (90), 140 (100), 114 (47), 95 (19), 83 (21), 69 (41), 55 (27).

### Example 187: rac-(3aR,5R,7aR)-5-butyl-1,3,3-trimethyloctahydrobenzo[c]isoxazole

Following the general procedure described in Example 81b: 4-butyl-7-methyloct-6-enal (821 mg, 4.18 mmol) (obtained from the corresponding α,β-unsaturated aldehyde using the general procedure described in Example 81a), *N*-methylhydroxylamine hydrochloride (524 mg, 6.27 mmol), K₂CO₃ (462 mg, 3.35 mmol) in toluene (50 mL) were reacted to give the title product (675 mg, 72 % yield) as a colorless oil.

GC/MS (EI): *m*/*z(%):* 225 (39) [*M*⁺], 210 (29), 179 (21), 123 (24), 109 (26), 95 (27), 86 (100), 67 (21), 55 (26). ¹H NMR (300 MHz, CDCl₃) δ 2.65 (s, 3H), 2.12 (dd, *J* = 10.9, 3.1 Hz, 1H), 1.95-1.77 (m, 3H), 1.70 (d, *J =* 12.2 Hz, 1H), 1.31 (d, *J =* 21.1 Hz, 11H), 1.09 (s, 3H), 0.98 - 0.75 (m, 5H). ¹³C NMR (75 MHz, CDCl₃) δ 79.5 (q), 72.1 (t), 57.7 (t), 44.8 (s), 37.4 (t), 36.6 (d), 31.6 (d), 31.0 (d), 29.5 (d), 28.6 (d), 28.0 (s), 25.3 (s), 23.0 (d), 14.2 (s).

### Example 188: rac-(3aR,5R,7aR)-1-isopropyl-3,3-dimethyl-5-propyloctahydrobenzo[c]isoxazole

Following the general procedure described in Example 81b: 7-methyl-4-propyloct-6-enal (1.50 g, 8.23 mmol) (obtained from the corresponding α,β-unsaturated aldehyde using the general procedure described in Example 81a), *N-*isopropylhydroxylamine (4.00 g, 53.30 mmol), K₂CO₃ (0.91 g, 6.58 mmol) in toluene (50 mL) were reacted to give the title product (1.02 g, 52 % yield) as a colorless oil.

GC/MS (El): *m*/*z(%):* 239 (25) [*M*⁺], 224 (100), 182 (4), 165 (7), 114 (31), 109 (35), 95 (21), 81 (20), 67 (20), 55 (18). ¹H NMR (300 MHz, CDCl₃) δ 2.89 (dt, *J =* 12.8, 6.4 Hz, 1H), 2.46 (td, *J =* 10.8, 3.3 Hz, 1H), 1.93 - 1.53 (m, 4H), 1.40 - 1.10 (m, 9H), 1.09 - 0.94 (m, 9H), 0.93 - 0.67 (m, 5H). ¹³C NMR (75 MHz, CDCl₃) δ 78.5 (q), 66.3 (t), 57.7 (t), 57.0 (t), 39.1 (d), 36.9 (t), 31.6 (d), 31.2 (d), 26.8 (s), 24.5 (s), 20.5 (s), 20.2 (d), 19.1 (s), 14.3 (s).

### Example 189: 1-ethyl-3,3,5,7-tetramethyl-5-(3-methylbut-2-en-1-yl)octahydrobenzo[c]isoxazole

Following the general procedure described in Example 81b: 2,4,7-trimethyl-4-(3-methylbut-2-en-1-yl)oct-6-enal (2.00 g, 7.95 mmol) (obtained from the corresponding α,β-unsaturated aldehyde using the general procedure described in Example 81a), *N-*ethylhydroxylamine hydrochloride (1.16 g, 11.93 mmol), K₂CO₃ (0.88 g, 6.36 mmol) in toluene (50 mL) were reacted to give the title product (1.03 g, 46 % yield) as a light yellow oil.

GC/MS (El): *m*/*z(%):* 279 (55) [*M*⁺], 264(80), 210 (7), 149 (11),140 (100), 114 (42), 69 (35), 55 (20).And GC/MS (EI): *m*/*z* (%):279 (50) [*M*⁺], 264(80), 208 (15), 152 (21), 140 (100), 114 (45), 69 (41), 55(22). ¹H NMR (300 MHz, CDCl₃) δ 5.09 (dt, *J* = 24.7, 7.2 Hz, 1H), 3.00 - 2.52 (m, 2H), 2.07 (d, *J =* 14.8 Hz, 2H), 1.83 (t, *J =* 9.7 Hz, 2H), 1.75-1.61 (m, 4H), 1.52 (s, 3H), 1.40 (d, *J =* 12.9 Hz, 1H), 1.33 - 1.09 (m, 7H), 1.07 - 0.76 (m, 11H). ¹³C NMR (75 MHz, CDCl₃) δ 133.3 (q),133.1 (q), 120.4 (t), 120.2 (t), 79.1 (q), 75.6 (t), 56.4 (d), 56.3 (d), 52.9 (t), 46.9 (d), 46.1 (d), 43.5 (d), 36.1 (d), 35.7 (d), 35.6 (d), 35.3 (q), 33.3 (t), 33.2 (t),29.2 (s), 26.5 (s), 26.0 (s), 24.5 (s), 23.8 (s), 20.2 (s), 18.0 (s), 17.9 (s), 13.7 (s).

### Example 190: 5-ethyl-1,3,3,5,7-pentamethyloctahydrobenzo[c]isoxazole

Following the general procedure described in Example 81b: 4-ethyl-2,4,7-trimethyloct-6-enal (584 mg, 2.439 mmol) (obtained from the corresponding α,β-unsaturated aldehyde using the general procedure described in Example 81a), *N*-methylhydroxylamine hydrochloride (306 mg, 3.66 mmol), K₂CO₃ (270 mg, 1.951 mmol) in toluene (50 mL) were reacted to give the title product (342 mg, 62 % yield) as a colorless oil.

GC/MS (EI): *m*/*z*(%):225 (26) [*M*⁺], 210 (38), 196 (7), 179 (12), 137 (9), 126 (100), 109 (20), 100 (68), 55 (28). ¹H NMR (300 MHz, CDCl₃) δ 2.86 - 2.46 (m, 3H), 2.16 - 1.82 (m, 2H), 1.69 (d, *J =* 7.1 Hz, 1H), 1.49 - 1.13 (m, 7H), 1.10-0.64 (m, 14H).¹³C NMR (75 MHz, CDCl₃) δ 79.2 (q), 79.1 (q), 78.2 (t), 78.0 (t), 53.8 (t), 53.5 (t), 48.9 (s), 46.5 (d), 46.2 (d), 38.0 (d), 35.9 (d), 35.2 (d), 34.4 (q), 34.2 (q), 32.8 (t), 32.5 (t), 29.5 (d), 28.5 (s), 26.9 (s), 24.9 (s), 24.8 (s), 23.0 (s), 20.2 (s), 7.9 (s).

### Example 191: 1,3,3,6-tetramethyloctahydrobenzofclisoxazole

Following the general procedure described in Example 81b: 3,7-dimethyloct-6-enal (3.125 g, 20.26 mmol), *N*-methylhydroxylamine hydrochloride (2.54 g, 30.40 mmol), K₂CO₃ (2.24 g, 16.21 mmol) in cyclohexane (80 mL) were reacted to give the title product (1.99 g, 54 % yield) as a colorless oil.

GC/MS (EI): *m*/*z(%):* 183 (71) [*M*⁺], 168 (54), 137 (94), 126 (9), 109 (15), 100 (100), 95 (43), 81 (44). ¹H NMR (300 MHz, CDCl₃) δ 2.58 (s, 3H), 2.14 (td, *J=* 10.9, 3.5 Hz, 1H), 1.81 - 1.55 (m, 4H), 1.49 - 1.28 (m, 1H), 1.27 - 1.10 (m, 4H), 1.05 (d, *J=* 9.8 Hz, 3H), 0.99 - 0.81 (m, 5H). ¹³C NMR (75 MHz, CDCl₃) δ 79.4 (q), 71.7 (t), 57.4 (t), 44.5 (s), 37.3 (d), 34.4 (d), 30.9 (t), 28.0, 25.2 (s), 24.5 (d), 21.9 (s).

### Example 192: 1,3,3,5,7-pentamethyl-5-(3-methylbut-2-en-1-vl)octahvdrobenzo[c]isoxazole

Following the general procedure described in Example 81b: 2,4,7-trimethyl-4-(3-methylbut-2-en-1-yl)oct-6-enal (2.00 g, 5.84 mmol) (obtained from the corresponding α,β-unsaturated aldehyde using the general procedure described in Example 81a), *N-*methylhydroxylamine hydrochloride (0.73 g, 8.76 mmol), K₂CO₃ (0.65 g, 4.67 mmol) in toluene (50 mL) were reacted to give the title product (1.04 g, 67 % yield) as a light yellow oil.

GC/MS (EI): *m*/*z(%):* 265 (81) [*M*⁺], 250 (78), 194 (8), 149 (18), 138 (19), 126 (100), 100 (57), 69 (41), 55 (20). And GC/MS (EI): *m*/*z(%):* 265(66) [*M*⁺], 250 (79), 194 (27), 153 (27),138 (31), 126 (100), 100 (63), 69 (52), 55 (28). ¹H NMR (300 MHz, CDCl₃) δ 5.10 (d, *J* = 25.1 Hz, 1H), 2.90 - 2.44 (m, 3H), 2.06 (s, 1H), 1.88 (t, *J =* 10.2 Hz, 2H), 1.66 (s, 4H), 1.54 (s, 3H), 1.48 - 0.62 (m, 17H). ¹³C NMR (75 MHz, CDCl₃) δ 133.4 (q), 133.1 (q),120.4 (t), 120.2 (t), 79.2 (q), 79.1 (q),78.1 (t), 77.9 (t), 53.7 (t), 48.8 (s), 46.8 (d), 46.1 (d), 43.5 (d), 36.1 (d), 35.7 (q), 35.6 (d),35.5 (d), 35.3 (q),32.8 (t), 29.2 (s), 26.9 (s), 26.1 (s), 24.8 (s), 23.8 (s), 20.1 (s), 18.0 (s), 17.9 (s).

### Example 193: 1-isopropyl-3,3,5,7-tetramethyloctahydrobenzo[c]isoxazole

Following the general procedure described in Example 81b: 2,4,7-trimethyloct-6-enal (2.83 g, 15.78 mmol), *N-*isopropylhydroxylamine hydrochloride (2.64 g, 23.67 mmol), K₂CO₃ (1.75 g, 12.62 mmol) in toluene (50 mL) were reacted to give the title product (1.63 g, 45 % yield) as a colorless oil.

GC/MS (EI): *m*/*z* (%): 225 (18) [*M*⁺], 210 (100), 168 (19), 151 (7), 128 (9), 109 (10), 95 (19) .¹H NMR (300 MHz, CDCl₃) δ 3.05 (dd, *J =* 13.0, 6.5 Hz, 1H), 2.22 (t, *J =* 9.9 Hz, 1H), 1.94 - 1.76 (m, 1H), 1.61 - 1.35 (m, 4H), 1.16 - 1.07 (m, 6H), 0.98 - 0.90 (m, 6H), 0.83 (dt, *J =* 14.1, 7.0 Hz, 6H), 0.69 (dd, *J* = 24.9, 12.7 Hz, 2H). ¹³C NMR (75 MHz, CDCl₃) δ 77.5 (q), 68.4 (t), 57.4 (t), 54.6 (t), 43.5 (d), 36.8 (t), 33.5 (d), 32.3 (t), 27.1 (s), 24.2 (s), 22.5 (s), 21.7 (s), 20.1 (s), 13.5 (s).

### Example 194: rac-(3aR,5R,7S,7aR)-1-isopropyl-3,3,5,7-tetramethyl-5-(3-methylbut-2-en-1-yl)octahydrobenzo[c]isoxazole

Following the general procedure described in Example 81b: 2,4,7-trimethyl-4-(3-methylbut-2-en-1-yl)oct-6-enal (2.02 g, 8.53 mmol) (obtained from the corresponding α,β-unsaturated aldehyde using the general procedure described in Example 81a), *N-*isopropylhydroxylamine hydrochloride (1.92 g, 25.6 mmol) in xylene (50 mL) were reacted to give the title product rac-(3aR,5R,7S,7aR)-1-isopropyl-3,3,5,7-tetramethyl-5-(3-methylbut-2-en-1-yl)octahydrobenzo[c]isoxazole (517 mg, 20 % yield) as a light yellow oil.

GC/MS (El): *m*/*z* (%): 293 (24) [*M*⁺], 278 (100), 154 (38), 128 (13), 107 (13), 95 (9), 83 (8), 69 (23), 55 (11). ¹H NMR (300 MHz, CDCl₃) δ 5.15 (t, *J =* 7.4 Hz, 1H), 3.12 (dt, *J* = 12.9, 6.5 Hz, 1H), 2.24 (t, *J =* 10.1 Hz, 1H), 2.16 - 2.01 (m, 1H), 1.87 (d, *J =* 7.5 Hz, 2H), 1.78 - 1.60 (m, 4H), 1.55 (s, 3H), 1.34 - 1.11 (m, 8H), 1.05 - 0.94 (m, 7H), 0.93 - 0.76 (m, 7H). ¹³C NMR (75 MHz, CDCl₃) δ 133.4 (q), 120.4 (t), 77.8 (q), 69.4 (t), 54.8 (t), 52.8 (t), 46.5 (d), 43.6 (d), 36.1 (d), 35.7 (d), 33.2 (t), 27.1 (s), 26.1 (s), 24.2 (s), 23.8 (s), 22.6 (s), 20.3 (s), 18.0 (s), 13.7 (s).

From the same reaction, two additional isomers were isolated: rac-(3aS,5R,7S,7aS)-1-isopropyl-3,3,5,7-tetramethyl-5-(3-methylbut-2-en-1-yl)octahydrobenzo[c]isoxazole (412 mg, 6 % yield) and rac-(3aS,5R,7R,7aS)-1-isopropyl-3,3,5,7-tetramethyl-5-(3-methylbut-2-en-1-yl)octahydrobenzo[c]isoxazole (412 mg, 15 % yield) as light yellow oils.

rac-(3aS,5R,7S,7aS)-1-isopropyl-3,3,5,7-tetramethyl-5-(3-methylbut-2-en-1-yl)octahydrobenzo[c]isoxazole : GC/MS (EI): *m*/*z(%):* 293 (21) [*M*⁺], 278 (73), 154 (100), 126 (51), 107 (33), 95 (22), 84 (26), 69 (99), 55 (24).

rac-(3aS,5R,7R,7aS)-1-isopropyl-3,3,5,7-tetramethyl-5-(3-methylbut-2-en-1-yl)octahydrobenzo[c]isoxazole : GC/MS (EI): *m*/*z* (%): 293 (20) [*M*⁺], 278 (100), 154 (60), 128 (18), 107 (22), 95 (22), 81 (18), 69 (79), 55 (22).

### Example 195: 1,3,3,7-tetramethyl-5-(3-methylbut-2-en-1 -yl)octahydrobenzo[c]isoxazole

Following the general procedure described in Example 81b: 2,7-dimethyl-4-(3-methylbut-2-en-1-yl)oct-6-enal (7.13 g, 28.8 mmol) (obtained from the corresponding α,β-unsaturated aldehyde using the general procedure described in Example 81a), N-methylhydroxylamine hydrochloride (3.61 g, 43.3 mmol), K₂CO₃ (3.19 g, 23.08 mmol) in toluene (60 mL) were reacted to give the title product (5.75 g, 79 % yield) as a colorless oil.

GC/MS (El): *m*/*z* (%): 251 (24) [*M*⁺], 236 (19), 193 (29), 180 (23),149 (40), 126 (100), 108 (42), 93 (58), 69 (38), 55 (18). ¹H NMR (300 MHz, CDCl₃) δ 5.02 (t, *J =* 7.3 Hz, 1H), 2.82 - 2.37 (m, 3H), 1.99 - 1.74 (m, 4H), 1.59 (s, 5H), 1.48 (s, 3H), 1.42 - 1.19 (m, 2H), 1.17 - 0.80 (m, 9H), 0.79 - 0.54 (m, 2H). ¹³C NMR (75 MHz, CDCl₃) δ 132.1 (q), 122.6 (t), 79.0 (q), 77.3 (t), 58.2 (t), 48.7 (s), 40.9 (d), 38.2 (t), 36.4 (t), 34.9 (d), 31.3 (d), 26.8 (s), 25.7 (s), 24.9 (s), 19.9 (s), 17.7 (s).

### Example 196: 1-isopropyl-5,7-dimethylhexahydro-1H-spiro[benzo[c]isoxazole-3,1'-cyclopentanel

Following the general procedure described in Example 81b: 6-cyclopentylidene-2,4-dimethylhexanal (215 mg, 1.11 mmol) (obtained from the corresponding α,β-unsaturated aldehyde using the general procedure described in Example 81a), *N-*isopropylhydroxylamine hydrochloride (185 mg, 1.66 mmol), K₂CO₃ (122 mg, 0.89 mmol) in toluene (50 mL) were reacted to give the title product (93 mg, 33 % yield) as a colorless oil.

GC/MS (El): *m*/*z(%):* 251 (34) [*M*⁺], 236 (100), 222 (31), 180 (26), 128 (41), 109 (22), 95 (28), 81 (18), 67 (14), 55 (19). ¹H NMR (300 MHz, CDCl₃) δ 3.13 (dt, *J* = 12.9, 6.5 Hz, 1H), 2.33 - 2.19 (m, 1H), 2.17 - 2.03 (m, 1H), 1.83 (dd, *J =* 11.9, 7.8 Hz, 1H), 1.75 - 1.34 (m, 11H), 1.27 - 1.14 (m, 3H), 1.04 (t, *J =* 8.5 Hz, 3H), 0.95 (t, *J =* 8.9 Hz, 6H), 0.90 - 0.68 (m, 2H). ¹³C NMR (75 MHz, CDCl₃) δ 88.9 (q), 69.4 (t), 55.5 (t), 55.0 (t), 43.8 (d), 37.1 (t), 36.6 (d), 34.5 (d), 33.9 (d), 32.5 (t), 24.9 (d), 23.7 (d), 22.7 (s), 22.0 (s), 20.3 (s),14.1 (s).

### Example 197: 1,3,3,5,7-pentamethyloctahydrobenzofclisoxazole

Following the general procedure described in Example 81b: 2,4,7-trimethyloct-6-enal (20.00 g, 102.00 mmol), *N*-methylhydroxylamine hydrochloride (12.78 g, 153.00 mmol), K₂CO₃ (11.28 g, 82.00 mmol) in toluene (300 ml) were reacted to give the title product (16.18 g, 80 % yield) as a colorless oil.

GC/MS (Isomer 1) (El): *m*/*z* (%): 197 (32) [*M*⁺], 182 (100), 151 (17), 126 (22), 109 (18), 100 (47), 60 (19). And GC/MS (Isomer 2) (EI): *m*/*z* (%): 197 (31) [*M*⁺], 182 (100), 151 (20), 126 (20), 109 (18), 100 (44), 60 (19). ¹H NMR (300 MHz, CDCl₃) δ 2.91 - 2.50 (m, 3H), 2.01 (ddd, *J* = 21.1, 15.3, 5.2 Hz, 3H), 1.55 (ddd, *J* = 18.9, 17.9, 9.5 Hz, 3H), 1.40 (dd, *J =* 10.7, 7.0 Hz, 1H), 1.30 - 1.05 (m, 5H), 1.04 - 0.64 (m, 8H). ¹³C NMR (75 MHz, CDCl₃) δ 79.2 (q), 79.1 (q), 77.9 (t), 77.2 (t), 58.4 (t), 51.9 (t), 48.9 (s), 43.2 (d), 40.1 (d), 36.6 (t), 33.7 (d), 32.5 (t), 31.9 (t), 30.9 (d), 27.7 (s), 26.9 (s), 26.8 (s), 25.0 (s), 24.8 (s), 21.8 (s), 20.1 (s), 20.0 (s), 19.2 (s).

### Example 198: 5,7-diethyl-1,3,3-trimethyloctahydrobenzofclisoxazole

Following the general procedure described in Example 81b: 2,4-diethyl-7-methyloct-6-enal (412 mg, 1.99 mmol) (obtained from the corresponding α,β-unsaturated aldehyde using the general procedure described in Example 81a), *N*-methylhydroxylamine hydrochloride (250 mg, 2.99 mmol), K₂CO₃ (220 mg, 1.60 mmol) in toluene (50 mL) were reacted to give the title product (0.30 mg, 66 % yield) as a colorless oil.

GC/MS (El): *m*/*z(%):* 225 (25) [*M*⁺], 210 (100), 179 (7), 126 (23), 114 (20), 98(11), 81 (9). And GC/MS (EI): *m*/*z* (%): 225 (17) [*M*⁺], 210 (19), 179 (6), 126 (100), 109 (23), 96 (25). ¹H NMR (300 MHz, CDCl₃) δ 2.84 - 2.53 (m, 3H), 2.22 -2.05 (m, 1H), 2.03- 1.43 (m, 5H), 1.41 - 1.23 (m, 4H), 1.23 - 1.05 (m, 6H), 0.99 (s, 1H), 0.94 - 0.76 (m, 7H). ¹³C NMR (75 MHz, CDCl₃) δ 79.4 (q), 76.1 (t), 75.6 (t), 58.8 (t), 52.8 (t), 49.4 (s), 43.4 (t), 39.2 (t), 39.1 (t), 36.4 (d), 35.0 (t), 33.4 (d), 31.3 (d), 29.5 (d), 28.7 (d), 26.8 (s), 26.7 (s), 26.0 (d), 25.4 (d), 25.0 (s), 24.8 (s), 12.7 (s), 11.8 (s), 11.0 (s), 10.5 (s).

### Example 199: 1,5,7-trimethylhexahydro-1H-spiro[benzo[c]isoxazole-3,1'-cyclopentane]

Following the general procedure described in Example 81b: 6-cyclopentylidene-2,4-dimethylhexanal (604 mg, 3.11 mmol) (obtained from the corresponding α,β-unsaturated aldehyde using the general procedure described in Example 81a), *N-*methylhydroxylamine hydrochloride (389 mg, 4.66 mmol), K₂CO₃ (344 mg, 2.49 mmol) in toluene (50 mL) were reacted to give the title product (303 mg, 44 % yield) as a colorless oil.

GC/MS (El): *m*/*z* (%): 223 (40) [*M*⁺], 181 (28), 152 (100), 139 (23), 122 (27), 95 (39), 82 (32), 67 (23), 55 (35). And GC/MS (EI): *m*/*z* (%): 223 (32) [*M*⁺], 181 (19), 152 (100), 139 (14), 122 (24), 95 (29), 82 (21), 67 (19), 55 (28). ¹H NMR (300 MHz, CDCl₃) δ 2.84-2.52 (m, 3H), 2.38 - 1.82 (m, 3H), 1.78 - 1.41 (m, 11H), 1.30 - 0.85 (m, 7H), 0.84-0.58 (m, 1H). ¹³C NMR (75 MHz, CDCl₃) δ 90.2 (q), 78.8 (t), 78.1 (t), 56.0 (t), 49.5 (t), 49.2 (t), 43.4 (d), 40.2 (d), 36.8 (t), 36.3 (d), 36.2 (d), 35.7 (d), 35.5 (d), 33.9 (d), 32.5 (t), 32.0 (t), 31.1 (d), 27.8 (t), 25.1 (d), 23.9 (d), 21.9 (s), 20.2 (s), 20.1 (s), 19.3 (s).

### Example 200: 1,3,3,5,7-pentamethyloctahydrobenzofclisoxazole hydrochloride

The flask was charged with 1,3,3,5,7-pentamethyloctahydrobenzo[*c*]isoxazole (313 mg, 1.59 mmol) and acetone (40 mL). Then 2.7 mL 1M HCl was added dropwise at r.t. Stirred overnight to obtain the title product (304 mg, 1.29 mmol, 81 % yield) as a white solid.

¹H NMR (300 MHz, CDCl₃) δ 14.92 (s, 1H), 3.15 - 2.86 (m, 3H), 2.83 - 2.59 (m, 1H), 2.56 - 2.31 (m, 1H), 2.27 - 1.90 (m, 1H), 1.88 - 1.68 (m, 2H), 1.66 - 1.31 (m, 5H), 1.20 (d, *J =* 3.1 Hz, 3H), 1.15 - 1.07 (m, 3H), 1.00 (d, *J =* 7.2 Hz, 1H), 0.92 (t, *J =* 7.2 Hz, 2H), 0.85 - 0.67 (m, 1H). ¹³C NMR (75 MHz, CDCl₃) δ 88.9 (q), 87.7 (q), 87.5 (q), 78.4 (t), 77.8 (t), 73.2 (t), 72.8 (t), 54.7 (t), 51.1 (t), 48.8 (t), 44.7 (s), 42.8 (d), 42.3 (d), 40.0 (d), 39.3 (s), 33.0 (d), 32.9 (d), 32.8 (t), 31.9 (t), 31.4 (t), 31.3 (t), 30.5 (s), 28.4 (t), 26.9 (t), 26.9 (t), 26.2 (s), 26.1 (s), 23.5 (s), 23.4 (s), 22.5 (s), 21.2 (s), 21.1 (s), 20.5 (s), 19.5 (s), 19.4 (s), 19.3 (s), 19.2 (s), 18.8 (s), 18.5 (s).

### Example 201: 1-isopropyl-3,3,5,7-tetramethyloctahydrobenzo[c]isoxazole hydrochloride

A flask was charged with 1-isopropyl-3,3,5,7-tetramethyloctahydrobenzo[c]isoxazole (185 mg, 0.81 mmol) and acetone (40 mL). Then 1.4mL 1M HCl was added dropwise at r.t. Stirred overnight to obtain the title product (180 mg, 0.69 mmol, 85 % yield) as a light brown solid.

¹H NMR (300 MHz, CDCl₃) δ 14.36 (s, 1H), 3.90 - 3.47 (m, 1H), 2.92 - 2.35 (m, 3H), 1.75 - 1.53 (m, 3H), 1.46 (d, *J* = 6.5 Hz, 3H), 1.39 (s, 3H), 1.19 (d, *J* = 6.5 Hz, 3H), 1.12 - 1.00 (m, 6H), 0.84 (d, *J =* 6.8 Hz, 3H), 0.79 - 0.60 (m, 2H). ¹³C NMR (75 MHz, CDCl₃) δ 86.0 (q), 70.9 (t), 56.7 (t), 53.1 (t), 43.1 (d), 32.9 (d), 32.1 (t), 31.0 (t), 26.4 (s), 23.0 (s), 21.2 (s), 19.2 (s), 18.7 (s), 13.6 (s).

### Example 202: 6-isopentyl-1,4,4,8-tetramethyloctahydro-2H-benzo[d][1,3]oxazine

A flask was charged with 40 mg Pd/C(10%), 1,3,3,7-tetramethyl-5-(3-methylbut-2-en-1-yl)octahydrobenzo[c]isoxazole (325 mg, 1.25 mmol) and methanol (50 mL), then the reaction mixture was stirred overnight at r.t. under hydrogen gas; after the reaction was filtered, washed with MTBE (50 mL * 2), the filtrate was concentrated to give the crude product. Flash chromatography (hexane/ MTBE = 4:1-1:1) gave the title product (88 mg, 0.33 mmol, 26 % yield) as a colorless oil.

GC/MS (El): *m*/*z* (%): 267 (8) [*M*⁺], 252 (7), 208 (2), 194 (2), 140 (100), 110 (14), 95 (6), 81 (3), 67 (3). ¹H NMR (300 MHz, CDCl₃) δ 4.65 (d, *J =* 10.8 Hz, 1H), 4.22 (d, *J =* 10.8 Hz, 1H), 2.49 - 2.22 (m, 4H), 1.79 - 1.54 (m, 3H), 1.52 - 1.36 (m, 2H), 1.28 - 1.06 (m, 11H), 0.97 - 0.79 (m, 9H), 0.76 - 0.47 (m, 2H). ¹³C NMR (75 MHz, CDCl₃) δ 81.2 (d), 74.6 (q), 63.8 (t), 41.6 (d), 40.5 (t), 37.1 (t), 36.3 (d), 34.9 (d), 34.0 (d), 33.9 (t), 32.8 (s), 29.1 (s), 28.4 (t), 22.8 (s), 22.7 (s), 18.6 (s), 18.6 (s).

In the same reaction, an additional product was isolated: 2-(5-isopentyl-3-methyl-2-(methylamino)cyclohexyl)propan-2-ol (178 mg, 0.66 mmol, 52 % yield) as colorless oil. GC/MS (El): *m*/*z* (%): 255 (12) [*M*⁺], 240 (10), 128 (34), 110 (25), 84 (100), 70 (15), 59 (7).

### Example 203 (not in accordance with the invention): 6-isopentyl-1,4,4,8-tetramethyloctahydro-2H-benzo[d][1,3]oxazin-2-one

A mixture of 2-(5-isopentyl-3-methyl-2-(methylamino)cyclohexyl)propan-2-ol (556 mg, 2.18 mmol) and di(1*H*-imidazol-1-yl)methanone (459 mg, 2.83 mmol) in 1,2-dichloroethane (40 mL) was stirred under Ar atmosphere at r.t. for 5 minutes. The mixture was heated to 80°C in Ar atmosphere overnight, and the reaction was monitored by GC and GC-MS. After cooled to room temperature, the reaction mixture was filtered and the filter was washed with MTBE. The combined filtrates were concentrated in vacuo to give crude product, which was purified by flash chromatography(PE:MTBE=1:2) to afford 6-isopentyl-1,4,4,8-tetramethyloctahydro-2*H-*benzo[*d*][1,3]oxazin-2-one (485 mg, 75 % yield) as a white solid.

GC/MS (EI): *m*/*z* (%): 281 (13) [*M*⁺], 238 (2), 222 (22), 194 (7), 154 (40), 126 (3), 110 (100), 84 (25), 55 (9). ¹H NMR (300 MHz, CDCl₃) δ 2.98 (s, 3H), 2.52 (t, *J =* 9.9 Hz, 1H), 1.78 - 1.50 (m, 4H), 1.48 - 1.29 (m, 2H), 1.24 (s, 3H), 1.19 - 1.00 (m, 10H), 0.83-0.73 (m, 7H), 0.72 - 0.56 (m, 1H). ¹³C NMR (75 MHz, CDCl₃) δ 155.5 (q), 79.2 (q), 65.0 (t), 49.1 (t), 42.9 (d), 40.0 (t), 38.2 (s), 37.1 (t), 36.1 (d), 34.3 (d), 33.4 (d), 28.1 (t), 27.2 (s), 22.5 (s), 22.4 (s), 22.3 (s), 20.4 (s).

### Example 204: rac-(3aR,5R,7S,7aR)-5-isopentyl-1-isopropyl-3,3,5,7-tetramethyloctahydrobenzofclisoxazole

Following the general procedure described in Example 81b: 4-isopentyl-2,4,7-trimethyloct-6-enal (1.317 g, 4.47 mmol), N-isopropylhydroxylamine (0.504 g, 6.71 mmol) in xylene (50 mL) were reacted at a reflux temperature to give the title product (438 mg, 31 % yield) as a colorless oil.

GC/MS (El): *m*/*z* (%): 295 (12) [*M*⁺], 280 (100), 154 (53), 128 (14), 109 (11), 95 (11), 69 (10), 55 (10). ¹H NMR (300 MHz, CDCl₃) δ 3.31 - 3.00 (m, 1H), 2.35 - 2.20 (m, 1H), 2.18 - 2.05 (m, 1H), 1.92 - 1.49 (m, 3H), 1.47 - 1.35 (m, 1H), 1.33 - 1.24 (m, 2H), 1.23 - 1.09 (m, 9H), 1.09 - 0.95 (m, 7H), 0.94 - 0.78 (m, 12H). ¹³C NMR (75 MHz, CDCl₃) δ 78.1(q), 69.5(d), 54.8(d), 52.7(d), 47.1(t), 43.7(t), 36.4(t), 34.2(q), 33.0(d), 32.6(t), 28.9(d), 27.1(s), 26.2(s), 24.2(s), 23.6(s), 22.8(d), 22.4(s), 20.4(s).

### Example 205: rac-(3aR,5S,7aR)-1,3,3,5-tetramethyl-5-(3-methylbut-2-en-1-yl)octahydrobenzo[clisoxazole

Following the general procedure described in Example 81b: 4,7-dimethyl-4-(3-methylbut-2-en-1-yl)oct-6-enal (0.589 g, 2.145 mmol) (obtained from the corresponding α,β-unsaturated aldehyde using the general procedure described in Example 81a), N-methylhydroxylamine hydrochloride (0.269 g, 3.22 mmol), K₂CO₃ (0.237 g, 1.716 mmol) in toluene (50 mL) were reacted to give the title product (165 mg, 28 % yield) as a light yellow oil.

GC/MS (El): *m*/*z(%):* 251 (55) [*M*⁺], 236 (100), 180 (18), 166 (23), 139 (21), 124 (56), 86 (69), 69 (26), 55 (17). ¹H NMR (300 MHz, CDCl₃) δ 5.14 (t, *J =* 7.2 Hz, 1H), 2.59 (s, 3H), 2.15 - 1.92 (m, 2H), 1.88 (d, *J =* 7.5 Hz, 2H), 1.72 - 1.58 (m, 4H), 1.54 (s, 3H), 1.48 - 1.35 (m, 2H), 1.32 - 1.08 (m, 6H), 1.02 (s, 3H), 0.83 (s, 3H). ¹³C NMR (75 MHz, CDCl₃) δ 133.4(q), 120.3(d), 79.4(q), 72.4(d), 52.7(d), 44.7(q), 43.6(t), 35.9(t), 35.4(t), 27.81(s), 26.11(s), 25.02(s), 24.9(t), 22.97(s), 17.93(s).

### Example 206: 5-(2,5-dimethoxyphenyl)-1,3,3,5,7-pentamethyloctahydrobenzofclisoxazole

The title compound was prepared following the general procedure described in Example 81b: 4-(2,5-dimethoxyphenyl)-2,4,7-trimethyloct-6-enal (obtained from the corresponding α,β-unsaturated aldehyde using the general procedure described in Example 81a), *N*-methylhydroxylamine hydrochloride and K₂CO₃ in toluene were reacted to give the title product (mixture of two diastereomers) as a light yellow oil.

GC/MS (Isomer 1) (EI): *m*/*z(%):* 333 (55) [*M*⁺], 246 (75), 178 (100), 163 (21),151 (17), 138 (25), 126 (23), 96 (8). GC/MS (Isomer 2) (EI): *m*/*z* (%): 333 (30) [*M*⁺], 246 (36), 178 (100), 163 (25),151 (19), 138 (15), 126 (45), 96 (20).

### Example 207: rac-(3aR,5R,7S,7aR)-1,3,3,5,7-pentamethyl-5-(m-tolyl)octahydrobenzo[c]isoxazole

The title compound was prepared following the general procedure described in Example 81b: 2,4,7-trimethyl-4-(m-tolyl)oct-6-enal (obtained from the corresponding α,β-unsaturated aldehyde using the general procedure described in Example 81a), *N-*methylhydroxylamine hydrochloride and K₂CO₃ in toluene were reacted to give the title product as a light yellow oil.

GC/MS (EI): *m*/*z* (%): 287 (1) [*M*⁺], 195 (35), 180 (44), 184 (20), 157 (12), 132 (100), 126 (35), 117 (22), 105 (20), 91 (23).

### Example 208: rac-(3aR,5R,7S,7aR)-1-isopropyl-5-(6-methoxypyridin-2-yl)-3,3,5,7-tetramethyloctahydrobenzo[c]isoxazole

The title compound was prepared following the general procedure described in Example 81b: 4-(6-methoxypyridin-2-yl)-2,4,7-trimethyloct-6-enal (obtained from the corresponding α,β-unsaturated aldehyde using the general procedure described in Example 81a) and *N*-isopropylhydroxylamine in toluene were reacted to give the title product as a light yellow oil.

GC/MS (EI): *m*/*z* (%): 332 (16) [*M*⁺], 317 (28), 259 (29), 231 (52), 150 (89), 125 (100), 110 (82), 84 (20).

### Example 209: 5-(2,4-dimethoxyphenyl)-1,3,3,5,7-pentamethyloctahydrobenzofclisoxazole

The title compound was prepared following the general procedure described in Example 81b: 4-(2,4-dimethoxyphenyl)-2,4,7-trimethyloct-6-enal (obtained from the corresponding α,β-unsaturated aldehyde using the general procedure described in Example 81a), *N*-methylhydroxylamine hydrochloride and K₂CO₃ in toluene were reacted to give the title product (mixture of two diastereomers) as a light yellow oil.

GC/MS (Isomer 1) (EI): *m*/*z (%):* 333 (52) [*M*⁺], 246 (50), 178 (100), 163 (53), 151 (27), 138 (55), 126 (15), 108 (42). GC/MS (Isomer 2) (EI): *m*/*z* (%): 333 (2) [*M*⁺], 195 (90), 180 (100), 178 (60), 163 (40), 151 (23), 138 (19), 126 (12).

### Example 210: 2-methyl-3-(1,3,3,5,7-pentamethyloctahydrobenzofclisoxazol-5-yl)benzonitrile

The title compound was prepared following the general procedure described in Example 81b: 2-methyl-3-(2,4,7-trimethyl-1-oxooct-6-en-4-yl)benzonitrile (obtained from the corresponding α,β-unsaturated aldehyde using the general procedure described in Example 81a), *N*-methylhydroxylamine hydrochloride and K₂CO₃ in toluene were reacted to give the title product (mixture of two diastereomers) as a light yellow oil.

GC/MS (Isomer 1) (EI): *m*/*z (%):* 312 (56) [*M*⁺], 210 (20), 144 (32), 126 (100),113 (32), 98 (55), 87 (95), 68 (27). GC/MS (Isomer 2) (EI): *m*/*z* (%): 312 (52) [*M*⁺], 210 (14), 144 (25), 126 (100), 113 (18), 98 (35), 87 (56), 68 (25).

### Example 211: rac-(3aR,5R,7S,7aR)-5-(3,5-dimethoxyphenyl)-1,3,3,5,7-pentamethyloctahydrobenzofclisoxazole

The title compound was prepared following the general procedure described in Example 81b: 4-(3,5-dimethoxyphenyl)-2,4,7-trimethyloct-6-enal (obtained from the corresponding α,β-unsaturated aldehyde using the general procedure described in Example 81a), *N*-methylhydroxylamine hydrochloride and K₂CO₃ in toluene were reacted to give the title product as a light yellow oil.

GC/MS (El): *m*/*z(%):* 333 (42) [*M*⁺], 246 (100), 231 (32), 204 (44),178 (83), 165 (15), 139 (22), 126 (28).

### Example 212: 1,3,3,5,7-pentamethyl-5-(pyridin-3-yl)octahydrobenzofclisoxazole

The title compound was prepared following the general procedure described in Example 81b: 2,4,7-trimethyl-4-(pyridin-3-yl)oct-6-enal (obtained from the corresponding α,β-unsaturated aldehyde using the general procedure described in Example 81a), *N*-methylhydroxylamine hydrochloride and K₂CO₃ in toluene were reacted to give the title product (mixture of three diastereomers) as a light yellow oil.

GC/MS (Isomer 1) (EI): *m*/*z* (%): 274 (55) [*M*⁺], 257 (45), 228 (14), 120 (100), 98 (37), 87 (14), 70 (12). GC/MS (Isomer 2) (El): *m*/*z* (%): 274 (15) [*M*⁺], 273 (69), 228 (12), 120 (100), 98 (18), 91 (12), 68 (22). GC/MS (Isomer 3) (EI): *m*/*z* (%): 274 (5) [*M*⁺], 273 (30), 228 (12), 187 (65), 149 (32), 120 (100), 98 (20).

### Example 213: rac-(3aR,5R,7S,7aR)-5-(4-fluoro-2-methoxyphenyl)-1,3,3,5,7-pentamethyloctahydrobenzofclisoxazole

The title compound was prepared following the general procedure described in Example 81b: 4-(4-fluoro-2-methoxyphenyl)-2,4,7-trimethyloct-6-enal (obtained from the corresponding α,β-unsaturated aldehyde using the general procedure described in Example 81a), *N*-methylhydroxylamine hydrochloride and K₂CO₃ in toluene were reacted to give the title product (mixture of three diastereomers) as a white solid.

GC/MS (EI): *m*/*z* (%): 321 (70) [*M*⁺], 219 (14), 180 (18), 153 (32), 139 (30), 126 (100), 100 (40), 87 (45).

### Example 214: rac-(3aR,5R,7S,7aR)-5-(2,6-dimethylphenyl)-1,3,3,5,7-pentamethyloctahydrobenzofclisoxazole

The title compound was prepared following the general procedure described in Example 81b: 4-(2,6-dimethylphenyl)-2,4,7-trimethyloct-6-enal (obtained from the corresponding α,β-unsaturated aldehyde using the general procedure described in Example 81a), *N*-methylhydroxylamine hydrochloride and K₂CO₃ in toluene were reacted to give the title product as a yellow oil.

GC/MS (EI): *m*/*z* (%): 301 (1) [*M*⁺], 300 (10), 214 (18), 195 (49), 180 (28), 146 (100), 126 (36), 96 (15).

### Example 4: Assay on TRPM8 modulators

A HEK293 cell line stably expressing hTRPM8 was generated according to Klein et al., (Chem. Senses 36: 649-658, 2011) and receptor activation was monitored by calcium imaging in a Flexstation. For Ca-imaging assays of TRPM8 channel activation, cells were seeded on day 0 at a density of 12000 cells per well in Dulbecco's modified Eagle medium (DMEM) containing 9% foetal bovine serum in black, clear bottom 96-well plates that had been coated with 0.001% polyethyleneimine (molecular weight = 60 000, Acros Organics). On day 2, agonists were evaluated via calcium imaging using Fluo-4. Briefly, growth medium was discarded, and the cells were incubated in the dark for 1 h at 37°C in 50 µL loading buffer consisting of 2.7 µM Fluo-4 AM (Invitrogen) and 2.5 µM probenecid (Sigma-Aldrich) in DMEM (without serum). After incubation, the plates were washed five times with 100 µL of assay buffer (in mM: 130 NaCl, 5 KCI, 10 HEPES, 2 CaCl₂, and 10 glucose, pH7.4.) and further incubated in the dark at room temperature for 30 min. The cells were then washed five times with 100 µL assay buffer and then calcium influx to serial dilutions of inventive compounds were measured in a Flexstation 3 (Molecular Devices). Receptor activation was initiated following addition of 20 µl of a 10-fold concentrated ligand stock solution, which is also prepared in assay buffer. Fluorescence was continuously monitored for 15 seconds prior to ligand addition and for 105 seconds after ligand addition, for a total of 120 seconds. Maximal receptor activation in relation to solvent control and relative to 31.6 µM menthol is determined. Data from serial dilutions were processed with a KNIME workflow to fit a sigmoidal dose-response curve and to extrapolate EC50 values.

TRPM8 agonist exhibiting an EC50 value below 35 µM are presented in Table 1 below.

**Table 1:**

| **Comp. No.** | **Chemical Name** | EC50 |
|---|---|---|
| 1 | 1,3,3,5,7-pentamethyl-5-(3-methylbut-2-en-1-yl)octahydrobenzo[c]isoxazole | ++ |
| 2 | 1,3,3,5,7-pentamethyl-5-(4-methylpyridin-3-yl)octahydrobenzo[c]isoxazole | ++ |
| 3 | 1,3,3,5,7-pentamethyl-5-(pyridin-3-yl)octahydrobenzo[c]isoxazole | + |
| 4 | 1,3,3,5,7-pentamethyloctahydrobenzo[c]isoxazole | + |
| 5 | 1,3,3,5,7-pentamethyloctahydrobenzo[c]isoxazole hydrochloride | + |
| 6 | 1,3,3,6-tetramethyloctahydrobenzo[c]isoxazole | + |
| 7 | 1,3,3-trimethyl-6-((R)-4-methylcyclohex-3-en-1-yl)octahydrobenzo[c]isoxazole | ++ |
| 8 | 1,5,7-trimethylhexahydro-1H-spiro[benzo[c]isoxazole-3,1'-cyclopentane] | + |
| 9 | 1-ethyl-3,3,5,7-tetramethyl-5-(3-methylbut-2-en-1-yl)octahydrobenzo[c]isoxazole | +++ |
| 10 | 1-isopropyl-3,3,5,7-tetramethyl-5-(3-methylbut-2-en-1-yl)octahydrobenzo[c]isoxazole | ++ |
| 11 | 1-isopropyl-3,3,5,7-tetramethyloctahydrobenzo[c]isoxazol-1-ium chloride | ++ |
| 12 | 1-isopropyl-3,3,5,7-tetramethyloctahydrobenzo[c]isoxazole | ++ |
| 13 | 1-isopropyl-5,7-dimethylhexahydro-1H-spiro[benzo[c]isoxazole-3,1'-cyclopentane] | + |
| 14 | 2-(1,3,3,5,7-pentamethyloctahydrobenzo[c]isoxazol-5-yl)benzonitrile | +++ |
| 15 | 2-methyl-3-(1,3,3,5,7-pentamethyloctahydrobenzo[c]isoxazol-5-yl)benzonitrile | ++ |
| 16 | 5-(2,4-dimethoxyphenyl)-1,3,3,5,7-pentamethyloctahydrobenzo[c]isoxazole | ++ |
| 17 | 5-(2,5-dimethoxyphenyl)-1,3,3,5,7-pentamethyloctahydrobenzo[c]isoxazole | +++ |
| 18 | 5-(6-methoxypyridin-2-yl)-1,3,3,5,7-pentamethyloctahydrobenzo[c]isoxazole | ++ |
| 19 | 5,7-diethyl-1,3,3-trimethyloctahydrobenzo[c]isoxazole | + |
| 20 | 5-ethyl-1,3,3,5,7-pentamethyloctahydrobenzo[c]isoxazole | + |
| 21 | 6-isopentyl-1,4,4,8-tetramethyloctahydro-2H-benzo[d][1,3]oxazin-2-one (not in accordance with the invention) | + |
| 22 | 6-isopentyl-1,4,4,8-tetramethyloctahydro-2H-benzo[d][1,3]oxazine | + |
| 23 | rac-(3aR,5R,7aR)-1,3,3-trimethyl-5-pentyloctahydrobenzo[c]isoxazole | ++ |
| 24 | rac-(3aR,5R,7aR)-1,3,3-trimethyl-5-propyloctahydrobenzo[c]isoxazole | ++ |
| 25 | rac-(3aR,5R,7aR)-1-ethyl-3,3-dimethyl-5-propyloctahydrobenzo[c]isoxazole | ++ |
| 26 | rac-(3aR,5R,7aR)-1-isopropyl-3,3-dimethyl-5-propyloctahydrobenzo[c]isoxazole | ++ |
| 27 | rac-(3aR,5R,7aR)-5-butyl-1,3,3-trimethyloctahydrobenzo[c]isoxazole | ++ |
| 28 | rac-(3aR,5R,7aR)-5-butyl-1-ethyl-3,3-dimethyloctahydrobenzo[c]isoxazole | ++ |
| 29 | rac-(3aR,5R,7aR)-5-butyl-1-isopropyl-3,3-dimethyloctahydrobenzo[c]isoxazole | + |
| 30 | rac-(3aR,5R,7aR)-5-ethyl-1-isopropyl-3,3-dimethyloctahydrobenzo[c]isoxazole | + |
| 31 | rac-(3aR,5R,7R,7aR)-1,3,3,5,7-pentamethyl-5-(2-(methylthio)phenyl)octahydrobenzo[c]isoxazole | + |
| 32 | rac-(3aR,5R,7R,7aR)-1-ethyl-3,3,5,7-tetramethyl-5-(3-methylbut-2-en-1-yl)octahydrobenzo[c]isoxazole | + |
| 33 | rac-(3aR,5R,7R,7aR)-1-ethyl-3,3,5,7-tetramethyl-5-(o-tolyl)octahydrobenzo[c]isoxazole | ++ |
| 34 | rac-(3aR,5R,7R,7aR)-5-(3-chloro-2-methylphenyl)-1,3,3,5,7-pentamethyloctahydrobenzo[c]isoxazole | ++ |
| 35 | rac-(3aR,5R,7R,7aR)-5-(4-fluoro-2-methylphenyl)-1,3,3,5,7-pentamethyloctahydrobenzo[c]isoxazole | + |
| 36 | rac-(3aR,5R,7R,7aR)-5-(4-methoxyphenyl)-1,3,3,5,7-pentamethyloctahydrobenzo[c]isoxazole | + |
| 37 | rac-(3aR,5R,7S,7aR)-1,3,3,5,7-pentamethyl-5-(2-(methylthio)phenyl)octahydrobenzo[c]isoxazole | +++ |
| 38 | rac-(3aR,5R,7S,7aR)-1,3,3,5,7-pentamethyl-5-(2,4,5-trimethylphenyl)octahydrobenzo[c]isoxazole | +++ |
| 39 | rac-(3aR,5R,7S,7aR)-1,3,3,5,7-pentamethyl-5-(2-methyl-5-(trifluoromethyl)phenyl)octahydrobenzo[c]isoxazole | ++ |
| 40 | rac-(3aR,5R,7S,7aR)-1,3,3,5,7-pentamethyl-5-(4-(methylthio)phenyl)octahydrobenzo[c]isoxazole | +++ |
| 41 | rac-(3aR,5R,7S,7aR)-1,3,3,5,7-pentamethyl-5-(5-methylthiophen-2-yl)octahydrobenzo[c]isoxazole | ++ |
| 42 | rac-(3aR,5R,7S,7aR)-1,3,3,5,7-pentamethyl-5-(5-methylthiophen-3-yl)octahydrobenzo[c]isoxazole | +++ |
| 43 | rac-(3aR,5R,7S,7aR)-1,3,3,5,7-pentamethyl-5-(m-tolyl)octahydrobenzo[c]isoxazole | +++ |
| 44 | rac-(3aR,5R,7S,7aR)-1,3,3,5,7-pentamethyl-5-(naphthalen-1-yl)octahydrobenzo[c]isoxazole | ++ |
| 45 | rac-(3aR,5R,7S,7aR)-1,3,3,5,7-pentamethyl-5-(naphthalen-2-yl)octahydrobenzo[c]isoxazole | +++ |
| 46 | rac-(3aR,5R,7S,7aR)-1,3,3,5,7-pentamethyl-5-(o-tolyl)octahydrobenzo[c]isoxazole | ++++ |
| 47 | rac-(3aR,5R,7S,7aR)-1,3,3,5,7-pentamethyl-5-(p-tolyl)octahydrobenzo[c]isoxazole | +++ |
| 48 | rac-(3aR,5R,7S,7aR)-1,3,3,5,7-pentamethyl-5-phenyloctahydrobenzo[c]isoxazole | +++ |
| 49 | rac-(3aR,5R,7S,7aR)-1,3,3,5,7-pentamethyl-5-propyloctahydrobenzo[c]isoxazole | ++ |
| 50 | rac-(3aR,5R,7S,7aR)-1,3,3,7-tetramethyl-5-(3-methylbut-2-en-1-yl)octahydrobenzo[c]isoxazole | +++ |
| 51 | rac-(3aR,5R,7S,7aR)-1,3,3,7-tetramethyl-5-(o-tolyl)octahydrobenzo[c]isoxazole | ++ |
| 52 | rac-(3aR,5R,7S,7aR)-1,3,3,7-tetramethyl-5-phenyloctahydrobenzo[c]isoxazole | +++ |
| 53 | rac-(3aR,5R,7S,7aR)-1-ethyl-3,3,5,7-tetramethyl-5-(3-methylbut-2-en-1-yl)octahydrobenzo[c]isoxazole | +++ |
| 54 | rac-(3aR,5R,7S,7aR)-1-ethyl-3,3,5,7-tetramethyl-5-(o-tolyl)octahydrobenzo[c]isoxazole | ++++ |
| 55 | rac-(3aR,5R,7S,7aR)-1-ethyl-3,3,5,7-tetramethyl-5-phenyloctahydrobenzo[c]isoxazole | +++ |
| 56 | rac-(3aR,5R,7S,7aR)-1-ethyl-3,3,5,7-tetramethyloctahydrobenzo[c]isoxazole | + |
| 57 | rac-(3aR,5R,7S,7aR)-1-ethyl-5-(2-methoxyphenyl)-3,3,7-trimethyloctahydrobenzo[c]isoxazole | ++++ |
| 58 | rac-(3aR,5R,7S,7aR)-1-isopropyl-3,3,5,7-tetramethyl-5-(3-methylbut-2-en-1-yl)octahydrobenzo[c]isoxazole | +++ |
| 59 | rac-(3aR,5R,7S,7aR)-1-isopropyl-3,3,5,7-tetramethyl-5-(o-tolyl)octahydrobenzo[c]isoxazole | +++ |
| 60 | rac-(3aR,5R,7S,7aR)-1-isopropyl-3,3,5,7-tetramethyl-5-phenyloctahydrobenzo[c]isoxazole | +++ |
| 61 | rac-(3aR,5R,7S,7aR)-1-isopropyl-3,3,5,7-tetramethyl-5-propyloctahydrobenzo[c]isoxazole | + |
| 62 | rac-(3aR,5R,7S,7aR)-1-isopropyl-3,3,7-trimethyl-5-(3-methylbut-2-en-1-yl)octahydrobenzo[c]isoxazole | ++ |
| 63 | rac-(3aR,5R,7S,7aR)-1-isopropyl-3,3,7-trimethyl-5-(o-tolyl)octahydrobenzo[c]isoxazole | ++ |
| 64 | rac-(3aR,5R,7S,7aR)-1-isopropyl-3,3,7-trimethyl-5-phenyloctahydrobenzo[c]isoxazole | +++ |
| 65 | rac-(3aR,5R,7S,7aR)-1-isopropyl-5-(2-methoxyphenyl)-3,3,5,7-tetramethyloctahydrobenzo[c]isoxazole | +++ |
| 66 | rac-(3aR,5R,7S,7aR)-1-isopropyl-5-(2-methoxyphenyl)-3,3,7-trimethyloctahydrobenzo[c]isoxazole | ++++ |
| 67 | rac-(3aR,5R,7S,7aR)-1-isopropyl-5-(4-methoxyphenyl)-3,3,5,7-tetramethyloctahydrobenzo[c]isoxazole | + |
| 68 | rac-(3aR,5R,7S,7aR)-1-isopropyl-5-(6-methoxypyridin-2-yl)-3,3,5,7-tetramethyloctahydrobenzo[c]isoxazole | ++ |
| 69 | rac-(3aR,5R,7S,7aR)-5-(2,3-dimethylphenyl)-1,3,3,5,7-pentamethyloctahydrobenzo[c]isoxazole | +++ |
| 70 | rac-(3aR,5R,7S,7aR)-5-(2,4-difluorophenyl)-1,3,3,5,7-pentamethyloctahydrobenzo[c]isoxazole | +++ |
| 71 | rac-(3aR,5R,7S,7aR)-5-(2,4-dimethylphenyl)-1,3,3,5,7-pentamethyloctahydrobenzo[c]isoxazole | +++ |
| 72 | rac-(3aR,5R,7S,7aR)-5-(2,4-dimethylphenyl)-1,3,3,7-tetramethyloctahydrobenzo[c]isoxazole | +++ |
| 73 | rac-(3aR,5R,7S,7aR)-5-(2,5-difluorophenyl)-1,3,3,5,7-pentamethyloctahydrobenzo[c]isoxazole | +++ |
| 74 | rac-(3aR,5R,7S,7aR)-5-(2,5-dimethylphenyl)-1,3,3,5,7-pentamethyloctahydrobenzo[c]isoxazole | ++++ |
| 75 | rac-(3aR,5R,7S,7aR)-5-(2,5-dimethylphenyl)-1,3,3,7-tetramethyloctahydrobenzo[c]isoxazole | ++ |
| 76 | rac-(3aR,5R,7S,7aR)-5-(2,6-dimethylphenyl)-1,3,3,5,7-pentamethyloctahydrobenzo[c]isoxazole | ++ |
| 77 | rac-(3aR,5R,7S,7aR)-5-(2-ethoxyphenyl)-1,3,3,5,7-pentamethyloctahydrobenzo[c]isoxazole | ++ |
| 78 | rac-(3aR,5R,7S,7aR)-5-(2-ethylphenyl)-1,3,3,5,7-pentamethyloctahydrobenzo[c]isoxazole | +++ |
| 79 | rac-(3aR,5R,7S,7aR)-5-(2-ethylphenyl)-1,3,3,7-tetramethyloctahydrobenzo[c]isoxazole | + |
| 80 | rac-(3aR,5R,7S,7aR)-5-(2-ethylphenyl)-1-isopropyl-3,3,7-trimethyloctahydrobenzo[c]isoxazole | ++ |
| 81 | rac-(3aR,5R,7S,7aR)-5-(2-fluorophenyl)-1,3,3,5,7-pentamethyloctahydrobenzo[c]isoxazole | +++ |
| 82 | rac-(3aR,5R,7S,7aR)-5-(2-methoxyphenyl)-1,3,3,5,7-pentamethyloctahydrobenzo[c]isoxazole | +++ |
| 83 | rac-(3aR,5R,7S,7aR)-5-(2-methoxyphenyl)-1,3,3,7-tetramethyloctahydrobenzo[c]isoxazole | ++++ |
| 84 | rac-(3aR,5R,7S,7aR)-5-(3,4-difluorophenyl)-1,3,3,5,7-pentamethyloctahydrobenzo[c]isoxazole | +++ |
| 85 | rac-(3aR,5R,7S,7aR)-5-(3,5-difluorophenyl)-1,3,3,5,7-pentamethyloctahydrobenzo[c]isoxazole | ++++ |
| 86 | rac-(3aR,5R,7S,7aR)-5-(3,5-dimethoxyphenyl)-1,3,3,5,7-pentamethyloctahydrobenzo[c]isoxazole | + |
| 87 | rac-(3aR,5R,7S,7aR)-5-(3,5-dimethylphenyl)-1,3,3,5,7-pentamethyloctahydrobenzo[c]isoxazole | ++ |
| 88 | rac-(3aR,5R,7S,7aR)-5-(3,5-dimethylphenyl)-1-isopropyl-3,3,5,7-tetramethyloctahydrobenzo[c]isoxazole | ++ |
| 89 | rac-(3aR,5R,7S,7aR)-5-(3-chloro-2-methylphenyl)-1,3,3,5,7-pentamethyloctahydrobenzo[c]isoxazole | +++ |
| 90 | rac-(3aR,5R,7S,7aR)-5-(3-chloro-2-methylphenyl)-1-isopropyl-3,3,7-trimethyloctahydrobenzo[c]isoxazole | +++ |
| 91 | rac-(3aR,5R,7S,7aR)-5-(3-fluoro-5-(trifluoromethyl)phenyl)-1,3,3,5,7-pentamethyloctahydrobenzo[c]isoxazole | ++ |
| 92 | rac-(3aR,5R,7S,7aR)-5-(3-fluorophenyl)-1,3,3,5,7-pentamethyloctahydrobenzo[c]isoxazole | +++ |
| 93 | rac-(3aR,5R,7S,7aR)-5-(3-isopropylphenyl)-1,3,3,5,7-pentamethyloctahydrobenzo[c]isoxazole | + |
| 94 | rac-(3aR,5R,7S,7aR)-5-(3-methoxyphenyl)-1,3,3,5,7-pentamethyloctahydrobenzo[c]isoxazole | +++ |
| 95 | rac-(3aR,5R,7S,7aR)-5-(4-(tert-butyl)phenyl)-1,3,3,5,7-pentamethyloctahydrobenzo[c]isoxazole | + |
| 96 | rac-(3aR,5R,7S,7aR)-5-(4-(tert-butyl)phenyl)-1,3,3,7-tetramethyloctahydrobenzo[c]isoxazole | + |
| 97 | rac-(3aR,5R,7S,7aR)-5-(4-(tert-butyl)phenyl)-1-isopropyl-3,3,7-trimethyloctahydrobenzo[c]isoxazole | + |
| 98 | rac-(3aR,5R,7S,7aR)-5-(4-chloro-2-methylphenyl)-1-isopropyl-3,3,7-trimethyloctahydrobenzo[c]isoxazole | +++ |
| 99 | rac-(3aR,5R,7S,7aR)-5-(4-fluoro-2-methoxyphenyl)-1,3,3,5,7-pentamethyloctahydrobenzo[c]isoxazole | ++++ |
| 100 | rac-(3aR,5R,7S,7aR)-5-(4-fluoro-2-methylphenyl)-1,3,3,5,7-pentamethyloctahydrobenzo[c]isoxazole | ++++ |
| 101 | rac-(3aR,5R,7S,7aR)-5-(4-fluoro-2-methylphenyl)-1,3,3,7-tetramethyloctahydrobenzo[c]isoxazole | +++ |
| 102 | rac-(3aR,5R,7S,7aR)-5-(4-fluorophenyl)-1,3,3,5,7-pentamethyloctahydrobenzo[c]isoxazole | ++++ |
| 103 | rac-(3aR,5R,7S,7aR)-5-(4-fluorophenyl)-1-isopropyl-3,3,5,7-tetramethyloctahydrobenzo[c]isoxazole | ++++ |
| 104 | rac-(3aR,5R,7S,7aR)-5-(4-methoxyphenyl)-1,3,3,5,7-pentamethyloctahydrobenzo[c]isoxazole | ++ |
| 105 | rac-(3aR,5R,7S,7aR)-5-(5-chloro-2-methylphenyl)-1,3,3,5,7-pentamethyloctahydrobenzo[c]isoxazole | ++++ |
| 106 | rac-(3aR,5R,7S,7aR)-5-(5-chloro-2-methylphenyl)-1-isopropyl-3,3,7-trimethyloctahydrobenzo[c]isoxazole | ++ |
| 107 | rac-(3aR,5R,7S,7aR)-5-(5-fluoro-2-methylphenyl)-1,3,3,5,7-pentamethyloctahydrobenzo[c]isoxazole | ++++ |
| 108 | rac-(3aR,5R,7S,7aR)-5-(5-fluoro-2-methylphenyl)-1,3,3,7-tetramethyloctahydrobenzo[c]isoxazole | ++ |
| 109 | rac-(3aR,5R,7S,7aR)-5-(benzo[d][1,3]dioxol-5-yl)-1,3,3,5,7-pentamethyloctahydrobenzo[c]isoxazole | ++ |
| 110 | rac-(3aR,5R,7S,7aR)-5-(furan-3-yl)-1,3,3,5,7-pentamethyloctahydrobenzo[c]isoxazole | ++ |
| 111 | rac-(3aR,5R,7S,7aR)-5,7-diethyl-1-isopropyl-3,3-dimethyloctahydrobenzo[c]isoxazole | ++ |
| 112 | rac-(3aR,5R,7S,7aR)-5-isopentyl-1-isopropyl-3,3,5,7-tetramethyloctahydrobenzo[c]isoxazole | +++ |
| 113 | rac-(3aR,5S,7aR)-1,3,3,5-tetramethyl-5-(3-methylbut-2-en-1-yl)octahydrobenzo[c]isoxazole | ++ |
| 114 | rac-(3aR,7aR)-1,3,3,4,5,7-hexamethyloctahydrobenzo[c]isoxazole | + |
| 115 | rac-(3aR,7aR)-1,3,3-trimethyl-6-phenyloctahydrobenzo[c]isoxazole | + |
| 116 | rac-(3aR,7aR)-1,5,7-triethyl-3,3-dimethyloctahydrobenzo[c]isoxazole | + |
| 117 | rac-(3aR,7aS)-1,3,3,4,5,7-hexamethyloctahydrobenzo[c]isoxazole | + |
| 118 | rac-(3aR,7S,7aR)-5,5-diethyl-1,3,3,7-tetramethylocta-hydrobenzo[c]isoxazole (not in accordance with the invention) | + |
| 119 | rac-(3aS,5R,7S,7aS)-5-(4-fluorophenyl)-1,3,3,5,7-pentamethyloctahydrobenzo[c]isoxazole | ++ |
| 120 | rac-(3aS,5S,7S,7aS)-1-ethyl-3,3,7-trimethyl-5-(3-methylbut-2-en-1-yl)octahydrobenzo[c]isoxazole | ++ |
| 121 | rac-2-chloro-5-((3aR,5R,7S,7aR)-1,3,3,5,7-pentamethyloctahydrobenzo[c]isoxazol-5-yl)benzonitrile | ++++ |
| 122 | rac-2-chloro-5-((3aS,5R,7R,7aS)-1,3,3,5,7-pentamethyloctahydrobenzo[c]isoxazol-5-yl)benzonitrile | + |
| 123 | rac-2-methyl-5-((3aR,5R,7S,7aR)-1,3,3,5,7-pentamethyloctahydrobenzo[c]isoxazol-5-yl)benzonitrile | +++ |
| 124 | rac-3-((3aR,5R,7S,7aR)-1,3,3,5,7-pentamethyloctahydrobenzo[c]isoxazol-5-yl)benzonitrile | ++++ |
| 125 | rac-3-((3aR,5R,7S,7aR)-1-ethyl-3,3,5,7-tetramethyloctahydrobenzo[c]isoxazol-5-yl)-4-methylbenzonitrile | ++++ |
| 126 | rac-3-((3aR,5R,7S,7aR)-1-isopropyl-3,3,5,7-tetramethyloctahydrobenzo[c]isoxazol-5-yl)-4-methylbenzonitrile | +++ |
| 127 | rac-3-((3aR,5R,7S,7aR)-1-isopropyl-3,3,5,7-tetramethyloctahydrobenzo[c]isoxazol-5-yl)benzonitrile | ++++ |
| 128 | rac-3-((3aR,5R,7S,7aR)-1-isopropyl-3,3,7-trimethyloctahydrobenzo[c]isoxazol-5-yl)-4-methoxybenzonitrile | ++++ |
| 129 | rac-3-chloro-5-((3aR,5R,7S,7aR)-1,3,3,5,7-pentamethyloctahydrobenzo[c]isoxazol-5-yl)benzonitrile | ++++ |
| 130 | rac-3-methyl-4-((3aR,5R,7S,7aR)-1,3,3,5,7-pentamethyloctahydrobenzo[c]isoxazol-5-yl)benzonitrile | +++ |
| 131 | rac-4-((3aR,5R,7S,7aR)-1,3,3,5,7-pentamethyloctahydrobenzo[c]isoxazol-5-yl)benzonitrile | +++ |
| 132 | rac-4-methoxy-3-((3aR,5R,7R,7aR)-1,3,3,5,7-pentamethyloctahydrobenzo[c]isoxazol-5-yl)benzonitrile | ++ |
| 133 | rac-4-methoxy-3-((3aR,5R,7S,7aR)-1,3,3,5,7-pentamethyloctahydrobenzo[c]isoxazol-5-yl)benzonitrile | ++++ |
| 134 | rac-4-methyl-2-((3aR,5R,7S,7aR)-1,3,3,5,7-pentamethyloctahydrobenzo[c]isoxazol-5-yl)benzonitrile | +++ |
| 135 | rac-4-methyl-3-((3aR,5R,7S,7aR)-1,3,3,5,7-pentamethyloctahydrobenzo[c]isoxazol-5-yl)benzonitrile | ++++ |
| 136 | rac-4-methyl-3-((3aR,5R,7S,7aR)-1,3,3,7-tetramethyloctahydrobenzo[c]isoxazol-5-yl)benzonitrile | +++ |
| 137 | rac-methyl 4-methyl-3-((3aR,5R,7S,7aR)-1,3,3,5,7-pentamethyloctahydrobenzo[c]isoxazol-5-yl)benzoate | +++ |
| 138 | rel-4-methyl-3-((3aR,5R,7S,7aR)-1,3,3,5,7-pentamethyloctahydrobenzo[c]isoxazol-5-yl)benzonitrile | ++++ |
| 139 | rel-4-methyl-3-((3aS,5S,7R,7aS)-1,3,3,5,7-pentamethyloctahydrobenzo[c]isoxazol-5-yl)benzonitrile | +++ |

| | | |
|---|---|---|
| ++++ EC₅₀ Value in the range of 0.05 µM and lower +++ EC₅₀ value in the range of 0.05 - 0.3 µM ++ EC₅₀ value in the range of 0.3 - 1.00 µM + EC₅₀ value in the range of 1.00 - 35 µM | | |

### Example 5: Sensory studies in aqueous solution

The compounds as listed below in Table 2 were dissolved at a concenteration of 1 wt-% in propylene glycol. These solutions were then dispensed in appropriate quantities into deionized water containing 0.5 wt-% Poloxamer 407 (which is a hydrophilic non-ionic surfactant, e.g., commercially available from SigmaAldrich) and 0.25 wt-% Cremaphor^{®} RH40 (obtained from BASF) as solubilizer, to obtain the desired final concentration of 40 ppm (parts per milion) of the respective compound.

A trained group of panelists evaluated the aqueous solutions containing test compound by swilling 20 mL of the solution in the mouth for 60 seconds, followed by spitting, without rinsing the mouth afterwards for the duration of the evaluation. Panelists evaluated and recorded the cooling performance as well as other sensorial and organoleptic attributes at different timepoints over a period of two hours. Cooling performance was rated on a scale from 0 to 10 with 0 being no effect and 10 being freezing. Scores were averaged for all panelists and the cooling intensity qualified as "N" for "none" (score of 0), "L" for "low" (score above 0, up to 1), "M" for "medium" (score above 1, up to 4), "S" for "strong" (score above 4, up to 8) and "E" for "extreme" (score above 8), which are provided in Table 2. None of the tested compounds showed any statistically significant bitterness or negative organoleptic features. All tasted samples developed cooling sensation starting during swilling (almost immediately upon contact with the mucosa) and before expectorating.

**Table 2:**

| Comp. No. | Chemical Name | Max. cooling (time of max cooling in minutes) | Cooling after 1h |
|---|---|---|---|
| 12 | 1-isopropyl-3,3,5,7-tetramethyloctahydrobenzo[c]isoxazole | M (5) | L |
| 4 | 1,3,3,5,7-pentamethyloctahydrobenzo[c]isoxazole | M (3) | L |
| 50 | rac-(3aR,5R,7S,7aR)-1,3,3,7-tetramethyl-5-(3-methylbut-2-en-1-yl)octahydrobenzo[c]isoxazole | M (10) | M |
| 26 | rac-(3aR,5R,7aR)-1-isopropyl-3,3-dimethyl-5-propyloctahydrobenzo[c]isoxazole | S (10) | M |
| 1 | 1,3,3,5,7-pentamethyl-5-(3-methylbut-2-en-1-yl)octahydrobenzo[c]isoxazole | M (20) | M |
| 9 | 1-ethyl-3,3,5,7-tetramethyl-5-(3-methylbut-2-en-1-yl)octahydrobenzo[c]isoxazole | M (10) | M |
| 64 | rac-(3aR,5R,7S,7aR)-1-isopropyl-3,3,7-trimethyl-5-phenyloctahydrobenzo[c]isoxazole | M (10) | M |
| 48 | rac-(3aR,5R,7S,7aR)-1,3,3,5,7-pentamethyl-5-phenyloctahydrobenzo[c]isoxazole | S (10) | M |
| 46 | rac-(3aR,5R,7S,7aR)-1,3,3,5,7-pentamethyl-5-(o-tolyl)octahydrobenzo[c]isoxazole | S (10) | M |
| 135 | rac-4-methyl-3-((3aR,5R,7S,7aR)-1,3,3,5,7-pentamethyloctahydrobenzo[c]isoxazol-5-yl)benzonitrile | E (10) | M |
| 124 | rac-3-((3aR,5R,7S,7aR)-1,3,3,5,7-pentamethyloctahydrobenzo[c]isoxazol-5-yl)benzonitrile | S (5) | M |
| 120 | rac-(3aS,5S,7S,7aS)-1-ethyl-3,3,7-trimethyl-5-(3-methylbut-2-en-1-yl)octahydrobenzo[c]isoxazole | M (10) | L |
| 111 | rac-(3aR,5R,7S,7aR)-5,7-diethyl-1-isopropyl-3,3-dimethyloctahydrobenzo[c]isoxazole | M (10) | L |
| 85 | rac-(3aR,5R,7S,7aR)-5-(3,5-difluorophenyl)-1,3,3,5,7-pentamethyloctahydrobenzo[c]isoxazole | M (10) | M |
| 83 | rac-(3aR,5R,7S,7aR)-5-(2-methoxyphenyl)-1,3,3,7-tetramethyloctahydrobenzo[c]isoxazole | S (10) | L |
| 66 | rac-(3aR,5R,7S,7aR)-1-isopropyl-5-(2-methoxyphenyl)-3,3,7-trimethyloctahydrobenzo[c]isoxazole | M (20) | M |
| 102 | rac-(3aR,5R,7S,7aR)-5-(4-fluorophenyl)-1,3,3,5,7-pentamethyloctahydrobenzo[c]isoxazole | S (10) | M |
| 7 | 1,3,3-trimethyl-6-(4-methylcyclohex-3-en-1-yl)octahydrobenzo[c]isoxazole | M (10) | L |

### Example 6: Sensory studies in model dentifrice

The compounds listed in Table 3 were dissolved at a concenteration of 1 wt-% in propylene glycol. These solutions were then dispersed in appropriate quantities to obtain the desired final concentration of 100 ppm (parts per milion) of the test compound into a model, unflavored dentifrice, the formula of which is given below (ingredients obtained from the suppliers respectively indicated in parenthesis).

| | wt% |
|---|---|
| Trisodium Phosphate dodecahydrate | 0.2 |
| Sorbitol | 50.0 |
| Precipitated Silica - Sorbosil^{™} AC77 *(Surfachem)* | 8.0 |
| Precipitated Silica - Sorbosil^{™} TC15 *(Surfachem)* | 9.0 |
| Sodium Carboxy Methyl Cellulose 9M31XF *(Ashland)* | 0.8 |
| Titanium Dioxide | 1.0 |
| Sodium Lauryl Sulphate | 1.5 |
| PEG 1500 *(Kilo)* | 5.0 |
| Demin Water | 24.5 |

A trained group of panelists evaluated the dentifrice containing a compound of formula (I), by brushing their teeth with 1 g of the dentifrice using a toothbrush for 60 seconds, followed by spitting, without rinsing the mouth afterwards for the duration of the evaluation. Panelists evaluated and recorded the cooling performance as well as other sensorial and organoleptic attributes at different timepoints over a period of two hours. Cooling performance was rated on a scale from 0 to 10 with 0 being no effect and 10 being freezing. Scores were averaged for all panelists and the cooling intensity qualified as "N" for "none" (score of 0), "L" for "low" (score above 0, up to 1), "M" for "medium" (score above 1, up to 4), "S" for "strong" (score above 4, up to 8) and "E" for "extreme" (score above 8), which are summarized below in Table 3. None of the tested compounds showed any statistically significant bitterness or negative organoleptic features. All tasted samples developed cooling sensation starting during brushing, before expectorating.

**Table 3:**

| Comp. No. | Chemical Name | Max. cooling (time of max cooling in minutes) | Cooling after 1h |
|---|---|---|---|
| 48 | rac-(3aR,5R,7S,7aR)-1,3,3,5,7-pentamethyl-5-phenyloctahydrobenzo[c]isoxazole | M (5) | L |
| 46 | rac-(3aR,5R,7S,7aR)-1,3,3,5,7-pentamethyl-5-(o-tolyl)octahydrobenzo[c]isoxazole | S (20) | M |
| 135 | rac-4-methyl-3-((3aR,5R,7S,7aR)-1,3,3,5,7-pentamethyloctahydrobenzo[c]isoxazol-5-yl)benzonitrile | S (5) | M |
| 102 | rac-(3aR,5R,7S,7aR)-5-(4-fluorophenyl)-1,3,3,5,7-pentamethyloctahydrobenzo[c]isoxazole | M (10) | M |

## Claims

1. Non-medical method of modulating of transient receptor potential channel melastatin member 8 (TRPM8) comprising bringing the receptor into contact with a compound of formula (la), a salt or solvate thereof 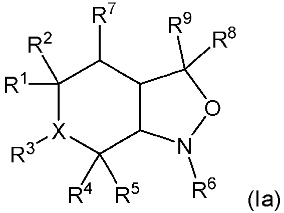 wherein
XisC,
R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
R¹ is selected from hydrogen,
C₁-C₆ alkyl,
C₁-C₇ alkyl substituted with one or two OH groups,
C₃-C₇ alkenyl,
phenyl optionally substituted with up to five substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, naphthyl optionally substituted with up to five substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, and
C₅-C₁₀ mono- or bicyclic aryl wherein 1 or 2 C-atoms are replaced by a hetero atom independently selected from sulfur, nitrogen, and oxygen, optionally substituted with up to three substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl;
**R²** is selected from hydrogen and methyl;
**R⁴, R⁵, and R**⁷ are independently selected from hydrogen and C₁-C₆ alkyl;
**R⁶ is** selected from hydrogen, C₁-C₆ alkyl, and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7- membered cycloalkyl ring.

2. Non-medical method according to claim 1 wherein the compound of formula (la), a salt or solvate thereof is a compound, wherein R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl.

3. Non-medical method according to claim 1 wherein the compound of formula (Ia) is selected from the group consisting of 4-methyl-3-(1,3,3,5,7-pentamethyloctahydrobenzo[c]isoxazol-5-yl)benzonitrile; 4-methoxy-3-(1,3,3,5,7-pentamethyloctahydrobenzo[c]isoxazol-5-yl)benzonitrile; 1-ethyl-5-(2-methoxyphenyl)-3,3,7-trimethyloctahydrobenzo[c]isoxazole; 5-(2-methoxyphenyl)-1,3,3,7-tetramethyloctahydrobenzo[c]isoxazole; 5-(3,5-difluorophenyl)-1,3,3,5,7-pentamethyloctahydrobenzo[c]isoxazole; 1,3,3,5,7-pentamethyloctahydrobenzo[c]isoxazol-5-yl)benzonitrile; 1,3,3,5,7-pentamethyl-5-(o-tolyl)octahydrobenzo[c]isoxazole; 5-(5-chloro-2-methylphenyl)-1,3,3,5,7-pentamethyloctahydrobenzo[c]isoxazole; 3-(1-isopropyl-3,3,7-trimethyloctahydrobenzo[c]isoxazol-5-yl)-4-methoxybenzonitrile; 5-(2,5-dimethylphenyl)-1,3,3,5,7-pentamethyloctahydrobenzo[c]isoxazole; 3-(1-ethyl-3,3,5,7-tetramethyloctahydrobenzo[c]isoxazol-5-yl)-4-methylbenzonitrile; 5-(5-fluoro-2-methylphenyl)-1,3,3,5,7-pentamethyloctahydrobenzo[c]isoxazole; 1-isopropyl-5-(2-methoxyphenyl)-3,3,7-trimethyloctahydrobenzo[c]isoxazole; 3-chloro-5-(1,3,3,5,7-pentamethyloctahydrobenzo[c]isoxazol-5-yl)benzonitrile; 3-(1-isopropyl-3,3,5,7-tetramethyloctahydrobenzo[c]isoxazol-5-yl)benzonitrile; 5-(3,4-difluorophenyl)-1,3,3,5,7-pentamethyloctahydrobenzo[c]isoxazole; 3-(1-isopropyl-3,3,5,7-tetramethyloctahydrobenzo[c]isoxazol-5-yl)-4-methylbenzonitrile; 5-(2-methoxyphenyl)-1,3,3,5,7-pentamethyloctahydrobenzo[c]isoxazole; 2-methyl-5-(1,3,3,5,7-pentamethyloctahydrobenzo[c]isoxazol-5-yl)benzonitrile; methyl 4-methyl-3-(1,3,3,5,7-pentamethyloctahydrobenzo[c]isoxazol-5-yl)benzoate; 1-isopropyl-3,3,5,7-tetramethyl-5-(o-tolyl)octahydrobenzo[c]isoxazole; 5-(3-fluorophenyl)-1,3,3,5,7-pentamethyloctahydrobenzo[c]isoxazole; 1,3,3,5,7-pentamethyl-5-phenyloctahydrobenzo[c]isoxazole; 1-isopropyl-3,3,7-trimethyl-5-phenyloctahydrobenzo[c]isoxazole; 5-(4-chloro-2-methylphenyl)-1-isopropyl-3,3,7-trimethyloctahydrobenzo[c]isoxazole; 5-(2,4-dimethylphenyl)-1,3,3,5,7-pentamethyloctahydrobenzo[c]isoxazole; 5-(4-fluoro-2-methylphenyl)-1,3,3,7-tetramethyloctahydrobenzo[c]isoxazole; 5-(2,5-difluorophenyl)-1,3,3,5,7-pentamethyloctahydrobenzo[c]isoxazole; 1-ethyl-3,3,5,7-tetramethyl-5-phenyloctahydrobenzo[c]isoxazole; 1,3,3,5,7-pentamethyl-5-(p-tolyl)octahydrobenzo[c]isoxazole; 1-isopropyl-3,3,5,7-tetramethyl-5-phenyloctahydrobenzo[c]isoxazole; 1,3,3,7-tetramethyl-5-phenyloctahydrobenzo[c]isoxazole; 1-isopropyl-5-(2-methoxyphenyl)-3,3,5,7-tetramethyloctahydrobenzo[c]isoxazole; 5-(2-fluorophenyl)-1,3,3,5,7-pentamethyloctahydrobenzo[c]isoxazole; 4-(1,3,3,5,7-pentamethyloctahydrobenzo[c]isoxazol-5-yl)benzonitrile; 3-methyl-4-(1,3,3,5,7-pentamethyloctahydrobenzo[c]isoxazol-5-yl)benzonitrile; 5-(2,4-difluorophenyl)-1,3,3,5,7-pentamethyloctahydrobenzo[c]isoxazole; 5-(2,5-dimethoxyphenyl)-1,3,3,5,7-pentamethyloctahydrobenzo[c]isoxazole; 5-(2,3-dimethylphenyl)-1,3,3,5,7-pentamethyloctahydrobenzo[c]isoxazole; 1,3,3,5,7-pentamethyl-5-(2,4,5-trimethylphenyl)octahydrobenzo[c]isoxazole; 1,3,3,5,7-pentamethyl-5-(4-(methylthio)phenyl)octahydrobenzo[c]isoxazole; 2-(1,3,3,5,7-pentamethyloctahydrobenzo[c]isoxazol-5-yl)benzonitrile; 5-(3-methoxyphenyl)-1,3,3,5,7-pentamethyloctahydrobenzo[c]isoxazole; 5-(3-chloro-2-methylphenyl)-1-isopropyl-3,3,7-trimethyloctahydrobenzo[c]isoxazole; 1,3,3,5,7-pentamethyl-5-(naphthalen-2-yl)octahydrobenzo[c]isoxazole; 5-(2,4-dimethylphenyl)-1,3,3,7-tetramethyloctahydrobenzo[c]isoxazole; 4-methyl-3-(1,3,3,7-tetramethyloctahydrobenzo[c]isoxazol-5-yl)benzonitrile; 1,3,3,7-tetramethyl-5-(3-methylbut-2-en-1-yl)octahydrobenzo[c]isoxazole; 5-(2-ethylphenyl)-1,3,3,5,7-pentamethyloctahydrobenzo[c]isoxazole; 1,3,3,5,7-pentamethyl-5-(m-tolyl)octahydrobenzo[c]isoxazole; 1,3,3,5,7-pentamethyl-5-(5-methylthiophen-3-yl)octahydrobenzo[c]isoxazole; 1-ethyl-3,3,7-trimethyl-5-(3-methylbut-2-en-1-yl)octahydrobenzo[c]isoxazole; 5-(2-ethylphenyl)-1-isopropyl-3,3,7-trimethyloctahydrobenzo[c]isoxazole; 1,3,3,5,7-pentamethyl-5-(3-methylbut-2-en-1-yl)octahydrobenzo[c]isoxazole; 5-(2,6-dimethylphenyl)-1,3,3,5,7-pentamethyloctahydrobenzo[c]isoxazole; 5-(benzo[d][1,3]dioxol-5-yl)-1,3,3,5,7-pentamethyloctahydrobenzo[c]isoxazole; 1-isopropyl-5-(6-methoxypyridin-2-yl)-3,3,5,7-tetramethyloctahydrobenzo[c]isoxazole; 5-(5-chloro-2-methylphenyl)-1-isopropyl-3,3,7-trimethyloctahydrobenzo[c]isoxazole; 5-butyl-1,3,3-trimethyloctahydrobenzo[c]isoxazole; 5-(2,4-dimethoxyphenyl)-1,3,3,5,7-pentamethyloctahydrobenzo[c]isoxazole; 1-isopropyl-3,3,7-trimethyl-5-(o-tolyl)octahydrobenzo[c]isoxazole; 1-isopropyl-3,3,7-trimethyl-5-(3-methylbut-2-en-1-yl)octahydrobenzo[c]isoxazole; 5-(5-fluoro-2-methylphenyl)-1,3,3,7-tetramethyloctahydrobenzo[c]isoxazole; 5-(3,5-dimethylphenyl)-1,3,3,5,7-pentamethyloctahydrobenzo[c]isoxazole; 1-isopropyl-3,3,5,7-tetramethyloctahydrobenzo[c]isoxazole; 1,3,3-trimethyl-6-(4-methylcyclohex-3-en-1-yl)octahydrobenzo[c]isoxazole; 5-(3-fluoro-5-(trifluoromethyl)phenyl)-1,3,3,5,7-pentamethyloctahydrobenzo[c]isoxazole; 1-isopropyl-3,3-dimethyl-5-propyloctahydrobenzo[c]isoxazole; 1,3,3-trimethyl-5-pentyloctahydrobenzo[c]isoxazole; 1-ethyl-3,3,5,7-tetramethyl-5-(o-tolyl)octahydrobenzo[c]isoxazole; 5-(3,5-dimethylphenyl)-1-isopropyl-3,3,5,7-tetramethyloctahydrobenzo[c]isoxazole; 5-(2,5-dimethylphenyl)-1,3,3,7-tetramethyloctahydrobenzo[c]isoxazole; 5,7-diethyl-1-isopropyl-3,3-dimethyloctahydrobenzo[c]isoxazole; 1,3,3,5,7-pentamethyl-5-(2-methyl-5-(trifluoromethyl)phenyl)octahydrobenzo[c]isoxazole; 1-ethyl-3,3-dimethyl-5-propyloctahydrobenzo[c]isoxazole; 1,3,3,5,7-pentamethyl-5-propyloctahydrobenzo[c]isoxazole; 1,3,3-trimethyl-5-propyloctahydrobenzo[c]isoxazole; 2-methyl-3-(1,3,3,5,7-pentamethyloctahydrobenzo[c]isoxazol-5-yl)benzonitrile; 1,3,3,7-tetramethyl-5-(o-tolyl)octahydrobenzo[c]isoxazole; 5-butyl-1-ethyl-3,3-dimethyloctahydrobenzo[c]isoxazole; 5-(4-fluorophenyl)-1,3,3,5,7-pentamethyloctahydrobenzo[c]isoxazole; 5-(furan-3-yl)-1,3,3,5,7-pentamethyloctahydrobenzo[c]isoxazole; 1-isopropyl-3,3,5,7-tetramethyloctahydrobenzo[c]isoxazol-1-ium chloride; 1,3,3,5,7-pentamethyl-5-(naphthalen-1-yl)octahydrobenzo[c]isoxazole; 5-(2-ethoxyphenyl)-1,3,3,5,7-pentamethyloctahydrobenzo[c]isoxazole; 5-(3-chloro-2-methylphenyl)-1,3,3,5,7-pentamethyloctahydrobenzo[c]isoxazole; 5-(6-methoxypyridin-2-yl)-1,3,3,5,7-pentamethyloctahydrobenzo[c]isoxazole; 1,3,3,5,7-pentamethyl-5-(5-methylthiophen-2-yl)octahydrobenzo[c]isoxazole; 1,3,3,5,7-pentamethyl-5-(4-methylpyridin-3-yl)octahydrobenzo[c]isoxazole; 1-ethyl-3,3,5,7-tetramethyloctahydrobenzo[c]isoxazole; 5-(2-ethylphenyl)-1,3,3,7-tetramethyloctahydrobenzo[c]isoxazole; 5-butyl-1-isopropyl-3,3-dimethyloctahydrobenzo[c]isoxazole; 1-ethyl-3,3,5,7-tetramethyl-5-(3-methylbut-2-en-1-yl)octahydrobenzo[c]isoxazole; 5-(4-fluoro-2-methylphenyl)-1,3,3,5,7-pentamethyloctahydrobenzo[c]isoxazole; 1-isopropyl-5,7-dimethylhexahydro-1H-spiro[benzo[c]isoxazole-3,1'-cyclopentane]; 1,5,7-triethyl-3,3-dimethyloctahydrobenzo[c]isoxazole; 1-isopropyl-3,3,5,7-tetramethyl-5-propyloctahydrobenzo[c]isoxazole; 6-isopentyl-1,4,4,8-tetramethyloctahydro-2H-benzo[d][1,3]oxazine; 5-(3,5-dimethoxyphenyl)-1,3,3,5,7-pentamethyloctahydrobenzo[c]isoxazole; 5,7-diethyl-1,3,3-trimethyloctahydrobenzo[c]isoxazole; 1-isopropyl-5-(4-methoxyphenyl)-3,3,5,7-tetramethyloctahydrobenzo[c]isoxazole; 5-(3-isopropylphenyl)-1,3,3,5,7-pentamethyloctahydrobenzo[c]isoxazole; 1,3,3-trimethyl-6-phenyloctahydrobenzo[c]isoxazole; 5-ethyl-1-isopropyl-3,3-dimethyloctahydrobenzo[c]isoxazole; 1,3,3,5,7-pentamethyl-5-(2-(methylthio)phenyl)octahydrobenzo[c]isoxazole; 1,5,7-trimethylhexahydro-1H-spiro[benzo[c]isoxazole-3,1'-cyclopentane]; 5-ethyl-1,3,3,5,7-pentamethyloctahydrobenzo[c]isoxazole; 5-(4-methoxyphenyl)-1,3,3,5,7-pentamethyloctahydrobenzo[c]isoxazole; 1,3,3,5,7-pentamethyloctahydrobenzo[c]isoxazole; 1,3,3,4,5,7-hexamethyloctahydrobenzo[c]isoxazole; 1,3,3,5,7-pentamethyl-5-(pyridin-3-yl)octahydrobenzo[c]isoxazole; 1,3,3,5,7-pentamethyloctahydrobenzo[c]isoxazole hydrochloride; 5-(4-fluoro-2-methoxyphenyl)-1,3,3,5,7-pentamethyloctahydrobenzo[c]isoxazole; 5-(4-fluorophenyl)-1-isopropyl-3,3,5,7-tetramethyloctahydrobenzo[c]isoxazole; 4-methyl-2-(1,3,3,5,7-pentamethyloctahydrobenzo[c]isoxazol-5-yl)benzonitrile; 1,3,3,5-tetramethyl-5-(3-methylbut-2-en-1-yl)octahydrobenzo[c]isoxazole; 1-isopropyl-3,3,5,7-tetramethyl-5-(3-methylbut-2-en-1-yl)octahydrobenzo[c]isoxazole; 5-isopentyl-1-isopropyl-3,3,5,7-tetramethyloctahydrobenzo[c]isoxazole; 2-chloro-5-(1,3,3,5,7-pentamethyloctahydrobenzo[c]isoxazol-5-yl)benzonitrile; 5-(4-(tert-butyl)phenyl)-1,3,3,5,7-pentamethyloctahydrobenzo[c]isoxazole; 5-(4-(tert-butyl)phenyl)-1,3,3,7-tetramethyloctahydrobenzo[c]isoxazole; 5-(4-(tert-butyl)phenyl)-1-isopropyl-3,3,7-trimethyloctahydrobenzo[c]isoxazole; and 1,3,3,6-tetramethyloctahydrobenzo[c]isoxazole, or a salt or solvate thereof.

4. A non-medical method of inducing a cooling sensation in a human or animal comprising contacting the human or animal with a compound of formula (la), or a salt or solvate thereof, as defined in claim 1 to 3.

5. A non-medical method of achieving a cooling effect on the skin or mucosa comprising contacting the skin or mucosa with a product comprising one or more compounds of formula (Ia) as defined in claim 1 to 3.

6. A consumer product comprising one or more compounds of formula (Ia) as defined in claim 1 to 3.

7. A pharmaceutical composition comprising one or more compounds of formula (Ia) as defined in claim 1 to 3.

8. The compound of formula (Ia) as defined in claim 1 to 3 for use as medicament.

9. A composition comprising a cool sensation wherein the composition comprises a compound of formula (la) 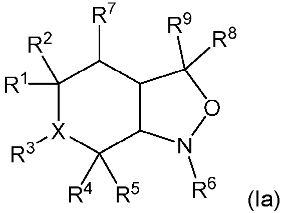 wherein
XisC,
R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
R¹ is selected from hydrogen,
C₁-C₆ alkyl,
C₁-C₇ alkyl substituted with one or two OH groups,
C₃-C₇ alkenyl,
phenyl optionally substituted with up to five substituents selected from the group consisting of halogen, C≡N, NO₂, C₁-C₆ alky, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, naphthyl optionally substituted with up to five substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, and
C₅-C₁₀ mono- or bicyclic aryl wherein 1 or 2 C-atoms are replaced by a hetero atom independently selected from sulfur, nitrogen, and oxygen, optionally substituted with up to three substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl;
**R²** is selected from hydrogen and methyl;
**R⁴, R⁵, and R⁷** are independently selected from hydrogen and C₁-C₆ alkyl; **R⁶ is** selected from hydrogen, C₁-C₆ alkyl, and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7- membered cycloalkyl ring.

10. A compound of formula (la), a salt or solvate thereof 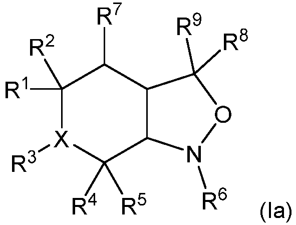 wherein
XisC,
R³ is selected from hydrogen, C₁-C₆ alkyl, C₁-C₇ alkyl substituted with one or two OH groups, C₃-C₇ cycloalkyl, C₃-C₇ cycloalkyl substituted with one or two OH groups, C₅-C₇ cycloalkenyl, C₅-C₇ cycloalkenyl substituted with one, two or three C₁-C₃ alkyl groups, and phenyl;
R¹ is selected from
C₁-C₆ alkyl,
C₁-C₇ alkyl substituted with one or two OH groups,
C₃-C₇ alkenyl,
phenyl optionally substituted with up to five substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, naphthyl optionally substituted with up to five substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl, and
C₅-C₁₀ mono- or bicyclic aryl wherein 1 or 2 C-atoms are replaced by a hetero atom independently selected from sulfur, nitrogen, and oxygen, optionally substituted with up to three substituents selected from the group consisting of halogen, C≡N, NO₂, C₁-C₆ alkyl, C₁-C₆ alkyl comprising up to 5 halogen atoms, C₁-C₆ alkyloxy, C₁-C₆ alkyloxy comprising up to 5 halogen atoms, C₃-C₇ cycloalkyl, -C(O)O-R¹⁰ wherein R¹⁰ is selected from hydrogen and C₁-C₃ alkyl, and -SR¹¹ wherein R¹¹ is selected from hydrogen and C₁-C₃ alkyl;
**R²** is selected from hydrogen and methyl;
**R⁴, R⁵, and R⁷** are independently selected from hydrogen and C₁-C₆ alkyl;
**R⁶ is** selected from hydrogen, C₁-C₆ alkyl, and C₃-C₇ cycloalkyl;
**R⁸ and R⁹** are independently selected from hydrogen, and C₁-C₆ alkly, or
R⁸ and R⁹ form together with the C-atom to which they are attached a 3, 4, 5, 6, or 7- membered cycloalkyl ring.

11. A compound according to claim 10 selected from the group consisting of of 4-methyl-3-(1,3,3,5,7-pentamethyloctahydrobenzo[c]isoxazol-5-yl)benzonitrile; 4-methoxy-3-(1,3,3,5,7-pentamethyloctahydrobenzo[c]isoxazol-5-yl)benzonitrile; 1-ethyl-5-(2-methoxyphenyl)-3,3,7-trimethyloctahydrobenzo[c]isoxazole; 5-(2-methoxyphenyl)-1,3,3,7-tetramethyloctahydrobenzo[c]isoxazole; 5-(3,5-difluorophenyl)-1,3,3,5,7-pentamethyloctahydrobenzo[c]isoxazole; 1,3,3,5,7-pentamethyloctahydrobenzo[c]isoxazol-5-yl)benzonitrile; 1,3,3,5,7-pentamethyl-5-(o-tolyl)octahydrobenzo[c]isoxazole; 5-(5-chloro-2-methylphenyl)-1,3,3,5,7-pentamethyloctahydrobenzo[c]isoxazole; 3-(1-isopropyl-3,3,7-trimethyloctahydrobenzo[c]isoxazol-5-yl)-4-methoxybenzonitrile; 5-(2,5-dimethylphenyl)-1,3,3,5,7-pentamethyloctahydrobenzo[c]isoxazole; 3-(1-ethyl-3,3,5,7-tetramethyloctahydrobenzo[c]isoxazol-5-yl)-4-methylbenzonitrile; 5-(5-fluoro-2-methylphenyl)-1,3,3,5,7-pentamethyloctahydrobenzo[c]isoxazole; 1-isopropyl-5-(2-methoxyphenyl)-3,3,7-trimethyloctahydrobenzo[c]isoxazole; 3-chloro-5-(1,3,3,5,7-pentamethyloctahydrobenzo[c]isoxazol-5-yl)benzonitrile; 3-(1-isopropyl-3,3,5,7-tetramethyloctahydrobenzo[c]isoxazol-5-yl)benzonitrile; 5-(3,4-difluorophenyl)-1,3,3,5,7-pentamethyloctahydrobenzo[c]isoxazole; 3-(1-isopropyl-3,3,5,7-tetramethyloctahydrobenzo[c]isoxazol-5-yl)-4-methylbenzonitrile; 5-(2-methoxyphenyl)-1,3,3,5,7-pentamethyloctahydrobenzo[c]isoxazole; 2-methyl-5-(1,3,3,5,7-pentamethyloctahydrobenzo[c]isoxazol-5-yl)benzonitrile; methyl 4-methyl-3-(1,3,3,5,7-pentamethyloctahydrobenzo[c]isoxazol-5-yl)benzoate; 1-isopropyl-3,3,5,7-tetramethyl-5-(o-tolyl)octahydrobenzo[c]isoxazole; 5-(3-fluorophenyl)-1,3,3,5,7-pentamethyloctahydrobenzo[c]isoxazole; 1,3,3,5,7-pentamethyl-5-phenyloctahydrobenzo[c]isoxazole; 1-isopropyl-3,3,7-trimethyl-5-phenyloctahydrobenzo[c]isoxazole; 5-(4-chloro-2-methylphenyl)-1-isopropyl-3,3,7-trimethyloctahydrobenzo[c]isoxazole; 5-(2,4-dimethylphenyl)-1,3,3,5,7-pentamethyloctahydrobenzo[c]isoxazole; 5-(4-fluoro-2-methylphenyl)-1,3,3,7-tetramethyloctahydrobenzo[c]isoxazole; 5-(2,5-difluorophenyl)-1,3,3,5,7-pentamethyloctahydrobenzo[c]isoxazole; 1-ethyl-3,3,5,7-tetramethyl-5-phenyloctahydrobenzo[c]isoxazole; 1,3,3,5,7-pentamethyl-5-(p-tolyl)octahydrobenzo[c]isoxazole; 1-isopropyl-3,3,5,7-tetramethyl-5-phenyloctahydrobenzo[c]isoxazole; 1,3,3,7-tetramethyl-5-phenyloctahydrobenzo[c]isoxazole; 1-isopropyl-5-(2-methoxyphenyl)-3,3,5,7-tetramethyloctahydrobenzo[c]isoxazole; 5-(2-fluorophenyl)-1,3,3,5,7-pentamethyloctahydrobenzo[c]isoxazole; 4-(1,3,3,5,7-pentamethyloctahydrobenzo[c]isoxazol-5-yl)benzonitrile; 3-methyl-4-(1,3,3,5,7-pentamethyloctahydrobenzo[c]isoxazol-5-yl)benzonitrile; 5-(2,4-difluorophenyl)-1,3,3,5,7-pentamethyloctahydrobenzo[c]isoxazole; 5-(2,5-dimethoxyphenyl)-1,3,3,5,7-pentamethyloctahydrobenzo[c]isoxazole; 5-(2,3-dimethylphenyl)-1,3,3,5,7-pentamethyloctahydrobenzo[c]isoxazole; 1,3,3,5,7-pentamethyl-5-(2,4,5-trimethylphenyl)octahydrobenzo[c]isoxazole; 1,3,3,5,7-pentamethyl-5-(4-(methylthio)phenyl)octahydrobenzo[c]isoxazole; 2-(1,3,3,5,7-pentamethyloctahydrobenzo[c]isoxazol-5-yl)benzonitrile; 5-(3-methoxyphenyl)-1,3,3,5,7-pentamethyloctahydrobenzo[c]isoxazole; 5-(3-chloro-2-methylphenyl)-1-isopropyl-3,3,7-trimethyloctahydrobenzo[c]isoxazole; 1,3,3,5,7-pentamethyl-5-(naphthalen-2-yl)octahydrobenzo[c]isoxazole; 5-(2,4-dimethylphenyl)-1,3,3,7-tetramethyloctahydrobenzo[c]isoxazole; 4-methyl-3-(1,3,3,7-tetramethyloctahydrobenzo[c]isoxazol-5-yl)benzonitrile; 1,3,3,7-tetramethyl-5-(3-methylbut-2-en-1-yl)octahydrobenzo[c]isoxazole; 5-(2-ethylphenyl)-1,3,3,5,7-pentamethyloctahydrobenzo[c]isoxazole; 1,3,3,5,7-pentamethyl-5-(m-tolyl)octahydrobenzo[c]isoxazole; 1,3,3,5,7-pentamethyl-5-(5-methylthiophen-3-yl)octahydrobenzo[c]isoxazole; 1-ethyl-3,3,7-trimethyl-5-(3-methylbut-2-en-1-yl)octahydrobenzo[c]isoxazole; 5-(2-ethylphenyl)-1-isopropyl-3,3,7-trimethyloctahydrobenzo[c]isoxazole; 1,3,3,5,7-pentamethyl-5-(3-methylbut-2-en-1-yl)octahydrobenzo[c]isoxazole; 5-(2,6-dimethylphenyl)-1,3,3,5,7-pentamethyloctahydrobenzo[c]isoxazole; 5-(benzo[d][1,3]dioxol-5-yl)-1,3,3,5,7-pentamethyloctahydrobenzo[c]isoxazole; 1-isopropyl-5-(6-methoxypyridin-2-yl)-3,3,5,7-tetramethyloctahydrobenzo[c]isoxazole; 5-(5-chloro-2-methylphenyl)-1-isopropyl-3,3,7-trimethyloctahydrobenzo[c]isoxazole; 5-butyl-1,3,3-trimethyloctahydrobenzo[c]isoxazole; 5-(2,4-dimethoxyphenyl)-1,3,3,5,7-pentamethyloctahydrobenzo[c]isoxazole; 1-isopropyl-3,3,7-trimethyl-5-(o-tolyl)octahydrobenzo[c]isoxazole; 1-isopropyl-3,3,7-trimethyl-5-(3-methylbut-2-en-1-yl)octahydrobenzo[c]isoxazole; 5-(5-fluoro-2-methylphenyl)-1,3,3,7-tetramethyloctahydrobenzo[c]isoxazole; 5-(3,5-dimethylphenyl)-1,3,3,5,7-pentamethyloctahydrobenzo[c]isoxazole; 1-isopropyl-3,3,5,7-tetramethyloctahydrobenzo[c]isoxazole; 1,3,3-trimethyl-6-(4-methylcyclohex-3-en-1-yl)octahydrobenzo[c]isoxazole; 5-(3-fluoro-5-(trifluoromethyl)phenyl)-1,3,3,5,7-pentamethyloctahydrobenzo[c]isoxazole; 1-isopropyl-3,3-dimethyl-5-propyloctahydrobenzo[c]isoxazole; 1,3,3-trimethyl-5-pentyloctahydrobenzo[c]isoxazole; 1-ethyl-3,3,5,7-tetramethyl-5-(o-tolyl)octahydrobenzo[c]isoxazole; 5-(3,5-dimethylphenyl)-1-isopropyl-3,3,5,7-tetramethyloctahydrobenzo[c]isoxazole; 5-(2,5-dimethylphenyl)-1,3,3,7-tetramethyloctahydrobenzo[c]isoxazole; 5,7-diethyl-1-isopropyl-3,3-dimethyloctahydrobenzo[c]isoxazole; 1,3,3,5,7-pentamethyl-5-(2-methyl-5-(trifluoromethyl)phenyl)octahydrobenzo[c]isoxazole; 1-ethyl-3,3-dimethyl-5-propyloctahydrobenzo[c]isoxazole; 1,3,3,5,7-pentamethyl-5-propyloctahydrobenzo[c]isoxazole; 1,3,3-trimethyl-5-propyloctahydrobenzo[c]isoxazole; 2-methyl-3-(1,3,3,5,7-pentamethyloctahydrobenzo[c]isoxazol-5-yl)benzonitrile; 1,3,3,7-tetramethyl-5-(o-tolyl)octahydrobenzo[c]isoxazole; 5-butyl-1-ethyl-3,3-dimethyloctahydrobenzo[c]isoxazole; 5-(4-fluorophenyl)-1,3,3,5,7-pentamethyloctahydrobenzo[c]isoxazole; 5-(furan-3-yl)-1,3,3,5,7-pentamethyloctahydrobenzo[c]isoxazole; 1-isopropyl-3,3,5,7-tetramethyloctahydrobenzo[c]isoxazol-1-ium chloride; 1,3,3,5,7-pentamethyl-5-(naphthalen-1-yl)octahydrobenzo[c]isoxazole; 5-(2-ethoxyphenyl)-1,3,3,5,7-pentamethyloctahydrobenzo[c]isoxazole; 5-(3-chloro-2-methylphenyl)-1,3,3,5,7-pentamethyloctahydrobenzo[c]isoxazole; 5-(6-methoxypyridin-2-yl)-1,3,3,5,7-pentamethyloctahydrobenzo[c]isoxazole; 1,3,3,5,7-pentamethyl-5-(5-methylthiophen-2-yl)octahydrobenzo[c]isoxazole; 1,3,3,5,7-pentamethyl-5-(4-methylpyridin-3-yl)octahydrobenzo[c]isoxazole; 1-ethyl-3,3,5,7-tetramethyloctahydrobenzo[c]isoxazole; 5-(2-ethylphenyl)-1,3,3,7-tetramethyloctahydrobenzo[c]isoxazole; 5-butyl-1-isopropyl-3,3-dimethyloctahydrobenzo[c]isoxazole; 1-ethyl-3,3,5,7-tetramethyl-5-(3-methylbut-2-en-1-yl)octahydrobenzo[c]isoxazole; 5-(4-fluoro-2-methylphenyl)-1,3,3,5,7-pentamethyloctahydrobenzo[c]isoxazole; 1-isopropyl-5,7-dimethylhexahydro-1H-spiro[benzo[c]isoxazole-3,1'-cyclopentane]; 1,5,7-triethyl-3,3-dimethyloctahydrobenzo[c]isoxazole; 1-isopropyl-3,3,5,7-tetramethyl-5-propyloctahydrobenzo[c]isoxazole; 6-isopentyl-1,4,4,8-tetramethyloctahydro-2H-benzo[d][1,3]oxazine; 5-(3,5-dimethoxyphenyl)-1,3,3,5,7-pentamethyloctahydrobenzo[c]isoxazole; 5,7-diethyl-1,3,3-trimethyloctahydrobenzo[c]isoxazole; 1-isopropyl-5-(4-methoxyphenyl)-3,3,5,7-tetramethyloctahydrobenzo[c]isoxazole; 5-(3-isopropylphenyl)-1,3,3,5,7-pentamethyloctahydrobenzo[c]isoxazole; 1,3,3-trimethyl-6-phenyloctahydrobenzo[c]isoxazole; 5-ethyl-1-isopropyl-3,3-dimethyloctahydrobenzo[c]isoxazole; 1,3,3,5,7-pentamethyl-5-(2-(methylthio)phenyl)octahydrobenzo[c]isoxazole; 1,5,7-trimethylhexahydro-1H-spiro[benzo[c]isoxazole-3,1'-cyclopentane]; 5-ethyl-1,3,3,5,7-pentamethyloctahydrobenzo[c]isoxazole; 5-(4-methoxyphenyl)-1,3,3,5,7-pentamethyloctahydrobenzo[c]isoxazole; 1,3,3,5,7-pentamethyloctahydrobenzo[c]isoxazole; 1,3,3,4,5,7-hexamethyloctahydrobenzo[c]isoxazole; 1,3,3,5,7-pentamethyl-5-(pyridin-3-yl)octahydrobenzo[c]isoxazole; 1,3,3,5,7-pentamethyloctahydrobenzo[c]isoxazole hydrochloride; 5-(4-fluoro-2-methoxyphenyl)-1,3,3,5,7-pentamethyloctahydrobenzo[c]isoxazole; 5-(4-fluorophenyl)-1-isopropyl-3,3,5,7-tetramethyloctahydrobenzo[c]isoxazole; 4-methyl-2-(1,3,3,5,7-pentamethyloctahydrobenzo[c]isoxazol-5-yl)benzonitrile; 1,3,3,5-tetramethyl-5-(3-methylbut-2-en-1-yl)octahydrobenzo[c]isoxazole; 1-isopropyl-3,3,5,7-tetramethyl-5-(3-methylbut-2-en-1-yl)octahydrobenzo[c]isoxazole; 5-isopentyl-1-isopropyl-3,3,5,7-tetramethyloctahydrobenzo[c]isoxazole; 2-chloro-5-(1,3,3,5,7-pentamethyloctahydrobenzo[c]isoxazol-5-yl)benzonitrile; 5-(4-(tert-butyl)phenyl)-1,3,3,5,7-pentamethyloctahydrobenzo[c]isoxazole; 5-(4-(tert-butyl)phenyl)-1,3,3,7-tetramethyloctahydrobenzo[c]isoxazole; 5-(4-(tert-butyl)phenyl)-1-isopropyl-3,3,7-trimethyloctahydrobenzo[c]isoxazole; and 1,3,3,6-tetramethyloctahydrobenzo[c]isoxazole, or a salt or solvate thereof.

## Patentansprüche

1. Nicht medizinisches Verfahren zur Modulierung von TRPM8 (Transient Rezeptor Potential Kanal Melastatin Member 8), umfassend das Inkontaktbringen des Rezeptors mit einer Verbindung der Formel (Ia), einem Salz oder Solvat davon 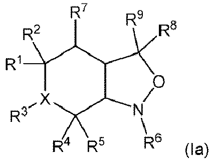 wobei
X für C steht,
R³ ausgewählt ist aus Wasserstoff, C₁-C₆-Alkyl, C₁-C₇-Alkyl, das durch eine oder zwei OH-Gruppen substituiert ist, C₃-C₇-Cycloalkyl, C₃-C₇-Cycloalkyl, das durch eine oder zwei OH-Gruppen substituiert ist, C₅-C₇-Cycloalkenyl, C₅-C₇-Cycloalkenyl, das durch eine, zwei oder drei C₁-C₃-Alkylgruppen substituiert ist, und Phenyl;
**R¹** ausgewählt ist aus Wasserstoff,
C₁-C₆-Alkyl,
C₁-C₇-Alkyl, das durch eine oder zwei OH-Gruppen substituiert ist,
C₃-C₇-Alkenyl,
Phenyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist,
Naphthyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist, und
mono- oder bicyclischem C₅-C₁₀-Aryl, wobei 1 oder 2 C-Atome durch ein unabhängig aus Schwefel, Stickstoff und Sauerstoff ausgewähltes Heteroatom ersetzt sind, das gegebenenfalls durch bis zu drei Substituenten, die aus der Gruppe bestehend aus Halogen, C≡N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist;
**R²** aus Wasserstoff und Methyl ausgewählt ist;
**R⁴, R⁵ und R⁷** unabhängig aus Wasserstoff und C₁-C₆-Alkyl ausgewählt sind;
**R⁶** aus Wasserstoff, C₁-C₆-Alkyl und C₃-C₇-Cycloalkyl ausgewählt ist;
**R⁸ und R⁹** unabhängig aus Wasserstoff und C₁-C₆-Alkyl ausgewählt ist oder
R⁸ und R⁹ zusammen mit dem C-Atom, an das sie gebunden sind, einen 3-, 4-, 5-, 6- oder 7-gliedrigen Cycloalkylring bilden.

2. Nicht medizinisches Verfahren nach Anspruch 1, wobei es sich bei der Verbindung der Formel (Ia), einem Salz oder Solvat davon um eine Verbindung handelt, in der R³ aus Wasserstoff, C₁-C₆-Alkyl, C₁-C₇-Alkyl, das durch eine oder zwei OH-Gruppen substituiert ist, C₃-C₇-Cycloalkyl, C₃-C₇-Cycloalkyl, das durch eine oder zwei OH-Gruppen substituiert ist, C₅-C₇-Cycloalkenyl, C₅-C₇-Cycloalkenyl, das durch eine, zwei oder drei C₁-C₃-Alkylgruppen substituiert ist, und Phenyl ausgewählt ist.

3. Nicht medizinisches Verfahren nach Anspruch 1, wobei die Verbindung der Formel (Ia) ausgewählt ist aus der Gruppe bestehend aus 4-Methyl-3-(1,3,3,5,7-pentamethyloctahydrobenzo[c]isoxazol-5-yl)benzonitril; 4-Methoxy-3-(1,3,3,5,7-pentamethyloctahydrobenzo[c]isoxazol-5-yl)benzonitril; 1-Ethyl-5-(2-methoxyphenyl)-3,3,7-trimethyloctahydrobenzo[c]isoxazol; 5-(2-Methoxyphenyl)-1,3,3,7-tetramethyloctahydrobenzo[c]isoxazol; 5-(3,5-Difluorphenyl)-1,3,3,5,7-pentamethyloctahydrobenzo[c]isoxazol; 1,3,3,5,7-Pentamethyloctahydrobenzo[c]isoxazol-5-yl)benzonitril; 1,3,3,5,7-Pentamethyl-5-(o-tolyl)octahydrobenzo[c]isoxazol; 5-(5-Chlor-2-methylphenyl)-1,3,3,5,7-pentamethyloctahydrobenzo[c]isoxazol; 3-(1-Isopropyl-3,3,7-trimethyloctahydrobenzo[c]isoxazol-5-yl)-4-methoxybenzonitril; 5-(2,5-Dimethylphenyl)-1,3,3,5,7-pentamethyloctahydrobenzo[c]isoxazol; 3-(1-Ethyl-3,3,5,7-tetramethyloctahydrobenzo[c]isoxazol-5-yl)-4-methylbenzonitril; 5-(5-Fluor-2-methylphenyl)-1,3,3,5,7-pentamethyloctahydrobenzo[c]isoxazol; 1-Isopropyl-5-(2-methoxyphenyl)-3,3,7-trimethyloctahydrobenzo[c]isoxazol; 3-Chlor-5-(1,3,3,5,7-pentamethyloctahydrobenzo[c]isoxazol-5-yl)benzonitril; 3-(1-Isopropyl-3,3,5,7-tetramethyloctahydrobenzo[c]isoxazol-5-yl)benzonitril; 5-(3,4-Difluorphenyl)-1,3,3,5,7-pentamethyloctahydrobenzo[c]isoxazol; 3-(1-Isopropyl-3,3,5,7-tetramethyloctahydrobenzo[c]isoxazol-5-yl)-4-methylbenzonitril; 5-(2-Methoxyphenyl)-1,3,3,5,7-pentamethyloctahydrobenzo[c]isoxazol; 2-Methyl-5-(1,3,3,5,7-pentamethyloctahydrobenzo[c]isoxazol-5-yl)benzonitril; 4-Methyl-3-(1,3,3,5,7-pentamethyloctahydrobenzo[c]isoxazol-5-yl)benzoesäuremethylester; 1-Isopropyl-3,3,5,7-tetramethyl-5-(o-tolyl)octahydrobenzo[c]isoxazol; 5-(3-Fluorphenyl)-1,3,3,5,7-pentamethyloctahydrobenzo[c]isoxazol; 1,3,3,5,7-Pentamethyl-5-phenyloctahydrobenzo[c]isoxazol; 1-Isopropyl-3,3,7-trimethyl-5-phenyloctahydrobenzo[c]isoxazol; 5-(4-Chlor-2-methylphenyl)-1-isopropyl-3,3,7-trimethyloctahydrobenzo[c]isoxazol; 5-(2,4-Dimethylphenyl)-1,3,3,5,7-pentamethyloctahydrobenzo[c]isoxazol; 5-(4-Fluor-2-methylphenyl)-1,3,3,7-tetramethyloctahydrobenzo[c]isoxazol; 5-(2,5-Difluorphenyl)-1,3,3,5,7-pentamethyloctahydrobenzo[c]isoxazol; 1-Ethyl-3,3,5,7-tetramethyl-5-phenyloctahydrobenzo[c]isoxazol; 1,3,3,5,7-Pentamethyl-5-(p-tolyl)octahydrobenzo[c]isoxazol; 1-Isopropyl-3,3,5,7-tetramethyl-5-phenyloctahydrobenzo[c]isoxazol; 1,3,3,7-Tetramethyl-5-phenyloctahydrobenzo[c]isoxazol; 1-Isopropyl-5-(2-methoxyphenyl)-3,3,5,7-tetramethyloctahydrobenzo[c]isoxazol; 5-(2-Fluorphenyl)-1,3,3,5,7-pentamethyloctahydrobenzo[c]isoxazol; 4-(1,3,3,5,7-Pentamethyloctahydrobenzo[c]isoxazol-5-yl)benzonitril; 3-Methyl-4-(1,3,3,5,7-pentamethyloctahydrobenzo[c]isoxazol-5-yl)benzonitril; 5-(2,4-Difluorphenyl)-1,3,3,5,7-pentamethyloctahydrobenzo[c]isoxazol; 5-(2,5-Dimethoxyphenyl)-1,3,3,5,7-pentamethyloctahydrobenzo[c]isoxazol; 5-(2,3-Dimethylphenyl)-1,3,3,5,7-pentamethyloctahydrobenzo[c]isoxazol; 1,3,3,5,7-Pentamethyl-5-(2,4,5-trimethylphenyl)octahydrobenzo[c]isoxazol; 1,3,3,5,7-Pentamethyl-5-(4-(methylthio)phenyl)octahydrobenzo[c]isoxazol; 2-(1,3,3,5,7-Pentamethyloctahydrobenzo[c]isoxazol-5-yl)benzonitril; 5-(3-Methoxyphenyl)-1,3,3,5,7-pentamethyloctahydrobenzo[c]isoxazol; 5-(3-Chlor-2-methylphenyl)-1-isopropyl-3,3,7-trimethyloctahydrobenzo[c]isoxazol; 1,3,3,5,7-Pentamethyl-5-(naphthalin-2-yl)octahydrobenzo[c]isoxazol; 5-(2,4-Dimethylphenyl)-1,3,3,7-tetramethyloctahydrobenzo[c]isoxazol; 4-Methyl-3-(1,3,3,7-tetramethyloctahydrobenzo[c]isoxazol-5-yl)benzonitril; 1,3,3,7-Tetramethyl-5-(3-methylbut-2-en-1-yl)octahydrobenzo[c]isoxazol; 5-(2-Ethylphenyl)-1,3,3,5,7-pentamethyloctahydrobenzo[c]isoxazol; 1,3,3,5,7-Pentamethyl-5-(m-tolyl)octahydrobenzo[c]isoxazol; 1,3,3,5,7-Pentamethyl-5-(5-methylthiophen-3-yl)octahydrobenzo[c]isoxazol; 1-Ethyl-3,3,7-trimethyl-5-(3-methylbut-2-en-1-yl)octahydrobenzo[c]isoxazol; 5-(2-Ethylphenyl)-1-isopropyl-3,3,7-trimethyloctahydrobenzo[c]isoxazol; 1,3,3,5,7-Pentamethyl-5-(3-methylbut-2-en-1-yl)octahydrobenzo[c]isoxazol; 5-(2,6-Dimethylphenyl)-1,3,3,5,7-pentamethyloctahydrobenzo[c]isoxazol; 5-(Benzo[d][1,3]dioxol-5-yl)-1,3,3,5,7-pentamethyloctahydrobenzo[c]isoxazol; 1-Isopropyl-5-(6-methoxypyridin-2-yl)-3,3,5,7-tetramethyloctahydrobenzo[c]isoxazol; 5-(5-Chlor-2-methylphenyl)-1-isopropyl-3,3,7-trimethyloctahydrobenzo[c]isoxazol; 5-Butyl-1,3,3-trimethyloctahydrobenzo[c]isoxazol; 5-(2,4-Dimethoxyphenyl)-1,3,3,5,7-pentamethyloctahydrobenzo[c]isoxazol; 1-Isopropyl-3,3,7-trimethyl-5-(o-tolyl)octahydrobenzo[c]isoxazol; 1-Isopropyl-3,3,7-trimethyl-5-(3-methylbut-2-en-1-yl)octahydrobenzo[c]isoxazol; 5-(5-Fluor-2-methylphenyl)-1,3,3,7-tetramethyloctahydrobenzo[c]isoxazol; 5-(3,5-Dimethylphenyl)-1,3,3,5,7-pentamethyloctahydrobenzo[c]isoxazol; 1-Isopropyl-3,3,5,7-tetramethyloctahydrobenzo[c]isoxazol; 1,3,3-Trimethyl-6-(4-methylcyclohex-3-en-1-yl)octahydrobenzo[c]isoxazol; 5-(3-Fluor-5-(trifluormethyl)phenyl)-1,3,3,5,7-pentamethyloctahydrobenzo[c]isoxazol; 1-Isopropyl-3,3-dimethyl-5-propyloctahydrobenzo[c]isoxazol; 1,3,3-Trimethyl-5-pentyloctahydrobenzo[c]isoxazol; 1-Ethyl-3,3,5,7-tetramethyl-5-(o-tolyl)octahydrobenzo[c]isoxazol; 5-(3,5-Dimethylphenyl)-1-isopropyl-3,3,5,7-tetramethyloctahydrobenzo[c]isoxazol; 5-(2,5-Dimethylphenyl)-1,3,3,7-tetramethyloctahydrobenzo[c]isoxazol; 5,7-Diethyl-1-isopropyl-3,3-dimethyloctahydrobenzo[c]isoxazol; 1,3,3,5,7-Pentamethyl-5-(2-methyl-5-(trifluormethyl)phenyl)octahydrobenzo[c]isoxazol; 1-Ethyl-3,3-dimethyl-5-propyloctahydrobenzo[c]isoxazol; 1,3,3,5,7-Pentamethyl-5-propyloctahydrobenzo[c]isoxazol; 1,3,3-Trimethyl-5-propyloctahydrobenzo[c]isoxazol; 2-Methyl-3-(1,3,3,5,7-pentamethyloctahydrobenzo[c]isoxazol-5-yl)benzonitril; 1,3,3,7-Tetramethyl-5-(o-tolyl)octahydrobenzo[c]isoxazol; 5-Butyl-1-ethyl-3,3-dimethyloctahydrobenzo[c]isoxazol; 5-(4-Fluorphenyl)-1,3,3,5,7-pentamethyloctahydrobenzo[c]isoxazol; 5-(Furan-3-yl)-1,3,3,5,7-pentamethyloctahydrobenzo[c]isoxazol; 1-Isopropyl-3,3,5,7-tetramethyloctahydrobenzo[c]isoxazol-1-iumchlorid; 1,3,3,5,7-Pentamethyl-5-(naphthalin-1-yl)octahydrobenzo[c]isoxazol; 5-(2-Ethoxyphenyl)-1,3,3,5,7-pentamethyloctahydrobenzo[c]isoxazol; 5-(3-Chlor-2-methylphenyl)-1,3,3,5,7-pentamethyloctahydrobenzo[c]isoxazol; 5-(6-Methoxypyridin-2-yl)-1,3,3,5,7-pentamethyloctahydrobenzo[c]isoxazol; 1,3,3,5,7-Pentamethyl-5-(5-methylthiophen-2-yl)octahydrobenzo[c]isoxazol; 1,3,3,5,7-Pentamethyl-5-(4-methylpyridin-3-yl)octahydrobenzo[c]isoxazol; 1-Ethyl-3,3,5,7-tetramethyloctahydrobenzo[c]isoxazol; 5-(2-Ethylphenyl)-1,3,3,7-tetramethyloctahydrobenzo[c]isoxazol; 5-Butyl-1-isopropyl-3,3-dimethyloctahydrobenzo[c]isoxazol; 1-Ethyl-3,3,5,7-tetramethyl-5-(3-methylbut-2-en-1-yl)octahydrobenzo[c]isoxazol; 5-(4-Fluor-2-methylphenyl)-1,3,3,5,7-pentamethyloctahydrobenzo[c]isoxazol; 1-Isopropyl-5,7-dimethylhexahydro-1H-spiro[benzo[c]isoxazol-3,1'-cyclopentan]; 1,5,7-Triethyl-3,3-dimethyloctahydrobenzo[c]isoxazol; 1-Isopropyl-3,3,5,7-tetramethyl-5-propyloctahydrobenzo[c]isoxazol; 6-Isopentyl-1,4,4,8-tetramethyloctahydro-2H-benzo[d][1,3]oxazin; 5-(3,5-Dimethoxyphenyl)-1,3,3,5,7-pentamethyloctahydrobenzo[c]isoxazol; 5,7-Diethyl-1,3,3-trimethyloctahydrobenzo[c]isoxazol; 1-Isopropyl-5-(4-methoxyphenyl)-3,3,5,7-tetramethyloctahydrobenzo[c]isoxazol; 5-(3-Isopropylphenyl)-1,3,3,5,7-pentamethyloctahydrobenzo[c]isoxazol; 1,3,3-Trimethyl-6-phenyloctahydrobenzo[c]isoxazol; 5-Ethyl-1-isopropyl-3,3-dimethyloctahydrobenzo[c]isoxazol; 1,3,3,5,7-Pentamethyl-5-(2-(methylthio)phenyl)octahydrobenzo[c]isoxazol; 1,5,7-Trimethylhexahydro-1H-spiro[benzo[c]isoxazol-3,1'-cyclopentan]; 5-Ethyl-1,3,3,5,7-pentamethyloctahydrobenzo[c]isoxazol; 5-(4-Methoxyphenyl)-1,3,3,5,7-pentamethyloctahydrobenzo[c]isoxazol; 1,3,3,5,7-Pentamethyloctahydrobenzo[c]isoxazol; 1,3,3,4,5,7-Hexamethyloctahydrobenzo[c]isoxazol; 1,3,3,5,7-Pentamethyl-5-(pyridin-3-yl)octahydrobenzo[c]isoxazol; 1,3,3,5,7-Pentamethyloctahydrobenzo[c]isoxazol-hydrochlorid; 5-(4-Fluor-2-methoxyphenyl)-1,3,3,5,7-pentamethyloctahydrobenzo[c]isoxazol; 5-(4-Fluorphenyl)-1-isopropyl-3,3,5,7-tetramethyloctahydrobenzo[c]isoxazol; 4-Methyl-2-(1,3,3,5,7-pentamethyloctahydrobenzo[c]isoxazol-5-yl)benzonitril; 1,3,3,5-Tetramethyl-5-(3-methylbut-2-en-1-yl)octahydrobenzo[c]isoxazol; 1-Isopropyl-3,3,5,7-tetramethyl-5-(3-methylbut-2-en-1-yl)octahydrobenzo[c]isoxazol; 5-Isopentyl-1-isopropyl-3,3,5,7-tetramethyloctahydrobenzo[c]isoxazol; 2-Chlor-5-(1,3,3,5,7-pentamethyloctahydrobenzo[c]isoxazol-5-yl)benzonitril; 5-(4-(tert-Butyl)phenyl)-1,3,3,5,7-pentamethyloctahydrobenzo[c]isoxazol; 5-(4-(tert-Butyl)phenyl)-1,3,3,7-tetramethyloctahydrobenzo[c]isoxazol; 5-(4-(tert-Butyl)phenyl)-1-isopropyl-3,3,7-trimethyloctahydrobenzo[c]isoxazol und 1,3,3,6-Tetramethyloctahydrobenzo[c]isoxazol oder einem Salz oder Solvat davon.

4. Nicht medizinisches Verfahren zur Herbeiführung eines kühlenden Gefühls bei einem Menschen oder Tier, umfassend das Inkontaktbringen des Menschen oder Tieres mit einer Verbindung der Formel (Ia) oder einem Salz oder Solvat davon gemäß Anspruch 1 bis 3.

5. Nicht medizinisches Verfahren zur Erzielung einer kühlenden Wirkung auf der Haut oder Schleimhaut, umfassend das Inkontaktbringen der Haut oder Schleimhaut mit einem Produkt, das eine oder mehrere Verbindungen der Formel (Ia) gemäß Anspruch 1 bis 3 umfasst.

6. Konsumprodukt, umfassend eine oder mehrere Verbindungen der Formel (Ia) gemäß Anspruch 1 bis 3.

7. Pharmazeutische Zusammensetzung, umfassend eine oder mehrere Verbindungen der Formel (Ia) gemäß Anspruch 1 bis 3.

8. Verbindung der Formel (Ia) gemäß Anspruch 1 bis 3 zur Verwendung als Medikament.

9. Zusammensetzung, umfassend ein kühlendes Gefühl, wobei die Zusammensetzung eine Verbindung der Formel (Ia) 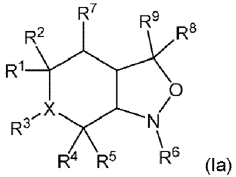 umfasst, wobei
X für C steht,
R³ ausgewählt ist aus Wasserstoff, C₁-C₆-Alkyl, C₁-C₇-Alkyl, das durch eine oder zwei OH-Gruppen substituiert ist, C₃-C₇-Cycloalkyl, C₃-C₇-Cycloalkyl, das durch eine oder zwei OH-Gruppen substituiert ist, C₅-C₇-Cycloalkenyl, C₅-C₇-Cycloalkenyl, das durch eine, zwei oder drei C₁-C₃-Alkylgruppen substituiert ist, und Phenyl;
R¹ ausgewählt ist aus Wasserstoff,
C₁-C₆-Alkyl,
C₁-C₇-Alkyl, das durch eine oder zwei OH-Gruppen substituiert ist,
C₃-C₇-Alkenyl,
Phenyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist,
Naphthyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist, und
mono- oder bicyclischem C₅-C₁₀-Aryl, wobei 1 oder 2 C-Atome durch ein unabhängig aus Schwefel, Stickstoff und Sauerstoff ausgewähltes Heteroatom ersetzt sind, das gegebenenfalls durch bis zu drei Substituenten, die aus der Gruppe bestehend aus Halogen, C≡N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist;
**R²** aus Wasserstoff und Methyl ausgewählt ist;
**R⁴, R⁵ und R⁷** unabhängig aus Wasserstoff und C₁-C₆-Alkyl ausgewählt sind;
**R⁶** aus Wasserstoff, C₁-C₆-Alkyl und C₃-C₇-Cycloalkyl ausgewählt ist;
**R⁸ und R⁹** unabhängig aus Wasserstoff und C₁-C₆-Alkyl ausgewählt ist oder
R⁸ und R⁹ zusammen mit dem C-Atom, an das sie gebunden sind, einen 3-, 4-, 5-, 6- oder 7-gliedrigen Cycloalkylring bilden.

10. Verbindung der Formel (Ia), Salz oder Solvat davon 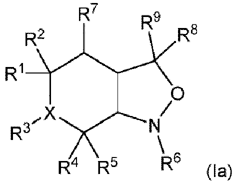 wobei
X für C steht,
R³ ausgewählt ist aus Wasserstoff, C₁-C₆-Alkyl, C₁-C₇-Alkyl, das durch eine oder zwei OH-Gruppen substituiert ist, C₃-C₇-Cycloalkyl, C₃-C₇-Cycloalkyl, das durch eine oder zwei OH-Gruppen substituiert ist, C₅-C₇-Cycloalkenyl, C₅-C₇-Cycloalkenyl, das durch eine, zwei oder drei C₁-C₃-Alkylgruppen substituiert ist, und Phenyl;
R¹ ausgewählt ist aus
C₁-C₆-Alkyl,
C₁-C₇-Alkyl, das durch eine oder zwei OH-Gruppen substituiert ist,
C₃-C₇-Alkenyl,
Phenyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist,
Naphthyl, das gegebenenfalls durch bis zu fünf Substituenten, die aus der Gruppe bestehend aus Halogen, C=N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist, und
mono- oder bicyclischem C₅-C₁₀-Aryl, wobei 1 oder 2 C-Atome durch ein unabhängig aus Schwefel, Stickstoff und Sauerstoff ausgewähltes Heteroatom ersetzt sind, das gegebenenfalls durch bis zu drei Substituenten, die aus der Gruppe bestehend aus Halogen, C≡N, NO₂, C₁-C₆-Alkyl, C₁-C₆-Alkyl mit bis zu 5 Halogenatomen, C₁-C₆-Alkyloxy, C₁-C₆-Alkyloxy mit bis zu 5 Halogenatomen, C₃-C₇-Cycloalkyl, -C(O)O-R¹⁰, wobei R¹⁰ aus Wasserstoff und C₁-C₃-Alkyl ausgewählt ist, und -SR¹¹, wobei R¹¹ aus Wasserstoff und C₁-C₃-Alkyl ausgewählt ist, ausgewählt sind, substituiert ist;
**R²** aus Wasserstoff und Methyl ausgewählt ist;
**R⁴, R⁵ und R⁷** unabhängig aus Wasserstoff und C₁-C₆-Alkyl ausgewählt sind;
**R⁶** aus Wasserstoff, C₁-C₆-Alkyl und C₃-C₇-Cycloalkyl ausgewählt ist;
**R⁸ und R⁹** unabhängig aus Wasserstoff und C₁-C₆-Alkyl ausgewählt ist oder
R⁸ und R⁹ zusammen mit dem C-Atom, an das sie gebunden sind, einen 3-, 4-, 5-, 6- oder 7-gliedrigen Cycloalkylring bilden.

11. Verbindung nach Anspruch 10, ausgewählt aus der Gruppe bestehend aus 4-Methyl-3-(1,3,3,5,7-pentamethyloctahydrobenzo[c]isoxazol-5-yl)benzonitril; 4-Methoxy-3-(1,3,3,5,7-pentamethyloctahydrobenzo[c]isoxazol-5-yl)benzonitril; 1-Ethyl-5-(2-methoxyphenyl)-3,3,7-trimethyloctahydrobenzo[c]isoxazol; 5-(2-Methoxyphenyl)-1,3,3,7-tetramethyloctahydrobenzo[c]isoxazol; 5-(3,5-Difluorphenyl)-1,3,3,5,7-pentamethyloctahydrobenzo[c]isoxazol; 1,3,3,5,7-Pentamethyloctahydrobenzo[c]isoxazol-5-yl)benzonitril; 1,3,3,5,7-Pentamethyl-5-(o-tolyl)octahydrobenzo[c]isoxazol; 5-(5-Chlor-2-methylphenyl)-1,3,3,5,7-pentamethyloctahydrobenzo[c]isoxazol; 3-(1-Isopropyl-3,3,7-trimethyloctahydrobenzo[c]isoxazol-5-yl)-4-methoxybenzonitril; 5-(2,5-Dimethylphenyl)-1,3,3,5,7-pentamethyloctahydrobenzo[c]isoxazol; 3-(1-Ethyl-3,3,5,7-tetramethyloctahydrobenzo[c]isoxazol-5-yl)-4-methylbenzonitril; 5-(5-Fluor-2-methylphenyl)-1,3,3,5,7-pentamethyloctahydrobenzo[c]isoxazol; 1-Isopropyl-5-(2-methoxyphenyl)-3,3,7-trimethyloctahydrobenzo[c]isoxazol; 3-Chlor-5-(1,3,3,5,7-pentamethyloctahydrobenzo[c]isoxazol-5-yl)benzonitril; 3-(1-Isopropyl-3,3,5,7-tetramethyloctahydrobenzo[c]isoxazol-5-yl)benzonitril; 5-(3,4-Difluorphenyl)-1,3,3,5,7-pentamethyloctahydrobenzo[c]isoxazol; 3-(1-Isopropyl-3,3,5,7-tetramethyloctahydrobenzo[c]isoxazol-5-yl)-4-methylbenzonitril; 5-(2-Methoxyphenyl)-1,3,3,5,7-pentamethyloctahydrobenzo[c]isoxazol; 2-Methyl-5-(1,3,3,5,7-pentamethyloctahydrobenzo[c]isoxazol-5-yl)benzonitril; 4-Methyl-3-(1,3,3,5,7-pentamethyloctahydrobenzo[c]isoxazol-5-yl)benzoesäuremethylester; 1-Isopropyl-3,3,5,7-tetramethyl-5-(o-tolyl)octahydrobenzo[c]isoxazol; 5-(3-Fluorphenyl)-1,3,3,5,7-pentamethyloctahydrobenzo[c]isoxazol; 1,3,3,5,7-Pentamethyl-5-phenyloctahydrobenzo[c]isoxazol; 1-Isopropyl-3,3,7-trimethyl-5-phenyloctahydrobenzo[c]isoxazol; 5-(4-Chlor-2-methylphenyl)-1-isopropyl-3,3,7-trimethyloctahydrobenzo[c]isoxazol; 5-(2,4-Dimethylphenyl)-1,3,3,5,7-pentamethyloctahydrobenzo[c]isoxazol; 5-(4-Fluor-2-methylphenyl)-1,3,3,7-tetramethyloctahydrobenzo[c]isoxazol; 5-(2,5-Difluorphenyl)-1,3,3,5,7-pentamethyloctahydrobenzo[c]isoxazol; 1-Ethyl-3,3,5,7-tetramethyl-5-phenyloctahydrobenzo[c]isoxazol; 1,3,3,5,7-Pentamethyl-5-(p-tolyl)octahydrobenzo[c]isoxazol; 1-Isopropyl-3,3,5,7-tetramethyl-5-phenyloctahydrobenzo[c]isoxazol; 1,3,3,7-Tetramethyl-5-phenyloctahydrobenzo[c]isoxazol; 1-Isopropyl-5-(2-methoxyphenyl)-3,3,5,7-tetramethyloctahydrobenzo[c]isoxazol; 5-(2-Fluorphenyl)-1,3,3,5,7-pentamethyloctahydrobenzo[c]isoxazol; 4-(1,3,3,5,7-Pentamethyloctahydrobenzo[c]isoxazol-5-yl)benzonitril; 3-Methyl-4-(1,3,3,5,7-pentamethyloctahydrobenzo[c]isoxazol-5-yl)benzonitril; 5-(2,4-Difluorphenyl)-1,3,3,5,7-pentamethyloctahydrobenzo[c]isoxazol; 5-(2,5-Dimethoxyphenyl)-1,3,3,5,7-pentamethyloctahydrobenzo[c]isoxazol; 5-(2,3-Dimethylphenyl)-1,3,3,5,7-pentamethyloctahydrobenzo[c]isoxazol; 1,3,3,5,7-Pentamethyl-5-(2,4,5-trimethylphenyl)octahydrobenzo[c]isoxazol; 1,3,3,5,7-Pentamethyl-5-(4-(methylthio)phenyl)octahydrobenzo[c]isoxazol; 2-(1,3,3,5,7-Pentamethyloctahydrobenzo[c]isoxazol-5-yl)benzonitril; 5-(3-Methoxyphenyl)-1,3,3,5,7-pentamethyloctahydrobenzo[c]isoxazol; 5-(3-Chlor-2-methylphenyl)-1-isopropyl-3,3,7-trimethyloctahydrobenzo[c]isoxazol; 1,3,3,5,7-Pentamethyl-5-(naphthalin-2-yl)octahydrobenzo[c]isoxazol; 5-(2,4-Dimethylphenyl)-1,3,3,7-tetramethyloctahydrobenzo[c]isoxazol; 4-Methyl-3-(1,3,3,7-tetramethyloctahydrobenzo[c]isoxazol-5-yl)benzonitril; 1,3,3,7-Tetramethyl-5-(3-methylbut-2-en-1-yl)octahydrobenzo[c]isoxazol; 5-(2-Ethylphenyl)-1,3,3,5,7-pentamethyloctahydrobenzo[c]isoxazol; 1,3,3,5,7-Pentamethyl-5-(m-tolyl)octahydrobenzo[c]isoxazol; 1,3,3,5,7-Pentamethyl-5-(5-methylthiophen-3-yl)octahydrobenzo[c]isoxazol; 1-Ethyl-3,3,7-trimethyl-5-(3-methylbut-2-en-1-yl)octahydrobenzo[c]isoxazol; 5-(2-Ethylphenyl)-1-isopropyl-3,3,7-trimethyloctahydrobenzo[c]isoxazol; 1,3,3,5,7-Pentamethyl-5-(3-methylbut-2-en-1-yl)octahydrobenzo[c]isoxazol; 5-(2,6-Dimethylphenyl)-1,3,3,5,7-pentamethyloctahydrobenzo[c]isoxazol; 5-(Benzo[d][1,3]dioxol-5-yl)-1,3,3,5,7-pentamethyloctahydrobenzo[c]isoxazol; 1-Isopropyl-5-(6-methoxypyridin-2-yl)-3,3,5,7-tetramethyloctahydrobenzo[c]isoxazol; 5-(5-Chlor-2-methylphenyl)-1-isopropyl-3,3,7-trimethyloctahydrobenzo[c]isoxazol; 5-Butyl-1,3,3-trimethyloctahydrobenzo[c]isoxazol; 5-(2,4-Dimethoxyphenyl)-1,3,3,5,7-pentamethyloctahydrobenzo[c]isoxazol; 1-Isopropyl-3,3,7-trimethyl-5-(o-tolyl)octahydrobenzo[c]isoxazol; 1-Isopropyl-3,3,7-trimethyl-5-(3-methylbut-2-en-1-yl)octahydrobenzo[c]isoxazol; 5-(5-Fluor-2-methylphenyl)-1,3,3,7-tetramethyloctahydrobenzo[c]isoxazol; 5-(3,5-Dimethylphenyl)-1,3,3,5,7-pentamethyloctahydrobenzo[c]isoxazol; 1-Isopropyl-3,3,5,7-tetramethyloctahydrobenzo[c]isoxazol; 1,3,3-Trimethyl-6-(4-methylcyclohex-3-en-1-yl)octahydrobenzo[c]isoxazol; 5-(3-Fluor-5-(trifluormethyl)phenyl)-1,3,3,5,7-pentamethyloctahydrobenzo[c]isoxazol; 1-Isopropyl-3,3-dimethyl-5-propyloctahydrobenzo[c]isoxazol; 1,3,3-Trimethyl-5-pentyloctahydrobenzo[c]isoxazol; 1-Ethyl-3,3,5,7-tetramethyl-5-(o-tolyl)octahydrobenzo[c]isoxazol; 5-(3,5-Dimethylphenyl)-1-isopropyl-3,3,5,7-tetramethyloctahydrobenzo[c]isoxazol; 5-(2,5-Dimethylphenyl)-1,3,3,7-tetramethyloctahydrobenzo[c]isoxazol; 5,7-Diethyl-1-isopropyl-3,3-dimethyloctahydrobenzo[c]isoxazol; 1,3,3,5,7-Pentamethyl-5-(2-methyl-5-(trifluormethyl)phenyl)octahydrobenzo[c]isoxazol; 1-Ethyl-3,3-dimethyl-5-propyloctahydrobenzo[c]isoxazol; 1,3,3,5,7-Pentamethyl-5-propyloctahydrobenzo[c]isoxazol; 1,3,3-Trimethyl-5-propyloctahydrobenzo[c]isoxazol; 2-Methyl-3-(1,3,3,5,7-pentamethyloctahydrobenzo[c]isoxazol-5-yl)benzonitril; 1,3,3,7-Tetramethyl-5-(o-tolyl)octahydrobenzo[c]isoxazol; 5-Butyl-1-ethyl-3,3-dimethyloctahydrobenzo[c]isoxazol; 5-(4-Fluorphenyl)-1,3,3,5,7-pentamethyloctahydrobenzo[c]isoxazol; 5-(Furan-3-yl)-1,3,3,5,7-pentamethyloctahydrobenzo[c]isoxazol; 1-Isopropyl-3,3,5,7-tetramethyloctahydrobenzo[c]isoxazol-1-iumchlorid; 1,3,3,5,7-Pentamethyl-5-(naphthalin-1-yl)octahydrobenzo[c]isoxazol; 5-(2-Ethoxyphenyl)-1,3,3,5,7-pentamethyloctahydrobenzo[c]isoxazol; 5-(3-Chlor-2-methylphenyl)-1,3,3,5,7-pentamethyloctahydrobenzo[c]isoxazol; 5-(6-Methoxypyridin-2-yl)-1,3,3,5,7-pentamethyloctahydrobenzo[c]isoxazol; 1,3,3,5,7-Pentamethyl-5-(5-methylthiophen-2-yl)octahydrobenzo[c]isoxazol; 1,3,3,5,7-Pentamethyl-5-(4-methylpyridin-3-yl)octahydrobenzo[c]isoxazol; 1-Ethyl-3,3,5,7-tetramethyloctahydrobenzo[c]isoxazol; 5-(2-Ethylphenyl)-1,3,3,7-tetramethyloctahydrobenzo[c]isoxazol; 5-Butyl-1-isopropyl-3,3-dimethyloctahydrobenzo[c]isoxazol; 1-Ethyl-3,3,5,7-tetramethyl-5-(3-methylbut-2-en-1-yl)octahydrobenzo[c]isoxazol; 5-(4-Fluor-2-methylphenyl)-1,3,3,5,7-pentamethyloctahydrobenzo[c]isoxazol; 1-Isopropyl-5,7-dimethylhexahydro-1H-spiro[benzo[c]isoxazol-3,1'-cyclopentan]; 1,5,7-Triethyl-3,3-dimethyloctahydrobenzo[c]isoxazol; 1-Isopropyl-3,3,5,7-tetramethyl-5-propyloctahydrobenzo[c]isoxazol; 6-Isopentyl-1,4,4,8-tetramethyloctahydro-2H-benzo[d][1,3]oxazin; 5-(3,5-Dimethoxyphenyl)-1,3,3,5,7-pentamethyloctahydrobenzo[c]isoxazol; 5,7-Diethyl-1,3,3-trimethyloctahydrobenzo[c]isoxazol; 1-Isopropyl-5-(4-methoxyphenyl)-3,3,5,7-tetramethyloctahydrobenzo[c]isoxazol; 5-(3-Isopropylphenyl)-1,3,3,5,7-pentamethyloctahydrobenzo[c]isoxazol; 1,3,3-Trimethyl-6-phenyloctahydrobenzo[c]isoxazol; 5-Ethyl-1-isopropyl-3,3-dimethyloctahydrobenzo[c]isoxazol; 1,3,3,5,7-Pentamethyl-5-(2-(methylthio)phenyl)octahydrobenzo[c]isoxazol; 1,5,7-Trimethylhexahydro-1H-spiro[benzo[c]isoxazol-3,1'-cyclopentan]; 5-Ethyl-1,3,3,5,7-pentamethyloctahydrobenzo[c]isoxazol; 5-(4-Methoxyphenyl)-1,3,3,5,7-pentamethyloctahydrobenzo[c]isoxazol; 1,3,3,5,7-Pentamethyloctahydrobenzo[c]isoxazol; 1,3,3,4,5,7-Hexamethyloctahydrobenzo[c]isoxazol; 1,3,3,5,7-Pentamethyl-5-(pyridin-3-yl)octahydrobenzo[c]isoxazol; 1,3,3,5,7-Pentamethyloctahydrobenzo[c]isoxazol-hydrochlorid; 5-(4-Fluor-2-methoxyphenyl)-1,3,3,5,7-pentamethyloctahydrobenzo[c]isoxazol; 5-(4-Fluorphenyl)-1-isopropyl-3,3,5,7-tetramethyloctahydrobenzo[c]isoxazol; 4-Methyl-2-(1,3,3,5,7-pentamethyloctahydrobenzo[c]isoxazol-5-yl)benzonitril; 1,3,3,5-Tetramethyl-5-(3-methylbut-2-en-1-yl)octahydrobenzo[c]isoxazol; 1-Isopropyl-3,3,5,7-tetramethyl-5-(3-methylbut-2-en-1-yl)octahydrobenzo[c]isoxazol; 5-Isopentyl-1-isopropyl-3,3,5,7-tetramethyloctahydrobenzo[c]isoxazol; 2-Chlor-5-(1,3,3,5,7-pentamethyloctahydrobenzo[c]isoxazol-5-yl)benzonitril; 5-(4-(tert-Butyl)phenyl)-1,3,3,5,7-pentamethyloctahydrobenzo[c]isoxazol; 5-(4-(tert-Butyl)phenyl)-1,3,3,7-tetramethyloctahydrobenzo[c]isoxazol; 5-(4-(tert-Butyl)phenyl)-1-isopropyl-3,3,7-trimethyloctahydrobenzo[c]isoxazol und 1,3,3,6-Tetramethyloctahydrobenzo[c]isoxazol oder einem Salz oder Solvat davon.

## Revendications

1. Procédé non médical de modulation de l'élément de mélastatine à canal potentiel de récepteur transitoire 8 (TRPM8) comprenant la mise en contact du récepteur avec un composé de formule (Ia), un sel ou un solvate correspondant 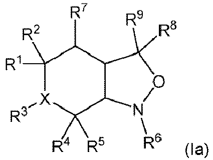
X étant C,
R³ étant choisi parmi hydrogène, C₁-C₆ alkyle, C₁-C₇ alkyle substitué par un ou deux groupes OH, C₃-C₇ cycloalkyle, C₃-C₇ cycloalkyle substitué par un ou deux groupes OH, C₅-C₇ cycloalcényle, C₅-C₇ cycloalcényle substitué par un, deux ou trois groupes C₁-C₃ alkyle, et phényle ;
R¹ étant choisi parmi hydrogène,
C₁-C₆ alkyle,
C₁-C₇ alkyle substitué par un ou deux groupes OH,
C₃-C₇ alcényle,
phényle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, Ci-Ce alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, naphtyle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, et
C₅-C₁₀ aryle monocyclique ou bicyclique, 1 ou 2 atomes de C étant remplacés par un hétéroatome indépendamment choisi parmi soufre, azote et oxygène, éventuellement substitué par jusqu'à trois substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle ;
R² étant choisi parmi hydrogène et méthyle ;
R⁴, R⁵, et R⁷ étant indépendamment choisis parmi hydrogène et C₁-C₆ alkyle ;
R⁶ étant choisi parmi hydrogène, C₁-C₆ alkyle, et C₃-C₇ cycloalkyle ;
R⁸ et R⁹ étant indépendamment choisis parmi hydrogène, et C₁-C₆ alkyle, ou
R⁸ et R⁹ formant conjointement avec l'atome de C auquel ils sont fixés un cycle cycloalkyle à 3, 4, 5, 6 ou 7 chaînons.

2. Procédé non médical selon la revendication 1, le composé de formule (Ia), un sel ou un solvate correspondant étant un composé, dans lequel R³ est choisi parmi hydrogène, C₁-C₆ alkyle, C₁-C₇ alkyle substitué par un ou deux groupes OH, C₃-C₇ cycloalkyle, C₃-C₇ cycloalkyle substitué par un ou deux groupes OH, C₅-C₇ cycloalcényle, C₅-C₇ cycloalcényle substitué par un, deux ou trois groupes C₁-C₃ alkyle, et phényle.

3. Procédé non médical selon la revendication 1, le composé de formule (Ia) étant choisi dans le groupe constitué par 4-méthyl-3-(1,3,3,5,7-pentaméthyloctahydrobenzo[c]isoxazol-5-yl)benzonitrile ; 4-méthoxy-3-(1,3,3,5,7-pentaméthyloctahydrobenzo[c]isoxazol-5-yl)benzonitrile ; 1-éthyl-5-(2-méthoxyphényl)-3,3,7-triméthyloctahydrobenzo[c]isoxazole ; 5-(2-méthoxyphényl)-1,3,3,7-tétraméthyloctahydrobenzo[c]isoxazole ; 5-(3,5-difluorophényl)-1,3,3,5,7-pentaméthyloctahydrobenzo[c]isoxazole ; 1,3,3,5,7-pentaméthyloctahydrobenzo[c]isoxazol-5-yl)benzonitrile ; 1,3,3,5,7-pentaméthyl-5-(o-tolyl)octahydrobenzo[c]isoxazole ; 5-(5-chloro-2-méthylphényl)-1,3,3,5,7-pentaméthyloctahydrobenzo[c]isoxazole; 3-(1-isopropyl-3,3,7-triméthyloctahydrobenzo[c]isoxazol-5-yl)-4-méthoxybenzonitrile ; 5-(2,5-diméthylphényl)-1,3,3,5,7-pentaméthyloctahydrobenzo[c]isoxazole ; 3-(1-éthyl-3,3,5,7-tétraméthyloctahydrobenzo[c]isoxazol-5-yl)-4-méthylbenzonitrile ; 5-(5-fluoro-2-méthylphényl)-1,3,3,5,7-pentaméthyloctahydrobenzo[c]isoxazole ; 1-isopropyl-5-(2-méthoxyphényl)-3,3,7-triméthyloctahydrobenzo[c]isoxazole ; 3-chloro-5-(1,3,3,5,7-pentaméthyloctahydrobenzo[c]isoxazol-5-yl)benzonitrile ; 3-(1-isopropyl-3,3,5,7-tétraméthyloctahydrobenzo[c]isoxazol-5-yl)benzonitrile ; 5-(3,4-difluorophényl)-1,3,3,5,7-pentaméthyloctahydrobenzo[c]isoxazole ; 3-(1-isopropyl-3,3,5,7-tétraméthyloctahydrobenzo[c]isoxazol-5-yl)-4-méthylbenzonitrile ; 5-(2-méthoxyphényl)-1,3,3,5,7-pentaméthyloctahydrobenzo[c]isoxazole ; 2-méthyl-5-(1,3,3,5,7-pentaméthyloctahydrobenzo[c]isoxazol-5-yl)benzonitrile ; méthyle 4-méthyl-3-(1,3,3,5,7-pentaméthyloctahydrobenzo[c]isoxazol-5-yl)benzoate ; 1-isopropyl-3,3,5,7-tétraméthyl-5-(o-tolyl)octahydrobenzo[c]isoxazole; 5-(3-fluorophényl)-1,3,3,5,7-pentaméthyloctahydrobenzo[c]isoxazole ; 1,3,3,5,7-pentaméthyl-5-phényloctahydrobenzo[c]isoxazole ; 1-isopropyl-3,3,7-triméthyl-5-phényloctahydrobenzo[c]isoxazole ; 5-(4-chloro-2-méthylphényl)-1-isopropyl-3,3,7-triméthyloctahydrobenzo[c]isoxazole ; 5-(2,4-diméthylphényl)-1,3,3,5,7-pentaméthyloctahydrobenzo[c]isoxazole; 5-(4-fluoro-2-méthylphényl)-1,3,3,7-tétraméthyloctahydrobenzo[c]isoxazole ; 5-(2,5-difluorophényl)-1,3,3,5,7-pentaméthyloctahydrobenzo[c]isoxazole ; 1-éthyl-3,3,5,7-tétraméthyl-5-phényloctahydrobenzo[c]isoxazole ; 1,3,3,5,7-pentaméthyl-5-(p-tolyl)octahydrobenzo[c]isoxazole ; 1-isopropyl-3,3,5,7-tétraméthyl-5-phényloctahydrobenzo[c]isoxazole ; 1,3,3,7-tétraméthyl-5-phényloctahydrobenzo[c]isoxazole ; 1-isopropyl-5-(2-méthoxyphényl)-3,3,5,7-tétraméthyloctahydrobenzo[c]isoxazole ; 5-(2-fluorophényl)-1,3,3,5,7-pentaméthyloctahydrobenzo[c]isoxazole ; 4-(1,3,3,5,7-pentaméthyloctahydrobenzo[c]isoxazol-5-yl)benzonitrile ; 3-méthyl-4-(1,3,3,5,7-pentaméthyloctahydrobenzo[c]isoxazol-5-yl)benzonitrile ; 5-(2,4-difluorophényl)-1,3,3,5,7-pentaméthyloctahydrobenzo[c]isoxazole ; 5-(2,5-diméthoxyphényl)-1,3,3,5,7-pentaméthyloctahydrobenzo[c]isoxazole ; 5-(2,3-diméthylphényl)-1,3,3,5,7-pentaméthyloctahydrobenzo[c]isoxazole ; 1,3,3,5,7-pentaméthyl-5-(2,4,5-triméthylphényl)octahydrobenzo[c]isoxazole ; 1,3,3,5,7-pentaméthyl-5-(4-(méthylthio)phényl)octahydrobenzo[c]isoxazole ; 2-(1,3,3,5,7-pentaméthyloctahydrobenzo[c]isoxazol-5-yl)benzonitrile ; 5-(3-méthoxyphényl)-1,3,3,5,7-pentaméthyloctahydrobenzo[c]isoxazole ; 5-(3-chloro-2-méthylphényl)-1-isopropyl-3,3,7-triméthyloctahydrobenzo[c]isoxazole ; 1,3,3,5,7-pentaméthyl-5-(naphtalén-2-yl)octahydrobenzo[c]isoxazole ; 5-(2,4-diméthylphényl)-1,3,3,7-tétraméthyloctahydrobenzo[c]isoxazole ; 4-méthyl-3-(1,3,3,7-tétraméthyloctahydrobenzo[c]isoxazol-5-yl)benzonitrile ; 1,3,3,7-tétraméthyl-5-(3-méthylbut-2-én-1-yl)octahydrobenzo[c]isoxazole ; 5-(2-éthylphényl)-1,3,3,5,7-pentaméthyloctahydrobenzo[c]isoxazole ; 1,3,3,5,7-pentaméthyl-5-(m-tolyl)octahydrobenzo[c]isoxazole ; 1,3,3,5,7-pentaméthyl-5-(5-méthylthiophén-3-yl)octahydrobenzo[c]isoxazole ; 1-éthyl-3,3,7-triméthyl-5-(3-méthylbut-2-én-1-yl)octahydrobenzo[c]isoxazole ; 5-(2-éthylphényl)-1-isopropyl-3,3,7-triméthyloctahydrobenzo[c]isoxazole ; 1,3,3,5,7-pentaméthyl-5-(3-méthylbut-2-én-1-yl)octahydrobenzo[c]isoxazole ; 5-(2,6-diméthylphényl)-1,3,3,5,7-pentaméthyloctahydrobenzo[c]isoxazole ; 5-(benzo[d][1,3]dioxol-5-yl)-1,3,3,5,7-pentaméthyloctahydrobenzo[c]isoxazole; 1-isopropyl-5-(6-méthoxypyridin-2-yl)-3,3,5,7-tétraméthyloctahydrobenzo[c]isoxazole; 5-(5-chloro-2-méthylphényl)-1-isopropyl-3,3,7-triméthyloctahydrobenzo[c]isoxazole ; 5-butyl-1,3,3-triméthyloctahydrobenzo[c]isoxazole ; 5-(2,4-diméthoxyphényl)-1,3,3,5,7-pentaméthyloctahydrobenzo[c]isoxazole ; 1-isopropyl-3,3,7-triméthyl-5-(o-tolyl)octahydrobenzo[c]isoxazole ; 1-isopropyl-3,3,7-triméthyl-5-(3-méthylbut-2-én-1-yl)octahydrobenzo[c]isoxazole ; 5-(5-fluoro-2-méthylphényl)-1,3,3,7-tétraméthyloctahydrobenzo[c]isoxazole ; 5-(3,5-diméthylphényl)-1,3,3,5,7-pentaméthyloctahydrobenzo[c]isoxazole ; 1-isopropyl-3,3,5,7-tétraméthyloctahydrobenzo[c]isoxazole ; 1,3,3-triméthyl-6-(4-méthylcyclohex-3-én-1-yl)octahydrobenzo[c]isoxazole ; 5-(3-fluoro-5-(trifluorométhyl)phényl)-1,3,3,5,7-pentaméthyloctahydrobenzo[c]isoxazole ; 1-isopropyl-3,3-diméthyl-5-propyloctahydrobenzo[c]isoxazole ; 1,3,3-triméthyl-5-pentyloctahydrobenzo[c]isoxazole ; 1-éthyl-3,3,5,7-tétraméthyl-5-(o-tolyl)octahydrobenzo[c]isoxazole ; 5-(3,5-diméthylphényl)-1-isopropyl-3,3,5,7-tétraméthyloctahydrobenzo[c]isoxazole ; 5-(2,5-diméthylphényl)-1,3,3,7-tétraméthyloctahydrobenzo[c]isoxazole ; 5,7-diéthyl-1-isopropyl-3,3-diméthyloctahydrobenzo[c]isoxazole ; 1,3,3,5,7-pentaméthyl-5-(2-méthyl-5-(trifluorométhyl)phényl)octahydrobenzo[c]isoxazole ; 1-éthyl-3,3-diméthyl-5-propyloctahydrobenzo[c]isoxazole ; 1,3,3,5,7-pentaméthyl-5-propyloctahydrobenzo[c]isoxazole; 1,3,3-triméthyl-5-propyloctahydrobenzo[c]isoxazole ; 2-méthyl-3-(1,3,3,5,7-pentaméthyloctahydrobenzo[c]isoxazol-5-yl)benzonitrile ; 1,3,3,7-tétraméthyl-5-(o-tolyl)octahydrobenzo[c]isoxazole ; 5-butyl-1-éthyl-3,3-diméthyloctahydrobenzo[c]isoxazole ; 5-(4-fluorophényl)-1,3,3,5,7-pentaméthyloctahydrobenzo[c]isoxazole ; 5-(furan-3-yl)-1,3,3,5,7-pentaméthyloctahydrobenzo[c]isoxazole ; chlorure de 1-isopropyl-3,3,5,7-tétraméthyloctahydrobenzo[c]isoxazol-1-ium ; 1,3,3,5,7-pentaméthyl-5-(naphtalén-1-yl)octahydrobenzo[c]isoxazole ; 5-(2-éthoxyphényl)-1,3,3,5,7-pentaméthyloctahydrobenzo[c]isoxazole ; 5-(3-chloro-2-méthylphényl)-1,3,3,5,7-pentaméthyloctahydrobenzo[c]isoxazole ; 5-(6-méthoxypyridin-2-yl)-1,3,3,5,7-pentaméthyloctahydrobenzo[c]isoxazole ; 1,3,3,5,7-pentaméthyl-5-(5-méthylthiophén-2-yl)octahydrobenzo[c]isoxazole ; 1,3,3,5,7-pentaméthyl-5-(4-méthylpyridin-3-yl)octahydrobenzo[c]isoxazole ; 1-éthyl-3,3,5,7-tétraméthyloctahydrobenzo[c]isoxazole ; 5-(2-éthylphényl)-1,3,3,7-tétraméthyloctahydrobenzo[c]isoxazole ; 5-butyl-1-isopropyl-3,3-diméthyloctahydrobenzo[c]isoxazole ; 1-éthyl-3,3,5,7-tétraméthyl-5-(3-méthylbut-2-én-1-yl)octahydrobenzo[c]isoxazole ; 5-(4-fluoro-2-méthylphényl)-1,3,3,5,7-pentaméthyloctahydrobenzo[c]isoxazole ; 1-isopropyl-5,7-diméthylhexahydro-1H-spiro[benzo[c]isoxazole-3,1'-cyclopentane] ; 1,5,7-triéthyl-3,3-diméthyloctahydrobenzo[c]isoxazole ; 1-isopropyl-3,3,5,7-tétraméthyl-5-propyloctahydrobenzo[c]isoxazole ; 6-isopentyl-1,4,4,8-tétraméthyloctahydro-2H-benzo[d][1,3]oxazine ; 5-(3,5-diméthoxyphényl)-1,3,3,5,7-pentaméthyloctahydrobenzo[c]isoxazole ; 5,7-diéthyl-1,3,3-triméthyloctahydrobenzo[c]isoxazole ; 1-isopropyl-5-(4-méthoxyphényl)-3,3,5,7-tétraméthyloctahydrobenzo[c]isoxazole ; 5-(3-isopropylphényl)-1,3,3,5,7-pentaméthyloctahydrobenzo[c]isoxazole ; 1,3,3-triméthyl-6-phényloctahydrobenzo[c]isoxazole ; 5-éthyl-1-isopropyl-3,3-diméthyloctahydrobenzo[c]isoxazole ; 1,3,3,5,7-pentaméthyl-5-(2-(méthylthio)phényl)octahydrobenzo[c]isoxazole ; 1,5,7-triméthylhexahydro-1H-spiro[benzo[c]isoxazole-3,1'-cyclopentane] ; 5-éthyl-1,3,3,5,7-pentaméthyloctahydrobenzo[c]isoxazole ; 5-(4-méthoxyphényl)-1,3,3,5,7-pentaméthyloctahydrobenzo[c]isoxazole ; 1,3,3,5,7-pentaméthyloctahydrobenzo[c]isoxazole ; 1,3,3,4,5,7-hexaméthyloctahydrobenzo[c]isoxazole ; 1,3,3,5,7-pentaméthyl-5-(pyridin-3-yl)octahydrobenzo[c]isoxazole ; 1,3,3,5,7-pentaméthyloctahydrobenzo[c]isoxazole hydrochloride ; 5-(4-fluoro-2-méthoxyphényl)-1,3,3,5,7-pentaméthyloctahydrobenzo[c]isoxazole ; 5-(4-fluorophényl)-1-isopropyl-3,3,5,7-tétraméthyloctahydrobenzo[c]isoxazole ; 4-méthyl-2-(1,3,3,5,7-pentaméthyloctahydrobenzo[c]isoxazol-5-yl)benzonitrile ; 1,3,3,5-tétraméthyl-5-(3-méthylbut-2-én-1-yl)octahydrobenzo[c]isoxazole ; 1-isopropyl-3,3,5,7-tétraméthyl-5-(3-méthylbut-2-én-1-yl)octahydrobenzo[c]isoxazole ; 5-isopentyl-1-isopropyl-3,3,5,7-tétraméthyloctahydrobenzo[c]isoxazole ; 2-chloro-5-(1,3,3,5,7-pentaméthyloctahydrobenzo[c]isoxazol-5-yl)benzonitrile ; 5-(4-(tert-butyl)phényl)-1,3,3,5,7-pentaméthyloctahydrobenzo[c]isoxazole ; 5-(4-(tert-butyl)phényl)-1,3,3,7-tétraméthyloctahydrobenzo[c]isoxazole ; 5-(4-(tert-butyl)phényl)-1-isopropyl-3,3,7-triméthyloctahydrobenzo[c]isoxazole ; et 1,3,3,6-tétraméthyloctahydrobenzo[c]isoxazole, ou un sel ou un solvate correspondant.

4. Procédé non médical d'induction d'une sensation rafraîchissante chez un humain ou un animal comprenant la mise en contact de l'humain ou de l'animal avec un composé de formule (Ia), ou un sel ou un solvate correspondant, tel que défini dans la revendication 1 à 3.

5. Procédé non médical pour obtenir un effet rafraîchissant sur la peau ou une muqueuse comprenant la mise en contact de la peau ou de la muqueuse avec un produit comprenant un ou plusieurs composés de formule (Ia) tels que définis dans la revendication 1 à 3.

6. Produit de consommation comprenant un ou plusieurs composés de formule (Ia) tels que définis dans la revendication 1 à 3.

7. Composition pharmaceutique comprenant un ou plusieurs composés de formule (Ia) tels que définis dans la revendication 1 à 3.

8. Composé de formule (Ia) tel que défini dans la revendication 1 à 3 pour une utilisation en tant que médicament.

9. Composition comprenant une sensation rafraîchissante, la composition comprenant un composé de formule (Ia) 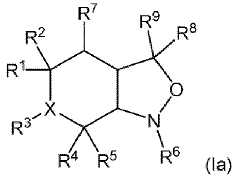
X étant C,
R³ étant choisi parmi hydrogène, C₁-C₆ alkyle, C₁-C₇ alkyle substitué par un ou deux groupes OH, C₃-C₇ cycloalkyle, C₃-C₇ cycloalkyle substitué par un ou deux groupes OH, C₅-C₇ cycloalcényle, C₅-C₇ cycloalcényle substitué par un, deux ou trois groupes C₁-C₃ alkyle, et phényle ;
R¹ étant choisi parmi hydrogène,
C₁-C₆ alkyle,
C₁-C₇ alkyle substitué par un ou deux groupes OH,
C₃-C₇ alcényle,
phényle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, Ci-Ce alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, naphtyle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, et
C₅-C₁₀ aryle monocyclique ou bicyclique, 1 ou 2 atomes de C étant remplacés par un hétéroatome indépendamment choisi parmi soufre, azote et oxygène, éventuellement substitué par jusqu'à trois substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle ;
R² étant choisi parmi hydrogène et méthyle ;
R⁴, R⁵, et R⁷ étant indépendamment choisis parmi hydrogène et C₁-C₆ alkyle ;
R⁶ étant choisi parmi hydrogène, C₁-C₆ alkyle, et C₃-C₇ cycloalkyle ;
R⁸ et R⁹ étant indépendamment choisis parmi hydrogène, et C₁-C₆ alkyle, ou
R⁸ et R⁹ formant conjointement avec l'atome de C auquel ils sont fixés un cycle cycloalkyle à 3, 4, 5, 6 ou 7 chaînons.

10. Composé de formule (Ia), sel ou solvate correspondant 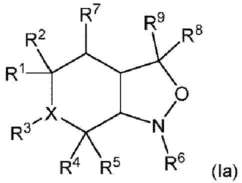
X étant C,
R³ étant choisi parmi hydrogène, C₁-C₆ alkyle, C₁-C₇ alkyle substitué par un ou deux groupes OH, C₃-C₇ cycloalkyle, C₃-C₇ cycloalkyle substitué par un ou deux groupes OH, C₅-C₇ cycloalcényle, C₅-C₇ cycloalcényle substitué par un, deux ou trois groupes C₁-C₃ alkyle, et phényle ;
R¹ étant choisi parmi
C₁-C₆ alkyle,
C₁-C₇ alkyle substitué par un ou deux groupes OH,
C₃-C₇ alcényle,
phényle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, Ci-Ce alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, naphtyle éventuellement substitué par jusqu'à cinq substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle, et
C₅-C₁₀ aryle monocyclique ou bicyclique, 1 ou 2 atomes de C étant remplacés par un hétéroatome indépendamment choisi parmi soufre, azote et oxygène, éventuellement substitué par jusqu'à trois substituants choisis dans le groupe constitué par halogène, C=N, NO₂, C₁-C₆ alkyle, C₁-C₆ alkyle comprenant jusqu'à 5 atomes d'halogène, C₁-C₆ alkyloxy, C₁-C₆ alkyloxy comprenant jusqu'à 5 atomes d'halogène, C₃-C₇ cycloalkyle, -C(O)O-R¹⁰, R¹⁰ étant choisi parmi hydrogène et C₁-C₃ alkyle, et -SR¹¹, R¹¹ étant choisi parmi hydrogène et C₁-C₃ alkyle ;
R² étant choisi parmi hydrogène et méthyle ;
R⁴, R⁵, et R⁷ étant indépendamment choisis parmi hydrogène et C₁-C₆ alkyle ;
R⁶ étant choisi parmi hydrogène, C₁-C₆ alkyle, et C₃-C₇ cycloalkyle ;
R⁸ et R⁹ étant indépendamment choisis parmi hydrogène, et C₁-C₆ alkyle, ou
R⁸ et R⁹ formant conjointement avec l'atome de C auquel ils sont fixés un cycle cycloalkyle à 3, 4, 5, 6 ou 7 chaînons.

11. Composé selon la revendication 10 choisi dans le groupe constitué par 4-méthyl-3-(1,3,3,5,7-pentaméthyloctahydrobenzo[c]isoxazol-5-yl)benzonitrile ; 4-méthoxy-3-(1,3,3,5,7-pentaméthyloctahydrobenzo[c]isoxazol-5-yl)benzonitrile ; 1-éthyl-5-(2-méthoxyphényl)-3,3,7-triméthyloctahydrobenzo[c]isoxazole ; 5-(2-méthoxyphényl)-1,3,3,7-tétraméthyloctahydrobenzo[c]isoxazole ; 5-(3,5-difluorophényl)-1,3,3,5,7-pentaméthyloctahydrobenzo[c]isoxazole ; 1,3,3,5,7-pentaméthyloctahydrobenzo[c]isoxazol-5-yl)benzonitrile ; 1,3,3,5,7-pentaméthyl-5-(o-tolyl)octahydrobenzo[c]isoxazole ; 5-(5-chloro-2-méthylphényl)-1,3,3,5,7-pentaméthyloctahydrobenzo[c]isoxazole; 3-(1-isopropyl-3,3,7-triméthyloctahydrobenzo[c]isoxazol-5-yl)-4-méthoxybenzonitrile ; 5-(2,5-diméthylphényl)-1,3,3,5,7-pentaméthyloctahydrobenzo[c]isoxazole ; 3-(1-éthyl-3,3,5,7-tétraméthyloctahydrobenzo[c]isoxazol-5-yl)-4-méthylbenzonitrile ; 5-(5-fluoro-2-méthylphényl)-1,3,3,5,7-pentaméthyloctahydrobenzo[c]isoxazole ; 1-isopropyl-5-(2-méthoxyphényl)-3,3,7-triméthyloctahydrobenzo[c]isoxazole ; 3-chloro-5-(1,3,3,5,7-pentaméthyloctahydrobenzo[c]isoxazol-5-yl)benzonitrile ; 3-(1-isopropyl-3,3,5,7-tétraméthyloctahydrobenzo[c]isoxazol-5-yl)benzonitrile ; 5-(3,4-difluorophényl)-1,3,3,5,7-pentaméthyloctahydrobenzo[c]isoxazole ; 3-(1-isopropyl-3,3,5,7-tétraméthyloctahydrobenzo[c]isoxazol-5-yl)-4-méthylbenzonitrile ; 5-(2-méthoxyphényl)-1,3,3,5,7-pentaméthyloctahydrobenzo[c]isoxazole ; 2-méthyl-5-(1,3,3,5,7-pentaméthyloctahydrobenzo[c]isoxazol-5-yl)benzonitrile ; méthyle 4-méthyl-3-(1,3,3,5,7-pentaméthyloctahydrobenzo[c]isoxazol-5-yl)benzoate ; 1-isopropyl-3,3,5,7-tétraméthyl-5-(o-tolyl)octahydrobenzo[c]isoxazole; 5-(3-fluorophényl)-1,3,3,5,7-pentaméthyloctahydrobenzo[c]isoxazole ; 1,3,3,5,7-pentaméthyl-5-phényloctahydrobenzo[c]isoxazole ; 1-isopropyl-3,3,7-triméthyl-5-phényloctahydrobenzo[c]isoxazole ; 5-(4-chloro-2-méthylphényl)-1-isopropyl-3,3,7-triméthyloctahydrobenzo[c]isoxazole ; 5-(2,4-diméthylphényl)-1,3,3,5,7-pentaméthyloctahydrobenzo[c]isoxazole; 5-(4-fluoro-2-méthylphényl)-1,3,3,7-tétraméthyloctahydrobenzo[c]isoxazole ; 5-(2,5-difluorophényl)-1,3,3,5,7-pentaméthyloctahydrobenzo[c]isoxazole ; 1-éthyl-3,3,5,7-tétraméthyl-5-phényloctahydrobenzo[c]isoxazole ; 1,3,3,5,7-pentaméthyl-5-(p-tolyl)octahydrobenzo[c]isoxazole ; 1-isopropyl-3,3,5,7-tétraméthyl-5-phényloctahydrobenzo[c]isoxazole ; 1,3,3,7-tétraméthyl-5-phényloctahydrobenzo[c]isoxazole ; 1-isopropyl-5-(2-méthoxyphényl)-3,3,5,7-tétraméthyloctahydrobenzo[c]isoxazole ; 5-(2-fluorophényl)-1,3,3,5,7-pentaméthyloctahydrobenzo[c]isoxazole ; 4-(1,3,3,5,7-pentaméthyloctahydrobenzo[c]isoxazol-5-yl)benzonitrile ; 3-méthyl-4-(1,3,3,5,7-pentaméthyloctahydrobenzo[c]isoxazol-5-yl)benzonitrile ; 5-(2,4-difluorophényl)-1,3,3,5,7-pentaméthyloctahydrobenzo[c]isoxazole ; 5-(2,5-diméthoxyphényl)-1,3,3,5,7-pentaméthyloctahydrobenzo[c]isoxazole ; 5-(2,3-diméthylphényl)-1,3,3,5,7-pentaméthyloctahydrobenzo[c]isoxazole ; 1,3,3,5,7-pentaméthyl-5-(2,4,5-triméthylphényl)octahydrobenzo[c]isoxazole ; 1,3,3,5,7-pentaméthyl-5-(4-(méthylthio)phényl)octahydrobenzo[c]isoxazole ; 2-(1,3,3,5,7-pentaméthyloctahydrobenzo[c]isoxazol-5-yl)benzonitrile ; 5-(3-méthoxyphényl)-1,3,3,5,7-pentaméthyloctahydrobenzo[c]isoxazole ; 5-(3-chloro-2-méthylphényl)-1-isopropyl-3,3,7-triméthyloctahydrobenzo[c]isoxazole ; 1,3,3,5,7-pentaméthyl-5-(naphtalén-2-yl)octahydrobenzo[c]isoxazole ; 5-(2,4-diméthylphényl)-1,3,3,7-tétraméthyloctahydrobenzo[c]isoxazole ; 4-méthyl-3-(1,3,3,7-tétraméthyloctahydrobenzo[c]isoxazol-5-yl)benzonitrile ; 1,3,3,7-tétraméthyl-5-(3-méthylbut-2-én-1-yl)octahydrobenzo[c]isoxazole ; 5-(2-éthylphényl)-1,3,3,5,7-pentaméthyloctahydrobenzo[c]isoxazole ; 1,3,3,5,7-pentaméthyl-5-(m-tolyl)octahydrobenzo[c]isoxazole ; 1,3,3,5,7-pentaméthyl-5-(5-méthylthiophén-3-yl)octahydrobenzo[c]isoxazole ; 1-éthyl-3,3,7-triméthyl-5-(3-méthylbut-2-én-1-yl)octahydrobenzo[c]isoxazole ; 5-(2-éthylphényl)-1-isopropyl-3,3,7-triméthyloctahydrobenzo[c]isoxazole ; 1,3,3,5,7-pentaméthyl-5-(3-méthylbut-2-én-1-yl)octahydrobenzo[c]isoxazole ; 5-(2,6-diméthylphényl)-1,3,3,5,7-pentaméthyloctahydrobenzo[c]isoxazole ; 5-(benzo[d] [1,3]dioxol-5-yl)-1,3,3,5,7-pentaméthyloctahydrobenzo[c]isoxazole ; 1-isopropyl-5-(6-méthoxypyridin-2-yl)-3,3,5,7-tétraméthyloctahydrobenzo[c]isoxazole; 5-(5-chloro-2-méthylphényl)-1-isopropyl-3,3,7-triméthyloctahydrobenzo[c]isoxazole ; 5-butyl-1,3,3-triméthyloctahydrobenzo[c]isoxazole ; 5-(2,4-diméthoxyphényl)-1,3,3,5,7-pentaméthyloctahydrobenzo[c]isoxazole ; 1-isopropyl-3,3,7-triméthyl-5-(o-tolyl)octahydrobenzo[c]isoxazole ; 1-isopropyl-3,3,7-triméthyl-5-(3-méthylbut-2-én-1-yl)octahydrobenzo[c]isoxazole ; 5-(5-fluoro-2-méthylphényl)-1,3,3,7-tétraméthyloctahydrobenzo[c]isoxazole ; 5-(3,5-diméthylphényl)-1,3,3,5,7-pentaméthyloctahydrobenzo[c]isoxazole ; 1-isopropyl-3,3,5,7-tétraméthyloctahydrobenzo[c]isoxazole ; 1,3,3-triméthyl-6-(4-méthylcyclohex-3-én-1-yl)octahydrobenzo[c]isoxazole ; 5-(3-fluoro-5-(trifluorométhyl)phényl)-1,3,3,5,7-pentaméthyloctahydrobenzo[c]isoxazole ; 1-isopropyl-3,3-diméthyl-5-propyloctahydrobenzo[c]isoxazole ; 1,3,3-triméthyl-5-pentyloctahydrobenzo[c]isoxazole ; 1-éthyl-3,3,5,7-tétraméthyl-5-(o-tolyl)octahydrobenzo[c]isoxazole ; 5-(3,5-diméthylphényl)-1-isopropyl-3,3,5,7-tétraméthyloctahydrobenzo[c]isoxazole ; 5-(2,5-diméthylphényl)-1,3,3,7-tétraméthyloctahydrobenzo[c]isoxazole ; 5,7-diéthyl-1-isopropyl-3,3-diméthyloctahydrobenzo[c]isoxazole ; 1,3,3,5,7-pentaméthyl-5-(2-méthyl-5-(trifluorométhyl)phényl)octahydrobenzo[c]isoxazole ; 1-éthyl-3,3-diméthyl-5-propyloctahydrobenzo[c]isoxazole ; 1,3,3,5,7-pentaméthyl-5-propyloctahydrobenzo[c]isoxazole ; 1,3,3-triméthyl-5-propyloctahydrobenzo[c]isoxazole ; 2-méthyl-3-(1,3,3,5,7-pentaméthyloctahydrobenzo[c]isoxazol-5-yl)benzonitrile ; 1,3,3,7-tétraméthyl-5-(o-tolyl)octahydrobenzo[c]isoxazole ; 5-butyl-1-éthyl-3,3-diméthyloctahydrobenzo[c]isoxazole ; 5-(4-fluorophényl)-1,3,3,5,7-pentaméthyloctahydrobenzo[c]isoxazole ; 5-(furan-3-yl)-1,3,3,5,7-pentaméthyloctahydrobenzo[c]isoxazole ; 1-isopropyl-3,3,5,7-tétraméthyloctahydrobenzo[c]isoxazol-1-ium chloride ; 1,3,3,5,7-pentaméthyl-5-(naphtalén-1-yl)octahydrobenzo[c]isoxazole ; 5-(2-éthoxyphényl)-1,3,3,5,7-pentaméthyloctahydrobenzo[c]isoxazole ; 5-(3-chloro-2-méthylphényl)-1,3,3,5,7-pentaméthyloctahydrobenzo[c]isoxazole ; 5-(6-méthoxypyridin-2-yl)-1,3,3,5,7-pentaméthyloctahydrobenzo[c]isoxazole ; 1,3,3,5,7-pentaméthyl-5-(5-méthylthiophén-2-yl)octahydrobenzo[c]isoxazole ; 1,3,3,5,7-pentaméthyl-5-(4-méthylpyridin-3-yl)octahydrobenzo[c]isoxazole ; 1-éthyl-3,3,5,7-tétraméthyloctahydrobenzo[c]isoxazole ; 5-(2-éthylphényl)-1,3,3,7-tétraméthyloctahydrobenzo[c]isoxazole ; 5-butyl-1-isopropyl-3,3-diméthyloctahydrobenzo[c]isoxazole ; 1-éthyl-3,3,5,7-tétraméthyl-5-(3-méthylbut-2-én-1-yl)octahydrobenzo[c]isoxazole ; 5-(4-fluoro-2-méthylphényl)-1,3,3,5,7-pentaméthyloctahydrobenzo[c]isoxazole ; 1-isopropyl-5,7-diméthylhexahydro-1H-spiro[benzo[c]isoxazole-3,1'-cyclopentane] ; 1,5,7-triéthyl-3,3-diméthyloctahydrobenzo[c]isoxazole ; 1-isopropyl-3,3,5,7-tétraméthyl-5-propyloctahydrobenzo[c]isoxazole ; 6-isopentyl-1,4,4,8-tétraméthyloctahydro-2H-benzo[d][1,3]oxazine ; 5-(3,5-diméthoxyphényl)-1,3,3,5,7-pentaméthyloctahydrobenzo[c]isoxazole ; 5,7-diéthyl-1,3,3-triméthyloctahydrobenzo[c]isoxazole ; 1-isopropyl-5-(4-méthoxyphényl)-3,3,5,7-tétraméthyloctahydrobenzo[c]isoxazole ; 5-(3-isopropylphényl)-1,3,3,5,7-pentaméthyloctahydrobenzo[c]isoxazole ; 1,3,3-triméthyl-6-phényloctahydrobenzo[c]isoxazole ; 5-éthyl-1-isopropyl-3,3-diméthyloctahydrobenzo[c]isoxazole ; 1,3,3,5,7-pentaméthyl-5-(2-(méthylthio)phényl)octahydrobenzo[c]isoxazole ; 1,5,7-triméthylhexahydro-1H-spiro[benzo[c]isoxazole-3,1'-cyclopentane] ; 5-éthyl-1,3,3,5,7-pentaméthyloctahydrobenzo[c]isoxazole ; 5-(4-méthoxyphényl)-1,3,3,5,7-pentaméthyloctahydrobenzo[c]isoxazole ; 1,3,3,5,7-pentaméthyloctahydrobenzo[c]isoxazole ; 1,3,3,4,5,7-hexaméthyloctahydrobenzo[c]isoxazole ; 1,3,3,5,7-pentaméthyl-5-(pyridin-3-yl)octahydrobenzo[c]isoxazole ; hydrochlorure de 1,3,3,5,7-pentaméthyloctahydrobenzo[c]isoxazole ; 5-(4-fluoro-2-méthoxyphényl)-1,3,3,5,7-pentaméthyloctahydrobenzo[c]isoxazole ; 5-(4-fluorophényl)-1-isopropyl-3,3,5,7-tétraméthyloctahydrobenzo[c]isoxazole ; 4-méthyl-2-(1,3,3,5,7-pentaméthyloctahydrobenzo[c]isoxazol-5-yl)benzonitrile ; 1,3,3,5-tétraméthyl-5-(3-méthylbut-2-én-1-yl)octahydrobenzo[c]isoxazole ; 1-isopropyl-3,3,5,7-tétraméthyl-5-(3-méthylbut-2-én-1-yl)octahydrobenzo[c]isoxazole ; 5-isopentyl-1-isopropyl-3,3,5,7-tétraméthyloctahydrobenzo[c]isoxazole ; 2-chloro-5-(1,3,3,5,7-pentaméthyloctahydrobenzo[c]isoxazol-5-yl)benzonitrile ; 5-(4-(tert-butyl)phényl)-1,3,3,5,7-pentaméthyloctahydrobenzo[c]isoxazole ; 5-(4-(tert-butyl)phényl)-1,3,3,7-tétraméthyloctahydrobenzo[c]isoxazole ; 5-(4-(tert-butyl)phényl)-1-isopropyl-3,3,7-triméthyloctahydrobenzo[c]isoxazole ; et 1,3,3,6-tétraméthyloctahydrobenzo[c]isoxazole, ou sel ou solvate correspondant.
